# EUROPEAN PATENT APPLICATION

(11) **EP 4 806 898 A1**
(43) Date of publication of application: **16.09.2026**
(21) Application number: 24889126.9
(22) Date of filing: 06.11.2024
(51) Int. Cl.: C07D 487/04, A61K 31/519, A61P 35/00, C07D 487/08, C07D 498/10, C07D 471/04, A61K 31/513, A61K 31/553, C07D 498/16, C07D 498/14

(54) **HETEROARYL DERIVATIVE COMPOUND AND USE THEREOF**

(30) Priority: 07.11.2023 KR 20230152783; 29.02.2024 KR 20240030152
(71) Applicant: Voronoi Inc., Incheon 21984 (KR)
(72) Inventor: HWANG, Seonah, Incheon 21984 (KR); LEE, Younho, Incheon 21984 (KR); JO, Seohyun, Incheon 21984 (KR); MA, Dahoon, Incheon 21984 (KR); OH, Serin, Incheon 21984 (KR); LEE, Suyoung, Incheon 21984 (KR); CHOI, Jinwoo, Incheon 21984 (KR); KIM, Daekwon, Incheon 21984 (KR)
(74) Representative: Reddie & Grose LLP
(86) International application number: PCT/KR2024/017446
(87) International publication number: WO 2025/100940

(57) **Abstract**

The present invention relates to heteroaryl derivative and uses thereof. The heteroaryl derivative of the present invention exhibits excellent inhibitory activity against USP1 protein and cells having BRCA1 mutation/deficiency, BRCA2 mutation/deficiency, or HR deficiency, and therefore can be effectively used for the treatment or prevention of diseases associated with USP1, PARP, BRCA1 mutation/deficiency, BRCA2 mutation/deficiency, or HR deficiency.

## Description

### [Technical Field]

The present invention relates to heteroaryl derivative compounds and medical uses thereof. Specifically, the present invention relates to heteroaryl derivative compounds selectively having USP1 inhibitory activity based on high binding affinity to the USP1 protein, and having activity to inhibit the proliferation of cells in which BRCA1 mutation/deficiency, BRCA2 mutation/deficiency, or HR deficiency.

### [Background Art]

The breast cancer 1 (BRCA1) and breast cancer 2 (BRCA2) genes are proteins involved in DNA damage repair and play an important role in repairing DNA double-strand breaks (DSBs) during homologous-recombination (HR). BRCA1 is required for C-terminal binding protein 1 interacting protein (CtIP)-mediated resection of double-strand breaks (DSBs) to generate single-stranded DNA (ssDNA) for homologous recombination via the Mre11-Rad50-Nbs1 (MRN) complex, and BRCA2 promotes homologous recombination by facilitating the subsequent loading of RAD51 onto ssDNA. In addition, BRCA1 and BRCA2 limit nucleic acid degradation and are therefore essential for protecting stalled replication forks. If mutation occurs in BRCA1 and BRCA2 genes, DNA damage repair does not occur normally, which increases the risk of developing various cancers including breast and ovarian cancer.

Since BRCA1 or BRCA2 work complementarily with poly ADP ribose polymerase 1 (PARP1), inhibition of PARP1 in BRCA1 or BRCA2 mutant cancers causes synthetic lethality, leading to the development of PARP1 inhibitors. However, there is a limitation in that therapeutic resistance to PARP inhibitors easily occurs due to restoration of homologous recombination or stabilization of replication forks, and a solution has been sought to overcome this limitation (Bunting et al., 2010; Ray Chaudhuri et al., 2016; Rondinelli et al., 2017; Xu et al., 2015).

Ubiquitin is a small protein, consisting of 76 amino acids, that is attached to target proteins through post-translational modification. Ubiquitination is a reversible process that involves a family of deubiquitinating enzymes (DUBs) that regulate a variety of cellular processes by deconjugating ubiquitin from substrates, which cleave the isopeptide bond between ubiquitin and the modified protein. More than 100 DUBs are known, and they are subdivided into six subfamilies. The largest of these is the ubiquitin-specific protease (USP) family, which contains 58 members. DUBs and their substrate proteins are often deregulated in cancer, and targeted specific DUB family members may exert antitumor activity by enhancing the ubiquitination and subsequent degradation of oncogenic substrates and the activity of other key proteins involved in tumor growth, survival, differentiation, and maintenance of the tumor microenvironment.

Ubiquitin-specific protease 1 (USP1), a USP subfamily of DUBs, regulates DNA damage repair by hydrolyzing ubiquitin from its substrates, and deubiquitinates proliferating cell nuclear antigen (PCNA) in the translesion synthesis (TLS) pathway and in the FANCI-FANCD2 complex in the Fanconi anemia (FA) pathway, respectively (Lim et al., Molecular Cell 72, 925-941, December 20, 2018). These two pathways are essential for the repair of DNA damage induced by DNA crosslinking agents, such as cisplatin and mitomycin C (MMC).

Further, USP1 is required for replication fork stabilization in BRCA1-deficient cells, and inhibition of USP1 in BRCA1-deficient cells increases synthetic lethality, making BRCA1-deficient tumors a prime target.

USP1 inhibition in BRCA1-deficient tumors that are resistant to PARP inhibitors may inhibit cancer cell growth. Therefore, there is a growing need to develop USP1 inhibitors capable of treating cancer by causing synthetic lethality, either alone or in combination with PARP inhibitors.

### [Disclosure]

### [Technical Problem]

An object of the present invention is to provide a heteroaryl derivative of a novel structure, a tautomer thereof, a stereoisomer thereof, or a pharmaceutically acceptable salt thereof.

Another object of the present invention is to provide a method for preparing the heteroaryl derivative compound.

Still another object of the present invention is to provide a pharmaceutical use of the heteroaryl derivative compound, and specifically, to provide a pharmaceutical composition comprising the heteroaryl derivative compound as an active ingredient for treating or preventing diseases associated with ubiquitin-specific protease 1 (USP1), poly (ADP-ribose) polymerase (PARP), breast cancer 1 (BRCA1) mutation/deficiency, breast cancer 2 (BRCA2) mutation/deficiency, or homologous-recombination (HR) deficiency, uses of the compound for treating or preventing diseases associated with USP1, PARP, BRCA1 mutation/deficiency, BRCA2 mutation/deficiency, or HR deficiency, or a method for treating or preventing diseases associated with USP1, PARP, BRCA1 mutation/deficiency, BRCA2 mutation/deficiency, or HR deficiency, the method comprising administering the compound.

### [Technical Solution]

In order to achieve the above purpose, the present inventors conducted extensive research with considerable efforts and completed the present invention by confirming that heteroaryl derivative compounds represented by Chemical Formula 1 described below selectively exhibited high binding affinity to ubiquitin-specific protease 1 (USP 1) protein and inhibited the proliferation of cells in which breast cancer 1 (BRCA1) mutation/deficiency, breast cancer 2 (BRCA2) mutation/deficiency, or homologous-recombination (HR) deficiency is activated.

### Heteroaryl derivative compound

The present invention provides a compound represented by the following Chemical Formula 1, a tautomer thereof, a stereoisomer thereof, or a pharmaceutically acceptable salt thereof: 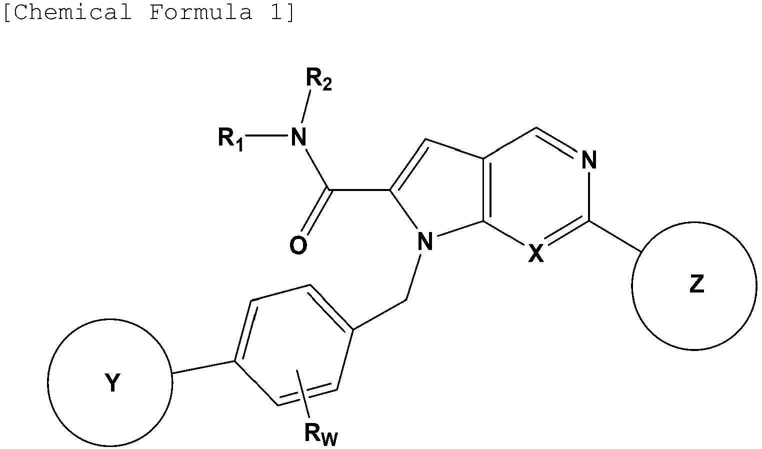 in Chemical Formula 1 above,
X is CH or N;
R_{W} is -H, -C₁₋₆alkyl, -C₁₋₆haloalkyl, -O-C₁₋₆alkyl, or - halo;
R₁ and R₂ are -H, -C₁₋₆alkyl, -C₁₋₆alkenyl, -C₁₋₆alkynyl, -C₁₋₆cyanoalkyl, -C₁₋₆aminoalkyl, -C₁₋₆hydroxyalkyl, -C₁₋₆haloalkyl, -OH, -C₁₋₆alkyl-O-C₁₋₆alkyl, -C₁₋₆alkyl-S-C₁₋₆alkyl, -C₁₋₆alkyl-C (=O) -C₁₋₆alkyl, -C₁₋₆alkyl-C (=O) -OC₁₋₆alkyl, -C₁₋₆alkyl-S(=O)₂-C₁₋₆alkyl, -(CH₂)ₙ- (3-6 membered cycloalkyl), - (CH₂)ₙ-(4-6 membered heterocycloalkyl), -(CH₂)n-aryl, or - (CH₂)n-(5-6 membered heteroaryl), wherein at least one H of the -C₁₋₆alkyl, -C₁₋₆alkenyl, or -C₁₋₆alkynyl may be substituted with deuterium, and at least one H of the - (CH₂)n- (3-6 membered cycloalkyl), -(CH₂)n- (4-6 membered heterocycloalkyl), -(CH₂)n-aryl, or -(CH₂)n-(5-6 membered heteroaryl) ring may be substituted with -C₁₋₆alkyl, -C₁₋₆aminoalkyl, -C₁₋₆hydroxyalkyl, -C₁₋₆haloalkyl, -halo, -NRₐR_{b}, -O-R_{c}, -C₁₋₆alkyl-O-C₁₋₆alkyl, -(CH₂)n-S(=O)₂-C₁₋₆alkyl, - (CH₂)n-(3-6 membered cycloalkyl), or -(CH₂)n- (4-6 membered heterocycloalkyl), wherein at least one of the -(CH₂)n-(3-6 membered cycloalkyl) or -(CH₂)n- (4-6 membered heterocycloalkyl) ring may be substituted with -C₁₋₆alkyl, - C₁₋₆haloalkyl, or -halo; or
the R₁ and R₂ may be linked to each other to form a 4-12 membered ring together with the N atom, wherein at least one H of the 4-12 membered ring may be substituted with -C₁₋₆alkyl, -C₁₋₆haloalkyl, -C₁₋₆alkyl-O-C₁₋₆alkyl, -(CH₂)n-S(=O)₂-C₁₋₆alkyl, -CN, -NRₐR_{b}, -O-R_{c}, -halo, -(3-6 membered cycloalkyl), or -(4-6 membered heterocycloalkyl), and - (CH₂)- of the 4-12 membered ring may be substituted with - C(=O)- or -S(=O)₂-;
Rₐ and R_{b} are -H or -C₁₋₆alkyl;
R_{c} is -H or -C₁₋₆alkyl;
n is 0, 1, 2, 3, or 4;
ring Z is -phenyl, -(5-10 membered heteroaryl), or -(5-10 membered heterohydroaryl), wherein the -(5-10 membered heteroaryl) or -(5-10 membered heterohydroaryl) ring comprises one or more N atoms, and at least one H of the - phenyl, -(5-10 membered heteroaryl), or -(5-10 membered heterohydroaryl) ring may be substituted with -C₁₋₆alkyl, - OH, -O-C₁₋₆alkyl, -O-C₁₋₆haloalkyl, -halo, -O-(3-6 membered cycloalkyl), or -(3-6 membered cycloalkyl), wherein at least one H of the -C₁₋₆alkyl or -O-C₁₋₆alkyl may be substituted with deuterium, and -(CH₂)- of the -(5-10 membered heterohydroaryl) ring may be substituted with -C(=O)-; and
ring Y is -(5-10 membered heteroaryl) or -(5-10 membered heterohydroaryl), wherein the -(5-10 membered heteroaryl) or -(5-10 membered heterohydroaryl) ring comprises one or more N atoms, and at least one H of the -(5-10 membered heteroaryl) or -(5-10 membered heterohydroaryl) ring may be substituted with -C₁₋₆alkyl, -C₁₋₆hydroxyalkyl, -C₁₋₆haloalkyl, -O-C₁₋₆alkyl, -COOH, -(3-6 membered cycloalkyl), -(4-6 membered heterocycloalkyl), or -(5-6 membered heteroaryl), wherein at least one H of the -C₁₋₆alkyl or -O-C₁₋₆alkyl may be substituted with deuterium, and at least one H of the -(3-6 membered cycloalkyl), -(4-6 membered heterocycloalkyl), -(5-6 membered heteroaryl) ring may be substituted with -C₁-₆alkyl, -C₁₋₆haloalkyl, or -halo.

According to an embodiment of the present invention, X may be N.

According to an embodiment of the present invention, R₁ and R₂ may be in the following ranges:
R₁ and R₂ are -H, -C₁₋₆alkyl, -C₁₋₆alkenyl, -C₁₋₆alkynyl, -C₁₋₆cyanoalkyl, -C₁₋₆aminoalkyl, -C₁₋₆hydroxyalkyl, -C₁₋₆haloalkyl, -OH, -C₁₋₆alkyl-O-C₁₋₆alkyl, -C₁₋₆alkyl-S-C₁₋₆alkyl, -C₁₋₆alkyl-C (=O)-C₁₋₆alkyl, -C₁₋₆alkyl-C (=O) -OC₁₋₆alkyl, -C₁₋₆alkyl-S(=O) ₂-C₁₋₆alkyl, -(CH₂)n- (3-6 membered cycloalkyl), - (CH₂)n-(4-6 membered heterocycloalkyl), -(CH₂)n-phenyl, - (CH₂)n-naphthalenyl, or -(CH₂)n-(5-6 membered heteroaryl), wherein at least one H of the -C₁₋₆alkyl, -C₁₋₆alkenyl, or - C₁₋₆alkynyl may be substituted with deuterium, and -(CH₂)- of the -(CH₂)n- (3-6 membered cycloalkyl) or -(CH₂)n- (4-6 membered heterocycloalkyl) ring may be substituted with - S(=O)₂-, and at least one H of the -(CH₂)n-(3-6 membered cycloalkyl), -(CH₂)n- (4-6 membered heterocycloalkyl), - (CH₂)n-phenyl, -(CH₂)n-naphthalenyl, or -(CH₂)n-(5-6 membered heteroaryl) ring may be substituted with -C₁₋₆alkyl, -C₁₋₆aminoalkyl, -C₁₋₆hydroxyalkyl, -C₁₋₆haloalkyl, -halo, -NRₐR_{b}, -O-R_{c}, -C₁₋₆alkyl-O-C₁₋₆alkyl, -(CH₂)n-S(=O)₂-C₁₋₆alkyl, - (CH₂)n- (3-6 membered cycloalkyl), or -(CH₂)n-(4-6 membered heterocycloalkyl), wherein at least one H of the -(CH₂)n-(3-6 membered cycloalkyl) or -(CH₂)n-(4-6 membered heterocycloalkyl) ring may be substituted with -C₁₋₆alkyl, - C₁₋₆haloalkyl, or -halo; or
the R₁ and R₂ may be linked to each other to form a - (4-6 membered heterocycloalkyl) or -(6-12 membered heterobicycloalkyl) ring together with the N atom, wherein the -(6-12 membered heterobicycloalkyl) ring is a fused ring, a spiro ring, or a bridged ring, and at least one H of the -(4-6 membered heterocycloalkyl), or -(6-12 membered heterobicycloalkyl) ring may be substituted with -C₁₋₆alkyl, -C₁₋₆haloalkyl, -C₁₋₆alkyl-O-C₁₋₆alkyl, -(CH₂)n-S(=O)₂-C₁₋₆alkyl, -CN, -NRₐR_{b}, -O-R_{c}, -halo, -(3-6 membered cycloalkyl), or - (4-6 membered heterocycloalkyl), and -(CH₂)- of the -(4-6 membered heterocycloalkyl) or -(6-12 membered heterobicycloalkyl) ring may be substituted with -S(=O)₂-;
Rₐ and R_{b} are -H or -C₁₋₆alkyl;
R_{c} is -H or -C₁₋₆alkyl; and
n is 0, 1, or 2.

According to an embodiment of the present invention, the ring Z may be in the following ranges: the ring Z is -phenyl, 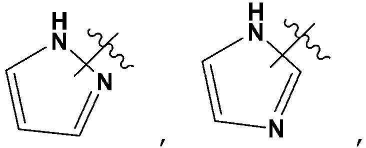 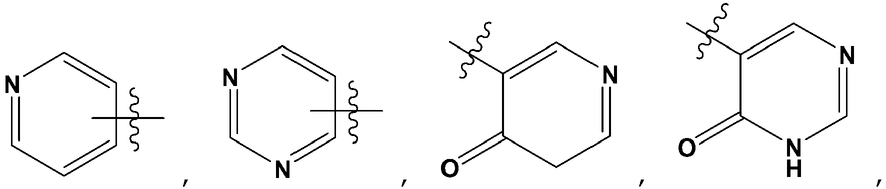 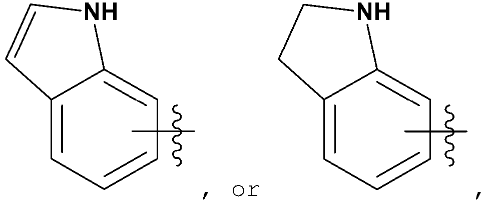 wherein at least one H of the ring Z may be substituted with -C₁₋₆alkyl, -OH, -O-C₁₋₆alkyl, -O-C₁₋₆haloalkyl, -halo, -O-(3-6 membered cycloalkyl), or -(3-6 membered cycloalkyl), wherein at least one H of the -C₁₋₆alkyl or -O-C₁₋₆alkyl may be substituted with deuterium, and -(CH₂)- of the -(5-10 membered heterohydroaryl) ring may be substituted with -C(=O)-.

More specifically, the ring Z may be -phenyl, 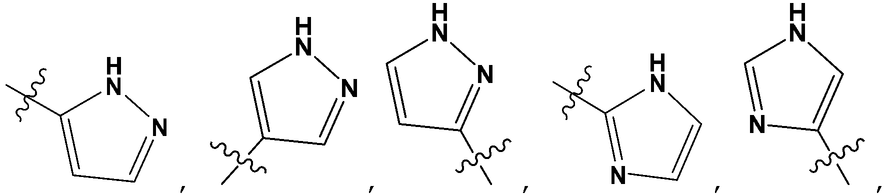 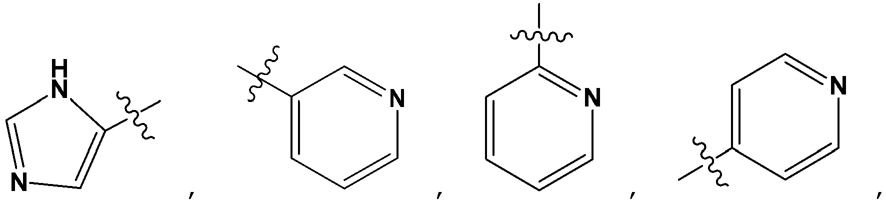 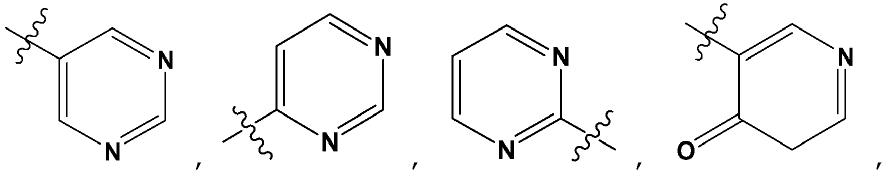 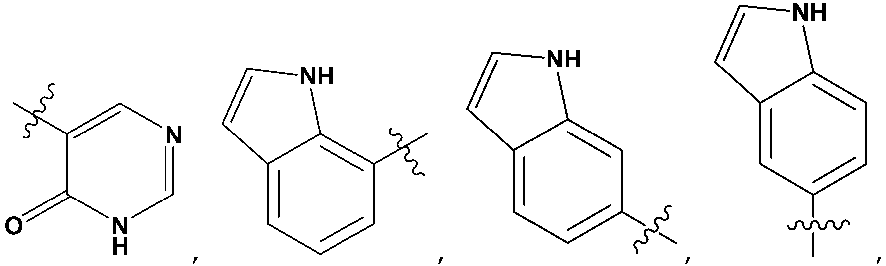 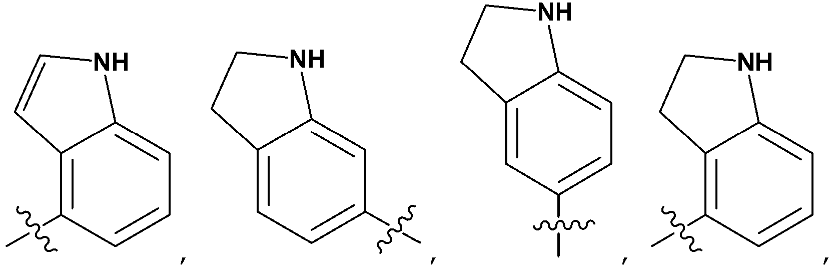 or

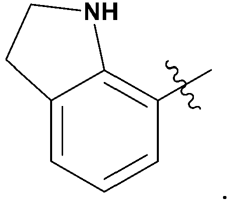

However, the ring Z is not limited thereto.

According to an embodiment of the present invention, the ring Y may be in the following ranges:
the ring Y is 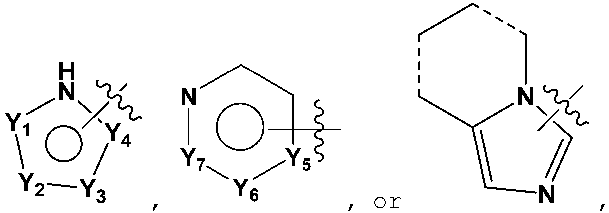 wherein at least one H of the ring Y may be substituted with -C₁₋₆alkyl, -C₁₋₆hydroxyalkyl, -C₁₋₆haloalkyl, -O-C₁₋₆alkyl, - COOH, -(3-6 membered cycloalkyl), -(4-6 membered heterocycloalkyl), or -(5-6 membered heteroaryl), wherein at least one H of the -C₁₋₆alkyl or -O-C₁₋₆alkyl may be substituted with deuterium, and at least one H of the -(3-6 membered cycloalkyl), -(4-6 membered heterocycloalkyl), -(5-6 membered heteroaryl) ring may be substituted with -C₁₋₆alkyl, -C₁₋₆haloalkyl, or -halo;
Y₁ to Y₇ are CH₂, CH, C, NH, N, O, or S; and
----- is a single bond or a double bond.

According to an embodiment of the present invention, the ring Y may be in the following ranges: the ring Y is 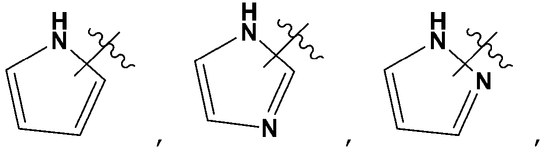 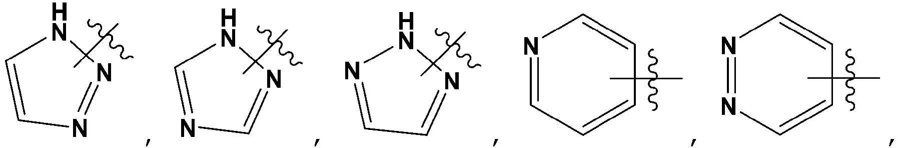 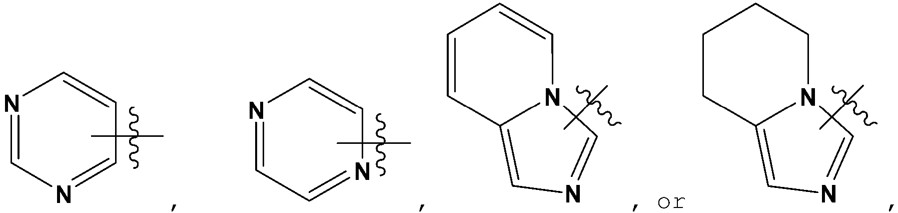 wherein at least one H of the ring Y may be substituted with -C₁₋₆alkyl, -C₁₋₆hydroxyalkyl, -C₁₋₆haloalkyl, -O-C₁₋₆alkyl, - COOH, -(3-6 membered cycloalkyl), -(4-6 membered heterocycloalkyl), or -(5-6 membered heteroaryl), wherein at least one H of the -C₁₋₆alkyl or -O-C₁₋₆alkyl may be substituted with deuterium, and at least one H of the -(3-6 membered cycloalkyl), -(4-6 membered heterocycloalkyl), -(5-6 membered heteroaryl) ring may be substituted with -C₁₋₆alkyl, -C₁₋₆haloalkyl, or -halo.

More specifically, the ring Y may be 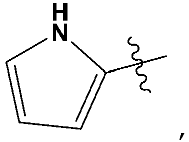 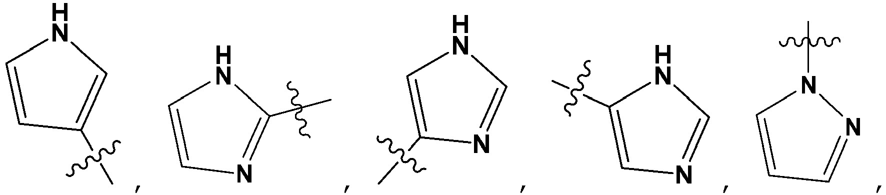 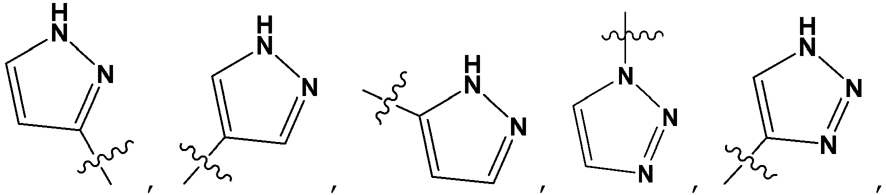 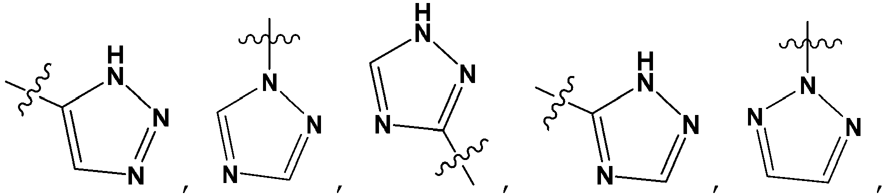 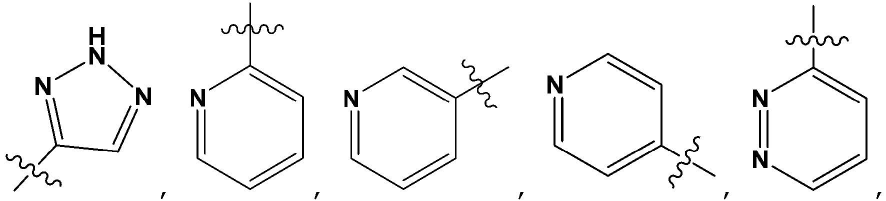 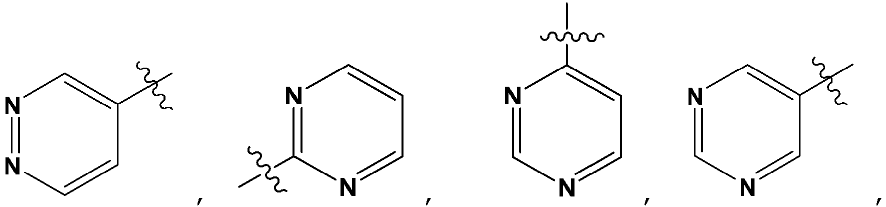 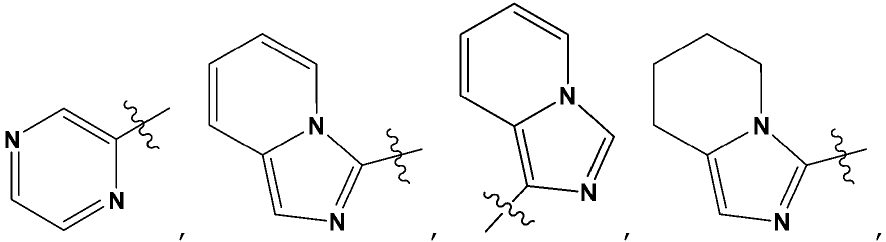 or

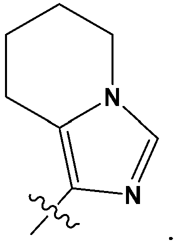

However, the ring Y is not limited thereto.

According to an embodiment of the present invention, the compound represented by Chemical Formula 1 above may be selected from the group consisting of compounds listed in Table 1 below.

In the present invention, all of the hydrogen atoms described may be substituted with its isotope, "deuterium", and any of the hydrogen atoms in the compounds according to Examples related to the present invention may also be substituted with "deuterium". In other words, the absence of the term "deuterium" for hydrogen contained in each substituent is not meant to exclude it, but is to be understood as falling within the scope of the present invention.

In the present invention, "alkyl" may refer to a saturated hydrocarbon, straight or branched chain, acyclic or cyclic, or a combination thereof, unless otherwise specified. For example, "C₁₋₆alkyl" may refer to an alkyl containing 1 to 6 carbon atoms. The acyclic alkyl may include, for example, methyl, ethyl, n-propyl, n-butyl, isopropyl, sec-butyl, isobutyl, tert-butyl, and the like, but is not limited thereto. The cyclic alkyl may be used interchangeably with "cycloalkyl" in the present specification, and may include, as an example, cyclopropyl, cyclobutyl, cyclopentyl, cyclohexyl, cycloheptyl, cyclooctyl, and the like, but is not limited thereto.

In the present invention, "alkenyl" and "alkynyl" may refer to unsaturated hydrocarbons, straight or branched chain, acyclic or cyclic, or a combination thereof. For example, "C₁₋₆alkenyl" may refer to an unsaturated hydrocarbon having 1 to 6 carbon atoms having one or more double bonds, and "C₁₋₆alkynyl" may refer to an unsaturated hydrocarbon having 1 to 6 carbon atoms having one or more triple bonds.

In the present invention, "alkoxy" may refer to an alkyl ether group of the form -(R'-O-R"), wherein R' may be selected from the group consisting of a single bond and C₁₋₆alkyl, and R" may be C₁₋₆alkyl. Here, alkyl is the same as defined above. For example, "C₁₋₆alkoxy" may refer to an alkoxy containing C₁₋₆alkyl, i.e., -(O-C₁₋₆alkyl) or -(C₁₋₆alkyl-O-C₁₋₆alkyl), and as an example, the alkoxy may include, but are not limited to, methoxy, ethoxy, n-propoxy, isopropoxy, n-butoxy, isobutoxy, sec-butoxy, tert-butoxy, and the like.

In the present invention, "halo" may be F, Cl, Br, or I.

In the present invention, "haloalkyl" may refer to a straight or branched chain alkyl (hydrocarbon) having one or more carbon atoms substituted with halo as defined herein. Examples of the haloalkyl include methyl, ethyl, propyl, isopropyl, isobutyl or n-butyl, independently substituted with one or more halogens, such as F, Cl, Br, or I, but are not limited to.

As used herein, "hydroxyalkyl" may refer to a straight or branched chain alkyl (hydrocarbon) having a carbon atom substituted with hydroxyl (-OH). Examples of the hydroxyalkyl include methyl, ethyl, propyl, isopropyl, isobutyl or n-butyl independently substituted with one or more -OH, but are not limited to.

As used herein, "aminoalkyl" may refer to a straight or branched chain alkyl (hydrocarbon) having a carbon atom substituted with amino(NR'R"). Here, R' and R" may each independently be selected from the group consisting of hydrogen and C₁₋₆alkyl, and the selected R' and R" may each independently be substituted or unsubstituted.

As used herein, "cyanoalkyl" may refer to a straight or branched chain alkyl (hydrocarbon) having a carbon atom substituted with cyano (-CN).

In the present invention, "alkylsulfonyl" refers to - (R'-S(=O)₂-R"), wherein R' may be selected from the group consisting of a single bond and C₁₋₆alkyl, and R" may be selected from the group consisting of hydroxyl and C₁₋₆alkyl. Each of the above selected R' and R" may be independently substituted or unsubstituted. Further, "C₀₋₆alkylsulfonyl" refers to a sulfonic acid group (-S(=O)₂OH) that does not contain alkyl or a sulfonyl group that contains C₁₋₆alkyl, i.e., -S(=O)₂-(C₁₋₆alkyl) or -(C₁₋₆alkyl)-S(=O)₂-(C₁₋₆alkyl), and may include methylsulfonyl, (methylsulfonyl)methyl, (methylsulfonyl)ethyl, ethylsulfonyl, (ethylsulfonyl)methyl, and (ethylsulfonyl)ethyl, but is not limited to.

In the present invention, "alkylcarbonyl" refers to - (R'-C(=O)-R"), wherein R' may be selected from the group consisting of a single bond and C₁₋₆alkyl, and R" may be selected from the group consisting of hydrogen and C₁₋₆alkyl. Each of the above selected R' and R" may be independently substituted or unsubstituted. Further, "C₀₋₆alkylcarbonyl" refers to an aldehyde group (-C(=O)H) that does not contain alkyl or a ketone group that contains C₁₋₆alkyl, i.e., -C(=O)-(C₁₋₆alkyl) or -(C₁₋₆alkyl) -C(=O)-(C₁₋₆alkyl) .

In the present invention, "cycloalkyl" may refer to a hydrocarbon ring that does not include a heteroatom (N, O, P, P(=O), or S, and the like) in the ring, and may be saturated or partially unsaturated. Here, when unsaturated, the cycloalkyl may be referred to as a cycloalkene. Unless otherwise stated, cycloalkyl may be a single ring or multi-rings such as spiro rings, bridged rings, or fused rings.

In the present invention, "heterocycloalkyl" may refer to a ring containing one or more selected from N, O, P, P(=O), and S in the ring, and may be saturated or partially unsaturated. Here, when unsaturated, the heterocycloalkyl may be referred to as a heterocycloalkene. Unless otherwise stated, the heterocycloalkyl may be a single ring. In addition, the term "heterocycloalkyl having 3 to 12 atoms" may refer to a heterocycloalkyl having 3 to 12 ring-forming atoms. For example, heterocycloalkyl may include pyrrolidine, imidazolidine, pyrazolidine, butyrolactam, valerolactam, imidazolidinone, hydantoin, dioxolane, phthalimide, piperidine, pyrimidine-2,4(1H,3H)-dione, 1,4-dioxane, morpholine, thiomorpholine, thiomorpholine-S-oxide, thiomorpholine-S,S-oxide, piperazine, pyran, pyridone, 3-pyrroline, thiopyran, pyrone, tetrahydrofuran, tetrahydrothiophene, and the like, but is not limited to.

In the present invention, "heterobicycloalkyl" may refer to a multi-ring, such as a spiro ring, bridged ring, or fused ring, containing at least one selected from N, O, P, P(=O), and S in the ring, and may be saturated or partially unsaturated. Here, when unsaturated, the heterobicycloalkyl may be referred to as a heterobicycloalkene. Examples of heterobicycloalkyl may include quinuclidine, tropane, 2-azaspiro[3.3]heptane, (1r,5s)-3-azabicyclo[3.2.1]octane, (1s,4s)-2-azabicyclo[2.2.2]octane, (1r,4r)-2-oxa-5-azabicyclo[2.2.2]octane, and the like, but are not limited to.

In the present invention, "arene" may refer to an aromatic hydrocarbon ring. The arene may be monocyclic arene or polycyclic arene. The number of carbon atoms forming the arene ring may be 5 or more and 30 or less, 5 or more and 20 or less, or 5 or more and 15 or less. Examples of arene may include benzene, naphthalene, fluorene, anthracene, phenanthrene, bibenzene, terbenzene, quaterbenzene, quinquebenzene, sexibenzene, triphenylene, pyrene, benzofluoranthene, chrysene, and the like, but are not limited to . In the present specification, a moiety obtained by removing one hydrogen atom from the above "arene" is referred to as "aryl".

In the present invention, "heteroarene" may be a ring containing one or more of O, N, P, Si, and S as heterogeneous elements. The number of carbon atoms forming the heteroarene ring may be 2 or more and 30 or less, or 2 or more and 20 or less. The heteroarene may be a monocyclic heteroarene or a polycyclic heteroarene. The polycyclic heteroarene may have, for example, a bicyclic or tricyclic structure. Examples of heteroarene may include thiophene, purine, pyrrole, pyrazole, imidazole, thiazole, oxazole, isothiazole, oxadiazole, triazole, pyridine, pyridin-2-one, pyridin-3-one, pyridin-4-one, bipyridyl, triazine, acridyl, pyridazine, pyrazine, quinoline, quinazoline, quinoxaline, phenoxazine, phthalazine, pyrimidine, pyridopyrimidine, pyridopyrazine, pyrazinopyrazine, isoquinoline, indole, carbazole, imidazopyridazine, imidazopyridine, imidazopyrimidine, pyrazolopyrimidine, imidazopyrazine or pyrazolopyridine, N-arylcarbazole, N-heteroarylcarbazole, N-alkylcarbazole, benzoxazole, benzoimidazole, benzocarbazole, benzothiophene, dibenzothiophene, thienothiophene, benzofuran, phenanthroline, isoxazole, thiadiazole, benzothiazole, tetrazole, phenothiazine, dibenzosilole, dibenzofuran, and the like, but are not limited to. In an embodiment of the present invention, the heteroarene may also include a bicyclic heterocycloarene including an arene ring fused to a heterocycloalkyl ring or a heteroarene fused to a cycloalkyl ring. In the present specification, a moiety obtained by removing one hydrogen atom from the above "heteroarene" is referred to as "heteroaryl".

In the present invention, "hydroarene" or "hydroaryl" is an aromatic hydrocarbon ring in which at least one double bond is saturated.

In the present invention, "heterohydroarene" refers to a polycyclic (bicyclic to tetracyclic) ring containing 1 to 5 heteroatoms selected from N, O, and S as ring-forming atoms, wherein at least one of the polycyclic rings may be a saturated or partially unsaturated ring, and at least the other ring may have an aromatic ring. As used herein, a moiety obtained by removing one hydrogen atom from the above "heterohydroarene" is referred to as "heterohydroaryl".

In the present invention, "ring" may be a single ring or a multi-ring. The multi-ring may be a spiro ring, a bridged ring, or a fused ring.

In the present invention, "stereoisomer" refers to compounds that have the same chemical or molecular formula but are sterically different. Stereoisomer in the present specification include optical isomer, enantiomer, diastereomer, cis/trans isomer, rotamer, and atropisomer, and each of these isomers, racemates, and mixtures thereof are also included within the scope of the present invention. For example, since the stereochemical structure of Chemical Formula 1 of the present invention is not specified, the stereoisomers of Chemical Formula 1 may be included. Unless otherwise specified, a solid bond ( 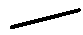 ) connected to an asymmetric carbon atom may include a wedge solid bond () or wedge dashed bond ( 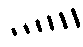 ) representing the absolute arrangement of stereocenters.

In the present invention, the term "tautomer", which is one of the structural isomers, refers to a compound that differs in structure and atomic arrangement but exists in equilibrium. Thus, the compounds of the present invention may comprise a variety of tautomers. Common tautomeric pairs include ketone-enol, amide-nitrile, lactam-lactim, amide-imidic acid in heterocyclic rings (e.g., in the nucleobases guanine, thymine, and cytosine) amine-enamine, and enamine-enamine. The following examples are included for illustrative purposes, and the present invention is not limited to these examples:

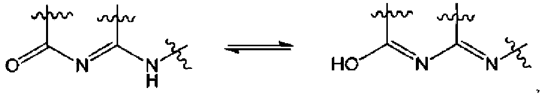

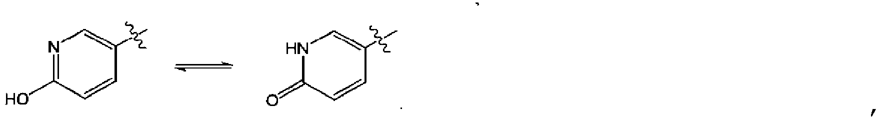

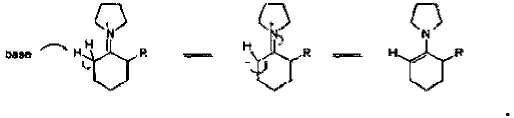

The compound represented by Chemical Formula 1 of the present invention may be present in the form of a "pharmaceutically acceptable salt". Thus, pharmaceutically acceptable salts of the compound represented by Chemical Formula 1 above are included in the scope of the compound of the present invention. The term "pharmaceutically acceptable salt" of the present invention refers to any organic or inorganic acid addition salt of the compound represented by Chemical Formula 1 at a concentration having a relatively non-toxic and harmless effective effect on patients in which side effects caused by these salts do not reduce the beneficial efficacy of the compound.

In particular, the pharmaceutically acceptable salt may be an acid addition salt formed from a free acid. Here, the acid addition salts may be obtained from inorganic acids such as hydrochloric acid, nitric acid, phosphoric acid, sulfuric acid, hydrobromic acid, hydroiodic acid, nitrous acid, phosphorous acid, and the like, non-toxic organic acids such as aliphatic mono- and dicarboxylates, phenyl-substituted alkanoates, hydroxy alkanoates and alkanedioates, aromatic acids, aliphatic and aromatic sulfonic acids, and the like, organic acids such as trifluoroacetic acid, acetate, benzoic acid, citric acid, lactic acid, maleic acid, gluconic acid, methanesulfonic acid, 4-toluenesulfonic acid, tartaric acid, fumaric acid, and the like.

Examples of the pharmaceutically acceptable salt may include sulfate, sulfite, nitrate, phosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, benzoate, phthalate, benzenesulfonate, toluenesulfonate, phenylacetate, phenylpropionate, phenylbutyrate, citrate, lactate, glycolate, malate, tartrate, mandelate, and the like.

The acid addition salt may be prepared by a conventional method, for example, may be prepared by dissolving the derivative represented by Chemical Formula 1 in an organic solvent such as methanol, ethanol, acetone, methylene chloride, acetonitrile, or the like, and adding an organic acid or an inorganic acid to form a precipitate, followed by filtering and drying the resulting precipitate, or may be prepared by distillation using a solvent and excess acid under reduced pressure, followed by drying and crystallizing the reaction product in an organic solvent.

Further, the pharmaceutically acceptable salt may be a salt obtained using a base or a metal salt. As an example of a metal salt, an alkali metal salt or an alkaline earth metal salt may be obtained by dissolving the compound in an excess alkali metal hydroxide or alkaline earth metal hydroxide solution, and filtering an insoluble compound salt, followed by evaporating and drying the filtrate. Sodium, potassium or calcium salts may be pharmaceutically suitable as alkali metal salts. In addition, the corresponding silver salt may be obtained by reacting an alkali metal or alkaline earth metal salt with a suitable silver salt (for example, silver nitrate) or may be prepared by salt production methods known in the art.

### Use of heteroaryl derivative compound

The present invention provides use of a compound represented by the following Chemical Formula 1, a tautomer thereof, a stereoisomer thereof, or a pharmaceutically acceptable salt thereof:

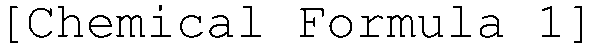

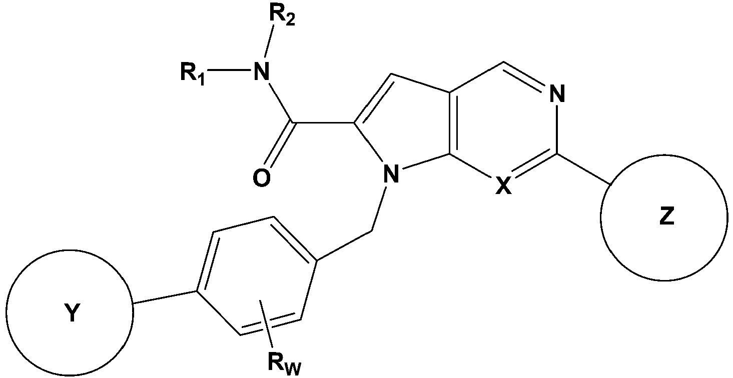

The Chemical Formula 1 is as defined above.

The compound represented by Chemical Formula 1 of the present invention, a tautomer thereof, a stereoisomer thereof, or a pharmaceutically acceptable salt thereof may inhibit the activity of USP1. Accordingly, the present invention provides a USP1 inhibitor comprising the compound represented by Chemical Formula 1, a tautomer thereof, a stereoisomer thereof, or a pharmaceutically acceptable salt thereof, as an active ingredient.

According to an embodiment of the present invention, the heteroaryl derivative represented by Chemical Formula 1 above exhibits excellent inhibitory activity against USP1, and therefore may be effectively used for the treatment or prevention of diseases associated with USP1, particularly cancer.

According to an embodiment of the present invention, the heteroaryl derivative represented by Chemical Formula 1 above has inhibitory activity in cells having BRCA1 mutation/deficiency, BRCA2 mutation/deficiency, or HR-deficiency, and therefore may be employed to treat or prevent diseases associated with USP1, PARP, BRCA1 mutation/deficiency, BRCA2 mutation/deficiency, or HR deficiency, particularly, cancer.

In the present invention, the cancer associated with USP1, PARP, BRCA1 mutation/deficiency, BRCA2 mutation/deficiency, or HR deficiency may be at least one selected from the group consisting of DNA damage repair pathway deficient cancer, homologous-recombination deficient cancer, BRCA1 mutant cancer, BRCA2 mutant cancer, PARP inhibitor-resistant or refractory cancer, BRCA1 deficient cancer, BRCA2 deficient cancer, ataxia telangiectasia mutated (ATM) mutant cancer, and mutant cancer in a gene encoding p53.

In the present invention, the cancer associated with the USP1, PARP, BRCA1 mutation/deficiency, BRCA2 mutation/deficiency, or HR deficiency may be a solid cancer or a hematological cancer. The cancer is not limited in type, but may be, for example, at least one selected from the group consisting of breast cancer, ovarian cancer, prostate cancer, pancreatic cancer, triple-negative breast cancer, cervical cancer, testicular cancer, fallopian tube cancer, cholangiocarcinoma, squamous cell carcinoma, lung cancer, lung adenocarcinoma, non-small cell lung cancer, small cell lung cancer, gastric cancer, colon cancer, rectal cancer, thyroid cancer, esophageal cancer, head and neck cancer, oral cancer, nasal cancer, throat cancer, kidney cancer, malignant pancreatic islet tumor, liver cancer, genitourinary tumor, endometrial cancer, peritoneal cancer, melanoma, Hodgkin's disease, non-Hodgkin's lymphoma, acute lymphoblastic leukemia, chronic lymphocytic leukemia, acute myeloid leukemia, hairy cell leukemia, multiple myeloma, neuroblastoma, Wilms' tumor, primary macroglobulinemia, bladder cancer, chronic myeloid leukemia, glioma, primary brain cancer, malignant melanoma, malignant carcinoid tumor, choriocarcinoma, mycosis fungoides, soft tissue sarcoma, osteogenic sarcoma, rhabdomyosarcoma, Kaposi sarcoma, fibrosarcoma, chondrosarcoma, hemangiosarcoma, Ewing sarcoma, bone cancer, malignant hypercalcemia, cervical hyperplasia, renal cell carcinoma, polycythemia vera, idiopathic thrombocythemia, adrenocortical carcinoma, skin cancer, metastatic bone cancer, metastatic brain cancer, meningioma, medulloblastoma, and spinal tumor. The cancer includes not only primary cancer but also metastatic cancer.

According to an embodiment of the present invention, the present invention provides a pharmaceutical composition for preventing or treating diseases associated with USP1, PARP, BRCA1 mutation/deficiency, BRCA2 mutation/deficiency, or HR deficiency, comprising: the compound represented by Chemical Formula 1 above, a tautomer thereof, a stereoisomer thereof, or a pharmaceutically acceptable salt thereof, as an active ingredient. Specifically, the disease associated with USP1, PARP, BRCA1 mutation/deficiency, BRCA2 mutation/deficiency, or HR deficiency may be cancer. The types of cancer are as described above.

The pharmaceutical composition of the present invention may further comprise at least one active ingredient exhibiting the same or similar efficacy in addition to the compound represented by Chemical Formula 1 above, a tautomer thereof, a stereoisomer, or a pharmaceutically acceptable salt thereof.

The pharmaceutical composition of the present invention may be used for clinical administration and may be prepared for administration in various oral and parenteral dosage forms.

In addition, according to an embodiment of the present invention, the present invention provides use of the compound represented by Chemical Formula 1 above, a tautomer thereof, a stereoisomer thereof, or a pharmaceutically acceptable salt thereof, for the manufacture of a medicament for the treatment or prevention of diseases associated with USP1, PARP, BRCA1 mutation/deficiency, BRCA2 mutation/deficiency, or HR deficiency. Specifically, the disease associated with USP1, PARP, BRCA1 mutation/deficiency, BRCA2 mutation/deficiency, or HR deficiency may be cancer. The types of cancer are as described above.

Further, according to an embodiment of the present invention, the present invention provides use of the compound represented by Chemical Formula 1 above, a tautomer thereof, a stereoisomer thereof, or a pharmaceutically acceptable salt thereof, for use in the manufacture of a medicament for the treatment or prevention of cancer. The types of cancer are as described above.

Further, according to an embodiment of the present invention, the present invention provides a method for treating or preventing diseases associated with USP1, PARP, BRCA1 mutation/deficiency, BRCA2 mutation/deficiency, or HR deficiency, comprising administering to a subject in need thereof a therapeutically effective amount of the compound represented by Chemical Formula 1 above, a tautomer thereof, a stereoisomer thereof, or a pharmaceutically acceptable salt thereof. The subject may be a mammal including a human. Specifically, the disease associated with USP1, PARP, BRCA1 mutation/deficiency, BRCA2 mutation/deficiency, or HR deficiency may be cancer. The types of cancer are as described above.

Further, according to an embodiment of the present invention, the present invention provides a method for treating or preventing cancer, comprising administering to a subject in need thereof a therapeutically effective amount of the compound represented by Chemical Formula 1 above, a tautomer thereof, a stereoisomer thereof, or a pharmaceutically acceptable salt thereof. The types of cancer are as described above.

Further, according to an embodiment of the present invention, the present invention provides a method for inhibiting USP1, comprising administering to a subject in need thereof a therapeutically effective amount of the compound represented by Chemical Formula 1 above, a tautomer thereof, a stereoisomer thereof, or a pharmaceutically acceptable salt thereof.

As used herein, the term "therapeutically effective amount" refers to an amount of the compound represented by Chemical Formula 1 that is effective for the treatment or prevention of diseases associated with USP1, PARP, BRCA1 mutation/deficiency, BRCA2 mutation/deficiency, or HR deficiency. Specifically, the "pharmaceutically effective amount" means an amount sufficient to treat a disease at a reasonable benefit/risk ratio applicable to medical treatment, and the effective dose level may be determined depending on factors including the subject type and severity, age, sex, type of diseases, the activity of the drug, the sensitivity to the drug, the time of administration, the route of administration, the rate of excretion, the duration of treatment, drugs used concurrently, and other factors well known in the medical field. The pharmaceutical composition of the present invention may be administered as an individual therapeutic agent or in combination with other therapeutic agents, or may be administered sequentially or simultaneously with a commercially available therapeutic agent. In addition, the pharmaceutical composition of the present invention may be administered singly or in multiple doses. In consideration of all of the above factors, it is important to administer an amount capable of obtaining the maximum effect with the minimum amount without side effects, which may be easily determined by those skilled in the art. The administration dose of the pharmaceutical composition of the present invention may be determined by specialists according to various factors such as the patient's condition, age, sex, complications, and the like. Since the active ingredient of the pharmaceutical composition of the present invention has excellent safety, the active ingredient may be used even above the predetermined dose.

As used herein, "prevention" refers to any action that suppresses or delays the occurrence, spread, and recurrence of the disease by administering the compound, and term "treatment" refers to any action in which the symptoms of the disease are improved or beneficially changed by administration of the compound.

Further, according to an embodiment of the present invention, the pharmaceutical composition may further comprise a pharmaceutically acceptable carrier, diluent, or excipient. In an embodiment, the present invention provides a pharmaceutical composition comprising the compound represented by Chemical Formula 1, or a pharmaceutically acceptable salt or a tautomer thereof, a stereoisomer thereof, and a pharmaceutically acceptable additive.

Examples of additives used in the pharmaceutical composition may include sweeteners, binders, solvents, solubilizers, wetting agents, emulsifiers, isotonic agents, absorbents, disintegrants, antioxidants, preservatives, lubricants, fillers, flavoring agents, and the like. For example, the additives may include lactose, dextrose, sucrose, mannitol, sorbitol, cellulose, glycine, silica, talc, stearic acid, stearin, magnesium stearate, magnesium aluminosilicate, starch, gelatin, gum tragacanth, alginic acid, sodium alginate, methylcellulose, sodium carboxymethylcellulose, agar, water, ethanol, polyethylene glycol, polyvinylpyrrolidone, sodium chloride, calcium chloride, orange essence, strawberry essence, vanilla flavor, and the like.

The pharmaceutical composition may be combined in various formulation forms for oral administration (for example, tablets, pills, powders, capsules, syrups or emulsions) or parenteral administration (for example, intramuscular, intravenous or subcutaneous injection).

For example, the pharmaceutical composition may be combined as a preparation for oral administration, and the additives used in this case may include cellulose, calcium silicate, corn starch, lactose, sucrose, dextrose, calcium phosphate, stearic acid, magnesium stearate, calcium stearate, gelatin, talc, surfactants, suspending agents, emulsifiers, diluents, and the like. In detail, solid preparations for oral administration include tablets, pills, powders, granules, capsules, and the like, and these solid preparations may be formulated by mixing at least one excipient in the composition, such as starch, calcium carbonate, sucrose, lactose, gelatin, and the like. In addition, lubricants such as magnesium stearate and talc may be used in addition to simple excipients. Further, examples of liquid preparations for oral administration may include suspensions, emulsions, syrups, and the like. Various excipients such as wetting agents, sweeteners, aromatics, preservatives, and the like, in addition to water and liquid paraffin that are commonly used simple diluents.

Further, preparations for parenteral administration may include sterilized aqueous solutions, non-aqueous solutions, suspensions, emulsions, freeze-dried preparations, suppositories. As the non-aqueous solvents and the suspensions, propylene glycol, polyethylene glycol, vegetable oils such as olive oil, and injectable esters such as ethyl oleate, and the like, may be used. As the base of the suppository, Witepsol, macrogol, Tween 61, cacao butter, laurin butter, glycerogelatin and the like, may be used. Meanwhile, conventional additives such as solubilizers, isotonic agents, suspending agents, emulsifiers, stabilizers, preservatives, and the like, may be contained in the injection.

It may also be formulated in combination preparations with other active agents to achieve synergistic action of the active ingredients.

Matters described in the use, composition, and treatment method of the present invention are equally applied unless they contradict each other.

### [Advantageous Effects]

The heteroaryl derivative compound of the present invention exhibits excellent inhibitory activity against USP1 protein and cells having BRCA1 mutation/deficiency, BRCA2 mutation/deficiency, or HR deficiency, and therefore can be effectively used for the treatment or prevention of diseases associated with USP1, PARP, BRCA1 mutation/deficiency, BRCA2 mutation/deficiency, or HR deficiency.

### [Best Mode]

Hereinafter, the present invention will be described in detail by Examples and Experimental Examples. However, the following Examples and Experimental Examples are only provided to illustrate the present invention, and the scope of the present invention is not limited thereto.

### <Analysis and purification conditions>

Compounds synthesized in Preparation Examples and Examples of the present invention were purified or subjected to structural analysis under the following conditions.

### 1. LC-MS, Prep-HPLC, MPLC

### Liquid Chromatography-Mass Spectrometry (LC-MS)

Waters UPLC system (ACQUITY UPLC PDA Detector) equipped with a mass QDa detector from Waters was used. Waters' ACQUITY UPLC^{®} BEH C18 (1.7 µm, 2.1 × 50 mm) column was used, and the column temperature was set at 30°C.
Water containing 0.1% formic acid was used as mobile phase A, and acetonitrile containing 0.1% formic acid was used as mobile phase B.
Gradient condition (10-100% B for 3 minutes, flow rate = 0.6 mL/min)

### Preparative High-Performance Liquid Chromatography (Prep-HPLC) for purification

The ACCQPrep HP150 equipment manufactured by Teledyne ISCO Inc., was used. Waters' XTERRA^{®} Prep RP18 OBD^{™} (10 µm, 30 × 300 mm) column was used, and the column temperature was set to room temperature.
Gradient condition (10-100% B for 120 minutes, flow rate = 42 mL/min)

### Medium Pressure Liquid Chromatography (MPLC) for purification

Medium pressure liquid chromatography was performed using Teledyne ISCO's CombiFlash Rf +UV.

### 2. NMR analysis

NMR analysis was performed using an NMR AVANCE NEO 400 MHz manufactured by Bruker, and data are expressed as ppm (parts per million, δ). The commercial reagents used were employed without further purification. In the present invention, room temperature or ambient temperature refers to a temperature of about 5°C to 40°C, as an example, 10°C to 30°C, and as another example, 20°C to 27°C, and is not strictly limited to the above range. Concentration under reduced pressure or solvent distillation was performed using a rotary evaporator.

### Preparation Example: Preparation of intermediate compound of the present invention

### Preparation Example 1. Preparation of (4-(1-isopropyl-4-(trifluoromethyl)-1H-imidazol-2-yl)phenyl)methanamine

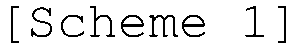

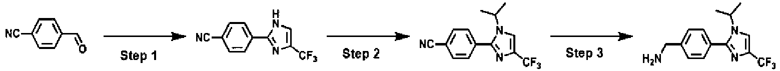

### [Step 1] Preparation of 4-(4-(trifluoromethyl)-1H-imidazol-2-yl)benzonitrile

3,3-Dibromo-1,1,1-trifluoropropan-2-one (22.74 mL, 185 mmol) was dissolved in water (70 mL), then sodium acetate (15.14 g, 185 mmol) was added and stirred at 100°C for 1 hour. The reaction mixture was cooled, and 4-formylbenzonitrile (22 g, 168 mmol) dissolved in methanol (489 mL) and ammonia water (183 mL, 4697 mmol) were then added at 0°C. The resulting reaction mixture was stirred at room temperature for 2 hours, and then stirred at 100°C for 2 hours. After confirming the formation of the target compound using LC-MS, the organic material was extracted with water and ethyl acetate, and the collected organic layer was dried over sodium sulfate to remove the remaining water and concentrated to obtain the target compound as a white solid (37 g, 84% yield). MS (ESI): m/z 238 [M+H]⁺

### [Step 2] Preparation of 4-(1-isopropyl-4-(trifluoromethyl)-1H-imidazol-2-yl)benzonitrile

The 4-(4-(trifluoromethyl)-1H-imidazol-2-yl)benzonitrile (2 g, 8.43 mmol) prepared in Step 1 was dissolved in dimethylformamide (DMF; 30 mL), and cesium carbonate (Cs₂CO₃; 8.24 g, 25.30 mmol) and 2-iodopropane (4.30 g, 25.30 mmol, 2.53 mL) were then added and stirred at 90°C for 12 hours. The reaction mixture was filtered, and the resulting filtrate was added to cold water (500 mL), and then extracted with ethyl acetate (200 mL × 2). The collected organic layer was washed with saturated sodium chloride solution (300 mL), and the resulting product was dried over anhydrous sodium sulfate to remove the remaining water and concentrated. The concentrated mixture was purified by MPLC (petroleum ether/ethyl acetate = 50/1 ~ 0/1) to obtain the target compound (3.8 g, 80% yield) as a yellow solid.

¹H NMR (400 MHz, DMSO-*d*₆): δ 8.29-8.25 (m, 1H), 8.08-7.93 (m, 2H), 7.85-7.72 (m, 2H), 4.55-4.43 (m, 1H), 1.43 (d, *J* = 6.4 Hz, 6H).

### [Step 3] Preparation of (4-(1-isopropyl-4-(trifluoromethyl)-1H-imidazol-2-yl)phenyl)methanamine

The 4-(1-isopropyl-4-(trifluoromethyl)-1H-imidazol-2-yl)benzonitrile (1.9 g, 6.80 mmol) prepared in Step 2 was dissolved in methanol (100 mL), and hydrochloric acid (HCl; 12 M, 1.13 mL) and palladium/carbon (Pd/C; 0.2 g, 187.93 µmol, 10% purity) were then added under nitrogen gas. The temperature of the reaction mixture was raised to 50°C and the resulting mixture was stirred under hydrogen gas (50 Psi) conditions for 12 hours. After confirming the completion of the reaction, the reaction mixture was filtered to obtain the target compound (3.2 g, crude, HCl) as a green solid.

¹H NMR (400 MHz, DMSO-*d*₆): δ 8.43 (s, 3H), 8.19 (s, 1H), 7.62 (s, 4H), 4.50 - 4.37 (m, 1H), 4.10 (s, 2H), 1.40 (d, *J* = 5.6 Hz, 6H).

### Preparation Example 2. Preparation of (4-(5-methyl-3-(trifluoromethyl)-1H-pyrazol-1-yl)phenyl)methanamine

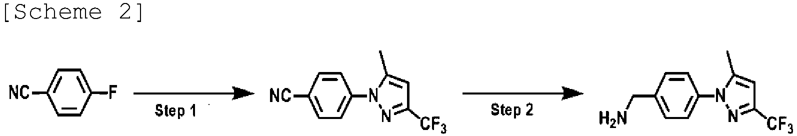

### [Step 1] 4-(5-methyl-3-(trifluoromethyl)-1H-pyrazol-1-yl)benzonitrile

4-Fluorobenzonitrile (645 mg, 5.33 mmol) was dissolved in dimethyl sulfoxide (DMSO; 6 mL), and 5-methyl-3-(trifluoromethyl)-1H-pyrazole (800 mg, 5.33 mmol) and potassium carbonate (K₂CO₃; 1.4 g, 10.66 mmol) were added, and the mixture was stirred at 100°C for 5 hours. After confirming the formation of the target compound using LC-MS, the reaction mixture was diluted with water and ethyl acetate, and the organic material was extracted with a saturated sodium chloride solution. To the collected organic layer, magnesium sulfate was added to remove the remaining water, and the organic layer obtained by filtration was concentrated under reduced pressure. The concentrated mixture was purified by MPLC (petroleum ether/ethyl acetate = 1/0 ~ 5/1) to obtain the target compound (1.2 g, 90 % yield) as a white solid. MS (ESI): m/z 252.0 [M+H]⁺

### [Step 2] (4-(5-methyl-3-(trifluoromethyl)-1H-pyrazol-1-yl)phenyl)methanamine

The 4-(5-methyl-3-(trifluoromethyl)-1H-pyrazol-1-yl)benzonitrile (1.2 g, 4.78 mmol) obtained in Step 1 was dissolved in tetrahydrofuran (THF; 16 mL), and lithium aluminum hydride (4.78 mL, 9.55 mmol) dissolved in tetrahydrofuran under nitrogen was added at 0°C, followed by stirring at room temperature for 1 hour. After confirming the formation of the target compound using LC-MS, water was slowly added to the reaction mixture, followed by stirring at room temperature for 30 minutes, and 15% aqueous sodium hydroxide solution was then added and stirred at room temperature for 30 minutes. The organic material was extracted with ethyl acetate, the collected organic layer was dried over sodium sulfate to remove the remaining water, followed by filtration, and the organic layer obtained by filtering was concentrated under reduced pressure. The concentrated mixture was purified by MPLC (petroleum ether/ethyl acetate = 1/0 ~ 0/1) to obtain the target compound (0.8 g, 66 % yield) as yellow liquid.

¹H NMR (400 MHz, CDCl₃): δ 7.52-7.37 (m, 4H), 6.45 (s, 1H), 3.95 (s, 2H), 2.34 (s, 3H), 1.55 (s, 2H); MS (ESI): m/z 256.0 [M+H]⁺

### Example: Preparation of compounds of the present invention

### Example 1. Preparation of 2-(4-cyclopropyl-6-methoxypyrimidin-5-yl)-7-(4-(1-isopropyl-4-(trifluoromethyl)-1H-imidazol-2-yl)benzyl)-N-methyl-7H-pyrrolo[2,3-d]pyrimidine-6-carboxamide

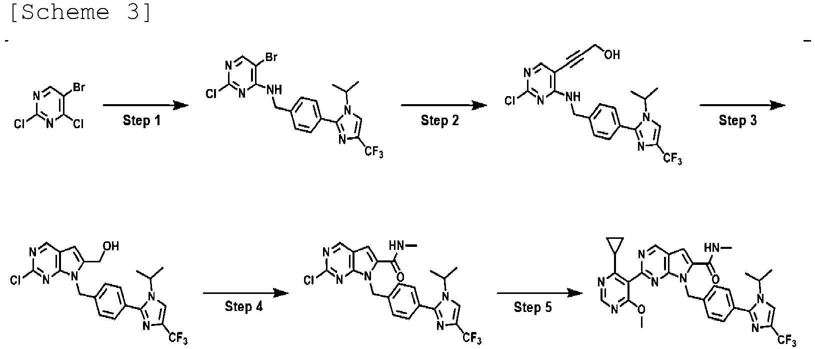

### [Step 1] Preparation of 5-bromo-2-chloro-N-(4-(1-isopropyl-4-(trifluoromethyl)-1H-imidazol-2-yl)benzyl)pyrimidin-4-amine

The (4-(1-isopropyl-4-(trifluoromethyl)-1H-imidazol-2-yl)phenyl)methanamine (3 g, 10.59 mmol) obtained in Preparation Example 1 was dissolved in acetonitrile (26 mL), and 5-bromo-2,4-dichloropyrimidine (2.4 g, 10.59 mmol) and DIPEA (5.55 mL, 31.8 mmol) were added and stirred at room temperature for 4 hours. After confirming the formation of the target compound using LC-MS, the reaction mixture was concentrated. The concentrated mixture was purified by MPLC (hexane/ethyl acetate = 20/1 ~ 1/1) to obtain the target compound (4.5 g, 90% yield) as a white solid. MS (ESI): m/z 474 [M+H]⁺

### [Step 2] Preparation of 3-(2-chloro-4-((4-(1-isopropyl-4-(trifluoromethyl)-1H-imidazol-2-yl)benzyl)amino)pyrimidin-5-yl)prop-2-yn-1-ol

The 5-bromo-2-chloro-N-(4-(1-isopropyl-4-(trifluoromethyl)-1H-imidazol-2-yl)benzyl)pyrimidin-4-amine (200 mg, 0.42 mmol) obtained in Step 1 was dissolved in THF (2.1 mL), and prop-2-yn-1-ol (48.6 µl, 0.84 mmol), TBAF (400 mg, 1.26 mmol), and Pd(dppf)Cl₂.DCM (69 mg, 0.084 mmol) were then added and stirred at 70°C for 5 hours. After confirming the formation of the target compound using LC-MS, the organic material was extracted with water and ethyl acetate. The collected organic layer was dried over sodium sulfate to remove the remaining water and concentrated. The concentrated mixture was purified by MPLC (hexane/ethyl acetate = 20/1 ~ 0/1) to obtain the target compound (60 mg, 32% yield) as a white solid. MS (ESI): m/z 450 [M+H]⁺

### [Step 3] Preparation of (2-chloro-7-(4-(1-isopropyl-4-(trifluoromethyl)-1H-imidazol-2-yl)benzyl)-7H-pyrrolo[2,3-d]pyrimidin-6-yl)methanol

The 3-(2-chloro-4-((4-(1-isopropyl-4-(trifluoromethyl)-1H-imidazol-2-yl)benzyl)amino)pyrimidin-5-yl)prop-2-yn-1-ol (60 mg, 0.13 mmol) obtained in Step 2 was dissolved in THF (1.33 mL), and TBAF (126 mg, 0.40 mmol) was then added and stirred at 70°C for 12 hours. After confirming the formation of the target compound using LC-MS, the organic material was extracted with water and ethyl acetate, and the collected organic layer was dried over sodium sulfate to remove the remaining water and concentrated. The concentrated mixture was purified by MPLC (hexane/ethyl acetate = 20/1 ~ 0/1) to obtain the target compound (41 mg, 68% yield) as a white solid. MS (ESI): m/z 450 [M+H]⁺

### [Step 4] Preparation of 2-chloro-7-(4-(1-isopropyl-4-(trifluoromethyl)-1H-imidazol-2-yl)benzyl)-N-methyl-7H-pyrrolo[2,3-d]pyrimidine-6-carboxamide

The (2-chloro-7-(4-(1-isopropyl-4-(trifluoromethyl)-1H-imidazol-2-yl)benzyl)-7H-pyrrolo[2,3-d]pyrimidin-6-yl)methanol (41 mg, 0.091 mmol) obtained in Step 3 was dissolved in DMF (0.9 mL), and methylamine solution (2.0 M in THF, 181 µl, 0.363 mmol) and sodium cyanide (NaCN; 4.44 mg, 0.091 mmol) were then added and stirred at room temperature for 4 minutes. Manganese dioxide (MnO₂; 403 mg, 4.08 mmol) was added to the reaction mixture and stirred at room temperature for 1 hour. After confirming the formation of the target compound using LC-MS, the organic material was extracted with water and ethyl acetate. The collected organic layer was dried over sodium sulfate to remove the remaining water and concentrated. The concentrated mixture was purified by MPLC (hexane/ethyl acetate = 20/1 ~ 0/1) to obtain the target compound (43 mg, 98% yield) as a white solid. MS (ESI): m/z 477 [M+H]⁺

### [Step 5] Preparation of 2-(4-cyclopropyl-6-methoxypyrimidin-5-yl)-7-(4-(1-isopropyl-4-(trifluoromethyl)-1H-imidazol-2-yl)benzyl)-N-methyl-7H-pyrrolo[2,3-d]pyrimidine-6-carboxamide

The 2-chloro-7-(4-(1-isopropyl-4-(trifluoromethyl)-1H-imidazol-2-yl)benzyl)-N-methyl-7H-pyrrolo[2,3-d]pyrimidine-6-carboxamide (43 mg, 0.09 mmol) obtained in Step 4 was dissolved in dioxane (2.5 mL) and water (5 mL), then 4-cyclopropyl-6-methoxy-5-(4,4,5,5-tetramethyl-1,3,2-dioxaborolan-2-yl)pyrimidine (37 mg, 0.135 mmol), K₃PO₄ (57.4 mg, 0.271 mmol), and Pd(dppf)Cl₂.DCM (7.4 mg, 9.02 µmol) were added, and the mixture was stirred at 90°C for 16 hours. After confirming the formation of the target compound using LC-MS, the reaction mixture was filtered through Celite and concentrated. The concentrated mixture was purified by prep-HPLC to obtain the target compound (30 mg, 56% yield) as a white solid.

¹H NMR (400 MHz, DMSO-*d*₆): δ 9.33 (s, 1H), 8.83-8.79 (m, 1H), 8.68 (s, 1H), 8.15 (s, 1H), 7.46 (d, *J* = 8.1 Hz, 2H), 7.35 (s, 1H), 7.29 (d, *J* = 8.1 Hz, 2H), 5.95 (s, 2H), 4.42-4.35 (m, 1H), 3.84 (s, 3H), 2.80 (d, *J* = 4.6 Hz, 3H), 1.66-1.60 (m, 1H), 1.37 (d, *J* = 6.6 Hz, 6H), 1.04-0.99 (m, 2H), 0.81-0.74 (m, 2H); MS (ESI): m/z 591 [M+H]⁺

### <Examples 1> to <Example 226>

All other compounds according to Examples of the present invention (Examples 2 to 226 compounds) were prepared in a similar manner to Example 1 above, and the chemical structural formulas, compound names, NMR, and LC-MS analysis results of respective compounds according to Examples above are summarized and shown in Table 1 below.

**[Table 1]**

| Ex am pl e | Chemical Structure | Compound name | ¹H NMR | LC-MS [M+H] + |
|---|---|---|---|---|
| 1 | 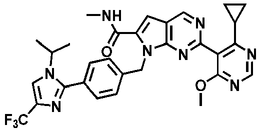 | 2-(4-cyclopropyl-6-methoxypyrimid in-5-yl)-7-(4-(1-isopropyl-4-(trifluorometh yl)-1H-imidazol-2-yl)benzyl)-N-methyl-7H-pyrrolo[2,3-d]pyrimidine-6-carboxamide | ¹H NMR (400 MHz, DMSO-*d*₆): δ 9.33 (s, 1H), 8.83-8.79 (m, 1H), 8.68 (s, 1H), 8.15 (s, 1H), 7.46 (d, *J* = 8.1 Hz, 2H), 7.35 (s, 1H), 7.29 (d, *J* = 8.1 Hz, 2H), 5.95 (s, 2H), 4.42-4.35 (m, 1H), 3.84 (s, 3H), 2.80 (d, *J* = 4.6 Hz, 3H), 1.66-1.60 (m, 1H), 1.37 (d, *J =* 6.6 Hz, 6H), 1.04-0.99 (m, 2H), 0.81-0.74 (m, 2H). | 591.2 |
| 2 | 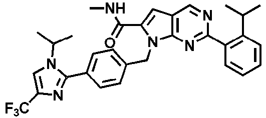 | 7-(4-(1-isopropyl-4-(trifluorometh yl)-1H-imidazol-2-yl)benzyl)-2-(2-isopropylpheny l)-N-methyl-7H-pyrrolo[2,3-d]pyrimidine-6-carboxamide | ¹H NMR (400 MHz, DMSO-*d*₆): δ 9.33 (s, 1H), 8.80 (q, *J* = 4.5 Hz, 1H), 8.15 (d, *J* = 1.4 Hz, 1H), 7.64 (dd, *J* = 7.8, 1.4 Hz, 1H), 7.49-7.39 (m, 4H), 7.35 (s, 1H), 7.28 (ddd, *J* = 8.3, 6.8, 1.8 Hz, 1H), 7.20 (d, *J* = 8.2 Hz, 2H), 5.99 (s, 2H), 4.41 (p, *J* = 6.6 Hz, 1H), 3.49 (p, *J* = 6.7 Hz, 1H), 2.80 (d, *J* = 4.6 Hz, 3H), 1.37 (s, 3H), 1.36 (s, 3H), 1.09 (s, 3H), 1.07 (s, 3H). | 561.3 |
| 3 | 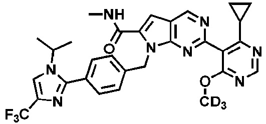 | 2-(4-cyclopropyl-6-(methoxy-d₃)pyrimidin-5-yl)-7-(4-(1-isopropyl-4-(trifluorometh yl)-1H-imidazol-2-yl)benzyl)-N-methyl-7H-pyrrolo[2,3-d]pyrimidine-6-carboxamide | ¹H NMR (400 MHz, CDCl₃) : δ 9.19 (s, 1H), 8.67 (s, 1H), 7.44 (s, 1H), 7.42 (s, 2H), 7.41 (s, 1H), 7.39 (t, *J* = 1.2 Hz, 1H), 6.90 (s, 1H), 6.15 (d, *J =* 5.1 Hz, 1H), 6.01 (s, 2H), 4.50 (p, *J* = 6.8 Hz, 1H), 2.99 (d, *J* = 4.9 Hz, 3H), 1.72 (tt, *J* = 8.4, 4.6 Hz, 1H), 1.42 (d, *J* = 6.7 Hz, 6H), 1.25-1.21 (m, 2H), 0.86 (dt, *J =* 8.1, 3.4 Hz, 2H) . | 594.2 |
| 4 | 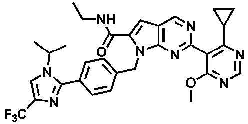 | 2-(4-cyclopropyl-6-methoxypyrimid in-5-yl)-N-ethyl-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, CDCl₃) : δ 9.18 (s, 1H), 8.67 (s, 1H), 7.46-7.43 (m, 1H), 7.43 (s, 1H), 7.41 (s, 1H), 7.38 (d, *J* = 1.4 Hz, 2H), 6.90 (s, 1H), 6.13 (t, *J* = 5.8 Hz, 1H), 6.00 (s, 2H), 4.49 (p, *J* = 6.7 Hz, 1H), 3.94 (s, 3H), 3.48-3.43 (m, 2H), 1.72 (tt, *J* = 8.3, 4.6 Hz, 1H), 1.42 (d, *J* = 6.8 Hz, 6H), 1.26-1.21 (m, 5H), 0.86 (dq, *J* = 7.0, 3.8 Hz, 2H). | 605.3 |
| 5 | 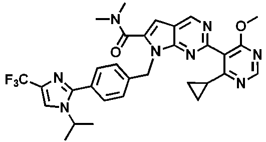 | 2-(4-cyclopropyl-6-methoxypyrimid in-5-yl)-7-(4-(1-isopropyl-4-(trifluorometh yl)-1H-imidazol-2-yl)benzyl)-N,N-dimethyl-7H-pyrrolo[2,3-d]pyrimidine-6-carboxamide | ¹H NMR (400 MHz, Methanol-*d*₄) : δ 9.24 (s, 1H), 8.67 (s, 1H), 7.93 (d, *J* = 1.3 Hz, 1H), 7.55-7.49 (m, 2H), 7.38 (d, *J* = 8.2 Hz, 2H), 7.03 (s, 1H), 5.75 (s, 2H), 4.50 (h, *J* = 6.7 Hz, 1H), 3.97 (s, 3H), 3.06 (s, 3H), 2.87 (s, 3H), 1.77 (tt, *J* = 8.4, 4.6 Hz, 1H), 1.45 (d, *J* = 6.7 Hz, 6H), 1.20 (t, *J* = 6.9 Hz, 2H), 0.93 (dt, *J* = 8.1, 3.4 Hz, 2H). | 605.0 |
| 6 | 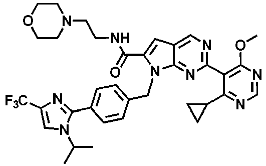 | 2-(4-cyclopropyl-6-methoxypyrimid in-5-yl)-7-(4-(1-isopropyl-4-(trifluorometh yl)-1H-imidazol-2-yl)benzyl)-N-(2-morpholinoethy l)-7H-pyrrolo[2,3-d]pyrimidine-6-carboxamide | ¹H NMR (400 MHz, Methanol-*d*₄) : δ 9.24 (s, 1H), 8.64 (s, 1H), 7.91 (d, *J* = 1.3 Hz, 1H), 7.47 (d, *J* = 8.2 Hz, 2H), 7.40 (d, *J* = 8.2 Hz, 2H), 7.32 (s, 1H), 6.05 (s, 2H), 4.52 (h, *J =* 6.9 Hz, 1H), 3.94 (s, 3H), 3.72 (t, *J* = 4.7 Hz, 4H), 3.57 (t, *J* = 6.6 Hz, 2H), 2.65 (s, 6H), 1.70 (tt, *J* = 8.4, 4.6 Hz, 1H), 1.44 (d, *J* = 6.7 Hz, 6H), 1.30-1.18 (m, 2H), 1.16 (tt, *J* = 6.5, 3.3 Hz, 2H), 0.87 (dt, *J =* 8.2, 3.4 Hz, 2H). | 690.0 |
| 7 | 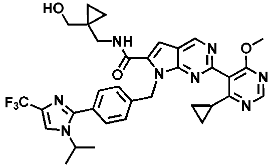 | 2-(4-cyclopropyl-6-methoxypyrimid in-5-yl)-N-((1-(hydroxymethyl )cyclopropyl)m ethyl)-7-(4-(1-isopropyl-4-(trifluoromethyl)-1H-imidazol-2-yl)benzyl)-7H-pyrrolo[2,3-d]pyrimidine-6-carboxamide | ¹H NMR (400 MHz, Methanol-*d*₄) : δ 9.27 (s, 1H), 8.76 (t, *J =* 5.9 Hz, 1H), 8.65 (s, 1H), 7.91 (d, *J* = 1.3 Hz, 1H), 7.47 (d, *J* = 8.3 Hz, 2H), 7.39 (d, *J* = 8.2 Hz, 2H), 7.33 (s, 1H), 6.05 (s, 2H), 4.51 (p, *J =* 6.7 Hz, 1H), 3.95 (s, 3H), 3.47-3.41 (m, 2H), 3.39 (s, 2H), 1.73 (tt, *J* = | 661.0 |
| 8 | 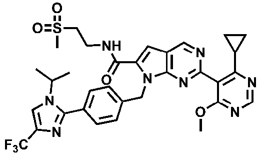 | 2-(4-cyclopropyl-6-methoxypyrimid in-5-yl)-7-(4-(1-isopropyl-4-(trifluorometh yl)-1H-imidazol-2-yl)benzyl)-N-(2-(methylsulfony l)ethyl)-7H-pyrrolo[2,3-d]pyrimidine-6-carboxamide | ¹H NMR (400 MHz, CDCl₃) : δ 9.22 (s, 1H), 8.67 (s, 1H), 7.43 (s, 1H), 7.41 (d, *J* = 1.7 Hz, 1H), 7.39 (d, *J* = 1.3 Hz, 1H), 7.34 (s, 1H), 7.32 (d, *J* = 1.7 Hz, 1H), 7.07 (t, *J* = 6.0 Hz, 1H), 7.03 (s, 1H), 6.00 (s, 2H), 4.49 (p, *J =* 6.7 Hz, 1H), 3.96 (dd, *J* = 6.9, 4.3 Hz, 2H), 3.93 (s, 3H), 3.20-3.15 (m, 2H), 3.01 (s, 3H), 1.72 (tt, *J* = 8.3, 4.6 Hz, 1H), 1.42 (d, *J* = 6.7 Hz, 6H), 1.22 (dt, *J* = 6.5, 3.3 Hz, 2H), 0.88-0.82 (m, 2H). 8.4, 4.7 Hz, 1H), 1.44 (d, *J* = 6.7 Hz, 6H), 1.16 (dt, *J* = 6.4, 3.3 Hz, 2H), 0.89 (dt, *J* = 8.2, 3.4 Hz, 2H), 0.56 (q, *J* = 4.0 Hz, 2H), 0.51-0.43 (m, 2H). | 683.2 |
| 9 | 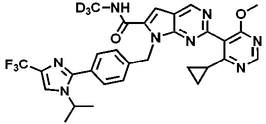 | 2-(4-cyclopropyl-6-methoxypyrimid in-5-yl)-7-(4-(1-isopropyl-4-(trifluorometh yl)-1H-imidazol-2-yl)benzyl)-N-(methyl-d₃)-7H-pyrrolo[2,3-d]pyrimidine-6-carboxamide | ¹H NMR (400 MHz, DMSO-*d*₆): δ 9.34 (s, 1H), 8.80 (s, 1H), 8.68 (s, 1H), 8.15 (d, *J* = 1.4 Hz, 1H), 7.46 (d, *J* = 8.0 Hz, 2H), 7.35 (s, 1H), 7.29 (d, *J* = 8.0 Hz, 2H), 5.96 (s, 2H), 4.39 (p, *J* = 6.7 Hz, 1H), 3.84 (s, 3H), 1.64 (dq, *J* = 8.2, 4.7, 4.2 Hz, 1H), 1.37 (d, *J* = 6.6 Hz, 6H), 1.02 (p, *J* = 3.7 Hz, 2H), 0.79 (dd, *J* = 8.1, 3.3 Hz, 2H). | 594.0 |
| 10 | 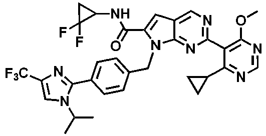 | 2-(4-cyclopropyl-6-methoxypyrimid in-5-yl)-N-(2,2-difluorocyclop ropyl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, Methanol-*d*₄): δ 9.25 (s, 1H), 8.64 (s, 1H), 7.90 (d, *J* = 1.3 Hz, 1H), 7.47 (d, *J* = 8.3 Hz, 2H), 7.41 (d, *J* = 8.3 Hz, 2H), 7.35 (s, 1H), 6.05 (s, 2H), 4.50 (p, *J =* 6.6 Hz, 1H), 3.94 (s, 3H), 1.71 (tt, *J* = 8.4, 4.7 Hz, 1H), 1.66-1.53 (m, 1H), 1.44 (dd, *J =* 6.7, 2.6 Hz, 6H), 1.31 (s, 2H), 1.16 (dt, *J* = 6.4, 3.3 Hz, 2H), 0.91-0.86 (m, 2H). | 653.0 |
| 11 | 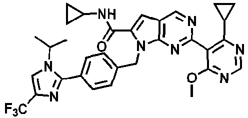 | N-cyclopropyl-2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, CDCl₃): δ 9.17 (s, 1H), 8.67 (s, 1H), 7.48 - 7.45 (m, 1H), 7.44 (d, *J* = 2.0 Hz, 1H), 7.42-7.38 (m, 3H), 6.87 (s, 1H), 6.29-6.24 (m, 1H), 6.01 (s, 2H), 4.50 (p, *J* = 6.7 Hz, 1H), 3.94 (s, 3H), 2.84 (td, *J* = 6.8, 3.2 Hz, 1H), 1.72 (tt, *J* = 8.3, 4.5 Hz, 1H), 1.42 (d, *J =* 6.7 Hz, 6H), 1.24 (td, *J* = 5.1, 4.4, 2.1 Hz, 2H), 0.93-0.88 (m, 2H), 0.86 (dd, *J* = 8.1, 3.1 Hz, 2H), 0.60 (d, *J* = 4.5 Hz, 2H). | 617.2 |
| 12 | 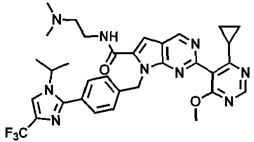 | 2-(4-cyclopropyl-6-methoxypyrimid in-5-yl)-N-(2-(dimethylamino )ethyl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, CDCl₃) : δ 9.19 (s, 1H), 8.67 (s, 1H), 7.45 (s, 1H), 7.43 (s, 1H), 7.41 (s, 1H), 7.40-7.37 (m, 2H), 6.98 (s, 1H), 6.90 (d, *J* = 5.2 Hz, 1H), 6.02 (s, 2H), 4.50 (h, *J* = 6.7 Hz, 1H), 3.94 (s, 3H), 3.48 (q, *J =* 5.6 Hz, 2H), 2.50 (t, *J* = 5.9 Hz, 2H), 2.26 (s, 6H), 1.73 (dq, *J* = 8.0, 4.1, 3.6 Hz, 1H), 1.41 (d, *J* = 6.8 Hz, 6H), 1.25-1.20 (m, 2H), 0.86 (td, *J* = 5.4, 2.7 Hz, 2H). | 648.3 |
| 13 | 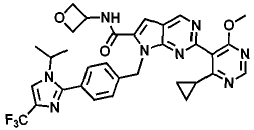 | 2-(4-cyclopropyl-6-methoxypyrimid in-5-yl)-7-(4-(1-isopropyl-4-(trifluorometh yl)-1H-imidazol-2-yl)benzyl) -N-(oxetan-3-yl)-7H-pyrrolo[2,3-d]pyrimidine-6-carboxamide | ¹H NMR (400 MHz, DMSO-*d*₆): δ 9.50 (d, *J* = 6.5 Hz, 1H), 9.38 (s, 1H), 8.68 (s, 1H), 8.15 (s, 1H), 7.50 (s, 1H), 7.46 (d, *J* = 8.2 Hz, 2H), 7.26 (d, *J* = 8.2 Hz, 2H), 5.91 (s, 2H), 5.04-4.97 (m, 1H), 4.81-4.76 (m, 2H), 4.59 - 4.55 (m, 2H), 4.40-4.36 (m, 1H), 3.84 (s, 3H), 1.67-1.63 (m, 1H), 1.36 (d, *J =* 6.6 Hz, 6H), 1.04-1.01 (m, 2H), 0.81-0.78 (m, 2H). | 633.2 |
| 14 | 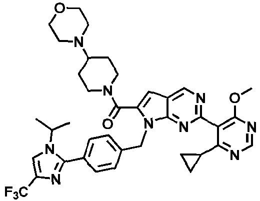 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (4-morpholinopipe ridin-1-yl)methanone | ¹H NMR (400 MHz, DMSO-*d₆*): δ 9.26 (s, 1H), 8.69 (s, 1H), 8.16 (s, 1H), 7.52 (d, *J* = 8.1 Hz, 2H), 7.25 (d, *J* = 8.1 Hz, 2H), 6.97 (s, 1H), 5.75-5.53 (m, 3H), 4.53-4.36 (m, 4H), 3.86 (s, 3H), 2.32-2.28 (m, 4H), 1.89 (s, 6H), 1.77-1.67 (m, 2H), 1.41-1.36 (m, 6H), 1.27-1.21 (m, 2H), 1.07-1.03 (m, 2H), 0.87-0.84 (m, 2H). | 730.3 |
| 15 | 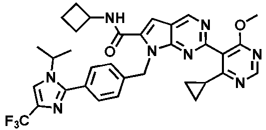 | N-cyclobutyl-2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-ds) : δ 9.34 (s, 1H), 9.00 (d, *J* = 7.6 Hz, 1H), 8.68 (s, 1H), 8.15 (d, *J* = 1.0 Hz, 1H), 7.45 (d, *J* = 8.2 Hz, 2H), 7.40 (s, 1H), 7.27 (d, *J* = 8.2 Hz, 2H), 5.91 (s, 2H), 4.43-4.35 (m, 2H), 3.84 (s, 3H), 2.25-2.20 (m, 2H), 2.09-2.03 (m, 2H), 1.71-1.63 (m, 3H), 1.36 (d, *J* = 6.6 Hz, 6H), 1.03-1.00 (m, 2H), 0.81-0.77 (m, 2H). | 631.3 |
| 16 | 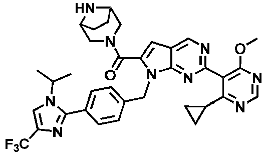 | (3,8-diazabicyclo[3 .2.1]octan-3-yl)(2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl)methanone | ¹H NMR (400 MHz, DMSO-*d₆*) : δ 9.26 (s, 1H), 8.67 (d, *J* = 9.7 Hz, 1H), 8.17 (s, 1H), 7.49 (d, *J* = 8.0 Hz, 2H), 7.25 (d, *J* = 8.1 Hz, 2H), 6.96 (s, 1H), 5.70-5.66 (m, 2H), 5.34 - 5.30 (m, 1H), 3.85 (s, 3H), 2.88-2.87 (m, 1H), 2.30-2.26 (m, 3H), 2.03-1.96 (m, 6H), 1.36 (d, 6H), 1.05-1.03 (m, 2H), 0.86-0.84 (m, 4H). | 672.3 |
| 17 | 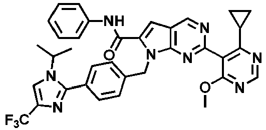 | 2-(4-cyclopropyl-6-methoxypyrimid in-5-yl)-7-(4-(1-isopropyl-4-(trifluorometh yl)-1H-imidazol-2-yl)benzyl)-N-phenyl-7H-pyrrolo[2,3-d]pyrimidine-6-carboxamide | ¹H NMR (400 MHz, CDCl₃) : δ 9.23 (s, 1H), 8.68 (s, 1H), 7.84 (s, 1H), 7.59 (t, *J* = 1.4 Hz, 1H), 7.58 (s, 1H), 7.48 (d, *J* = 1.8 Hz, 1H), 7.46 (d, *J* = 2.0 Hz, 1H), 7.42 (d, *J* = 2.0 Hz, 1H), 7.40 (d, *J* = 1.7 Hz, 2H), 7.38 (d, *J* = 1.7 Hz, 1H), 7.37 (d, *J* = 1.3 Hz, 1H), 7.23-7.17 (m, 1H), 7.10 (s, 1H), 6.05 (s, 2H), 4.46 (p, J = 6.7 Hz, 1H), 3.95 (s, 3H), 1.74 (tt, *J* = 8.3, 4.6 Hz, 1H), 1.39 (d, *J* = 6.7 Hz, 6H), 1.25-1.22 (m, 2H), 0.88 (dt, *J* = 8.1, 3.4 Hz, 2H). | 653.3 |
| 18 | 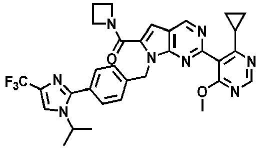 | azetidin-1-yl(2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl)methanone | ¹H NMR (400 MHz, DMSO-*d*₆): δ 9.30 (s, 1H), 8.69 (s, 1H), 8.16 (s, 1H), 7.50 (d, *J* = 7.9 Hz, 2H), 7.28 (d, *J* = 8.0 Hz, 2H), 7.18 (s, 1H), 5.84 (s, 2H), 4.41 (p, *J* = 6.7 Hz, 1H), 4.31 (t, *J* = 7.6 Hz, 2H), 4.06 (t, *J* = 7.7 Hz, 2H), 3.84 (s, 3H), 2.24 (q, *J* = 7.7 Hz, 2H), 1.65 (dq, *J* = 8.1, 4.0, 3.5 Hz, 1H), 1.38 (s, 3H), 1.37 (s, 3H), 1.05-1.00 (m, 2H), 0.80 (dd, *J* = 7.8, 3.5 Hz, 2H). | 617.2 |
| 19 | 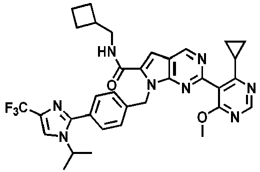 | N-(cyclobutylmet hyl)-2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.29 (s, 1H), 8.78 (t, *J* = 5.9 Hz, 1H), 8.64 (s, 1H), 8.11 (d, *J* = 1.4 Hz, 1H), 7.44-7.37 (m, 2H), 7.29 (s, 1H), 7.25 (d, *J* = 8.1 Hz, 2H), 5.89 (s, 2H), 4.33 (p, *J* = 6.6 Hz, 1H), 3.80 (s, 3H), 3.25 (d, *J* = 6.7 Hz, 2H), 1.90 (ddd, *J* = 8.3, 5.9, 2.9 Hz, 2H), 1.79-1.71 (m, 2H), 1.70-1.56 (m, 4H), 1.32 (d, *J* = 6.6 Hz, 6H), 0.98 (p, *J* = 3.7 Hz, 2H), 0.78-0.73 (m, 2H). | 645.3 |
| 20 | 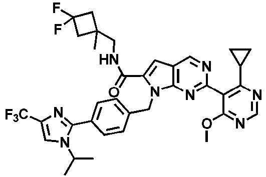 | 2-(4-cyclopropyl-6-methoxypyrimid in-5-yl)-N-((3,3-difluoro-1-methylcyclobut yl)methyl)-7-(4- (1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.37 (s, 1H), 8.99 (t, *J* = 6.3 Hz, 1H), 8.69 (s, 1H), 8.16 (d, *J* = 1.5 Hz, 1H), 7.47-7.43 (m, 2H), 7.42 (s, 1H), 7.28 (d, *J* = 8.2 Hz, 2H), 5.95 (s, 2H), 4.38 (p, *J* = 6.7 Hz, 1H), 3.85 (s, 3H), 3.37 (d, *J* = 6.3 Hz, 2H), 2.67-2.60 (m, 2H), 2.23 (dd, *J* = 14.7, 10.2 Hz, 2H), 1.65 (td, *J* = 8.1, 4.1 Hz, 1H), 1.37 (d, *J* = 6.6 Hz, 6H), 1.16 (s, 3H), 1.05-1.01 (m, 2H), 0.81 (dd, *J* = 8.0, 3.2 Hz, 2H). | 695.3 |
| 21 | 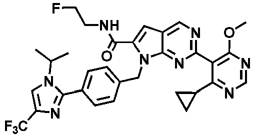 | 2-(4-cyclopropyl-6-methoxypyrimid in-5-yl)-N-(2-fluoroethyl)-7-(4-(1-isopropyl-4-(trifluoromethyl)-1H-imidazol-2-yl)benzyl)-7H-pyrrolo[2,3-d]pyrimidine-6-carboxamide | ¹H NMR (400 MHz, DMSO-*d*₆): δ 9.36 (s, 1H), 9.10 (t, *J* = 5.7 Hz, 1H), 8.69 (s, 1H), 8.16 (d, *J* = 1.4 Hz, 1H), 7.49-7.44 (m, 2H), 7.42 (s, 1H), 7.30 (d, *J* = 8.2 Hz, 2H), 5.95 (s, 2H), 4.60 (t, *J* = 5.0 Hz, 1H), 4.48 (t, *J* = 5.0 Hz, 1H), 4.39 (p, *J* = 6.6 Hz, 1H), 3.85 (s, 3H), 3.62 (q, *J* = 5.2 Hz, 1H), 3.55 (q, *J* = 5.2 Hz, 1H), 1.66 (tt, *J* = 8.2, 4.6 Hz, 1H), 1.37 (d, *J* = 6.6 Hz, 6H), 1.03 (dq, *J* = 6.1, 3.5 Hz, 2H), 0.81 (dt, *J* = 8.3, 3.4 Hz, 2H). | 623.1 |
| 22 | 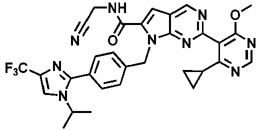 | N-(cyanomethyl) - 2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, Methanol-*d*₄): δ 9.37 (t, *J* = 5.7 Hz, 1H), 9.29 (s, 1H), 8.66 (s, 1H), 7.90 (d, *J* = 1.3 Hz, 1H), 7.51-7.40 (m, 4H), 7.37 (s, 1H), 6.07 (s, 2H), 4.51 (p, *J* = 6.7 Hz, 1H), 4.35 (t, *J* = 2.9 Hz, 2H), 3.95 (s, 3H), 1.74 (tt, *J* = 8.4, 4.6 Hz, 1H), 1.43 (d, *J* = 6.7 Hz, 6H), 1.17 (dt, *J* = 6.4, 3.3 Hz, 2H), 0.90 (dt, *J* = 8.1, 3.3 Hz, 2H). | 616.0 |
| 23 | 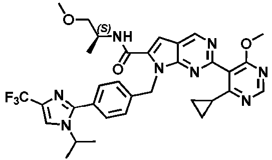 | (S)-2-(4-cyclopropyl-6-methoxypyrimid in-5-yl)-7-(4-(1-isopropyl-4-(trifluorometh yl)-1H-imidazol-2-yl)benzyl)-N-(1-methoxypropan-2-yl)-7H-pyrrolo[2,3-d]pyrimidine-6-carboxamide | ¹H NMR (400 MHz, Methanol-*d*₄): δ 9.24 (s, 1H), 8.65 (s, 1H), 8.58 (d, *J* = 8.2 Hz, 1H), 7.91 (d, *J* = 1.4 Hz, 1H), 7.47 (d, *J* = 8.3 Hz, 2H), 7.41 (d, *J* = 8.2 Hz, 2H), 7.30 (s, 1H), 6.09-5.95 (m, 2H), 4.50 (p, *J* = 6.7 Hz, 1H), 3.95 (s, 3H), 3.53-3.40 (m, 3H), 3.38 (s, 3H), 1.71 (tt, *J* = 8.4, 4.6 Hz, 1H), 1.44 (d, *J* = 6.7 Hz, 6H), 1.16 (dq, *J* = 6.3, 3.5 Hz, 2H), 0.88 (dq, *J* = 6.8, 3.7 Hz, 2H). | 649.0 |
| 24 | 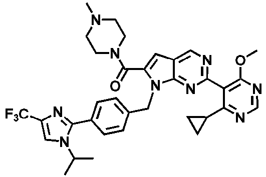 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (4-methylpiperazi n-1-yl)methanone | ¹H NMR (400 MHz, Methanol-*d*₄): δ 9.24 (s, 1H), 8.66 (s, 1H), 7.93 (dd, *J* = 4.8, 1.3 Hz, 1H), 7.59-7.51 (m, 2H), 7.37 (d, *J* = 7.9 Hz, 2H), 7.04 (s, 1H), 5.78 (s, 2H), 4.54 (p, *J* = 6.7 Hz, 2H), 3.96 (d, *J* = 1.3 Hz, 3H), 3.60 (s, 4H), 2.80 (s, 4H) 2.60 (s, 3H), 1.75 (tt, *J* = 8.2, 4.5 Hz, 1H), 1.46 (d, *J* = 6.7 Hz, 7H), 1.19 (dt, *J* = 6.5, 3.2 Hz, 2H), 0.93 (dt, *J* = 8.0, 3.4 Hz, 2H). | 660.0 |
| 25 | 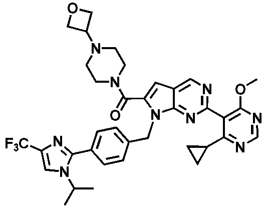 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (4-(oxetan-3-yl)piperazin-1-yl)methanone | ¹H NMR (400 MHz, Methanol-*d*₄): δ 9.22 (s, 1H), 8.65 (s, 1H), 7.94 (d, *J* = 1.4 Hz, 1H), 7.57-7.50 (m, 2H), 7.39-7.32 (m, 2H), 6.98 (s, 1H), 5.77 (s, 2H), 4.62 (t, *J* = 6.6 Hz, 2H), 4.59-4.53 (m, 1H), 4.51 (t, *J* = 6.1 Hz, 2H), 3.96 (s, 3H), 3.75 (s, 2H), 3.51 (q, *J* = 7.1 Hz, 1H), 3.43 (p, *J* = 6.2 Hz, 2H), 2.30 (s, 2H), 1.90 (s, 2H), 1.75 (tt, *J* = 8.5, 4.7 Hz, 1H), 1.47 (d, *J* = 6.6 Hz, 6H), 1.24-1.15 (m, 2H), 0.93 (dq, *J* = 7.0, 3.8 Hz, 2H). | 702.0 |
| 26 | 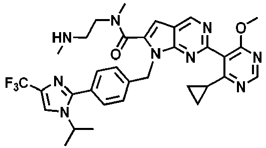 | 2-(4-cyclopropyl-6-methoxypyrimid in-5-yl)-7-(4-(1-isopropyl-4-(trifluorometh yl)-1H-imidazol-2-yl)benzyl)-N-methyl-N-(2-(methylamino)e thyl)-7H-pyrrolo[2,3-d]pyrimidine-6-carboxamide | ¹H NMR (400 MHz, Methanol-*d*₄): δ 9.24 (s, 1H), 8.65 (s, 1H), 7.94 (d, *J* = 1.4 Hz, 1H), 7.52 (d, *J* = 8.0 Hz, 2H), 7.37 (s, 2H), 7.10 (s, 1H), 5.78 (s, 2H), 4.51 (p, *J* = 6.7 Hz, 1H), 3.95 (s, 3H), 3.71 (s, 2H), 3.00 (s, 6H), 2.66 (s, 2H), 1.77-1.70 (m, 1H), 1.45 (d, *J* = 6.7 Hz, 6H), 1.18 (dd, *J* = 4.6, 2.7 Hz, 2H), 0.91 (dd, *J* = 8.1, 3.2 Hz, 2H). | 648.0 |
| 27 | 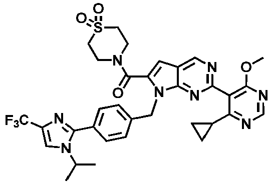 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (1,1-dioxidothiomor pholino)methan one | ¹H NMR (400 MHz, Methanol-*d*₄): δ 9.27 (s, 1H), 8.66 (s, 1H), 7.93 (d, *J* = 1.3 Hz, 1H), 7.59-7.52 (m, 2H), 7.40 (d, *J* = 8.2 Hz, 2H), 7.15 (s, 1H), 5.79 (s, 2H), 4.55 (p, *J* = 6.6 Hz, 1H), 3.96 (s, 7H), 3.22-2.97 (m, 2H), 2.79 (d, *J* = 35.1 Hz, 2H), 1.77 (tt, *J* = 8.5, 4.6 Hz, 1H), 1.47 (d, *J* = 6.7 Hz, 6H), 1.22-1.16 (m, 2H), 0.94 (dq, *J* = 6.9, 3.7 Hz, 2H). | 695.0 |
| 28 | 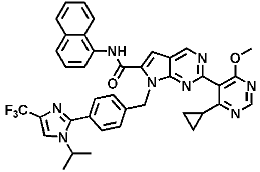 | 2-(4-cyclopropyl-6-methoxypyrimid in-5-yl)-7-(4-(1-isopropyl-4-(trifluoromethyl)-1H-imidazol-2-yl)benzyl)-N-(naphthalen-1-yl)-7H-pyrrolo[2,3-d]pyrimidine-6-carboxamide | ¹H NMR (400 MHz, Methanol-*d*₄): δ 9.35 (s, 1H), 8.67 (s, 1H), 7.95 (dd, *J* = 7.1, 2.3 Hz, 1H), 7.93-7.86 (m, 3H), 7.69 (s, 1H), 7.60-7.48 (m, 6H), 7.44 (d, *J* = 8.0 Hz, 2H), 6.10 (s, 2H), 4.49 (p, *J* = 6.8 Hz, 1H), 3.97 (s, 3H), 1.77 (tt, *J* = 8.3, 4.5 Hz, 1H), 1.40 (d, *J* = 6.7 Hz, 6H), 1.19 (dt, *J* = 6.4, 3.3 Hz, 2H), 0.92 (dq, *J* = 7.1, 3.8 Hz, 2H). | 703.0 |
| 29 | 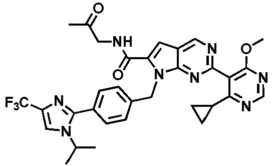 | 2-(4-cyclopropyl-6-methoxypyrimid in-5-yl)-7-(4-(1-isopropyl-4-(trifluorometh yl)-1H-imidazol-2-yl)benzyl)-N-(2-oxopropyl)-7H-pyrrolo[2,3-d]pyrimidine-6-carboxamide | ¹H NMR (400 MHz, Methanol-*d*₄): δ 9.26 (s, 1H), 8.97 (t, *J* = 5.9 Hz, 1H), 8.65 (s, 1H), 7.90 (d, *J* = 1.4 Hz, 1H), 7.49-7.41 (m, 4H), 7.39 (s, 1H), 6.04 (s, 2H), 4.52 (p, *J* = 6.8 Hz, 1H), 4.27-4.21 (m, 2H), 3.94 (s, 3H), 2.20 (s, 3H), 1.71 (tt, *J* = 8.4, 4.7 Hz, 1H), 1.44 (d, *J* = 6.7 Hz, 6H), 1.19-1.12 (m, 2H), 0.88 (dt, *J* = 8.1, 3.4 Hz, 2H). | 633.0 |
| 30 | 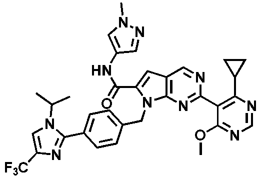 | 2-(4-cyclopropyl-6-methoxypyrimid in-5-yl)-7-(4-(1-isopropyl-4-(trifluorometh yl)-1H-imidazol-2-yl)benzyl)-N-(1-methyl-1H-pyrazol-4-yl)-7H-pyrrolo[2,3-d]pyrimidine-6-carboxamide | ¹H NMR (400 MHz, DMSO-*d*₆): δ 9.38 (s, 1H), 8.68 (s, 1H), 8.14 (d, *J* = 0.9 Hz, 1H), 8.07 (s, 1H), 7.55 (d, *J* = 12.2 Hz, 2H), 7.46 (d, *J* = 8.2 Hz, 2H), 7.31 (d, *J* = 8.2 Hz, 2H), 6.01 (s, 2H), 4.41-4.34 (m, 1H), 3.84 (d, *J* = 8.3 Hz, 6H), 1.69-1.63 (m, 1H), 1.35 (d, *J* = 6.6 Hz, 6H), 1.05-1.00 (m, 3.5 Hz, 2H), 0.82-0.77 (m, 2H). | 657.2 |
| 31 | 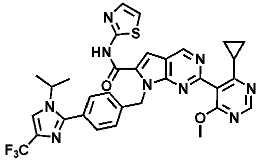 | 2-(4-cyclopropyl-6-methoxypyrimid in-5-yl)-7-(4-(1-isopropyl-4-(trifluorometh yl)-1H-imidazol-2-yl)benzyl)-N-(thiazol-2-yl)-7H-pyrrolo[2,3-d]pyrimidine-6-carboxamide | ¹H NMR (400 MHz, DMSO-*d*₆): δ 9.16 (s, 1H), 8.66 (s, 1H), 8.10 (s, 1H), 7.96 (s, 2H), 7.41 (s, 2H), 7.28 (d, *J* = 3.7 Hz, 1H), 7.22 (s, 1H), 6.72 (d, *J* = 3.7 Hz, 1H), 6.37 (s, 2H), 4.41-4.36 (m, 1H), 3.84 (s, 3H), 1.68-1.63 (m, 1H), 1.34 (d, *J* = 6.6 Hz, 6H), 1.02-0.98 (m, 2H), 0.79 - 0.74 (m, 2H). | 660.2 |
| 32 | 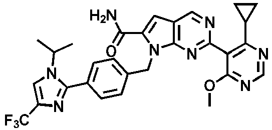 | 2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.27 (s, 1H), 8.61 (s, 1H), 8.24 (s, 1H), 8.08 (d, *J* = 0.9 Hz, 1H), 7.62 (s, 1H), 7.41- 7.37 (m, 3H), 7.22 (d, *J* = 8.2 Hz, 2H), 5.91 (s, 2H), 4.36-4.29 (m, 1H), 3.77 (s, 3H), 1.59-1.53 (m, 1H), 1.30 (d, *J* = 6.6 Hz, 6H), 0.97-0.93 (m, *J* = 7.2, 3.4 Hz, 2H), 0.74-0.69 (m, 2H). | 577.2 |
| 33 | 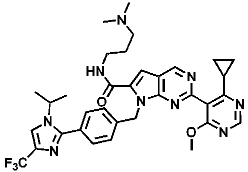 | 2-(4-cyclopropyl-6-methoxypyrimid in-5-yl)-N-(3-(dimethylamino propyl) -7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): 9.26 (s, 1H), 8.81 (t, *J* = 5.6 Hz, 1H), 8.61 (s, 1H), 8.08 (s, 1H), 7.39 (d, *J* = 8.2 Hz, 2H), 7.26 (s, 1H), 7.22 (d, *J* = 8.2 Hz, 2H), 5.87 (s, 2H), 4.35-4.29 (m, 1H), 3.77 (s, 3H), 2.18-2.14 (m, 2H), 2.04 (s, 6H), 1.64-1.48 (m, 4H), 1.30 (d, *J* = 6.6 Hz, 6H), 1.17 (s, 1H), 0.97-0.94 (m, 2H), 0.75-0.70 (m, 2H). | 662.3 |
| 34 | 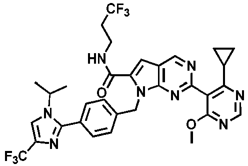 | 2-(4-cyclopropyl-6-methoxypyrimid in-5-yl)-7-(4-(1-isopropyl-4-(trifluorometh yl)-1H-imidazol-2-yl)benzyl)-N-(3,3,3-trifluoropropy l)-7H-pyrrolo[2,3-d]pyrimidine-6-carboxamide | ¹H NMR (400 MHz, DMSO-*d*₆): δ 9.29 (s, 1H), 9.00 (t, *J* = 5.7 Hz, 1H), 8.61 (s, 1H), 8.08 (s, 1H), 7.39 (d, *J* = 8.2 Hz, 2H), 7.28 (s, 1H), 7.22 (d, *J* = 8.2 Hz, 2H), 5.87 (s, 2H), 4.35-4.28 (m, 1H), 3.77 (s, 3H), 3.47-3.42 (m, 2H), 2.56-2.45 (m, 2H), 1.61-1.55 (m, 1H), 1.29 (d, *J* = 6.6 Hz, 6H), 0.98-0.94 (m, *J* = 6.9, 4.0 Hz, 2H), 0.75-0.70 (m, *J* = 6.4, 3.2 Hz, 2H). | 673.2 |
| 35 | 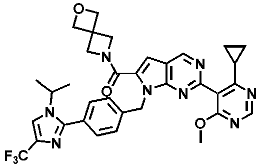 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (2-oxa-6-azaspiro[3.3]h eptan-6-yl)methanone | ¹H NMR (400 MHz, DMSO-*d₆*): δ 9.23 (d, *J* = 10.3 Hz, 1H), 8.61 (d, *J* = 1.7 Hz, 1H), 8.09 (s, 1H), 7.43-7.39 (m, 2H), 7.24-7.18 (m, 2H), 7.11 (s, 1H), 5.76 (d, *J* = 18.2 Hz, 2H), 4.59 (d, *J* = 12.6 Hz, 2H), 4.41 (s, 1H), 4.36-4.30 (m, 1H), 4.07 (d, *J* = 70.8 Hz, 2H), 3.80-3.69 (m, 4H), 3.40 (s, 2H), 1.59-1.54 (m, 1H), 1.30 (d, *J* = 6.6 Hz, 6H), 0.97-0.93 (m, 2H), 0.75-0.70 (m, 2H). | 659.2 |
| 36 | 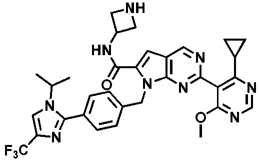 | N-(azetidin-3-yl)-2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d₆*): δ 9.29 (s, 1H), 9.23 (d, *J* = 7.1 Hz, 1H), 8.62-8.60 (m, 1H), 8.08 (s, 1H), 7.40 (s, 2H), 7.38 (s, 1H), 7.20 (d, *J* = 8.1 Hz, 2H), 5.84 (s, 2H), 4.66-4.57 (m, 1H), 4.34-4.29 (m, 1H), 3.77 (s, 3H), 3.58-3.55 (m, 2H), 3.51-3.49 (m, 3H), 1.59-1.55 (m, 1H), 1.30 (d, *J* = 6.6 Hz, 6H), 0.95 (s, 2H), 0.74-0.70 (m, 2H). | 632.3 |
| 37 | 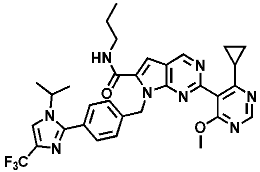 | 2-(4-cyclopropyl-6-methoxypyrimid in-5-yl)-7-(4-(1-isopropyl-4-(trifluorometh yl)-1H-imidazol-2-yl)benzyl)-N-propyl-7H-pyrrolo[2,3-d]pyrimidine-6-carboxamide | ¹H NMR (400 MHz, DMSO-*d₆*): δ 9.26 (s, 1H), 8.77 (t, *J* = 5.7 Hz, 1H), 8.61 (s, 1H), 8.08 (s, 1H), 7.39 (d, *J* = 8.2 Hz, 2H), 7.27 (s, 1H), 7.22 (d, *J* = 8.1 Hz, 2H), 5.87 (s, 2H), 4.34-4.29 (m, 1H), 3.77 (s, 3H), 3.18-3.13 (m, 4H), 1.60-1.56 (m, 1H), 1.48-1.43 (m, 2H), 1.31-1.28 (m, 6H), 0.79 (t, *J* = 7.4 Hz, 3H), 0.75-0.71 (m, 2H). | 619.2 |
| 38 | 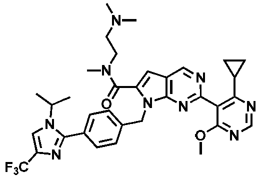 | 2-(4-cyclopropyl-6-methoxypyrimid in-5-yl)-N-(2-(dimethylamino )ethyl)-7-(4-(1-isopropyl-4-(trifluorometh yl)-1H-imidazol-2-yl)benzyl)-N-methyl-7H-pyrrolo [2,3-d]pyrimidine-6-carboxamide | ¹H NMR (400 MHz, DMSO-*d₆*): δ 9.18 (s, 1H), 8.61 (s, 1H), 8.09 (s, 1H), 7.42 (d, *J* = 7.8 Hz, 2H), 7.23 (d, *J* = 8.1 Hz, 2H), 6.93 (s, 1H), 5.55 (s, 2H), 4.36-4.29 (m, 1H), 3.78 (s, 3H), 2.86 (s, 3H), 2.11 (s, 3H), 1.90-1.85 (m, 2H), 1.75 (s, 2H), 1.63-1.58 (m, 1H), 1.30 (d, *J* = 6.6 Hz, 6H), 0.98-0.94 (m, 2H), 0.77-0.72 (m, 2H). | 662.3 |
| 39 | 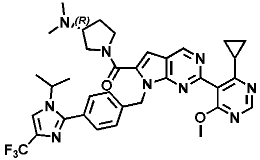 | (R)-(2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (3-(dimethylamino )pyrrolidin-1-yl)methanone | ¹H NMR (400 MHz, DMSO-*d₆*): δ 9.19 (s, 1H), 8.62 (s, 1H), 8.09 (s, 1H), 7.42 (d, *J* = 7.8 Hz, 2H), 7.17 (d, *J* = 8.2 Hz, 2H), 7.07 (d, *J* = 13.9 Hz, 1H), 5.72-5.49 (m, 2H), 4.36-4.29 (m, 1H), 3.78 (s, 3H), 3.66-3.52 (m, 2H), 3.07-3.04 (m, 1H), 2.61-2.54 (m, 7.5 Hz, 1H), 2.37-2.30 (m, 1H), 2.06 (s, 3H), 1.95 (s, 3H), 1.66-1.55 (m, 2H), 1.49-1.37 (m, 1H), 1.31 (d, *J* = 8.4, 4.8 Hz, 6H), 0.99-0.96 (m, 2H), 0.79-0.75 (m, 2H). | 674.3 |
| 40 | 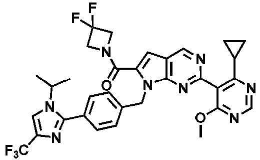 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (3,3-difluoroazetid in-1-yl)methanone | ¹H NMR (400 MHz, DMSO-*d₆*): δ 9.26 (s, 1H), 8.62 (d, *J* = 2.9 Hz, 1H), 8.08 (d, *J* = 1.0 Hz, 1H), 7.41 (d, *J* = 8.2 Hz, 2H), 7.27 (s, 1H), 7.22 (d, *J* = 8.2 Hz, 2H), 5.75 (s, 2H), 4.61 (d, *J* = 122.4 Hz, 4H), 4.36-4.30 (m, 1H), 3.77 (s, 3H), 1.59-1.54 (m, 1H), 1.30 (d, *J* = 6.6 Hz, 6H), 0.97-0.93 (m, 2H), 0.75-0.70 (m, 2H). | 653.2 |
| 41 | 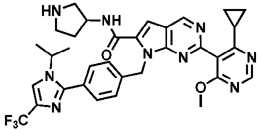 | 2-(4-cyclopropyl-6-methoxypyrimid in-5-yl)-7-(4-(1-isopropyl-4-(trifluorometh yl)-1H-imidazol-2-yl)benzyl)-N-(pyrrolidin-3-yl)-7H-pyrrolo[2,3-d]pyrimidine-6-carboxamide | ¹H NMR (400 MHz, CDCl₃): δ 9.17 (d, *J* = 1.1 Hz, 1H), 8.67 (s, 1H), 7.43 (d, *J* = 2.1 Hz, 1H), 7.41 (d, *J* = 2.2 Hz, 1H), 7.39 (s, 1H), 7.34 (d, *J* = 7.8 Hz, 2H), 6.78 (d, *J* = 17.4 Hz, 1H), 5.84 (s, 2H), 4.48 (dt, *J* = 13.5, 7.0 Hz, 1H), 3.95 (s, 3H), 3.74 (dt, *J* = 12.8, 6.2 Hz, 1H), 3.64 (d, *J* = 17.2 Hz, 1H), 3.60-3.52 (m, 1H), 3.51-3.43 (m, 1H), 3.42-3.31 (m, 1H), 2.95 (dd, *J* = 10.4, 5.1 Hz, 1H), 2.11-1.87 (m, 1H), 1.74 (tt, *J* = 8.3, 4.6 Hz, 1H), 1.69-1.61 (m, 1H), 1.42 (dd, *J* = 7.2, 2.9 Hz, 6H), 1.23 (dd, *J* = 4.5, 2.7 Hz, 2H), 0.88-0.84 (m, 2H). | 646.2 |
| 42 | 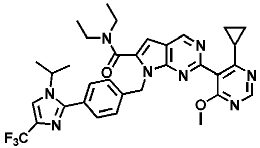 | 2-(4-cyclopropyl-6-methoxypyrimid in-5-yl)-N,N-diethyl-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, CDCl₃): δ 9.18 (s, 1H), 8.67 (s, 1H), 7.46-7.39 (m, 3H), 7.35 (d, *J* = 8.2 Hz, 2H), 6.70 (s, 1H), 5.76 (s, 2H), 4.46 (p, *J* = 6.7 Hz, 1H), 3.95 (s, 3H), 3.49 (s, 2H), 3.15 (s, 2H), 1.75 (tt, *J* = 8.4, 4.6 Hz, 1H), 1.41 (d, *J* = 6.7 Hz, 6H), 1.24 (dd, *J* = 5.6, 2.7 Hz, 2H), 1.10 (d, *J* = 41.6 Hz, 6H), 0.90-0.85 (m, 2H). | 633.3 |
| 43 | 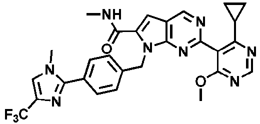 | 2-(4-cyclopropyl-6-methoxypyrimid in-5-yl)-N-methyl-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, CDCl₃) : δ 9.19 (s, 1H), 8.67 (s, 1H), 7.49 (d, *J* = 8.2 Hz, 2H), 7.42 (d, *J* = 8.4 Hz, 2H), 7.29-7.27 (m, 1H), 6.90 (s, 1H), 6.15 (d, *J* = 5.4 Hz, 1H), 6.01 (s, 2H), 3.94 (s, 3H), 3.72 (s, 3H), 2.99 (d, *J* = 4.9 Hz, 3H), 1.73 (tt, *J* = 8.3, 4.6 Hz, 1H), 1.22 (dt, *J* = 6.5, 3.3 Hz, 2H), 0.86 (dq, *J* = 6.8, 3.8 Hz, 2H). | 563.2 |
| 44 | 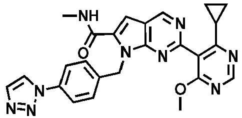 | 7-(4-(1H-1,2,3-triazol-1-yl)benzyl)-2-(4-cyclopropyl-6-methoxypyrimid in-5-yl)-N-methyl-7H-pyrrolo[2,3-d]pyrimidine-6-carboxamide | ¹H NMR (400 MHz, CDCl₃): δ 9.20 (s, 1H), 8.67 (s, 1H), 7.93 (d, *J* = 1.1 Hz, 1H), 7.82 (d, *J* = 1.1 Hz, 1H), 7.64-7.60 (m, 2H), 7.56-7.52 (m, 2H), 6.93 (s, 1H), 6.20 (s, 1H), 6.01 (s, 2H), 3.94 (s, 3H), 3.02 (d, *J* = 4.9 Hz, 3H), 1.71 (td, *J* = 8.2, 4.2 Hz, 1H), 1.27-1.20 (m, 2H), 0.88-0.83 (m, 2H). | 482.2 |
| 45 | 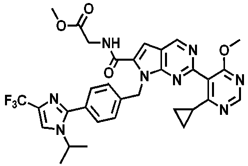 | methyl (2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carbonyl)glyci nate | ¹H NMR (400 MHz, DMSO-*d*₆): δ 9.37 (d, *J* = 4.3 Hz, 2H), 8.69 (s, 1H), 8.16 (d, *J* = 1.4 Hz, 1H), 7.46 (d, *J* = 7.8 Hz, 3H), 7.31 (d, *J* = 8.0 Hz, 2H), 5.94 (s, 2H), 4.41 (p, *J* = 6.6 Hz, 1H), 4.07 (d, *J* = 5.8 Hz, 2H), 3.85 (s, 3H), 3.67 (s, 3H), 1.66 (tt, *J* = 8.3, 4.6 Hz, 1H), 1.37 (d, *J* = 6.6 Hz, 6H), 1.04 (dp, *J* = 6.0, 3.4 Hz, 2H), 0.81 (dt, *J* = 8.1, 3.4 Hz, 2H). | 649.1 |
| 46 | 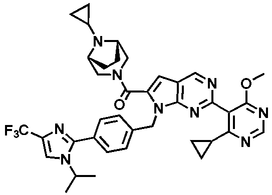 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl)((1R,5S)-8-cyclopropyl-3,8-diazabicyclo[3 .2.1]octan-3-yl)methanone | ¹H NMR (400 MHz, DMSO-*d*₆): δ 9.28 (s, 1H), 8.69 (s, 1H), 8.18 (d, *J* = 1.4 Hz, 1H), 7.51 (d, *J* = 8.0 Hz, 2H), 7.27 (d, *J* = 8.0 Hz, 2H), 6.99 (s, 1H), 5.71 (q, *J* = 15.2 Hz, 2H), 4.39 (p, *J* = 6.6 Hz, 1H), 4.21 (d, *J* = 12.6 Hz, 1H), 3.86 (s, 3H), 3.50 (d, *J* = 13.0 Hz, 2H), 3.28 (d, *J* = 12.9 Hz, 2H), 3.07 (s, 1H), 2.92-2.87 (m, 1H), 1.80 (td, *J* = 6.7, 3.4 Hz, 2H), 1.70 (tt, *J* = 8.3, 4.6 Hz, 1H), 1.39 (dd, *J* = 6.9, 3.7 Hz, 6H), 1.05 (p, *J* = 4.4, 4.0 Hz, 2H), 0.86 (dd, *J* = 7.3, 4.6 Hz, 2H), 0.82 (d, *J* = 10.1 Hz, 2H), 0.39 (d, *J* = 6.6 Hz, 2H), 0.32 (d, *J* = 7.8 Hz, 2H). | 712.1 |
| 47 | 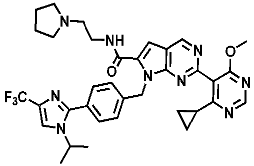 | 2-(4-cyclopropyl-6-methoxypyrimid in-5-yl)-7-(4-(1-isopropyl-4-(trifluorometh yl)-1H-imidazol-2-yl)benzyl)-N-(2-(pyrrolidin-1-yl)ethyl)-7H-pyrrolo[2,3-d]pyrimidine-6-carboxamide | ¹H NMR (400 MHz, DMSO-*d*₆): δ 9.34 (s, 1H), 8.83 (t, *J* = 5.8 Hz, 1H), 8.69 (s, 1H), 8.16 (d, *J* = 1.4 Hz, 1H), 7.46 (d, *J* = 8.2 Hz, 2H), 7.35 (s, 1H), 7.31 (d, *J* = 8.1 Hz, 2H), 5.95 (s, 2H), 4.40 (p, *J* = 6.6 Hz, 1H), 3.85 (s, 3H), 3.39 (q, *J* = 6.5 Hz, 2H), 2.56 (t, *J* = 6.8 Hz, 2H), 2.46 (q, *J* = 4.3, 3.2 Hz, 4H), 1.70-1.62 (m, *J* = 4.5 Hz, 5H), 1.37 (d, *J* = 6.6 Hz, 6H), 1.03 (dq, *J* = 6.0, 3.5 Hz, 2H), 0.81 (dt, *J* = 8.2, 3.4 Hz, 2H). | 674.1 |
| 48 | 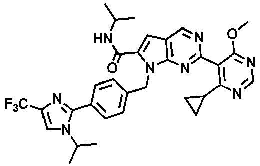 | 2-(4-cyclopropyl-6-methoxypyrimid in-5-yl)-N-isopropyl-7-(4- (1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.33 (s, 1H), 8.69 (s, 1H), 8.63 (d, *J* = 7.9 Hz, 1H), 8.15 (d, *J* = 1.4 Hz, 1H), 7.46 (d, *J* = 8.2 Hz, 2H), 7.34 (s, 1H), 7.29 (d, *J* = 8.0 Hz, 2H), 5.92 (s, 2H), 4.38 (p, *J* = 6.7 Hz, 1H), 4.09 (dq, *J* = 13.4, 6.7 Hz, 1H), 3.85 (s, 3H), 1.65 (tt, *J* = 8.3, 4.6 Hz, 1H), 1.37 (d, *J* = 6.8 Hz, 6H), 1.16 (d, *J* = 6.6 Hz, 6H), 1.03 (p, *J* = 3.6 Hz, 2H), 0.81 (dq, *J* = 6.9, 3.4 Hz, 2H). | 619.1 |
| 49 | 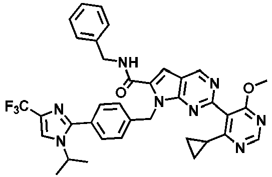 | N-benzyl-2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.42 (t, *J* = 6.0 Hz, 1H), 9.36 (s, 1H), 8.69 (s, 1H), 8.17 (d, *J* = 1.4 Hz, 1H), 7.48-7.42 (m, 3H), 7.35-7.23 (m, 7H), 5.96 (s, 2H), 4.50 (d, *J* = 6.0 Hz, 2H), 4.39 (p, *J* = 6.6 Hz, 1H), 3.85 (s, 3H), 1.66 (tt, *J* = 8.2, 4.6 Hz, 1H), 1.38 (d, *J* = 6.6 Hz, 6H), 1.04 (p, *J* = 3.6 Hz, 2H), 0.81 (dt, *J* = 8.2, 3.4 Hz, 2H). | 667.1 |
| 50 | 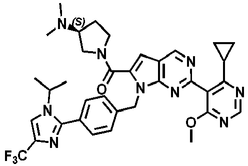 | (S)-(2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (3-(dimethylamino )pyrrolidin-1-yl)methanone | ¹H NMR (400 MHz, CDCl₃): δ 9.18 (d, *J* = 11.5 Hz, 1H), 8.67 (s, 1H), 7.45-7.39 (m, 3H), 7.35 (t, *J* = 8.4 Hz, 2H), 6.77 (d, *J* = 3.5 Hz, 1H), 6.01-5.67 (m, 2H), 4.49 (p, *J* = 6.6 Hz, 1H), 3.95 (s, 3H), 3.93-3.72 (m, 1H), 3.50-3.18 (m, 3H), 2.27 (s, 3H), 2.15 (s, 3H), 1.75 (d, *J* = 9.6 Hz, 1H), 1.46-1.41 (m, 6H), 1.31 (d, *J* = 5.9 Hz, 1H), 1.27 (s, 1H), 1.24 (dd, *J* = 4.6, 2.7 Hz, 2H), 0.89 (d, *J* = 3.0 Hz, 1H), 0.88-0.85 (m, 2H). | 674.3 |
| 51 | 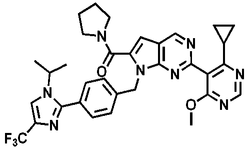 | (2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl)(pyrrolidin -1-yl)methanone | ¹H NMR (400 MHz, CDCl₃): δ 9.17 (s, 1H), 8.67 (s, 1H), 7.44-7.39 (m, 3H), 7.34 (d, *J* = 8.2 Hz, 2H), 6.78 (s, 1H), 5.85 (s, 2H), 4.47 (p, *J* = 6.7 Hz, 1H), 3.95 (s, 3H), 3.58 (t, *J* = 7.0 Hz, 2H), 3.33 (t, *J* = 6.7 Hz, 2H), 1.91 (p, *J* = 6.6 Hz, 2H), 1.75 (td, *J* = 7.9, 7.4, 4.7 Hz, 3H), 1.42 (d, *J* = 6.7 Hz, 6H), 1.23 (dt, *J* = 6.5, 3.3 Hz, 2H), 0.90-0.83 (m, 2H). | 631.2 |
| 52 | 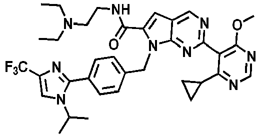 | 2-(4-cyclopropyl-6-methoxypyrimid in-5-yl)-N-(2-(diethylamino) ethyl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, Methanol-*d*₄): δ 9.28 (s, 1H), 8.64 (s, 1H), 7.92 (d, *J* = 1.3 Hz, 1H), 7.47 (d, *J* = 7.9 Hz, 3H), 7.43-7.34 (m, 2H), 6.07 (s, 2H), 4.50 (p, *J* = 6.7 Hz, 1H), 3.94 (s, 3H), 3.71 (t, *J* = 6.5 Hz, 2H), 3.21 (s, 6H), 1.70 (tt, *J* = 8.7, 4.7 Hz, 1H), 1.44 (d, *J* = 6.7 Hz, 6H), 1.30 (t, *J* = 7.2 Hz, 6H), 1.15 (dt, *J* = 6.4, 3.3 Hz, 2H), 0.86 (dt, *J* = 8.1, 3.4 Hz, 2H). | 676.0 |
| 53 | 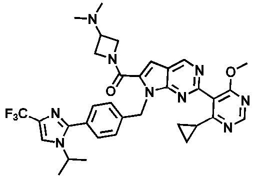 | (2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (3-(dimethylamino )azetidin-1-yl)methanone | ¹H NMR (400 MHz, Methanol-*d*₄): δ 9.26 (s, 1H), 8.64 (s, 1H), 7.92 (d, *J* = 1.4 Hz, 1H), 7.53-7.46 (m, 2H), 7.39 (d, *J* = 8.2 Hz, 2H), 7.24 (s, 1H), 5.97 (d, *J* = 30.9 Hz, 2H), 4.53 (p, *J* = 6.7 Hz, 1H), 4.37 (d, *J* = 41.1 Hz, 2H), 3.94 (s, 3H), 3.51 (q, *J* = 7.0 Hz, 3H), 2.49 (s, 6H), 1.70 (tt, *J* = 8.4, 4.6 Hz, 1H), 1.45 (d, *J* = 6.7 Hz, 6H), 1.16 (dt, *J* = 6.3, 4.2 Hz, 2H), 0.87 (dq, *J* = 6.9, 3.7 Hz, 2H). | 660.0 |
| 54 | 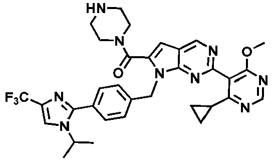 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (piperazin-1-yl)methanone | ¹H NMR (400 MHz, DMSO-*d*₆): δ 9.29 (s, 1H), 8.69 (s, 1H), 8.17 (d, *J* = 1.3 Hz, 1H), 7.54-7.48 (m, 2H), 7.29 (d, *J* = 8.0 Hz, 2H), 7.06 (s, 1H), 5.64 (s, 2H), 4.40 (p, *J* = 6.7 Hz, 1H), 3.85 (s, 3H), 3.61 (d, *J* = 51.9 Hz, 4H), 2.95 (s, 4H), 1.67 (td, *J* = 8.1, 4.2 Hz, 1H), 1.38 (d, *J* = 6.6 Hz, 6H), 1.04 (t, *J* = 3.7 Hz, 2H), 0.83 (dq, *J* = 6.9, 3.4 Hz, 2H). | 646.0 |
| 55 | 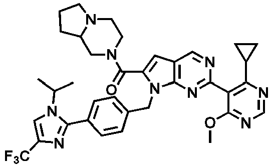 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (hexahydrop yrrolo[1,2-a]pyrazin-2 (1H) - yl)methanone | ¹H NMR (400 MHz, CDCl₃): δ 9.18 (s, 1H), 8.67 (s, 1H), 7.44 (d, *J* = 8.2 Hz, 2H), 7.39 (d, *J* = 1.3 Hz, 1H), 7.34 (d, *J* = 7.9 Hz, 2H), 6.67 (s, 1H), 5.77 (dd, *J* = 32.7, 13.6 Hz, 2H), 4.73 (d, *J* = 44.8 Hz, 1H), 4.49 (hept, *J* = 6.7 Hz, 1H), 3.95 (s, 3H), 3.84 (dd, *J* = 39.6, 17.9 Hz, 1H), 3.11-2.46 (m, 4H), 2.08 (q, *J* = 8.9 Hz, 1H), 1.75 (tt, *J* = 8.3, 4.6 Hz, 4H), 1.43 (dd, *J* = 6.7, 3.4 Hz, 6H), 1.28-1.21 (m, 4H), 0.90-0.84 (m, 3H). | 686.3 |
| 56 | 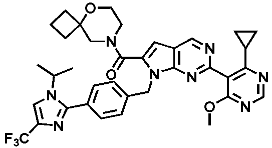 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (5-oxa-8-azaspiro[3.5]n onan-8-yl)methanone | ¹H NMR (400 MHz, CDCl₃) : δ 9.20 (s, 1H), 8.67 (s, 1H), 7.49-7.43 (m, 2H), 7.40 (d, *J* = 1.3 Hz, 1H), 7.33 (d, *J* = 7.9 Hz, 2H), 6.70 (s, 1H), 5.80 (s, 2H), 4.47 (p, *J* = 6.7 Hz, 1H), 3.95 (s, 3H), 3.78 - 3.19 (m, 6H), 2.03-1.65 (m, 7H), 1.43 (d, *J* = 6.6 Hz, 6H), 1.26-1.21 (m, 2H), 0.89-0.84 (m, 2H). | 687.2 |
| 57 | 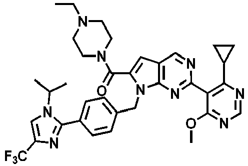 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (4-ethylpiperazin -1-yl)methanone | ¹H NMR (400 MHz, CDCl₃): δ 9.17 (s, 1H), 8.67 (s, 1H), 7.48-7.43 (m, 2H), 7.40 (d, *J* = 1.3 Hz, 1H), 7.35 (d, *J* = 8.2 Hz, 2H), 6.66 (s, 1H), 5.77 (s, 2H), 4.50 (p, *J* = 6.7 Hz, 1H), 3.95 (s, 3H), 3.76 (s, 2H), 3.41 (s, 2H), 2.36 (p, *J* = 9.0, 7.2 Hz, 4H), 2.02 (d, *J* = 12.4 Hz, 2H), 1.75 (tt, *J* = 8.3, 4.6 Hz, 1H), 1.43 (d, *J* = 6.7 Hz, 6H), 1.24 (dd, *J* = 4.4, 2.7 Hz, 2H), 1.02 (t, *J* = 7.1 Hz, 3H), 0.88 (dd, *J* = 8.1, 3.0 Hz, 2H). | 674.3 |
| 58 | 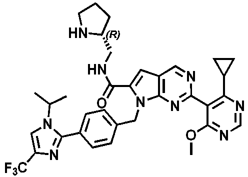 | (R) -2- (4-cyclopropyl-6-methoxypyrimid in-5-yl)-7-(4-(1-isopropyl-4-(trifluorometh yl)-1H-imidazol-2-yl)benzyl)-N-(pyrrolidin-2-ylmethyl)-7H-pyrrolo[2,3-d]pyrimidine-6-carboxamide | ¹H NMR (400 MHz, DMSO-*d₆*): δ 9.34 (s, 1H), 8.93 (s, 1H), 8.69 (s, 1H), 8.15 (s, 1H), 7.46 (d, *J* = 7.9 Hz, 2H), 7.39 (s, 1H), 7.30 (d, *J* = 7.8 Hz, 2H), 5.94 (s, 2H), 4.43-4.37 (m, 2H), 3.85 (s, 3H), 2.85-2.74 (m, 4H), 1.76-1.57 (m, 6H), 1.37 (d, *J* = 6.5 Hz, 6H), 1.05-1.00 (m, 2H), 0.83-0.78 (m, 2H). | 660.3 |
| 59 | 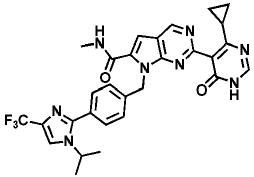 | 2-(4-cyclopropyl-6-oxo-1,6-dihydropyrimid in-5-yl)-7-(4-(1-isopropyl-4-(trifluorometh yl)-1H-imidazol-2-yl)benzyl)-N-methyl-7H-pyrrolo[2,3-d]pyrimidine-6-carboxamide | ¹H NMR (400 MHz, DMSO-*d*₆): δ 12.54 (s, 1H), 9.30 (s, 1H), 8.79 (q, *J* = 4.5 Hz, 1H), 8.17-8.09 (m, 2H), 7.44 (d, *J* = 8.2 Hz, 2H), 7.32 (s, 1H), 7.26 (d, *J* = 8.1 Hz, 2H), 5.95 (s, 2H), 4.40 (p, *J* = 6.6 Hz, 1H), 2.79 (d, *J* = 4.6 Hz, 3H), 1.48 (t, *J* = 4.2 Hz, 1H), 1.36 (d, *J* = 6.6 Hz, 6H), 0.98-0.92 (m, 2H), 0.69 (dq, *J* = 10.7, 3.5 Hz, 2H). | 577.2 |
| 60 | 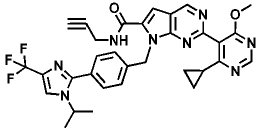 | 2-(4-cyclopropyl-6-methoxypyrimid in-5-yl)-7-(4-(1-isopropyl-4-(trifluorometh yl)-1H-imidazol-2-yl)benzyl)-N-(prop-2-yn-1-yl)-7H-pyrrolo[2,3-d]pyrimidine-6-carboxamide | ¹H NMR (400 MHz, DMSO-*d*₆): δ 9.34 (d, *J* = 7.5 Hz, 2H), 8.68 (s, 1H), 8.15 (d, *J* = 1.5 Hz, 1H), 7.49-7.43 (m, 2H), 7.41 (s, 1H), 7.31 (d, *J* = 8.0 Hz, 2H), 5.94 (s, 2H), 4.39 (p, *J* = 6.6 Hz, 1H), 4.08 (dd, *J* = 5.6, 2.5 Hz, 2H), 3.84 (s, 3H), 3.20 (t, *J* = 2.5 Hz, 1H), 1.65 (tt, *J* = 8.3, 4.6 Hz, 1H), 1.37 (s, 3H), 1.36 (s, 3H), 1.02 (p, *J* = 3.7 Hz, 2H), 0.80 (dd, *J* = 8.0, 3.3 Hz, 2H). | 615.2 |
| 61 | 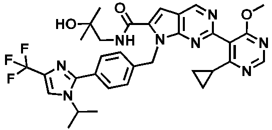 | 2-(4-cyclopropyl-6-methoxypyrimid in-5-yl)-N-(2-hydroxy-2-methylpropyl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.34 (s, 1H), 8.69 (s, 1H), 8.64 (t, *J* = 6.3 Hz, 1H), 8.16 (d, *J* = 1.4 Hz, 1H), 7.48-7.41 (m, 3H), 7.29 (d, *J* = 8.1 Hz, 2H), 5.94 (s, 2H), 4.38 (p, *J* = 6.6 Hz, 1H), 3.85 (s, 3H), 3.65 (s, 1H), 3.26 (d, *J* = 6.3 Hz, 2H), 1.65 (tt, *J* = 8.1, 4.6 Hz, 1H), 1.38 (s, 3H), 1.36 (s, 3H), 1.07 (s, 6H), 1.05-1.01 (m, 2H), 0.84-0.78 (m, 2H). | 649.3 |
| 62 | 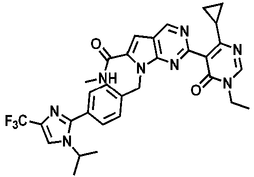 | 2-(4-cyclopropyl-1-ethyl-6-oxo-1,6-dihydropyrimid in-5-yl)-7-(4-(1-isopropyl-4-(trifluorometh yl)-1H-imidazol-2-yl)benzyl)-N-methyl-7H-pyrrolo[2,3-d]pyrimidine-6-carboxamide | ¹H NMR (400 MHz, DMSO-*d*₆) δ 9.30 (s, 1H), 8.80 (d, *J* = 4.7 Hz, 1H), 8.44 (s, 1H), 8.14 (d, *J* = 1.4 Hz, 1H), 7.47-7.40 (m, 2H), 7.33 (s, 1H), 7.24 (d, *J* = 8.0 Hz, 2H), 5.95 (s, 2H), 4.40 (p, *J* = 6.6 Hz, 1H), 3.91 (q, *J* = 7.1 Hz, 2H), 2.79 (d, *J* = 4.6 Hz, 3H), 1.43 (dt, *J* = 8.3, 3.6 Hz, 1H), 1.36 (d, *J* = 6.6 Hz, 6H), 1.25 (t, *J* = 7.0 Hz, 3H), 0.97-0.92 (m, 2H), 0.69 (dd, *J* = 8.0, 3.2 Hz, 2H). | 605.2 |
| 63 | 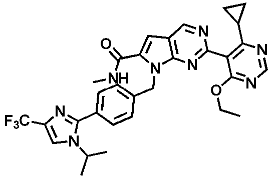 | 2-(4-cyclopropyl-6-ethoxypyrimidi n-5-yl)-7-(4-(1-isopropyl-4-(trifluorometh yl)-1H-imidazol-2-yl)benzyl)-N-methyl-7H-pyrrolo[2,3-d]pyrimidine-6-carboxamide | ¹H NMR (400 MHz, DMSO-*d*₆): δ 9.34 (d, *J* = 7.5 Hz, 2H), 8.68 (s, 1H), 8.15 (d, *J* = 1.5 Hz, 1H), 7.49-7.43 (m, 2H), 7.41 (s, 1H), 7.31 (d, *J* = 8.0 Hz, 2H), 5.94 (s, 2H), 4.39 (p, *J* = 6.6 Hz, 1H), 4.08 (dd, *J* = 5.6, 2.5 Hz, 2H), 3.84 (s, 3H), 3.20 (t, *J* = 2.5 Hz, 1H), 1.65 (tt, *J* = 8.3, 4.6 Hz, 1H), 1.37 (s, 3H), 1.36 (s, 3H), 1.02 (p, *J* = 3.7 Hz, 2H), 0.80 (dd, *J* = 8.0, 3.3 Hz, 2H). | 615.2 |
| 64 | 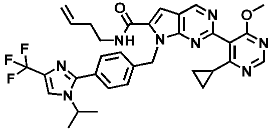 | N- (but-3-en-1-yl)-2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.34 (s, 1H), 8.69 (s, 1H), 8.64 (t, *J* = 6.3 Hz, 1H), 8.16 (d, *J* = 1.4 Hz, 1H), 7.48-7.41 (m, 3H), 7.29 (d, *J* = 8.1 Hz, 2H), 5.94 (s, 2H), 4.38 (p, *J* = 6.6 Hz, 1H), 3.85 (s, 3H), 3.65 (s, 1H), 3.26 (d, *J* = 6.3 Hz, 2H), 1.65 (tt, *J* = 8.1, 4.6 Hz, 1H), 1.38 (s, 3H), 1.36 (s, 3H), 1.07 (s, 6H), 1.05-1.01 (m, 2H), 0.84-0.78 (m, 2H). | 631.3 |
| 65 | 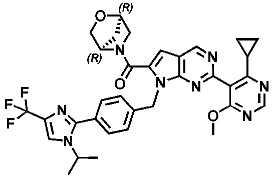 | ((1R,4R)-2-oxa-5-azabicyclo[2.2 .1]heptan-5-yl)(2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl)methanone | ¹H NMR (400 MHz, DMSO-*d*₆): δ 9.30 (s, 1H), 8.80 (d, *J* = 4.7 Hz, 1H), 8.44 (s, 1H), 8.14 (d, *J* = 1.4 Hz, 1H), 7.47-7.40 (m, 2H), 7.33 (s, 1H), 7.24 (d, *J* = 8.0 Hz, 2H), 5.95 (s, 2H), 4.40 (p, *J* = 6.6 Hz, 1H), 3.91 (q, *J* = 7.1 Hz, 2H), 2.79 (d, *J* = 4.6 Hz, 3H), 1.43 (dt, *J* = 8.3, 3.6 Hz, 1H), 1.36 (d, *J* = 6.6 Hz, 6H), 1.25 (t, *J* = 7.0 Hz, 3H), 0.97-0.92 (m, 2H), 0.69 (dd, *J* = 8.0, 3.2 Hz, 2H). | 659.3 |
| 66 | 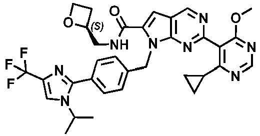 | (S)-2-(4-cyclopropyl-6-methoxypyrimid in-5-yl)-7-(4-(1-isopropyl-4-(trifluorometh yl)-1H-imidazol-2-yl)benzyl)-N-(oxetan-2-ylmethyl)-7H-pyrrolo[2,3-d]pyrimidine-6-carboxamide | ¹H NMR (400 MHz, DMSO-*d*₆): δ 9.35 (s, 1H), 9.07 (t, *J* = 5.9 Hz, 1H), 8.69 (s, 1H), 8.15 (d, *J* = 1.4 Hz, 1H), 7.49-7.41 (m, 3H), 7.29 (d, *J* = 8.2 Hz, 2H), 5.95 (s, 2H), 4.83 (p, *J* = 6.0 Hz, 1H), 4.52-4.45 (m, 1H), 4.38 (ddd, *J* = 12.2, 7.7, 4.4 Hz, 2H), 3.85 (s, 3H), 3.58 (dt, *J* = 14.0, 5.9 Hz, 1H), 3.47 (dt, *J* = 14.0, 5.2 Hz, 1H), 2.63-2.55 (m, 1H), 2.35 (td, *J* = 9.4, 5.3 Hz, 1H), 1.65 (tt, *J* = 8.2, 4.6 Hz, 1H), 1.38 (s, 3H), 1.36 (s, 3H), 1.03 (p, *J* = 3.6 Hz, 2H), 0.81 (dq, *J* = 6.8, 3.4 Hz, 2H). | 647.2 |
| 67 | 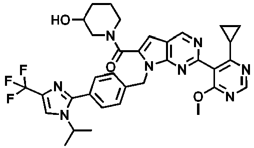 | (2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (3-hydroxypiperid in-1-yl)methanone | ¹H NMR (400 MHz, DMSO-*d*₆): δ 9.28 (s, 1H), 8.70 (s, 1H), 8.17 (d, *J* = 1.5 Hz, 1H), 7.50 (d, *J* = 8.1 Hz, 2H), 7.29 (d, *J* = 8.0 Hz, 2H), 6.98 (d, *J* = 16.3 Hz, 1H), 5.70 (d, *J* = 15.2 Hz, 1H), 5.55 (d, *J* = 17.7 Hz, 1H), 4.93 (d, *J* = 95.3 Hz, 1H), 4.38 (d, *J* = 9.5 Hz, 1H), 3.86 (s, 4H), 3.59-3.42 (m, 2H), 3.21 (d, *J* = 34.4 Hz, 2H), 2.96 (d, *J* = 42.4 Hz, 2H), 1.82-1.63 (m, 3H), 1.39 (s, 3H), 1.37 (s, 3H), 1.05 (t, *J* = 3.8 Hz, 2H), 0.85 (s, 2H). | 661.3 |
| 68 | 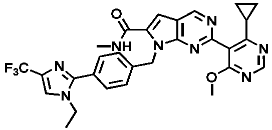 | 2-(4-cyclopropyl-6-methoxypyrimid in-5-yl)-7-(4-(1-ethyl-4-(trifluorometh yl)-1H-imidazol-2-yl)benzyl)-N-methyl-7H-pyrrolo[2,3-d]pyrimidine-6-carboxamide | ¹H NMR (400 MHz, DMSO-*d*₆): δ 9.34 (s, 1H), 8.82 (q, *J* = 4.5 Hz, 1H), 8.68 (s, 1H), 8.00 (d, *J* = 1.5 Hz, 1H), 7.57-7.48 (m, 2H), 7.35 (s, 1H), 7.28 (d, *J* = 8.1 Hz, 2H), 5.95 (s, 2H), 4.03 (q, *J* = 7.3 Hz, 2H), 3.84 (s, 3H), 2.80 (d, *J* = 4.6 Hz, 3H), 1.65 (tt, *J* = 8.2, 4.6 Hz, 1H), 1.29 (t, *J* = 7.2 Hz, 3H), 1.02 (dq, *J* = 6.2, 3.5 Hz, 2H), 0.80 (dq, *J* = 6.9, 3.4 Hz, 2H). | 577.2 |
| 69 | 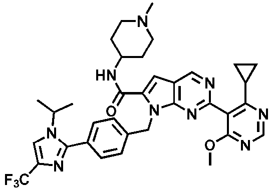 | 2-(4-cyclopropyl-6-methoxypyrimid in-5-yl)-7-(4-(1-isopropyl-4-(trifluorometh yl)-1H-imidazol-2-yl)benzyl)-N-(1-methylpiperidi n-4-yl)-7H-pyrrolo[2,3-d]pyrimidine-6-carboxamide | ¹H NMR (400 MHz, CDCl₃): δ 9.18 (s, 1H), 8.67 (s, 1H), 7.44-7.38 (m, 5H), 6.93 (s, 1H), 5.99 (s, 3H), 4.49 (p, *J* = 6.7 Hz, 1H), 3.94 (s, 4H), 2.81 (d, *J* = 11.5 Hz, 2H), 2.29 (s, 3H), 2.16-2.09 (m, 2H), 2.01 (d, *J* = 12.7 Hz, 2H), 1.71(dq, *J* = 8.3, 4.6, 4.2 Hz, 1H), 1.59-1.50 (m, 2H), 1.42 (d, *J* = 6.6 Hz, 6H), 1.23 (dd, *J* = 4.5, 2.7 Hz, 2H), 0.88-0.82 (m, 2H). | 674.3 |
| 70 | 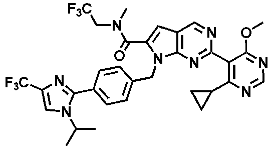 | 2-(4-cyclopropyl-6-methoxypyrimid in-5-yl)-7-(4-(1-isopropyl-4-(trifluorometh yl)-1H-imidazol-2-yl)benzyl)-N-methyl-N-(2,2,2-trifluoroethyl )-7H-pyrrolo[2,3-d]pyrimidine-6-carboxamide | ¹H NMR (400 MHz, DMSO-*d*₆): δ 9.31 (s, 1H), 8.69 (s, 1H), 8.16 (d, *J* = 1.4 Hz, 1H), 7.51-7.45 (m, 2H), 7.24 (d, *J* = 7.7 Hz, 2H), 7.20 (s, 1H), 5.63 (s, 2H), 4.39 (p, *J* = 6.3 Hz, 3H), 3.85 (s, 3H), 3.09 (s, 3H), 1.67 (tt, *J* = 8.2, 4.6 Hz, 1H), 1.37 (d, *J* = 6.6 Hz, 6H), 1.03 (p, *J* = 3.7 Hz, 2H), 0.82 (dq, *J* = 10.3, 3.5 Hz, 2H). | 673.1 |
| 71 | 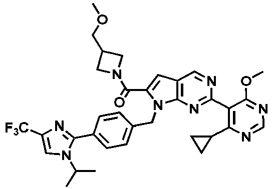 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (3-(methoxymethyl )azetidin-1-yl)methanone | ¹H NMR (400 MHz, DMSO-*d*₆): δ 9.29 (s, 1H), 8.68 (s, 1H), 8.16 (s, 1H), 7.89 (s, 1H), 7.50 (d, *J* = 8.2 Hz, 2H), 7.28 (d, *J* = 8.0 Hz, 2H), 5.83 (d, *J* = 2.1 Hz, 2H), 4.42 (dt, *J* = 13.0, 6.5 Hz, 1H), 4.37-4.31 (m, 1H), 3.98 (tq, *J* = 9.1, 4.0 Hz, 2H), 3.84 (s, 3H), 3.78-3.72 (m, 2H), 3.70 (s, 3H), 2.86 (s, 2H), 1.65 (tt, *J* = 8.2, 4.6 Hz, 1H), 1.38 (d, *J* = 6.6 Hz, 6H), 1.03 (p, *J* = 3.5 Hz, 2H), 0.80 (dq, *J* = 7.0, 3.5 Hz, 2H). | 661.1 |
| 72 | 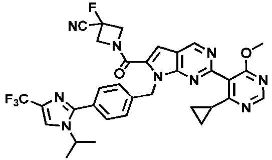 | 1-(2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carbonyl)-3-fluoroazetidin e-3-carbonitrile | ¹H NMR (400 MHz, DMSO-*d*₆): δ 9.26 (s, 1H), 8.61 (s, 1H), 8.08 (d, *J* = 1.4 Hz, 1H), 7.43-7.38 (m, 2H), 7.26 (s, 1H), 7.24 (d, *J* = 8.1 Hz, 2H), 5.73 (s, 2H), 5.03 (s, 2H), 4.74 (s, 2H), 4.32 (q, *J* = 6.7 Hz, 1H), 3.76 (s, 3H), 1.58-1.54 (m, 1H), 1.30 (d, *J* = 6.6 Hz, 6H), 0.97-0.92 (m, 2H), 0.71 (dd, *J* = 8.1, 3.2 Hz, 2H). | 660.1 |
| 73 | 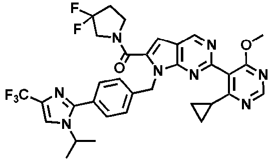 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl)(3,3-difluoropyrrol idin-1-yl)methanone | ¹H NMR (400 MHz, DMSO-*d*₆): δ 9.30 (s, 1H), 8.69 (s, 1H), 8.16 (s, 1H), 7.48 (t, *J* = 7.3 Hz, 2H), 7.26 (s, 3H), 5.72-5.66 (m, 2H), 4.43 - 4.35 (m, 1H), 3.94 (q, *J* = 14.6, 14.1 Hz, 2H), 3.85 (s, 3H), 3.76-3.62 (m, 3H), 3.47 (d, *J* = 7.8 Hz, 2H), 1.66 (td, *J* = 8.1, 4.2 Hz, 1H), 1.40-1.33 (m, 6H), 1.04 (p, *J* = 3.7 Hz, 2H), 0.82 (dt, *J* = 6.9, 3.5 Hz, 2H). | 667.1 |
| 74 | 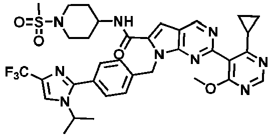 | 2-(4-cyclopropyl-6-methoxypyrimid in-5-yl)-7-(4-(1-isopropyl-4-(trifluorometh yl)-1H-imidazol-2-yl)benzyl)-N-(1-(methylsulfony l)piperidin-4-yl)-7H-pyrrolo[2,3-d]pyrimidine-6-carboxamide | ¹H NMR (400 MHz, CDCl₃): δ 9.20 (s, 1H), 8.67 (s, 1H), 7.39 (d, *J* = 1.9 Hz, 5H), 6.96 (s, 1H), 6.07 (d, *J* = 7.9 Hz, 1H), 5.99 (s, 2H), 4.48 (p, *J* = 6.7 Hz, 1H), 4.10-3.97 (m, 1H), 3.94 (s, 3H), 3.83 | 738.2 |
| 75 | 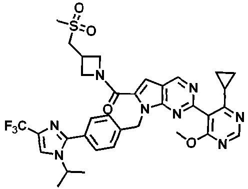 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (3-((methylsulfon yl)methyl)azet idin-1-yl)methanone | ¹H NMR (400 MHz, CDCl₃): δ 9.21 (s, 1H), 8.67 (s, 1H), 7.49-7.45 (m, 2H), 7.41 (d, *J* = 1.3 Hz, 1H), 7.33-7.28 (m, 2H), 6.76 (s, 1H), 6.05 (d, *J* = 14.9 Hz, 1H), 5.75 (d, *J* = 14.9 Hz, 1H), 4.46 (h, *J* = 6.7 Hz, 1H), 4.30 (s, 2H), 3.95 (s, 3H), 3.78 (d, *J* = 6.7 Hz, 2H), 3.22 (p, *J* = 6.9 Hz, 1H), 3.12 (dd, *J* = 18.7, 7.7 Hz, 2H), 2.95 (s, 3H), 1.74 (tt, *J* = 8.4, 4.6 Hz, 1H), 1.43 (s, 6H), 1.24 (d, *J* = 4.8 Hz, 2H), 0.88-0.83 (m, 2H). (d, *J* = 12.4 Hz, 2H), 2.86 (dd, *J* = 12.1, 2.5 Hz, 2H), 2.82 (s, 3H), 2.12 (d, *J* = 11.6 Hz, 2H), 1.72 (tt, *J* = 8.3, 4.7 Hz, 1H), 1.61 (dd, *J* = 12.0, 4.0 Hz, 2H), 1.42 (d, *J* = 6.7 Hz, 6H), 1.25-1.20 (m, 2H), 0.86 (dq, *J* = 7.0, 3.9 Hz, 2H). | 709.2 |
| 76 | 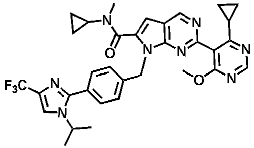 | N-cyclopropyl-2-(4-cyclopropyl-6-methoxypyrimid in-5-yl)-7-(4-(1-isopropyl-4-(trifluorometh yl)-1H-imidazol-2-yl)benzyl)-N-methyl-7H-pyrrolo[2,3-d]pyrimidine-6-carboxamide | ¹H NMR (400 MHz, CDCl₃): δ 9.17 (s, 1H), 8.68 (s, 1H), 7.44-7.37 (m, 3H), 7.34 (d, *J* = 8.2 Hz, 2H), 6.83 (s, 1H), 5.85 (s, 2H), 4.46 (p, *J* = 6.7 Hz, 1H), 3.97 (s, 3H), 3.06 (s, 3H), 2.80 (tt, *J* = 7.1, 3.8 Hz, 1H), 1.79 (tt, *J* = 8.4, 4.7 Hz, 1H), 1.41 (d, *J* = 6.7 Hz, 6H), 1.24 (dt, *J* = 6.5, 3.9 Hz, 3H), 0.95-0.85 (m, 3H), 0.58 (s, 2H). | 631.2 |
| 77 | 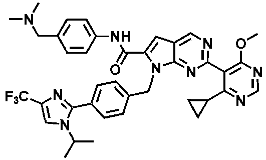 | 2-(4-cyclopropyl-6-methoxypyrimid in-5-yl)-N-(4-((dimethylamin o)methyl)pheny l)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 10.69 (s, 1H), 9.43 (s, 1H), 8.70 (s, 1H), 8.15 (d, *J* = 1.4 Hz, 1H), 7.76 (d, *J* = 8.1 Hz, 2H), 7.66 (s, 1H), 7.50-7.44 (m, 2H), 7.37 (d, *J* = 8.0 Hz, 2H), 7.31 (d, *J* = 8.1 Hz, 2H), 5.97 (s, 2H), 4.38 (p, *J* = 6.7 Hz, 1H), 3.86 (s, 3H), 3.68 (s, 2H), 2.44-2.29 (m, 7H), 1.67 (tt, *J* = 8.1, 4.6 Hz, 1H), 1.35 (d, *J* = 6.6 Hz, 6H), 1.09-1.00 (m, 2H), 0.81 (dq, *J* = 7.0, 3.5 Hz, 2H). | 710.2 |
| 78 | 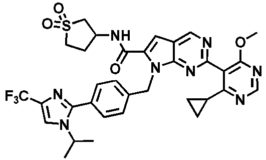 | 2-(4-cyclopropyl-6-methoxypyrimid in-5-yl)-N-(1,1-dioxidotetrahy drothiophen-3-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.38 (s, 1H), 9.14 (d, *J* = 7.1 Hz, 1H), 8.69 (s, 1H), 8.16 (d, *J* = 1.4 Hz, 1H), 7.47 (d, *J* = 8.2 Hz, 2H), 7.43 (s, 1H), 7.30 (d, *J* = 8.0 Hz, 2H), 5.92 (s, 2H), 4.70 (h, *J* = 7.6 Hz, 1H), 4.39 (p, *J* = 6.7 Hz, 1H), 3.85 (s, 3H), 3.54 (dd, *J* = 13.5, 8.0 Hz, 1H), 3.20 (ddd, *J* = 17.0, 8.9, 4.9 Hz, 2H), 3.04 (dd, *J* = 13.5, 7.6 Hz, 1H), 2.44 (dd, *J* = 12.9, 6.3 Hz, 1H), 2.27-2.13 (m, 1H), 1.66 (tt, *J* = 8.3, 4.7 Hz, 1H), 1.38 (dd, *J* = 6.7, 3.3 Hz, 6H), 1.04 (dq, *J* = 6.1, 3.5 Hz, 2H), 0.90-0.78 (m, 2H). | 695.1 |
| 79 | 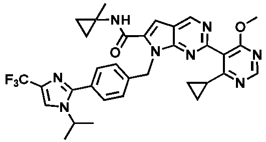 | 2-(4-cyclopropyl-6-methoxypyrimid in-5-yl)-7-(4-(1-isopropyl-4-(trifluorometh yl)-1H-imidazol-2-yl)benzyl)-N-(1-methylcyclopro pyl)-7H-pyrrolo[2,3-d]pyrimidine-6-carboxamide | ¹H NMR (400 MHz, DMSO-*d*₆): δ 9.31 (s, 1H), 9.00 (s, 1H), 8.69 (s, 1H), 8.16 (d, *J* = 1.4 Hz, 1H), 7.47 (d, *J* = 8.1 Hz, 2H), 7.33-7.25 (m, 3H), 5.92 (s, 2H), 4.39 (p, *J* = 6.7 Hz, 1H), 3.85 (s, 3H), 1.64 (td, *J* = 8.1, 4.2 Hz, 1H), 1.40-1.33 (m, 8H), 1.06-1.00 (m, 2H), 0.82 (dt, *J* = 8.1, 3.4 Hz, 2H), 0.70 (d, *J* = 4.7 Hz, 2H), 0.64 (d, *J* = 4.8 1Hz, 2H). | 631.2 |
| 80 | 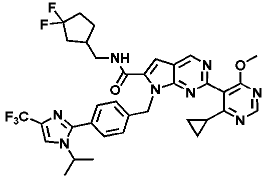 | 2-(4-cyclopropyl-6-methoxypyrimid in-5-yl)-N-((3,3-difluorocyclop entyl)methyl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.35 (s, 1H), 8.93 (t, *J* = 5.9 Hz, 1H), 8.69 (s, 1H), 8.16 (d, *J* = 1.4 Hz, 1H), 7.46 (d, *J* = 8.0 Hz, 2H), 7.36 (s, 1H), 7.29 (d, *J* = 8.0 Hz, 2H), 5.93 (s, 2H), 4.39 (p, *J* = 6.6 Hz, 1H), 3.85 (s, 3H), 2.45-2.38 (m, 2H), 2.22-2.01 (m, 4H), 1.94-1.75 (m, 2H), 1.70-1.60 (m, 1H), 1.56-1.41 (m, 1H), 1.37 (d, *J* = 6.6 Hz, 6H), 1.03 (d, *J* = 4.6 Hz, 2H), 0.81 (dd, *J* = 7.9, 3.5 Hz, 2H). | 695.2 |
| 81 | 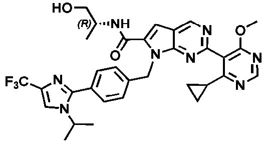 | (R) -2- (4-cyclopropyl-6-methoxypyrimid in-5-yl)-N-(1-hydroxypropan-2-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.33 (s, 1H), 8.69 (s, 1H), 8.52 (d, *J* = 8.2 Hz, 1H), 8.16 (d, *J* = 1.5 Hz, 1H), 7.50-7.43 (m, 2H), 7.37 (s, 1H), 7.31 (d, *J* = 8.0 Hz, 2H), 5.92 (s, 2H), 4.77 (s, 1H), 4.39 (p, *J* = 6.7 Hz, 1H), 3.85 (s, 3H), 3.49-3.41 (m, 1H), 3.41 - 3.35 (m, 2H), 1.65 (dq, *J* = 8.2, 4.8, 4.2 Hz, 1H), 1.37 (d, *J* = 6.6 Hz, 6H), 1.12 (d, *J* = 6.7 Hz, 3H), 1.05-1.01 (m, 2H), 0.80 (dt, *J* = 6.8, 3.4 Hz, 2H). | 635.2 |
| 82 | 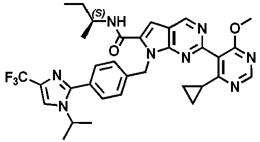 | (S)-N-(sec-butyl)-2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.33 (s, 1H), 8.69 (s, 1H), 8.56 (d, *J* = 8.4 Hz, 1H), 8.16 (d, *J* = 1.4 Hz, 1H), 7.48-7.43 (m, 2H), 7.34 (s, 1H), 7.30 (d, *J* = 8.1 Hz, 2H), 5.93 (s, 2H), 4.38 (p, *J* = 6.6 Hz, 1H), 3.92 (dt, *J* = 14.2, 7.0 Hz, 1H), 3.85 (s, 3H), 1.66 (tt, *J* = 8.1, 4.6 Hz, 1H), 1.54-1.46 (m, 2H), 1.37 (dd, *J* = 6.6, 1.6 Hz, 6H), 1.13 (d, *J* = 6.6 Hz, 3H), 1.04 (dq, *J* = 6.0, 3.6 Hz, 2H), 0.85 (d, *J* = 7.3 Hz, 3H), 0.81 (d, *J* = 7.1 Hz, 2H). | 633.1 |
| 83 | 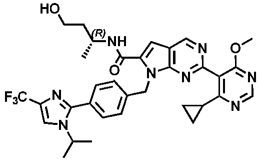 | (R)-2-(4-cyclopropyl-6-methoxypyrimid in-5-yl)-N-(4-hydroxybutan-2-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, CDCl₃): δ 9.19 (s, 1H), 8.67 (s, 1H), 7.41-7.37 (m, 3H), 7.32 (d, *J* = 8.1 Hz, 2H), 6.91 (s, 1H), 6.21 (d, *J* = 8.4 Hz, 1H), 4.49 (p, *J* = 6.7 Hz, 1H), 4.25 (d, *J* = 7.1 Hz, 1H), 3.95 (s, 3H), 3.55 (s, 2H), 3.39 (s, 2H), 1.87 (dt, *J* = 9.7, 4.2 Hz, 1H), 1.74 (tt, *J* = 8.3, 4.6 Hz, 1H), 1.42 (dd, *J* = 6.7, 5.4 Hz, 6H), 1.28 (d, *J* = 6.6 Hz, 3H), 1.24 (dq, *J* = 7.0, 4.3, 3.6 Hz, 2H), 0.88 (dd, *J* = 7.8, 3.0 Hz, 2H). | 649.1 |
| 84 | 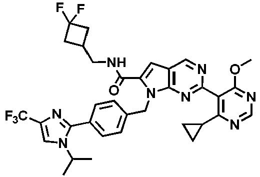 | 2-(4-cyclopropyl-6-methoxypyrimid in-5-yl)-N-((3,3-difluorocyclob utyl)methyl) - 7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.36 (s, 1H), 8.98 (t, *J* = 5.9 Hz, 1H), 8.69 (s, 1H), 8.16 (d, *J* = 1.4 Hz, 1H), 7.49-7.44 (m, 2H), 7.37 (s, 1H), 7.29 (d, *J* = 8.0 Hz, 2H), 5.94 (s, 2H), 4.39 (p, *J* = 6.6 Hz, 1H), 3.85 (s, 3H), 3.41 (t, *J* = 6.0 Hz, 2H), 2.60 (ddd, *J* = 13.7, 6.0, 3.6 Hz, 2H), 2.44-2.27 (m, 4H), 1.66 (tt, *J* = 8.2, 4.6 Hz, 1H), 1.37 (d, *J* = 6.6 Hz, 6H), 1.03 (dq, *J* = 6.1, 3.5 Hz, 2H), 0.81 (dt, *J* = 8.2, 3.4 Hz, 2H). | 680.0 |
| 85 | 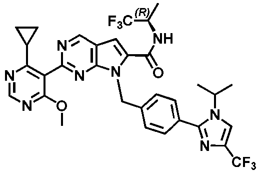 | (R)-2-(4-cyclopropyl-6-methoxypyrimid in-5-yl)-7-(4-(1-isopropyl-4-(trifluorometh yl)-1H-imidazol-2-yl)benzyl)-N-(1,1,1-trifluoropropa n-2-yl)-7H-pyrrolo[2,3-d]pyrimidine-6-carboxamide | ¹H NMR (400 MHz, DMSO-*d₆*): δ 9.34 (s, 1H), 9.24 (d, *J* = 8.7 Hz, 1H), 8.65 (s, 1H), 8.11 (s, 1H), 7.47 (s, 1H), 7.41 (d, *J* = 8.1 Hz, 2H), 7.24 (d, *J* = 8.1 Hz, 2H), 5.87 (s, 2H), 4.85-4.76 (m, 1H), 4.37-4.30 (m, 1H), 3.81 (s, 3H), 1.65-1.59 (m, 1H), 1.34-1.30 (m, 9H), 0.99 (s, 2H), 0.79-0.74 (m, 2H). | 673.2 |
| 86 | 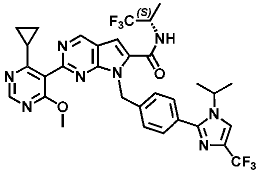 | (S)-2-(4-cyclopropyl-6-methoxypyrimid in-5-yl)-7-(4-(1-isopropyl-4-(trifluorometh yl)-1H-imidazol-2-yl)benzyl)-N-(1,1,1-trifluoropropa n-2-yl)-7H-pyrrolo[2,3-d]pyrimidine-6-carboxamide | ¹H NMR (400 MHz, DMSO-*d₆*): δ 9.34 (s, 1H), 9.24 (d, *J* = 8.8 Hz, 1H), 8.65 (s, 1H), 8.11 (s, 1H), 7.47 (s, 1H), 7.41 (d, *J* = 8.2 Hz, 2H), 7.24 (d, *J* = 8.2 Hz, 2H), 5.87 (s, 2H), 4.85-4.76 (m, 1H), 4.37-4.30 (m, 1H), 3.81 (s, 3H), 1.64-1.58 (m, 1H), 1.34-1.30 (m, 9H), 1.01-0.97 (m, 2H), 0.79-0.74 (m, 2H). | 673.3 |
| 87 | 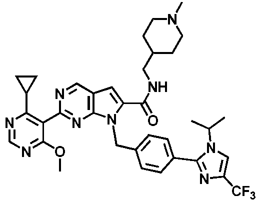 | 2-(4-cyclopropyl-6-methoxypyrimid in-5-yl)-7-(4-(1-isopropyl-4-(trifluorometh yl)-1H-imidazol-2-yl)benzyl)-N-((1-methylpiperidi n-4-yl)methyl)-7H-pyrrolo[2,3-d]pyrimidine-6-carboxamide | ¹H NMR (400 MHz, DMSO-*d₆*): δ 9.33 (s, 1H), 8.84 (t, *J* = 5.8 Hz, 1H), 8.73-8.65 (m, 1H), 8.16 (d, *J* = 0.9 Hz, 1H), 7.46 (d, *J* = 8.2 Hz, 2H), 7.35 (s, 1H), 7.28 (d, *J* = 8.2 Hz, 2H), 5.93 (s, 2H), 4.43-4.37 (m, 1H), 3.85 (s, 3H), 3.15 (t, *J* = 6.3 Hz, 2H), 2.74-2.69 (m, 2H), 2.11 (s, 3H), 1.79-1.72 (m, 2H), 1.68-1.63 (m, 1H), 1.58-1.53 (m, 2H), 1.49-1.43 (m, 1H), 1.37 (d, *J* = 6.6 Hz, 6H), 1.19-1.10 (m, 2H), 1.06-1.01 (m, 2H), 0.84-0.79 (m, 2H). | 688.3 |
| 88 | 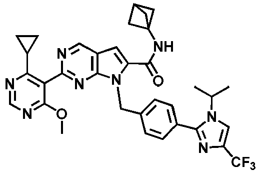 | N-(bicyclo[1.1.1 ]pentan-1-yl)-2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.29 (s, 2H), 8.63 (s, 1H), 8.11 (s, 1H), 7.42 (d, *J* = 8.2 Hz, 2H), 7.33 (s, 1H), 7.22 (d, *J* = 8.2 Hz, 2H), 5.88 (s, 2H), 4.39 - 4.31 (m, 1H), 3.79 (s, 3H), 2.44 (s, 1H), 2.06 (s, 6H), 1.61-1.55 (m, 1H), 1.33 (d, *J* = 6.6 Hz, 6H), 0.99-0.95 (m, 2H), 0.76 - 0.71 (m, 2H). | 643.3 |
| 89 | 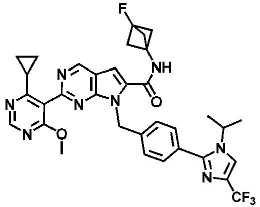 | 2-(4-cyclopropyl-6-methoxypyrimid in-5-yl)-N-(3-fluorobicyclo[ 1.1.1]pentan-1-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d₆*): 9.48 (s, 1H), 9.31 (s, 1H), 8.63 (s, 1H), 8.11 (s, 1H), 7.42 (d, *J* = 8.2 Hz, 2H), 7.37 (s, 1H), 7.21 (d, *J* = 8.2 Hz, 2H), 5.87 (s, 2H), 4.38-4.31 (m, 1H), 3.79 (s, 3H), 2.39 (d, *J* = 2.1 Hz, 6H), 1.62-1.56 (m, 1H), 1.33 (d, *J* = 6.6 Hz, 6H), 0.99-0.95 (m, 2H), 0.76-0.71 (m, 2H). | 661.3 |
| 90 | 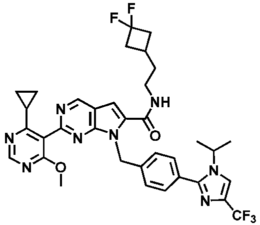 | 2-(4-cyclopropyl-6-methoxypyrimid in-5-yl)-N-(2-(3,3-difluorocyclob utyl)ethyl)-7-(4- (1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d₆*): δ 9.34 (s, 1H), 8.85 (t, *J* = 5.7 Hz, 1H), 8.68 (s, 1H), 8.15 (d, *J* = 0.9 Hz, 1H), 7.46 (d, *J* = 8.2 Hz, 2H), 7.35 (s, 1H), 7.29 (d, *J* = 8.2 Hz, 2H), 5.95 (s, 2H), 4.43-4.36 (m, 1H), 3.85 (s, 3H), 3.27-3.23 (m, 2H), 2.68-2.61 (m, 2H), 2.29-2.18 (m, 2H), 2.10 (s, 1H), 1.73-1.68 (m, 2H), 1.64 (m, 1H), 1.37 (d, *J* = 6.6 Hz, 6H), 1.05-1.01 (m, *J* = 6.9, 4.0 Hz, 2H), 0.83-0.78 (m, 2H). | 695.3 |
| 91 | 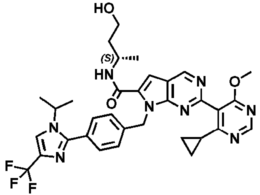 | (S)-2-(4-cyclopropyl-6-methoxypyrimid in-5-yl)-N-(4-hydroxybutan-2-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, CDCl₃): δ 9.19 (s, 1H), 8.67 (s, 1H), 7.40 (d, *J* = 8.4 Hz, 3H), 7.32 (d, *J* = 8.0 Hz, 2H), 6.91 (s, 1H), 6.25 (d, *J* = 8.4 Hz, 1H), 6.12 (d, *J* = 14.8 Hz, 1H), 5.87 (d, *J* = 14.8 Hz, 1H), 4.48 (q, *J* = 6.7 Hz, 1H), 4.28-4.19 (m, 1H), 3.95 (s, 3H), 3.59 (dd, *J* = 6.2, 3.8 Hz, 1H), 3.38 (tt, *J* = 11.0, 5.2 Hz, 2H), 1.86 (dddd, *J* = 12.7, 9.5, 6.2, 3.5 Hz, 1H), 1.74 (tt, *J* = 8.3, 4.6 Hz, 1H), 1.47 (dd, *J* = 9.4, 4.6 Hz, 1H), 1.44-1.41 (m, 6H), 1.28 (d, *J* = 6.6 Hz, 3H), 1.24 (dt, *J* = 4.5, 2.7 Hz, 2H), 0.88 (dd, *J* = 7.8, 3.2 Hz, 2H). | 649.3 |
| 92 | 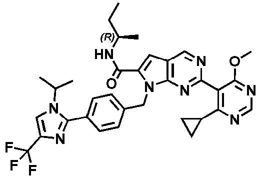 | (R)-N-(sec-butyl)-2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, CDCl₃): δ 9.18 (s, 1H), 8.67 (s, 1H), 7.45 (d, *J* = 8.2 Hz, 2H), 7.39 (dd, *J* = 8.4, 2.0 Hz, 3H), 6.90 (s, 1H), 6.00 (s, 2H), 5.88 (d, *J* = 8.5 Hz, 1H), 4.48 (p, *J* = 6.7 Hz, 1H), 4.13-4.03 (m, 1H), 3.94 (s, 3H), 1.72 (tt, *J* = 8.3, 4.6 Hz, 1H), 1.59 (d, *J* = 7.3 Hz, 1H), 1.54 (s, 1H), 1.41 (d, *J* = 6.7 Hz, 6H), 1.26-1.23 (m, 2H), 1.21 (d, *J* = 6.5 Hz, 3H), 0.94 (t, *J* = 7.4 Hz, 3H), 0.88-0.84 (m, 2H). | 633.3 |
| 93 | 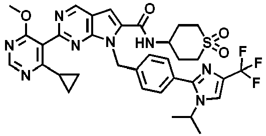 | 2-(4-cyclopropyl-6-methoxypyrimid in-5-yl)-N-(1,1-dioxidotetrahy dro-2H-thiopyran-4-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.36 (s, 1H), 8.82 (d, *J* = 7.9 Hz, 1H), 8.70 (s, 1H), 8.16 (d, *J* = 1.4 Hz, 1H), 7.51-7.44 (m, 2H), 7.41 (s, 1H), 7.31 (d, *J* = 8.1 Hz, 2H), 5.92 (s, 2H), 4.39 (p, *J* = 6.6 Hz, 1H), 4.26-4.16 (m, 1H), 3.86 (s, 3H), 3.29 (d, *J* = 4.0 Hz, 2H), 3.12 (d, *J* = 13.3 Hz, 2H), 2.10 (dddd, *J* = 20.0, 14.6, 10.4, 3.8 Hz, 4H), 1.66 (tt, *J* = 8.1, 4.6 Hz, 1H), 1.39 (s, 3H), 1.37 (s, 3H), 1.04 (p, *J* = 3.6 Hz, 2H), 0.82 (dq, *J* = 7.0, 3.5 Hz, 2H). | 709.2 |
| 94 | 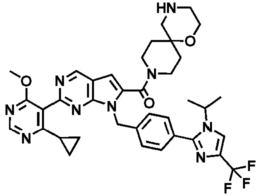 | (2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl)(1-oxa-4,9-diazaspiro[5.5 ]undecan-9-yl)methanone | ¹H NMR (400 MHz, DMSO-*d*₆): δ 9.27 (s, 1H), 8.70 (s, 1H), 8.16 (d, *J* = 1.4 Hz, 1H), 7.51 (d, *J* = 8.0 Hz, 2H), 7.27 (d, *J* = 8.0 Hz, 2H), 6.98 (s, 1H), 5.63 (q, *J* = 15.3 Hz, 2H), 4.40 (p, *J* = 6.6 Hz, 1H), 4.14 (d, *J* = 12.8 Hz, 1H), 3.87 (s, 3H), 3.49 (s, 3H), 3.25-3.04 (m, 2H), 2.61 (s, 2H), 2.46 (d, *J* = 8.7 Hz, 2H), 1.98 (d, *J* = 13.6 Hz, 1H), 1.71 (tt, *J* = 8.2, 4.7 Hz, 1H), 1.60 (d, *J* = 13.2 Hz, 1H), 1.39 (s, 3H), 1.37 (s, 3H), 1.33-1.24 (m, 2H), 1.09-1.02 (m, 2H), 0.86 (qd, *J* = 6.7, 3.0 Hz, 3H). | 716.2 |
| 95 | 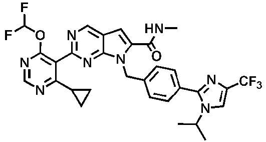 | 2-(4-cyclopropyl-6-(difluorometho xy)pyrimidin-5-yl)-7-(4-(1-isopropyl-4-(trifluorometh yl)-1H-imidazol-2-yl)benzyl)-N-methyl-7H-pyrrolo[2,3-d]pyrimidine-6-carboxamide | ¹H NMR (400 MHz, DMSO-*d*₆): δ 9.33 (s, 1H), 8.78 (d, *J* = 4.9 Hz, 1H), 8.75 (s, 1H), 8.09 (s, 1H), 7.38 (d, *J* = 8.0 Hz, 2H), 7.31 (s, 1H), 7.25 (d, *J* = 8.0 Hz, 2H), 5.90 (s, 2H), 5.26 (t, *J* = 4.8 Hz, 1H), 4.33 (p, *J* = 6.6 Hz, 1H), 2.75 (d, *J* = 4.5 Hz, 3H), 1.74 (dt, *J* = 8.2, 4.9 Hz, 1H), 1.30 (d, *J* = 6.6 Hz, 6H), 1.05-1.01 (m, 2H), 0.84 (dd, *J* = 7.8, 3.4 Hz, 2H). | 627.2 |
| 96 | 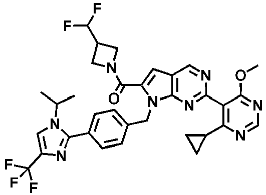 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (3-(difluoromethy l)azetidin-1-yl)methanone | ¹H NMR (400 MHz, CDCl₃) : δ 9.20 (s, 1H), 8.67 (s, 1H), 7.46-7.36 (m, 5H), 6.83 (s, 1H), 6.14-5.81 (m, 3H), 4.50 (p, *J* = 6.7 Hz, 1H), 4.25 (s, 4H), 3.94 (s, 3H), 3.19-3.04 (m, 1H), 1.73 (tt, *J* = 8.3, 4.6 Hz, 1H), 1.42 (d, *J* = 6.6 Hz, 6H), 1.25-1.21 (m, 2H), 0.87 (d, *J* = 7.8 Hz, 2H). | 667.2 |
| 97 | 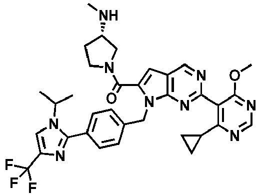 | (S)-(2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (3-(methylamino)p yrrolidin-1-yl)methanone | ¹H NMR (400 MHz, CDCl₃): δ 9.17 (d, *J* = 1.7 Hz, 1H), 8.67 (s, 1H), 7.41 (dd, *J* = 8.4, 6.5 Hz, 3H), 7.35 (d, *J* = 8.0 Hz, 2H), 6.78 (d, *J* = 12.0 Hz, 1H), 5.83 (d, *J* = 3.9 Hz, 2H), 4.52-4.44 (m, 1H), 3.95 (s, 3H), 3.72 (dt, *J* = 12.6, 6.3 Hz, 1H), 3.61-3.26 (m, 3H), 3.12-2.99 (m, 1H), 2.37 (d, *J* = 62.0 Hz, 3H), 1.98 (ddq, *J* = 86.4, 13.1, 6.6 Hz, 1H), 1.74 (td, *J* = 8.4, 4.3 Hz, 1H), 1.59 (s, 2H), 1.42 (dd, *J* = 6.8, 3.8 Hz, 6H), 1.26-1.22 (m, 2H), 0.90-0.85 (m, 2H). | 660.2 |
| 98 | 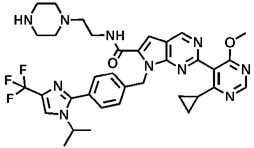 | 2-(4-cyclopropyl-6-methoxypyrimid in-5-yl)-7-(4-(1-isopropyl-4-(trifluorometh yl)-1H-imidazol-2-yl)benzyl) -N-(2-(piperazin-1-yl)ethyl)-7H-pyrrolo[2,3-d]pyrimidine-6-carboxamide | ¹H NMR (400 MHz, DMSO-*d*₆): δ 9.35 (s, 1H), 8.79 (t, *J* = 5.8 Hz, 1H), 8.69 (s, 1H), 8.16 (s, 1H), 7.47 (d, *J* = 8.1 Hz, 2H), 7.38-7.27 (m, 3H), 5.95 (s, 2H), 4.40 (p, *J* = 6.7 Hz, 1H), 3.85 (s, 3H), 3.40 (t, *J* = 6.5 Hz, 3H), 2.68 (t, *J* = 4.8 Hz, 4H), 2.43 (t, *J* = 6.8 Hz, 2H), 2.39-2.29 (m, 4H), 1.65 (tt, *J* = 8.2, 4.7 Hz, 1H), 1.39 (s, 3H), 1.37 (s, 3H), 1.03 (p, *J* = 3.8 Hz, 2H), 0.81 (dt, *J* = 7.9, 3.5 Hz, 2H). | 689.3 |
| 99 | 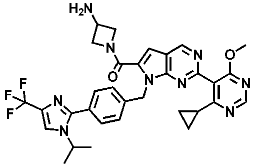 | (3-aminoazetidin-1-yl)(2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl)methanone | ¹H NMR (400 MHz, DMSO-*d*₆): δ 9.30 (s, 1H), 8.69 (s, 1H), 8.16 (d, *J* = 1.5 Hz, 1H), 7.50 (d, *J* = 8.1 Hz, 2H), 7.29 (d, *J* = 8.1 Hz, 2H), 7.17 (s, 1H), 5.84 (s, 2H), 4.41 (dt, *J* = 10.9, 6.9 Hz, 2H), 4.22 (t, *J* = 8.4 Hz, 1H), 3.94 (d, *J* = 8.3 Hz, 1H), 3.85 (s, 3H), 3.76-3.64 (m, 2H), 2.23 (s, 2H), 1.65 (tt, *J =* 8.3, 4.7 Hz, 1H), 1.39 (s, 3H), 1.37 (s, 3H), 1.03 (p, *J* = 3.7 Hz, 2H), 0.80 (dq, *J* = 7.0, 3.4 Hz, 2H). | 632.2 |
| 10 0 | 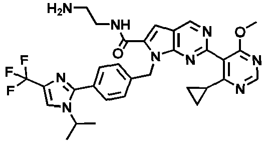 | N-(2-aminoethyl)-2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.34 (s, 1H), 8.79 (d, *J* = 6.4 Hz, 1H), 8.69 (s, 1H), 8.16 (s, 1H), 7.47 (d, *J* = 8.0 Hz, 2H), 7.38 (s, 1H), 7.31 (d, *J* = 7.9 Hz, 2H), 5.94 (s, 2H), 4.40 (p, *J* = 6.6 Hz, 1H), 3.85 (s, 3H), 3.32-3.26 (m, 4H), 2.69 (t, *J* = 6.4 Hz, 2H), 1.65 (tt, *J* = 8.3, 4.7 Hz, 1H), 1.38 (s, 3H), 1.37 (s, 3H), 1.07-1.00 (m, 2H), 0.81 (dd, *J* = 7.8, 3.5 Hz, 2H). | 620.2 |
| 10 1 | 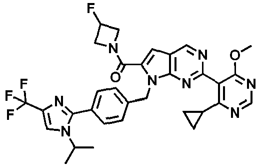 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (3-fluoroazetidin -1-yl)methanone | ¹H NMR (400 MHz, DMSO-*d*₆): δ 9.32 (s, 1H), 8.69 (s, 1H), 8.16 (d, *J* = 1.4 Hz, 1H), 7.52-7.45 (m, 2H), 7.32-7.24 (m, 3H), 5.84 (d, *J* = 7.2 Hz, 2H), 5.45 (dtt, *J* = 57.3, 6.2, 3.2 Hz, 1H), 4.64 (d, *J* = 20.0 Hz, 1H), 4.41 (p, *J* = 6.7 Hz, 3H), 4.12 (dd, *J* = 25.1, 12.0 Hz, 1H), 3.84 (s, 3H), 1.65 (tt, *J* = 8.2, 4.6 Hz, 1H), 1.38 (s, 3H), 1.37 (s, 3H), 1.03 (dq, *J* = 6.1, 3.5 Hz, 2H), 0.80 (dt, *J* = 8.2, 3.4 Hz, 2H). | 635.2 |
| 10 2 | 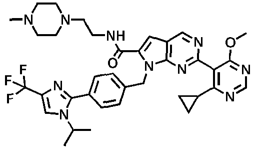 | 2-(4-cyclopropyl-6-methoxypyrimid in-5-yl)-7-(4-(1-isopropyl-4-(trifluorometh yl)-1H-imidazol-2-yl)benzyl)-N-(2-(4-methylpiperazi n-1-yl)ethyl)-7H-pyrrolo[2,3-d]pyrimidine-6-carboxamide | ¹H NMR (400 MHz, DMSO-*d*₆): δ 9.34 (s, 1H), 8.79 (t, *J* = 5.8 Hz, 1H), 8.69 (s, 1H), 8.15 (d, *J* = 1.4 Hz, 1H), 7.46 (d, *J* = 8.0 Hz, 2H), 7.36-7.26 (m, 3H), 5.94 (s, 2H), 4.40 (p, *J* = 6.7 Hz, 1H), 3.85 (s, 3H), 3.41-3.36 (m, 2H), 2.43 (dt, *J* = 13.2, 6.7 Hz, 6H), 2.31 (d, *J* = 24.0 Hz, 4H), 2.11 (s, 3H), 1.65 (tt, *J* = 8.3, 4.6 Hz, 1H), 1.38 (s, 3H), 1.37 (s, 3H), 1.03 (p, *J* = 3.6 Hz, 2H), 0.80 (dq, *J* = 6.9, 3.4 Hz, 2H). | 703.3 |
| 10 3 | 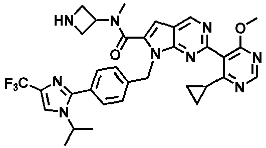 | N-(azetidin-3-yl)-2-(4-cyclopropyl-6-methoxypyrimid in-5-yl)-7-(4-(1-isopropyl-4-(trifluoromethyl) -1H-imidazol-2-yl)benzyl)-N-methyl-7H-pyrrolo[2,3-d]pyrimidine-6-carboxamide | ¹H NMR (400 MHz, DMSO-*d*₆): δ 9.32 (s, 1H), 8.70 (s, 1H), 8.18 (d, *J* = 1.4 Hz, 1H), 7.50 (d, *J* = 8.1 Hz, 2H), 7.26 (d, *J* = 8.0 Hz, 2H), 7.06 (s, 1H), 5.60 (s, 2H), 4.40 (p, *J* = 6.6 Hz, 1H), 4.16 (s, 2H), 3.85 (s, 3H), 3.33 (s, 3H), 3.06 (s, 2H), 1.70-1.62 (m, 1H), 1.38 (d, *J* = 6.6 Hz, 6H), 1.24 (s, 1H), 1.04 (s, 2H), 0.82 (s, 2H). | 646.2 |
| 10 4 | 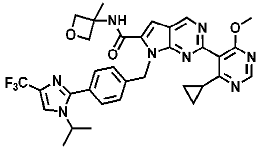 | 2-(4-cyclopropyl-6-methoxypyrimid in-5-yl)-7-(4-(1-isopropyl-4-(trifluorometh yl)-1H-imidazol-2-yl)benzyl)-N-(3-methyloxetan-3-yl)-7H-pyrrolo[2,3-d]pyrimidine-6-carboxamide | ¹H NMR (400 MHz, DMSO-*d*₆): δ 9.36 (s, 1H), 9.25 (s, 1H), 8.69 (s, 1H), 8.16 (d, *J* = 1.4 Hz, 1H), 7.51-7.43 (m, 2H), 7.37 (s, 1H), 7.28 (d, *J* = 8.1 Hz, 2H), 5.91 (s, 2H), 4.68 (d, *J* = 6.4 Hz, 2H), 4.38 (dd, *J* = 6.6, 3.6 Hz, 3H), 3.86 (s, 3H), 1.67 (dq, *J* = 8.1, 4.1, 3.5 Hz, 1H), 1.58 (s, 3H), 1.37 (d, *J* = 6.6 Hz, 6H), 1.07-1.00 (m, 2H), 0.85-0.79 (m, 2H). | 647.2 |
| 10 5 | 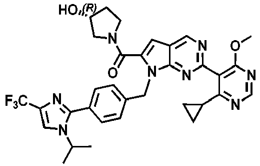 | (R) - (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (3-hydroxypyrroli din-1-yl)methanone | ¹H NMR (400 MHz, DMSO-*d*₆): δ 9.27 (d, *J* = 2.6 Hz, 1H), 8.69 (d, *J* = 1.5 Hz, 1H), 8.16 (s, 1H), 7.47 (dd, *J* = 8.2, 3.5 Hz, 2H), 7.27 (dd, *J* = 8.1, 4.7 Hz, 2H), 7.15 (d, *J* = 5.7 Hz, 1H), 5.70 (ddd, *J* = 48.5, 15.5, 10.7 Hz, 2H), 4.99 (s, 1H), 4.39 (p, *J* = 6.5 Hz, 1H), 3.85 (d, *J* = 3.8 Hz, 3H), 3.57 (ddt, *J* = 17.9, 12.9, 6.4 Hz, 4H), 1.96-1.89 (m, 1H), 1.83 (s, 1H), 1.74-1.61 (m, 2H), 1.37 (dd, *J* = 7.2, 3.3 Hz, 6H), 1.04 (p, *J* = 3.3 Hz, 2H), 0.83 (dq, *J* = 7.8, 3.5 Hz, 2H). | 647.1 |
| 10 6 | 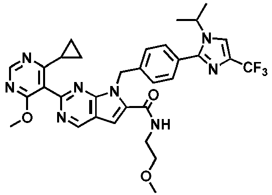 | 2-(4-cyclopropyl-6-methoxypyrimid in-5-yl)-7-(4-(1-isopropyl-4-(trifluorometh yl)-1H-imidazol-2-yl)benzyl)-N-(2-methoxyethyl)-7H-pyrrolo[2,3-d]pyrimidine-6-carboxamide | ¹H NMR (400 MHz, DMSO-*d*₆): δ 9.34 (s, 1H), 8.93 (t, *J* = 5.0 Hz, 1H), 8.69 (s, 1H), 8.16 (d, *J* = 1.5 Hz, 1H), 7.49-7.43 (m, 2H), 7.38 (s, 1H), 7.31 (d, *J* = 8.0 Hz, 2H), 5.95 (s, 2H), 4.39 (p, *J* = 6.6 Hz, 1H), 3.85 (s, 3H), 3.45 (h, *J* = 5.0 Hz, 4H), 3.27 (s, 3H), 1.65 (tt, *J* = 8.1, 4.6 Hz, 1H), 1.37 (d, *J* = 6.7 Hz, 6H), 1.03 (p, *J* = 3.6 Hz, 2H), 0.80 (dq, *J* = 9.9, 3.5 Hz, 2H). | 635.1 |
| 10 7 | 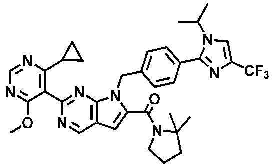 | (2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl)(2,2-dimethylpyrrol idin-1-yl)methanone | ¹H NMR (400 MHz, DMSO-*d*₆): δ 9.25 (s, 1H), 8.69 (s, 1H), 8.17 (d, *J* = 1.4 Hz, 1H), 7.51 (d, *J* = 8.1 Hz, 2H), 7.23 (d, *J* = 7.9 Hz, 2H), 7.02 (s, 1H), 5.67 (s, 2H), 4.38 (q, *J* = 6.6 Hz, 1H), 3.86 (s, 3H), 1.97 (dd, *J* = 15.6, 8.0 Hz, 2H), 1.76 (d, *J* = 6.8 Hz, 2H), 1.69 (dt, *J* = 8.1, 3.6 Hz, 1H), 1.54 (t, *J* = 6.8 Hz, 2H), 1.43-1.35 (m, 12H), 1.05 (p, *J* = 3.5 Hz, 2H), 0.86 (dq, *J* = 7.0, 3.5 Hz, 2H). | 659.1 |
| 10 8 | 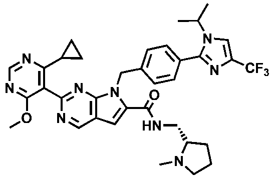 | (S)-2-(4-cyclopropyl-6-methoxypyrimid in-5-yl)-7-(4-(1-isopropyl-4-(trifluorometh yl)-1H-imidazol-2-yl)benzyl)-N-((1-methylpyrrolid in-2-yl)methyl)-7H-pyrrolo[2,3-d]pyrimidine-6-carboxamide | ¹H NMR (400 MHz, DMSO-*d*₆): δ 9.40 (s, 1H), 9.09 (t, *J* = 5.9 Hz, 1H), 8.69 (s, 1H), 8.17 (d, *J* = 1.4 Hz, 1H), 7.49-7.46 (m, 3H), 7.28 (d, *J* = 8.0 Hz, 2H), 5.95 (s, 2H), 4.39 (p, *J* = 6.7 Hz, 1H), 3.85 (s, 3H), 3.79 (s, 5H), 2.92 (s, 2H), 2.89 (s, 3H), 2.11 (d, *J* = 13.0 Hz, 2H), 1.67-1.62 (m, 1H), 1.38 (d, *J* = 6.7 Hz, 6H), 1.03 (p, *J* = 3.6 Hz, 2H), 0.80 (dq, *J* = 6.9, 3.2 Hz, 2H). | 674.1 |
| 10 9 | 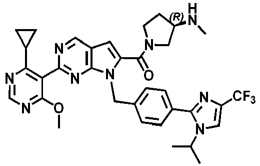 | (R) - (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (3-(methylamino)p yrrolidin-1-yl)methanone | ¹H NMR (400 MHz, DMSO-*d₆*): δ 9.27 (d, *J* = 4.4 Hz, 1H), 8.69 (s, 1H), 8.16 (s, 1H), 7.49 (d, *J* = 6.7 Hz, 2H), 7.27 (d, *J* = 8.1 Hz, 2H), 7.13 (d, *J* = 2.9 Hz, 1H), 5.78-5.65 (m, 2H), 4.42-4.37 (m, 1H), 3.86 (d, *J* = 2.0 Hz, 3H), 3.58-3.45 (m, 4H), 3.15-3.09 (m, 1H), 2.26 (d, *J* = 3.4 Hz, 2H), 2.13 (d, *J* = 4.3 Hz, 2H), 1.90 (s, 3H), 1.77-1.64 (m, 3H), 1.38 (d, *J* = 6.5 Hz, 6H), 1.06-1.02 (m, 2H), 0.86-0.81 (m, 2H). | 660.3 |
| 11 0 | 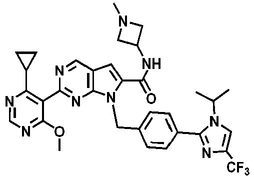 | 2-(4-cyclopropyl-6-methoxypyrimid in-5-yl)-7-(4-(1-isopropyl-4-(trifluorometh yl)-1H-imidazol-2-yl)benzyl)-N-(1-methylazetidin -3-yl)-7H-pyrrolo[2,3-d]pyrimidine-6-carboxamide | ¹H NMR (400 MHz, DMSO-*d₆*): δ 9.27 (d, *J* = 4.4 Hz, 1H), 8.69 (s, 1H), 8.16 (s, 1H), 7.49 (d, *J* = 6.7 Hz, 2H), 7.27 (d, *J* = 8.1 Hz, 2H), 7.13 (d, *J* = 2.9 Hz, 1H), 5.78-5.65 (m, 2H), 4.42-4.37 (m, 1H), 3.86 (d, *J* = 2.0 Hz, 3H), 3.58-3.45 (m, 4H), 3.15-3.09 (m, 1H), 2.26 (d, *J* = 3.4 Hz, 2H), 2.13 (d, *J* = 4.3 Hz, 2H), 1.90 (s, 3H), 1.77-1.64 (m, 3H), 1.38 (d, *J* = 6.5 Hz, 6H), 1.06-1.02 (m, 2H), 0.86-0.81 (m, 2H). | 646.2 |
| 11 1 | 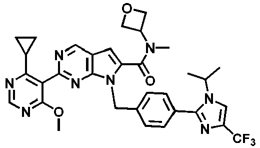 | 2-(4-cyclopropyl-6-methoxypyrimid in-5-yl)-7-(4-(1-isopropyl-4-(trifluorometh yl)-1H-imidazol-2-yl)benzyl)-N-methyl-N-(oxetan-3-yl)-7H-pyrrolo[2,3-d]pyrimidine-6-carboxamide | ¹H NMR (400 MHz, DMSO-*d₆*): δ 9.41 (s, 1H), 8.69 (s, 1H), 8.16 (s, 1H), 7.62 (d, *J* = 16.2 Hz, 1H), 7.49 (d, *J* = 8.1 Hz, 2H), 7.29 (d, *J* = 7.6 Hz, 2H), 5.94 (s, 2H), 4.50-4.13 (m, 3H), 3.90 (s, 1H), 3.85 (s, 3H), 2.73-2.67 (m, 1H), 2.44-2.41 (m, 1H), 2.33 (d, *J* = 5.7 Hz, 3H), 1.69-1.64 (m, 1H), 1.37 (d, *J* = 6.6 Hz, 6H), 1.03 (s, 2H), 0.83-0.78 (m, 2H). | 647.3 |
| 11 2 | 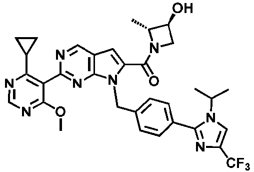 | (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl)((2R,3S)-3-hydroxy-2-methylazetidin -1-yl)methanone | ¹H NMR (400 MHz, DMSO-*d*₆): δ 9.29 (s, 1H), 8.69 (s, 1H), 8.17-8.15 (m, 1H), 7.49 (d, *J* = 8.2 Hz, 2H), 7.26 (d, *J* = 8.2 Hz, 2H), 7.19 (s, 1H), 5.82 (s, 2H), 4.57-4.31 (m, 3H), 4.02-3.96 (m, 1H), 3.85 (s, 3H), 1.86 (s, 3H), 1.69-1.64 (m, 1H), 1.37 (d, *J* = 6.6 Hz, 6H), 1.24 (s, 2H), 1.03 (s, 2H), 0.84-0.78 (m, 2H). | 647.2 |
| 11 3 | 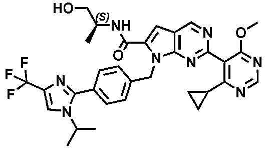 | (S)-2-(4-cyclopropyl-6-methoxypyrimid in-5-yl)-N-(1-hydroxypropan-2-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.33 (s, 1H), 8.69 (s, 1H), 8.52 (d, *J* = 8.2 Hz, 1H), 8.16 (d, *J* = 1.4 Hz, 1H), 7.46 (d, *J* = 8.0 Hz, 2H), 7.37 (s, 1H), 7.31 (d, *J* = 8.0 Hz, 2H), 5.92 (s, 2H), 4.77 (t, *J* = 5.8 Hz, 1H), 4.39 (p, *J* = 6.6 Hz, 1H), 4.02 (dt, *J* = 13.6, 6.6 Hz, 1H), 3.85 (s, 3H), 3.45 (dt, *J* = 11.2, 5.8 Hz, 1H), 3.35 (d, *J* = 7.8 Hz, 1H), 1.65 (tt, *J* = 8.2, 4.7 Hz, 1H), 1.38 (s, 3H), 1.36 (s, 3H), 1.12 (d, *J* = 6.7 Hz, 3H), 1.03 (t, *J* = 3.6 Hz, 2H), 0.81 (dd, *J* = 8.1, 3.3 Hz, 2H). | 635.2 |
| 11 4 | 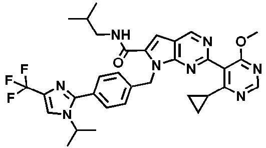 | 2-(4-cyclopropyl-6-methoxypyrimid in-5-yl)-N-isobutyl-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.34 (s, 1H), 8.85 (t, *J* = 5.9 Hz, 1H), 8.69 (s, 1H), 8.16 (d, *J* = 1.5 Hz, 1H), 7.46 (d, *J* = 8.0 Hz, 2H), 7.36 (s, 1H), 7.29 (d, *J* = 8.0 Hz, 2H), 5.94 (s, 2H), 4.38 (p, *J* = 6.6 Hz, 1H), 3.85 (s, 3H), 3.10 (t, *J* = 6.4 Hz, 2H), 1.84 (hept, *J* = 6.8 Hz, 1H), 1.66 (tt, *J* = 8.2, 4.6 Hz, 1H), 1.38 (s, 3H), 1.36 (s, 3H), 1.04 (p, *J* = 3.8 Hz, 2H), 0.88 (s, 3H), 0.86 (s, 3H), 0.82 (dt, *J* = 7.9, 3.2 Hz, 2H). | 633.3 |
| 11 5 | 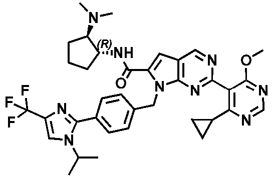 | 2-(4-cyclopropyl-6-methoxypyrimid in-5-yl)-N-((1R,2R)-2-(dimethylamino )cyclopentyl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.34 (s, 1H), 8.81 (d, *J* = 8.7 Hz, 1H), 8.69 (s, 1H), 8.16 (d, *J* = 1.4 Hz, 1H), 7.46 (d, *J* = 8.0 Hz, 2H), 7.36 - 7.27 (m, 3H), 5.94 (d, *J* = 2.4 Hz, 2H), 4.38 (p, *J* = 6.6 Hz, 1H), 4.24 (p, *J* = 7.6 Hz, 1H), 3.86 (s, 3H), 2.73 (d, *J* = 7.8 Hz, 1H), 2.15 (s, 6H), 1.94 (dt, *J* = 14.4, 7.1 Hz, 1H), 1.79 (dd, *J* = 12.1, 6.6 Hz, 1H), 1.65 (td, *J* = 7.7, 4.0 Hz, 2H), 1.62-1.56 (m, 1H), 1.53-1.45 (m, 2H), 1.38 (s, 3H), 1.37 (s, 3H), 1.04 (p, *J* = 3.7 Hz, 2H), 0.82 (dq, *J* = 6.9, 3.5 Hz, 2H). | 688.3 |
| 11 6 | 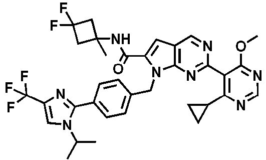 | 2-(4-cyclopropyl-6-methoxypyrimid in-5-yl)-N-(3,3-difluoro-1-methylcyclobutyl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.36 (s, 1H), 9.12 (s, 1H), 8.70 (s, 1H), 8.16 (d, *J* = 1.4 Hz, 1H), 7.48 (d, *J* = 8.1 Hz, 2H), 7.36 (s, 1H), 7.29 (d, *J* = 8.1 Hz, 2H), 5.91 (s, 2H), 4.39 (p, *J* = 6.7 Hz, 1H), 3.86 (s, 3H), 2.96 (q, *J* = 13.9 Hz, 2H), 2.72 (td, *J* = 13.5, 9.3 Hz, 2H), 1.66 (tt, *J* = 8.2, 4.6 Hz, 1H), 1.51 (s, 3H), 1.38 (s, 3H), 1.36 (s, 3H), 1.04 (dq, *J* = 6.1, 3.6 Hz, 2H), 0.83 (dt, *J* = 8.2, 3.3 Hz, 2H). | 681.2 |
| 11 7 | 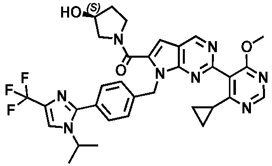 | (S)-(2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (3-hydroxypyrroli din-1-yl)methanone | ¹H NMR (400 MHz, DMSO-*d*₆): δ 9.27 (d, *J* = 2.7 Hz, 1H), 8.70 (d, *J* = 1.6 Hz, 1H), 8.16 (s, 1H), 7.48 (dd, *J* = 8.1, 3.4 Hz, 2H), 7.28 (dd, *J* = 8.1, 4.7 Hz, 2H), 7.15 (d, *J* = 5.6 Hz, 1H), 5.70 (ddd, *J* = 48.4, 15.5, 11.0 Hz, 2H), 5.02 (dd, *J* = 34.0, 3.4 Hz, 1H), 4.40 (h, *J* = 6.5 Hz, 1H), 4.25 (d, *J* = 43.3 Hz, 1H), 3.86 (d, *J* = 3.8 Hz, 3H), 3.56 (td, *J* = 14.1, 12.7, 6.3 Hz, 2H), 3.43-3.35 (m, 2H), 1.97-1.81 (m, 1H), 1.73-1.64 (m, 2H), 1.39 (s, 3H), 1.37 (s, 3H), 1.04 (dp, *J* = 6.1, 3.5 Hz, 2H), 0.83 (tt, *J* = 7.7, 3.6 Hz, 2H). | 647.2 |
| 11 8 | 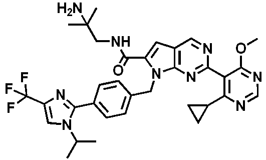 | N-(2-amino-2-methylpropyl)-2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.35 (s, 1H), 8.69 (s, 1H), 8.66 (s, 1H), 8.16 (d, *J* = 1.4 Hz, 1H), 7.45 (d, *J* = 8.1 Hz, 3H), 7.29 (d, *J* = 8.1 Hz, 2H), 5.94 (s, 2H), 4.39 (p, *J* = 6.6 Hz, 1H), 3.86 (s, 3H), 3.19 (s, 2H), 1.67 (tt, *J* = 16.8, 8.3 Hz, 3H), 1.38 (s, 3H), 1.37 (s, 3H), 1.04 (p, *J* = 3.6 Hz, 2H), 0.98 (s, 6H), 0.82 (dd, *J* = 7.8, 3.4 Hz, 2H). | 648.3 |
| 11 9 | 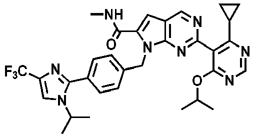 | 2-(4-cyclopropyl-6-isopropoxypyri midin-5-yl)-7-(4- (1-isopropyl-4-(trifluorometh yl)-1H-imidazol-2-yl)benzyl)-N-methyl-7H-pyrrolo[2,3-d]pyrimidine-6-carboxamide | ¹H NMR (400 MHz, DMSO-*d*₆): δ 9.35 (s, 1H), 8.81 (d, *J* = 4.9 Hz, 1H), 8.63 (s, 1H), 8.16 (d, *J* = 1.4 Hz, 1H), 7.45 (d, *J* = 8.1 Hz, 2H), 7.35 (s, 1H), 7.21 (d, *J* = 8.0 Hz, 2H), 5.97 (s, 2H), 5.29 (p, *J* = 6.2 Hz, 1H), 4.39 (p, *J* = 6.6 Hz, 1H), 2.80 (d, *J* = 4.5 Hz, 3H), 1.62 (tt, *J* = 8.2, 4.7 Hz, 1H), 1.38 (s, 3H), 1.36 (s, 3H), 1.10 (s, 3H), 1.08 (s, 3H), 1.02 (p, *J* = 3.6 Hz, 2H), 0.79 (dq, *J* = 6.9, 3.4 Hz, 2H). | 619.2 |
| 12 0 | 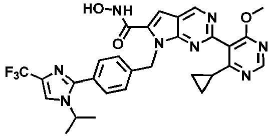 | 2-(4-cyclopropyl-6-methoxypyrimid in-5-yl)-N-hydroxy-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 11.59 (s, 1H), 9.33 (s, 1H), 8.68 (s, 1H), 8.16 (s, 1H), 7.51-7.44 (m, 2H), 7.33-7.23 (m, 3H), 5.94 (s, 2H), 4.40 (p, *J* = 6.6 Hz, 1H), 3.84 (s, 3H), 1.64 (td, *J* = 8.2, 4.2 Hz, 1H), 1.37 (d, *J* = 6.6 Hz, 6H), 1.02 (p, *J* = 3.5 Hz, 2H), 0.79 (dd, *J* = 7.8, 3.5 Hz, 2H). | 593.1 |
| 12 1 | 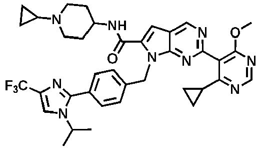 | 2-(4-cyclopropyl-6-methoxypyrimid in-5-yl)-N-(1-cyclopropylpip eridin-4-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.36 (s, 1H), 8.92 (s, 1H), 8.69 (s, 1H), 8.17 (s, 1H), 7.47 (d, *J* = 8.1 Hz, 2H), 7.43-7.38 (m, 1H), 7.30 (d, *J* = 7.9 Hz, 2H), 5.91 (d, *J* = 8.0 Hz, 2H), 4.38 (p, *J* = 6.5 Hz, 1H), 4.04 (d, *J* = 7.2 Hz, 1H), 3.86 (s, 3H), 3.55 (d, *J* = 12.1 Hz, 2H), 2.78 (s, 1H), 2.12-1.97 (m, 4H), 1.78 (s, 2H), 1.65 (td, *J* = 8.2, 4.2 Hz, 1H), 1.38 (d, *J* = 6.7 Hz, 6H), 1.03 (dd, *J* = 14.6, 8.4 Hz, 4H), 0.88-0.80 (m, 4H). | 700.2 |
| 12 2 | 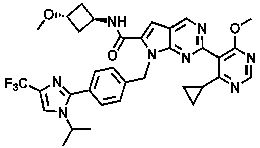 | 2-(4-cyclopropyl-6-methoxypyrimid in-5-yl)-7-(4-(1-isopropyl-4-(trifluorometh yl)-1H-imidazol-2-yl)benzyl)-N-((1r,3r)-3-methoxycyclobu tyl)-7H-pyrrolo[2,3-d]pyrimidine-6-carboxamide | ¹H NMR (400 MHz, DMSO-*d*₆): δ 9.35 (s, 1H), 9.05 (d, *J* = 7.0 Hz, 1H), 8.69 (s, 1H), 8.16 (d, *J* = 1.4 Hz, 1H), 7.50-7.43 (m, 2H), 7.41 (s, 1H), 7.28 (d, *J* = 8.0 Hz, 2H), 5.92 (s, 2H), 4.41 (dp, *J* = 13.1, 7.0, 6.5 Hz, 2H), 3.85 (s, 3H), 3.16 (s, 3H), 2.26 (t, *J* = 6.5 Hz, 4H), 1.65 (tt, *J* = 8.3, 4.6 Hz, 1H), 1.37 (d, *J* = 6.6 Hz, 6H), 1.03 (q, *J* = 3.5 Hz, 2H), 0.80 (dd, *J* = 8.0, 3.4 Hz, 2H). | 661.2 |
| 12 3 | 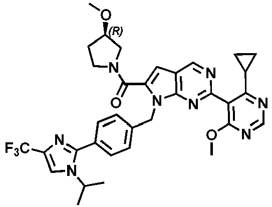 | (R) - (2- (4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (3-methoxypyrroli din-1-yl)methanone | ¹H NMR (400 MHz, CDCl₃) : δ 9.19 (s, 1H), 8.68 (s, 1H), 7.44-7.31 (m, 5H), 6.80 (d, *J =* 14.1 Hz, 1H), 5.91 (dd, *J* = 14. 9, 10.8 Hz, 1H), 5.77 (dd, *J =* 14.9, 3.9 Hz, 1H), 4.48 (h, *J* = 6.5 Hz, 1H), 3.95 (s, 3H), 3.86-3.68 (m, 2H), 3.62-3.46 (m, 2H), 3.26 (d, *J =* 68.1 Hz, 3H), 1.97 (dd, *J* = 8.7, 4.6 Hz, 1H), 1.76-1.72 (m, 3H), 1.44-1.40 (m, 6H), 1.25-1.22 (m, 2H), 0.88-0.85 (m, 2H). | 661.3 |
| 12 4 | 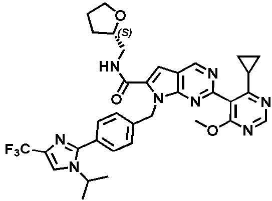 | (S)-2-(4-cyclopropyl-6-methoxypyrimid in-5-yl)-7-(4-(1-isopropyl-4-(trifluorometh yl)-1H-imidazol-2-yl)benzyl)-N-((tetrahydrofu ran-2-yl)methyl)-7H-pyrrolo[2,3-d]pyrimidine-6-carboxamide | ¹H NMR (400 MHz, CDCl₃): δ 9.19 (s, 1H), 8.67 (s, 1H), 7.45-7.37 (m, 5H), 6.98 (s, 1H), 6.54 (t, *J* = 5.8 Hz, 1H), 6.01 (s, 2H), 4.49 (p, *J* = 6.7 Hz, 1H), 4.03 (qd, *J* = 7.1, 3.1 Hz, 1H), 3.94 (s, 3H), 3.88 (dt, *J* = 8.5, 6.6 Hz, 1H), 3.80-3.72 (m, 2H), 3.30 (ddd, *J* = 13.9, 7.6, 4.9 Hz, 1H), 2.08-1.98 (m, 1H), 1.95-1.87 (m, 2H), 1.72 (tt, *J* = 8.3, 4.6 Hz, 1H), 1.53 (t, *J* = 7.7 Hz, 1H), 1.41 (dd, *J* = 6.7, 1.3 Hz, 6H), 1.24-1.20 (m, 2H), 0.85 (dq, *J* = 7.0, 3.8 Hz, 2H). | 661.3 |
| 12 5 | 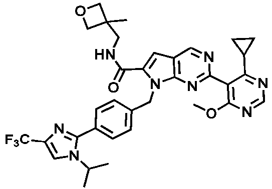 | 2-(4-cyclopropyl-6-methoxypyrimid in-5-yl)-7-(4-(1-isopropyl-4-(trifluorometh yl)-1H-imidazol-2-yl)benzyl)-N-((3-methyloxetan-3-yl)methyl)-7H-pyrrolo[2,3-d]pyrimidine-6-carboxamide | ¹H NMR (400 MHz, CDCl₃) : δ 9.20 (s, 1H), 8.67 (s, 1H), 7.40 (d, *J* = 9.7 Hz, 5H), 7.01 (s, 1H), 6.57 (t, *J* = 6.1 Hz, 1H), 6.02 (s, 2H), 4.53-4.42 (m, 5H), 3.94 (s, 3H), 3.60 (d, *J* = 6.1 Hz, 2H), 1.72 (tt, *J* = 8.4, 4.6 Hz, 1H), 1.42 (d, *J* = 6.7 Hz, 6H), 1.31 (s, 3H), 1.23 (dd, J *=* 4.5, 2.7 Hz, 2H), 0.87-0.86 (m, 2H). | 661.2 |
| 12 6 | 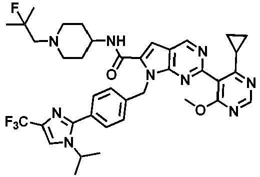 | 2-(4-cyclopropyl-6-methoxypyrimid in-5-yl)-N-(1-(2-fluoro-2-methylpropyl)p iperidin-4-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, CDCl₃) : δ 9.18 (s, 1H), 8.67 (s, 1H), 7.45-7.37 (m, 5H), 6.90 (s, 1H), 5.99 (s, 2H), 5.97 (s, 1H), 4.49 (p, *J* = 6.7 Hz, 1H), 3.94 (s, 3H), 2.94-2.84 (m, 2H), 2.44 (d, *J =* 22.5 Hz, 2H), 2.33 (td, *J* = 12.0, 2.5 Hz, 2H), 1.96 (d, *J =* 11.9 Hz, 2H), 1.71 (dd, J *=* 9.0, 4.6 Hz, 2H), 1.54 (dd, *J* = 11.9, 3.6 Hz, 2H), 1.42 (d, J = 6.7 Hz, 6H), 1.38 (s, 3H), 1.33 (s, 3H), 1.23 (dd, J *=* 4.6, 2.7 Hz, 2H), 0.86 (dd, *J =* 8.1, 3.1 Hz, 2H). | 734.3 |
| 12 7 | 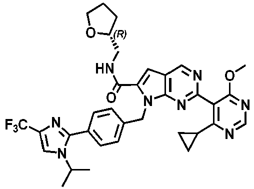 | (R) -2- (4-cyclopropyl-6-methoxypyrimid in-5-yl)-7-(4-(1-isopropyl-4-(trifluorometh yl)-1H-imidazol-2-yl)benzyl)-N-((tetrahydrofu ran-2-yl)methyl)-7H-pyrrolo[2,3-d]pyrimidine-6-carboxamide | ¹H NMR (400 MHz, DMSO-*d*₆): δ 9.34 (s, 1H), 8.94 (t, *J =* 5.9 Hz, 1H), 8.69 (s, 1H), 8.16 (d, *J* = 1.4 Hz, 1H), 7.46 (d, *J* = 8.1 Hz, 2H), 7.40 (s, 1H), 7.30 (d, *J* = 8.0 Hz, 2H), 5.95 (s, 2H), 4.39 (p, J = 6.6 Hz, 1H), 4.00-3.94 (m, 1H), 3.85 (s, 3H), 3.81-3.73 (m, 2H), 3.70 (s, 4H), 3.68-3.59 (m, 2H), 1.65 (tt, J = 8.3, 4.7 Hz, 1H), 1.37 (d, *J* = 6.6 Hz, 6H), 1.03 (p, *J* = 3.6 Hz, 2H), 0.81 (dq, *J* = 6.9, 3.4 Hz, 2H). | 661.1 |
| 12 8 | 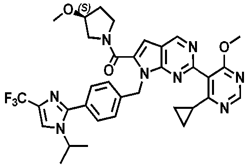 | (S)-(2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidin-6-yl) (3-methoxypyrroli din-1-yl)methanone | ¹H NMR (400 MHz, DMSO-*d*₆): δ 9.27 (d, J= 7.0 Hz, 1H), 8.69 (s, 1H), 8.16 (s, 1H), 7.48 (dd, J = 8.3, 2.2 Hz, 2H), 7.26 (d, *J =* 7.9 Hz, 2H), 7.17 (d, *J =* 3.7 Hz, 1H), 5.79-5.64 (m, 2H), 4.39 (td, J = 6.7, 3.6 Hz, 1H), 3.85 (d, *J =* 2.7 Hz, 3H), 3.73 (d, *J =* 2.2 Hz, 1H), 3.61-3.41 (m, 4H), 3.25 (d, *J* = 1.8 Hz, 3H), 3.10 (s, 2H), 1.67 (dp, *J* = 7.5, 4.1, 3.7 Hz, 1H), 1.40-1.33 (m, 6H), 1.04 (dd, *J* = 4.9, 2.8 Hz, 2H), 0.83 (ddd, *J* = 8.5, 5.4, 2.9 Hz, 2H). | 661.1 |
| 12 9 | 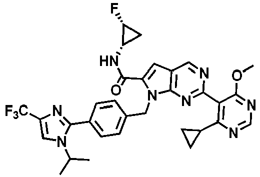 | 2-(4-cyclopropyl-6-methoxypyrimid in-5-yl)-N-((1S,2R)-2-fluorocyclopro pyl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.35 (s, 1H), 8.99 (d, *J =* 3.8 Hz, 1H), 8.69 (s, 1H), 8.16 (d, *J* = 1.4 Hz, 1H), 7.48-7.44 (m, 2H), 7.42 (s, 1H), 7.32 (d, *J* = 8.0 Hz, 2H), 5.94 (s, 2H), 4.96-4.68 (m, 2H), 4.39 (p, *J* = 6.6 Hz, 1H), 3.85 (s, 3H), 1.66 (tt, *J =* 8.3, 4.6 Hz, 1H), 1.37 (dd, *J =* 6.6, 2.0 Hz, 6H), 1.20-1.12 (m, 2H), 1.03 (dq, *J =* 6.0, 3.5 Hz, 2H), 0.81 (dq, *J* = 6.7, 3.4 Hz, 2H). | 635.0 |
| 13 0 | 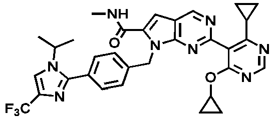 | 2- (4-cyclopropoxy-6-cyclopropylpyr imidin-5-yl)-7-(4-(1-isopropyl-4-(trifluorometh yl)-1H-imidazol-2-yl)benzyl)-N-methyl-7H-pyrrolo[2,3-d]pyrimidine-6-carboxamide | ¹H NMR (400 MHz, DMSO-*d*₆): δ 9.33 (s, 1H), 8.81 (d, *J =* 4.8 Hz, 1H), 8.71 (s, 1H), 8.16 (d, *J* = 1.4 Hz, 1H), 7.51-7.43 (m, 2H), 7.35 (d, *J =* 2.7 Hz, 1H), 7.23 (d, *J* = 8.0 Hz, 2H), 5.95 (s, 2H), 4.42 (p, *J =* 6.6 Hz, 1H), 4.32 (tt, *J* = 6.2, 2.9 Hz, 1H), 2.80 (d, *J =* 4.6 Hz, 3H), 1.65 (dt, *J* = 8.0, 3.6 Hz, 1H), 1.39 (s, 3H), 1.37 (s, 3H), 1.06-1.00 (m, 2H), 0.83-0.78 (m, 2H), 0.67 (d, *J =* 6.6 Hz, 2H), 0.42 (q, J = 4.0, 2.7 Hz, 2H). | 617.2 |
| 13 1 | 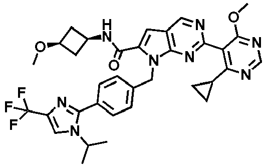 | 2-(4-cyclopropyl-6-methoxypyrimid in-5-yl)-7-(4-(1-isopropyl-4-(trifluorometh yl)-1H-imidazol-2-yl)benzyl)-N-((1s,3s)-3-methoxycyclobu tyl)-7H-pyrrolo[2,3-d]pyrimidine-6-carboxamide | ¹H NMR (400 MHz, DMSO-*d*₆): δ 9.40 (s, 1H), 9.05 (d, *J =* 7.6 Hz, 1H), 8.74 (s, 1H), 8.21 (d, *J* = 1.5 Hz, 1H), 7.52 (d, *J* = 8.2 Hz, 2H), 7.47 (s, 1H), 7.33 (d, *J* = 8.2 Hz, 2H), 5.98 (s, 2H), 4.45 (p, J = 6.6 Hz, 1H), 4.09 (hept, *J* = 8.0, 7.5 Hz, 1H), 3.90 (s, 3H), 3.68 (p, *J* = 7.1 Hz, 1H), 3.21 (s, 3H), 2.67 (dtd, *J* = 9.2, 6.8, 2.8 Hz, 2H), 2.00 (dt, *J =* 11.8, 8.8 Hz, 2H), 1.70 (tt, *J* = 8.3, 4.7 Hz, 1H), 1.43 (s, 3H), 1.42 (s, 3H), 1.08 (p, *J* = 3.8 Hz, 2H), 0.86 (dt, *J* = 8.1, 3.3 Hz, 2H). | 661.3 |
| 13 2 | 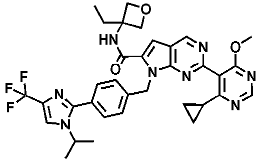 | 2-(4-cyclopropyl-6-methoxypyrimid in-5-yl)-N-(3-ethyloxetan-3-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.36 (s, 1H), 9.24 (s, 1H), 8.69 (s, 1H), 8.16 (d, *J* = 1.4 Hz, 1H), 7.48 (d, *J* = 8.3 Hz, 2H), 7.39 (s, 1H), 7.28 (d, *J* = 8.1 Hz, 2H), 5.90 (s, 2H), 4.64 (d, *J =* 6.5 Hz, 2H), 4.40 (dd, *J* = 18.2, 6.6 Hz, 3H), 3.86 (s, 3H), 3.82 (s, 3H), 2.02 (q, *J =* 7.3 Hz, 2H), 1.67 (tt, *J* = 8.2, 4.7 Hz, 1H), 1.38 (s, 3H), 1.36 (s, 3H), 1.04 (p, *J =* 3.6 Hz, 2H), 0.83 (d, *J* = 5.0 Hz, 2H). | 661.2 |
| 13 3 | 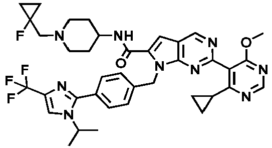 | 2-(4-cyclopropyl-6-methoxypyrimid in-5-yl)-N-(1-((1-fluorocyclopro pyl)methyl) pip eridin-4-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.33 (s, 1H), 8.69 (s, 1H), 8.66 (d, *J* = 7.8 Hz, 1H), 8.16 (s, 1H), 7.47 (d, *J* = 8.0 Hz, 2H), 7.36 (s, 1H), 7.30 (d, *J* = 8.0 Hz, 2H), 5.92 (s, 2H), 4.39 (p, *J* = 6.6 Hz, 1H), 3.85 (s, 3H), 3.80-3.71 (m, 1H), 2.99 (d, *J* = 11.2 Hz, 2H), 2.72 (s, 1H), 2.67 (s, 1H), 2.16 (t, *J* = 11.4 Hz, 2H), 1.78 (d, *J* = 12.3 Hz, 2H), 1.66 (dq, *J =* 8.1, 4.0, 3.5 Hz, 1H), 1.61-1.51 (m, 2H), 1.38 (s, 3H), 1.36 (s, 3H), 1.05-0.94 (m, 4H), 0.83-0.79 (m, 2H), 0.65 (td, *J =* 8.0, 6.0 Hz, 2H). | 732.3 |
| 13 4 | 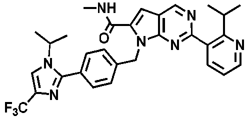 | 7-(4-(1-isopropyl-4-(trifluorometh yl)-1H-imidazol-2-yl)benzyl)-2-(2-isopropylpyrid in-3-yl)-N-methyl-7H-pyrrolo[2,3-d]pyrimidine-6-carboxamide | ¹H NMR (400 MHz, CDCl₃): δ 9.17 (s, 1H), 8.67 (dd, *J =* 4.7, 1.8 Hz, 1H), 8.09 (dd, *J =* 7.8, 1.9 Hz, 1H), 7.43-7.37 (m, 3H), 7.34 (d, *J =* 8.2 Hz, 2H), 7.25-7.23 (m, 1H), 6.90 (s, 1H), 6.18 (d, *J* = 5.3 Hz, 1H), 6.02 (s, 2H), 4.51 (p, *J* = 6.7 Hz, 1H), 3.77 (p, *J* = 6.7 Hz, 1H), 2.99 (d, *J* = 4.9 Hz, 3H), 1.41 (d, *J* = 6.7 Hz, 6H), 1.30 (d, *J* = 6.8 Hz, 6H). | 562.2 |
| 13 5 | 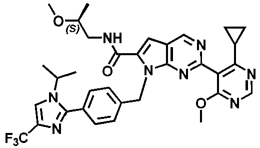 | (S)-2-(4-cyclopropyl-6-methoxypyrimid in-5-yl)-7-(4-(1-isopropyl-4-(trifluoromethyl)-1H-imidazol-2-yl)benzyl)-N-(2-methoxypropyl) -7H-pyrrolo[2,3-d]pyrimidine-6-carboxamide | ¹H NMR (400 MHz, DMSO-*d*₆): δ 9.29 (s, 1H), 8.87-8.83 (m, 1H), 8.64 (s, 1H), 8.11 (s, 1H), 7.43-7.38 (m, 3H), 7.34 (s, 1H), 5.90 (s, 2H), 4.35-4.32 (m, 1H), 3.80 (s, 3H), 3.20 (s, 3H), 1.77 (s, 3H), 1.63-1.58 (m, 1H), 1.32 (d, *J* = 6.6 Hz, 6H), 1.19 (s, 1H), 1.01 (d, *J =* 6.1 Hz, 3H), 0.90 (s, 1H), 0.78-0.74 (m, 2H), 0.68-0.60 (m, 1H). | 649.3 |
| 13 6 | 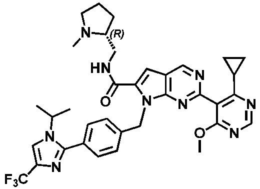 | (R) -2- (4-cyclopropyl-6-methoxypyrimid in-5-yl)-7-(4-(1-isopropyl-4-(trifluorometh yl)-1H-imidazol-2-yl)benzyl)-N-((1-methylpyrrolid in-2-yl)methyl)-7H-pyrrolo[2,3-d]pyrimidine-6-carboxamide | ¹H NMR (400 MHz, DMSO-*d₆*) : δ 9.34 (s, 1H), 8.77 (t, *J =* 5.9 Hz, 1H), 8.69 (s, 1H), 8.16 (s, 1H), 7.46 (d, *J* = 8.1 Hz, 2H), 7.36 (s, 1H), 7.29 (d, *J* = 8.1 Hz, 2H), 5.95 (s, 2H), 4.41-4.37 (m, 1H), 3.85 (s, 3H), 2.95-2.90 (m, 2H), 2.29 (s, 3H), 2.16-2.07 (m, 2H), 1.83-1.72 (m, 2H), 1.65-1.54 (m, 4H), 1.37 (d, *J* = 6.6 Hz, 6H), 1.03 (s, 2H), 0.83-0.78 (m, 2H). | 674.3 |
| 13 7 | 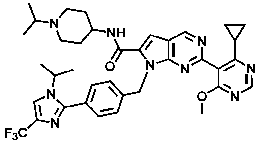 | 2-(4-cyclopropyl-6-methoxypyrimid in-5-yl)-7-(4-(1-isopropyl-4-(trifluorometh yl)-1H-imidazol-2-yl)benzyl)-N-(1-isopropylpiper idin-4-yl)-7H-pyrrolo[2,3-d]pyrimidine-6-carboxamide | ¹H NMR (400 MHz, DMSO-*d*₆): δ 9.32 (s, 1H), 8.66 (d, *J =* 15.4 Hz, 2H), 8.15 (s, 1H), 7.45 (d, *J* = 8.0 Hz, 2H), 7.36-7.27 (m, 3H), 5.90 (s, 2H), 4.38 (d, J = 6.8 Hz, 1H), 3.84 (s, 3H), 2.77 (d, *J* = 9.1 Hz, 3H), 2.15 (t, *J =* 10.9 Hz, 2H), 1.88 (s, 3H), 1.77 (d, *J* = 10.2 Hz, 2H), 1.65 (s, 1H), 1.48 (d, *J* = 11.2 Hz, 2H), 1.36 (d, *J* = 6.4 Hz, 6H), 0.95 (d, *J* = 6.3 Hz, 6H), 0.82 (s, 2H). | 702.3 |
| 13 8 | 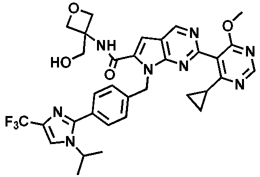 | 2-(4-cyclopropyl-6-methoxypyrimid in-5-yl)-N-(3-(hydroxymethyl )oxetan-3-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, CDCl₃) : & 9.24 (s, 1H), 8.68 (s, 1H), 7.44-7.41 (m, 1H), 7.38 (d, *J* = 7.9 Hz, 2H), 7.15 (d, *J* = 8.0 Hz, 2H), 7.06 (s, 1H), 6.97 (s, 1H), 5.92 (s, 2H), 4.72 (s, 1H), 4.69 (d, *J* = 6.8 Hz, 2H), 4.53 (p, *J* = 6.7 Hz, 1H), 4.41 (d, *J* = 6.8 Hz, 2H), 3.95 (s, 3H), 3.64 (s, 2H), 1.77 (tt, *J =* 8.3, 4.6 Hz, 1H), 1.46 (d, *J* = 6.7 Hz, 6H), 1.25 (dd, *J* = 4.5, 2.6 Hz, 2H), 0.92 (dq, *J =* 7.1, 3.9 Hz, 2H). | 663.2 |
| 13 9 | 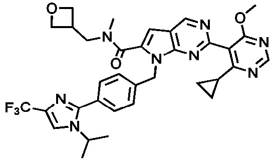 | 2-(4-cyclopropyl-6-methoxypyrimid in-5-yl)-7-(4-(1-isopropyl-4-(trifluorometh yl)-1H-imidazol-2-yl)benzyl)-N-methyl-N-(oxetan-3-ylmethyl)-7H-pyrrolo[2,3-d]pyrimidine-6-carboxamide | ¹H NMR (400 MHz, CDCl₃): δ 9.19 (s, 1H), 8.67 (s, 1H), 7.46 (d, *J* = 7.9 Hz, 2H), 7.41 (d, *J* = 1.4 Hz, 1H), 7.31 (s, 2H), 6.71 (s, 1H), 5.78 (s, 2H), 4.80-4.44 (m, 5H), 3.95 (s, 3H), 3.77 (d, *J* = 11.9 Hz, 2H), 3.26 (s, 1H), 2.89 (s, 3H), 1.78-1.70 (m, 1H), 1.43 (d, *J* = 6.7 Hz, 6H), 1.24 (dd, *J =* 4.5, 2.7 Hz, 2H), 0.90-0.85 (m, 2H). | 661.2 |
| 14 0 | 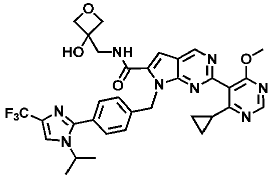 | 2-(4-cyclopropyl-6-methoxypyrimid in-5-yl)-N-((3-hydroxyoxetan-3-yl)methyl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.36 (s, 1H), 8.96 (t, *J =* 6.2 Hz, 1H), 8.69 (s, 1H), 8.16 (s, 1H), 7.49-7.42 (m, 3H), 7.30 (d, *J =* 8.1 Hz, 2H), 5.95 (s, 2H), 5.90 (s, 1H), 4.44 (d, *J =* 6.4 Hz, 2H), 4.37 (d, *J* = 6.5 Hz, 3H), 3.85 (s, 3H), 1.66 (tt, *J* = 8.3, 4.6 Hz, 1H), 1.37 (d, *J* = 6.6 Hz, 6H), 1.07-1.02 (m, 2H), 0.81 (dq, *J* = 9.8, 3.4 Hz, 2H) . | 663.2 |
| 14 1 | 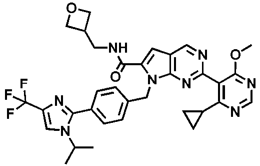 | 2-(4-cyclopropyl-6-methoxypyrimid in-5-yl)-7-(4-(1-isopropyl-4-(trifluorometh yl)-1H-imidazol-2-yl)benzyl)-N-(oxetan-3-ylmethyl) -7H-pyrrolo[2,3-d]pyrimidine-6-carboxamide | ¹H NMR (400 MHz, DMSO-*d*₆): δ 9.35 (s, 1H), 8.98 (t, *J =* 5.8 Hz, 1H), 8.69 (s, 1H), 8.16 (d, *J* = 1.5 Hz, 1H), 7.47 (d, *J* = 8.2 Hz, 2H), 7.36 (s, 1H), 7.29 (d, *J* = 7.9 Hz, 2H), 5.94 (s, 2H), 4.60 (dd, *J =* 7.8, 6.0 Hz, 2H), 4.39 (p, *J* = 6.6 Hz, 1H), 4.31 (t, *J* = 6.0 Hz, 2H), 3.85 (s, 3H), 3.55 (t, *J* = 6.4 Hz, 2H), 3.15 (p, *J* = 6.9 Hz, 1H), 1.65 (tt, *J* = 8.3, 4.7 Hz, 1H), 1.38 (s, 3H), 1.37 (s, 3H), 1.04 (p, *J* = 3.7 Hz, 2H), 0.81 (dd, *J =* 7.9, 3.2 Hz, 2H). | 647.2 |
| 14 2 | 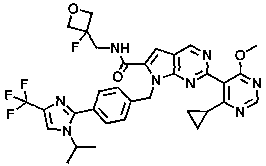 | 2-(4-cyclopropyl-6-methoxypyrimid in-5-yl)-N-((3-fluorooxetan-3-yl)methyl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.43 (s, 1H), 9.23 (t, *J =* 6.0 Hz, 1H), 8.74 (s, 1H), 8.21 (d, *J* = 1.4 Hz, 1H), 7.52 (d, *J* = 8.5 Hz, 3H), 7.33 (d, *J* = 8.0 Hz, 2H), 6.00 (s, 2H), 4.65 (dq, *J* = 20.2, 8.1 Hz, 4H), 4.44 (p, *J* = 6.6 Hz, 1H), 3.94-3.84 (m, 5H), 1.71 (tt, *J =* 8.2, 4.6 Hz, 1H), 1.43 (s, 3H), 1.42 (s, 3H), 1.09 (dq, *J =* 6.1, 3.5 Hz, 2H), 0.90-0.85 (m, 2H). | 665.2 |
| 14 3 | 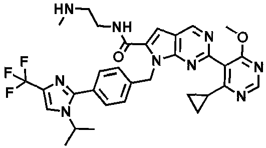 | 2-(4-cyclopropyl-6-methoxypyrimid in-5-yl)-7-(4-(1-isopropyl-4-(trifluorometh yl)-1H-imidazol-2-yl)benzyl) -N-(2-(methylamino)e thyl)-7H-pyrrolo[2,3-d]pyrimidine-6-carboxamide | ¹H NMR (400 MHz, DMSO-*d₆*): δ 9.40 (s, 1H), 8.86 (t, *J =* 5.8 Hz, 1H), 8.74 (s, 1H), 8.21 (s, 1H), 7.52 (d, *J* = 8.1 Hz, 2H), 7.43 (s, 1H), 7.36 (d, *J* = 8.0 Hz, 2H), 5.99 (s, 2H), 4.45 (p, *J* = 6.7 Hz, 1H), 3.90 (s, 3H), 3.42 (d, *J =* 7.3 Hz, 2H), 2.72 (t, *J* = 6.4 Hz, 2H), 2.37 (s, 3H), 1.70 (tt, *J* = 8.3, 4.6 Hz, 1H), 1.44 (s, 3H), 1.42 (s, 3H), 1.13-1.04 (m, 2H), 0.85 (dd, *J* = 7.9, 3.4 Hz, 2H). | 634.2 |
| 14 4 | 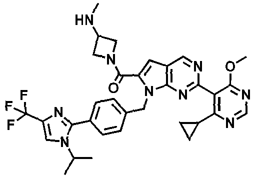 | (2- (4-cyclopropyl-6-methoxypyrimid in-5-yl)-7-(4-(1-isopropyl-4-(trifluorometh yl)-1H-imidazol-2-yl) benzyl) -7H-pyrrolo[2,3-d]pyrimidin-6-yl) (3-(methylamino)a zetidin-1-yl)methanone | ¹H NMR (400 MHz, DMSO-*d*₆): δ 9.35 (s, 1H), 8.74 (s, 1H), 8.21 (d, *J* = 1.4 Hz, 1H), 7.55 (d, *J* = 8.0 Hz, 2H), 7.34 (d, *J* = 8.0 Hz, 2H), 7.24 (s, 1H), 5.89 (d, *J* = 2.8 Hz, 2H), 4.46 (dq, *J* = 13.5, 7.7, 7.2 Hz, 2H), 4.21 (dd, *J =* 10.2, 7.6 Hz, 1H), 4.06 (dd, *J =* 9.0, 4.9 Hz, 1H), 3.89 (s, 3H), 3.78 (dd, *J =* 10.4, 5.0 Hz, 1H), 3.54 (h, *J* = 6.7, 6.1 Hz, 1H), 2.26 (s, 3H), 1.70 (tt, *J =* 8.3, 4.6 Hz, 1H), 1.44 (s, 3H), 1.42 (s, 3H), 1.08 (p, *J =* 3.7 Hz, 2H), 0.86 (dt, *J* = 8.0, 3.7 Hz, 2H) . | 646.2 |
| 14 5 | 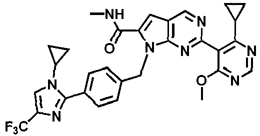 | 7-(4-(1-cyclopropyl-4-(trifluorometh yl) -1H-imidazol-2-yl)benzyl)-2-(4-cyclopropyl-6-methoxypyrimid in-5-yl)-N-methyl-7H-pyrrolo[2,3-d]pyrimidine-6-carboxamide | ¹H NMR (400 MHz, DMSO-*d*₆): δ 9.34 (s, 1H), 8.82 (d, *J =* 4.7 Hz, 1H), 8.68 (s, 1H), 7.91 (s, 1H), 7.79 (d, *J* = 8.3 Hz, 2H), 7.34 (s, 1H), 7.27 (d, *J* = 8.3 Hz, 2H), 5.95 (s, 2H), 3.84 (s, 3H), 3.70-3.66 (m, 1H), 2.80 (d, *J* = 4.5 Hz, 3H), 1.66-1.63 (m, 1H), 1.03 (s, 2H), 0.98-0.94 (m, 2H), 0.90-0.87 (m, 2H), 0.83-0.78 (m, 2H). | 589.1 |
| 14 6 | 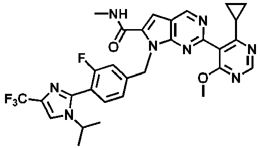 | 2-(4-cyclopropyl-6-methoxypyrimid in-5-yl)-7-(3-fluoro-4-(1-isopropyl-4-(trifluorometh yl)-1H-imidazol-2-yl)benzyl)-N-methyl-7H-pyrrolo[2,3-d]pyrimidine-6-carboxamide | ¹H NMR (400 MHz, DMSO-*d*₆): δ 9.36 (s, 1H), 8.90-8.83 (m, 1H), 8.68 (s, 1H), 8.23 (s, 1H), 7.47 (t, *J =* 7.8 Hz, 1H), 7.38 (s, 1H), 7.22 (d, *J =* 10.8 Hz, 1H), 7.11 (d, *J* = 8.0 Hz, 1H), 5.95 (s, 2H), 4.12-4.05 (m, 1H), 3.83 (s, 3H), 2.82 (d, *J* = 4.6 Hz, 3H), 1.67-1.63 (m, 1H), 1.33 (d, *J =* 6.6 Hz, 6H), 1.05-1.01 (m, 2H), 0.83-0.78 (m, 2H). | 609.2 |
| 14 7 | 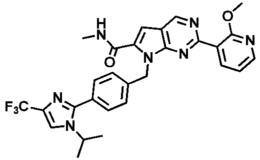 | 7-(4-(1-isopropyl-4-(trifluorometh yl)-1H-imidazol-2-yl)benzyl)-2-(2-methoxypyridin -3-yl)-N-methyl-7H-pyrrolo[2,3-d]pyrimidine-6-carboxamide | ¹H NMR (400 MHz, CDCl₃): δ 9.16 (s, 1H), 8.29 (dd, *J =* 4.9, 2.0 Hz, 1H), 8.21 (dd, *J* = 7.4, 2.0 Hz, 1H), 7.48 (d, *J* = 8.1 Hz, 2H), 7.43 (d, *J* = 8.0 Hz, 2H), 7.40-7.37 (m, 1H), 7.05 (dd, *J* = 7.4, 4.9 Hz, 1H), 6.86 (s, 1H), 6.16 (d, *J =* 5.6 Hz, 1H), 6.02 (s, 2H), 4.51 (p, *J* = 6.7 Hz, 1H), 4.07 (s, 3H), 2.99 (d, *J* = 4.9 Hz, 3H), 1.41 (d, *J* = 6.7 Hz, 6H). | 550.1 |
| 14 8 | 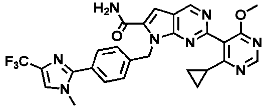 | 2-(4-cyclopropyl-6-methoxypyrimid in-5-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.34 (s, 1H), 8.69 (s, 1H), 8.31 (s, 1H), 7.91 (d, *J* = 1.4 Hz, 1H), 7.69 (s, 1H), 7.64-7.59 (m, 2H), 7.44 (s, 1H), 7.28 (d, *J* = 8.1 Hz, 2H), 5.98 (s, 2H), 3.85 (s, 3H), 3.73 (s, 3H), 1.65 (tt, *J =* 8.2, 4.6 Hz, 1H), 1.03 (dq, *J* = 5.9, 3.6 Hz, 2H), 0.81 (dq, *J* = 10.1, 3.5 Hz, 2H). | 549.0 |
| 14 9 | 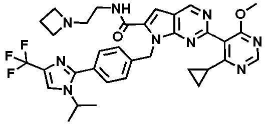 | N-(2-(azetidin-1-yl)ethyl)-2-(4-cyclopropyl-6-methoxypyrimid in-5-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, CDCl₃) : δ 9.20 (s, 1H), 8.67 (s, 1H), 7.46-7.37 (m, 5H), 7.00 (s, 1H), 6.79 (s, 1H), 6.01 (s, 2H), 4.49 (p, *J* = 6.7 Hz, 1H), 3.94 (s, 3H), 3.37 (q, *J* = 5.5 Hz, 2H), 3.22 (t, *J* = 7.0 Hz, 4H), 2.59 (t, *J* = 5.7 Hz, 2H), 2.09 (p, *J* = 7.0 Hz, 2H), 1.72 (tt, *J =* 8.4, 4.7 Hz, 1H), 1.41 (d, *J =* 6.7 Hz, 6H), 1.24-1.20 (m, 2H), 0.85 (dq, *J =* 6.9, 3.8 Hz, 2H). | 660.3 |
| 15 0 | 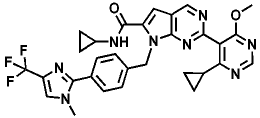 | N-cyclopropyl-2-(4-cyclopropyl-6-methoxypyrimid in-5-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.38 (d, *J =* 0.9 Hz, 1H), 8.89 (d, *J* = 4.2 Hz, 1H), 8.74 (s, 1H), 7.97 (t, *J* = 1.3 Hz, 1H), 7.67 (d, *J* = 8.0 Hz, 2H), 7.43-7.31 (m, 3H), 5.99 (s, 2H), 3.90 (s, 3H), 3.79 (s, 3H), 2.90 (tt, *J* = 7.8, 3.9 Hz, 1H), 1.70 (tt, *J* = 8.1, 4.8 Hz, 1H), 1.09 (p, *J =* 3.7 Hz, 2H), 0.87 (td, *J* = 6.8, 3.5 Hz, 2H), 0.79 (td, *J* = 7.0, 4.7 Hz, 2H), 0.64 (q, *J* = 3.8, 3.2 Hz, 2H) . | 589.2 |
| 15 1 | 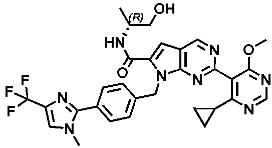 | (R) -2- (4-cyclopropyl-6-methoxypyrimid in-5-yl)-N-(1-hydroxypropan-2-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.33 (s, 1H), 8.69 (s, 1H), 8.52 (d, *J* = 8.1 Hz, 1H), 7.91 (d, *J* = 1.5 Hz, 1H), 7.61 (d, *J* = 8.1 Hz, 2H), 7.37 (s, 1H), 7.31 (d, *J =* 8.1 Hz, 2H), 5.92 (s, 2H), 4.78 (t, *J* = 5.7 Hz, 1H), 4.02 (p, *J* = 6.6 Hz, 1H), 3.86 (s, 3H), 3.73 (s, 3H), 3.46 (dt, *J =* 11.2, 5.8 Hz, 1H), 3.36 (d, *J* = 6.3 Hz, 1H), 1.66 (tt, *J =* 8.2, 4.6 Hz, 1H), 1.13 (d, *J* = 6.7 Hz, 3H), 1.04 (p, *J* = 3.7 Hz, 2H), 0.82 (dd, *J* = 8.0, 3.4 Hz, 2H) | 607.2 |
| 15 2 | 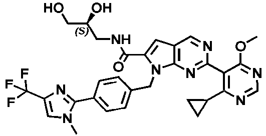 | (S)-2-(4-cyclopropyl-6-methoxypyrimid in-5-yl)-N-(2,3-dihydroxypropy 1)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.34 (s, 1H), 8.79 (t, *J =* 5.9 Hz, 1H), 8.69 (s, 1H), 7.91 (d, *J* = 1.5 Hz, 1H), 7.65 - 7.58 (m, 2H), 7.40 (s, 1H), 7.32 (d, *J* = 8.3 Hz, 2H), 5.94 (s, 2H), 4.84 (d, *J* = 5.0 Hz, 1H), 4.59 (t, *J* = 5.7 Hz, 1H), 3.86 (s, 3H), 3.74 (s, 3H), 3.65 (dt, *J* = 6.8, 5.0 Hz, 1H), 3.42 (dt, *J* = 13.4, 5.5 Hz, 1H), 3.35 (d, *J* = 5.8 Hz, 2H), 3.21 (dt, *J* = 13.1, 6.3 Hz, 1H), 1.66 (tt, *J* = 8.3, 4.7 Hz, 1H), 1.04 (dq, *J =* 6.0, 3.6 Hz, 2H), 0.82 (dq, *J* = 7.0, 3.5 Hz, 2H). | 623.2 |
| 15 3 | 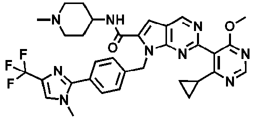 | 2-(4-cyclopropyl-6-methoxypyrimid in-5-yl)-7-(4-(1-methyl-4-(trifluorometh yl)-1H-imidazol-2-yl)benzyl)-N-(1-methylpiperidi n-4-yl)-7H-pyrrolo[2,3-d]pyrimidine-6-carboxamide | ¹H NMR (400 MHz, DMSO-*d*₆): δ 9.33 (s, 1H), 8.69 (s, 1H), 8.66 (d, *J* = 7.9 Hz, 1H), 7.91 (d, *J* = 1.4 Hz, 1H), 7.64-7.57 (m, 2H), 7.36 (s, 1H), 7.30 (d, *J* = 8.2 Hz, 2H), 5.91 (s, 2H), 3.86 (s, 3H), 3.73 (s, 4H), 2.76 (d, *J* = 11.0 Hz, 2H), 2.16 (s, 3H), 1.99 - 1.89 (m, 2H), 1.76 (dd, *J =* 12.9, 3.9 Hz, 2H), 1.66 (tt, *J* = 8.3, 4.6 Hz, 1H), 1.56 (qd, *J* = 12.0, 3.8 Hz, 2H), 1.04 (dq, *J =* 6.0, 3.5 Hz, 2H), 0.83 (dq, *J* = 10.1, 3.5 Hz, 2H). | 646.2 |
| 15 4 | 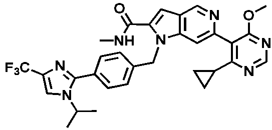 | 6-(4-cyclopropyl-6-methoxypyrimid in-5-yl)-1-(4-(1-isopropyl-4-(trifluorometh yl)-1H-imidazol-2-yl)benzyl)-N-methyl-1H-pyrrolo[3,2-c]pyridine-2-carboxamide | ¹H NMR (400 MHz, DMSO-*d*₆): δ 9. 06 (d, *J* = 1.0 Hz, 1H), 8.76 (d, *J* = 4.8 Hz, 1H), 8.63 (s, 1H), 8.15 (d, *J* = 1.4 Hz, 1H), 7.75 (s, 1H), 7.49 (d, *J* = 8.2 Hz, 2H), 7.35 (s, 1H), 7.27 (d, *J =* 8.0 Hz, 2H), 5.98 (s, 2H), 4.40 (p, *J* = 6.6 Hz, 1H), 3.78 (s, 3H), 2.82 (d, *J* = 4.6 Hz, 3H), 1.73 (tt, *J* = 8.2, 4.7 Hz, 1H), 1.38 (s, 3H), 1.36 (s, 3H), 1.00 (p, *J* = 3.8 Hz, 2H), 0.81-0.75 (m, 2H). | 590.2 |
| 15 5 | 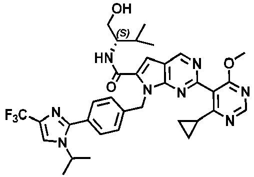 | (S)-2-(4-cyclopropyl-6-methoxypyrimid in-5-yl)-N-(1-hydroxy-3-methylbutan-2-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.33 (s, 1H), 8.69 (s, 1H), 8.41 (d, *J* = 9.0 Hz, 1H), 8.16 (d, *J* = 1.4 Hz, 1H), 7.45 (d, *J* = 8.2 Hz, 2H), 7.39 (s, 1H), 7.32 (d, *J* = 8.1 Hz, 2H), 5.92 (d, *J* = 2.3 Hz, 2H), 4.65 (t, *J* = 5.5 Hz, 1H), 4.38 (p, *J* = 6.6 Hz, 1H), 3.85 (s, 3H), 3.83 - 3.77 (m, 1H), 3.51 (dt, *J* = 11.7, 5.4 Hz, 2H), 1.89 (dt, *J =* 13.6, 6.8 Hz, 1H), 1.65 (tt, *J* = 8.3, 4.7 Hz, 1H), 1.37 (dd, *J* = 6.6, 1.7 Hz, 6H), 1.04 (dq, *J* = 5.6, 3.4 Hz, 2H), 0.89 (d, *J* = 6.8 Hz, 3H), 0.83 (s, 3H), 0.82-0.78 (m, 2H). | 663.1 |
| 15 6 | 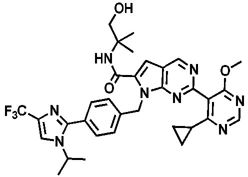 | 2-(4-cyclopropyl-6-methoxypyrimid in-5-yl)-N-(1-hydroxy-2-methylpropan-2-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.30 (s, 1H), 8.69 (s, 1H), 8.16 (d, *J* = 1.4 Hz, 1H), 7.94 (s, 1H), 7.48-7.43 (m, 2H), 7.32 (d, *J =* 8.0 Hz, 2H), 7.29 (s, 1H), 5.87 (s, 2H), 4.86 (t, *J* = 5.9 Hz, 1H), 4.39 (p, *J* = 6.6 Hz, 1H), 3.85 (s, 3H), 3.50 (d, *J =* 5.9 Hz, 2H), 1.65 (td, *J* = 8.0, 4.0 Hz, 1H), 1.37 (d, *J* = 6.6 Hz, 6H), 1.30 (s, 6H), 1.04 (dd, *J* = 4.7, 2.8 Hz, 2H), 0.84-0.79 (m, 2H). | 649.1 |
| 15 7 | 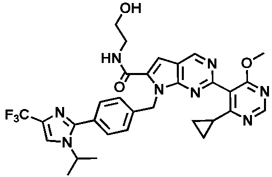 | 2-(4-cyclopropyl-6-methoxypyrimid in-5-yl)-N-(2-hydroxyethyl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.33 (s, 1H), 8.68 (d, *J =* 4.7 Hz, 2H), 8.16 (d, *J =* 1.5 Hz, 1H), 7.46 (d, *J =* 8.0 Hz, 2H), 7.37 (s, 1H), 7.30 (d, *J* = 8.1 Hz, 2H), 5.92 (s, 2H), 4.38 (p, *J* = 6.6 Hz, 1H), 3.85 (s, 3H), 2.99 (dt, *J =* 12.9, 3.5 Hz, 2H), 1.75 (dd, *J* = 13.0, 3.8 Hz, 2H), 1.65 (tt, *J* = 8.2, 4.7 Hz, 1H), 1.37 (d, *J =* 6.6 Hz, 6H), 1.04 (p, *J* = 3.7 Hz, 2H), 0.82 (dt, *J =* 8.0, 3.5 Hz, 2H). | 621.1 |
| 15 8 | 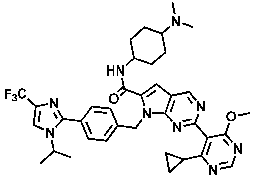 | 2-(4-cyclopropyl-6-methoxypyrimid in-5-yl)-N-(4-(dimethylamino )cyclohexyl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, CDCl₃) : δ 9.17 (s, 1H), 8.67 (s, 1H), 7.45-7.38 (m, 5H), 6.89 (s, 1H), 5.99 (s, 2H), 5.93 (d, *J* = 8.5 Hz, 1H), 4.49 (p, *J* = 6.7 Hz, 1H), 3.94 (s, 3H), 3.85 (ddd, *J* = 11.6, 7.8, 4.0 Hz, 1H), 2.32 (s, 6H), 2.23 (s, 1H), 2.13 (d, *J* = 12.4 Hz, 2H), 1.97 (s, 2H), 1.72 (t, *J* = 4.4 Hz, 1H), 1.42 (d, *J* = 6.7 Hz, 6H), 1.38 (d, *J =* 11.7 Hz, 2H), 1.25 (d, *J* = 2.4 Hz, 2H), 1.24-1.21 (m, 2H), 0.88-0.84 (m, 2H). | 702.1 |
| 15 9 | 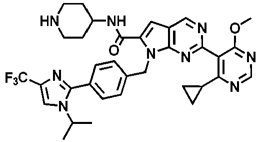 | 2-(4-cyclopropyl-6-methoxypyrimid in-5-yl)-7-(4-(1-isopropyl-4-(trifluorometh yl)-1H-imidazol-2-yl)benzyl)-N-(piperidin-4-yl)-7H-pyrrolo[2,3-d]pyrimidine-6-carboxamide | | 660.3 |
| 16 0 | 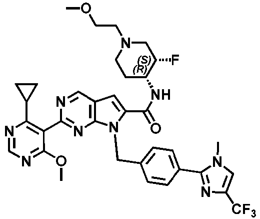 | 2-(4-cyclopropyl-6-methoxypyrimid in-5-yl)-N-((3S,4R)-3-fluoro-1-(2-methoxyethyl)p iperidin-4-yl)-7-(4-(1-methyl-4-(trifluorometh yl)-1H-imidazol-2-yl)benzyl)-7H-pyrrolo[2,3-d]pyrimidine-6-carboxamide | 1H NMR (400 MHz, DMSO-*d*₆): δ 9.32 (d, *J* = 18.5 Hz, 1H), 8.69 (s, 1H), 8.13 (s, 1H), 7.91 (s, 1H), 7.60 (t, *J* = 7.8 Hz, 2H), 7.45 (d, *J* = 13.4 Hz, 1H), 7.28 (t, *J =* 7.7 Hz, 2H), 5.91 (s, 2H), 3.72 (s, 3H), 2.20-2.13 (m, 2H), 2.01-1.99 (m, 1H), 1.84 (s, 6H), 1.68-1.40 (m, 6H), 1.10-0.91 (m, 4H), 0.85 (m, 2H), 0.79-0.57 (m, 2H). | 708.3 |
| 16 1 | 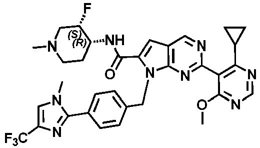 | 2-(4-cyclopropyl-6-methoxypyrimid in-5-yl)-N-((3S,4R)-3-fluoro-1-methylpiperidi n-4-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.35 (s, 1H), 8.87 (d, *J* = 7.5 Hz, 1H), 8.69 (s, 1H), 7.92 (s, 1H), 7.61 (d, *J* = 8.3 Hz, 2H), 7.48 (s, 1H), 7.29 (d, *J* = 8.4 Hz, 2H), 5.91 (s, 2H), 4.76 (d, *J* = 49.6 Hz, 1H), 3.97 (d, *J* = 31.5 Hz, 1H), 3.86 (s, 3H), 3.73 (s, 3H), 3.04 (t, *J =* 11.5 Hz, 1H), 2.81 (d, *J =* 10.0 Hz, 1H), 2.25-1.94 (m, 3H), 2.18 (s, 3H), 1.67-1.58 (m, 2H), 1.05-1.03 (m, 2H), 0.84-0.82 (m, 2H). | 664.3 |
| 16 2 | 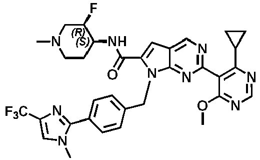 | 2-(4-cyclopropyl-6-methoxypyrimid in-5-yl)-N-((3R, 4S) -3-fluoro-1-methylpiperidi n-4-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.34 (s, 1H), 8.88 (d, *J* = 7.4 Hz, 1H), 8.69 (s, 1H), 7.91 (s, 1H), 7.60 (d, *J* = 8.0 Hz, 2H), 7.47 (s, 1H), 7.28 (d, *J* = 8.4 Hz, 2H), 5.91 (s, 2H), 4.87-4.68 (m, 1H), 4.05-3.94 (m, 1H), 3.85 (s, 3H), 3.72 (s, 3H), 3.11-3.02 (m, 1H), 2.90-2.79 (m, 1H), 2.21 (s, 3H), 2.04-1.95 (m, 1H), 1.70-1.59 (m, 2H), 1.29-1.17 (m, 2H), 1.06-1.00 (m, 2H), 0.86-0.79 (m, 2H). | 664.3 |
| 16 3 | 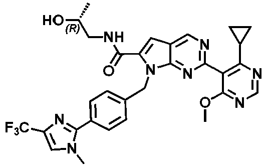 | (R)-2-(4-cyclopropyl-6-methoxypyrimid in-5-yl)-N-(2-hydroxypropyl) -7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.33 (s, 1H), 8.81 (d, 1H), 8.68 (s, 1H), 7.91 (s, 1H), 7.60 (d, *J* = 8.2 Hz, 2H), 7.39 (s, 1H), 7.29 (d, *J* = 8.1 Hz, 2H), 5.92 (s, 2H), 4.79 (d, *J* = 4.8 Hz, 1H), 3.84 (s, 3H), 3.83-3.72 (m, 1H), 3.72 (s, 3H), 3.21 (d, *J* = 6.2 Hz, 2H), 1.64 (s, 1H), 1.04 (s, 3H), 1.02 (s, 2H). 0.82 (s, 2H). | 607.2 |
| 16 4 | 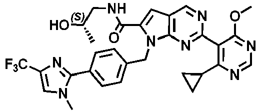 | (S)-2-(4-cyclopropyl-6-methoxypyrimid in-5-yl)-N-(2-hydroxypropyl) -7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.33 (s, 1H), 8.82 (t, *J =* 5.9 Hz, 1H), 8.68 (s, 1H), 7.91 (s, 1H), 7.64-7.56 (m, 2H), 7.39 (s, 1H), 7.34-7.24 (m, 2H), 5.92 (s, 2H), 4.79 (d, *J* = 4.8 Hz, 1H), 3.84 (s, 3H), 3.81-3.73 (m, 1H), 3.72 (s, 3H), 3.20 (t, *J* = 6.0 Hz, 2H), 1.64 (dt, *J =* 8.0, 3.5 Hz, 1H), 1.03 (d, *J =* 6.1 Hz, 3H), 1.02-1.01 (m, 2H), 0.83-0.79 (m, 2H) . | 607.2 |
| 16 5 | 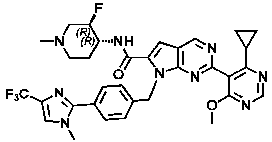 | 2-(4-cyclopropyl-6-methoxypyrimid in-5-yl)-N-((3R,4R)-3-fluoro-1-methylpiperidi n-4-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.36 (s, 1H), 8.92 (d, *J =* 8.4 Hz, 1H), 8.70 (s, 1H), 7.92 (d, *J* = 1.4 Hz, 1H), 7.65-7.57 (m, 2H), 7.38 (s, 1H), 7.35-7.29 (m, 2H), 5.92 (d, *J* = 2.9 Hz, 2H), 4.67-4.58 (m, 0.5H), 4.52-4.47 (m, 0.5H), 3.98-3.93 (m, 1H), 3.86 (s, 3H), 3.73 (s, 3H), 3.18-3.08 (m, 1H), 2.72 (d, *J =* 11.3 Hz, 1H), 2.24 (s, 3H), 2.08-1.96 (m, 2H), 1.88-1.79 (m, 1H), 1.67 (tt, *J* = 8.2, 4.7 Hz, 1H), 1.58 (dt, *J* = 12.4, 6.2 Hz, 1H), 1.07-1.03 (m, 2H), 0.86-0.81 (m, 2H). | 664.2 |
| 16 6 | 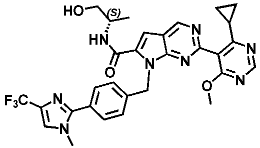 | (S)-2-(4-cyclopropyl-6-methoxypyrimid in-5-yl)-N-(1-hydroxypropan-2-yl)-7-(4-(1-methyl-4-(trifluoromethyl)-1H-imidazol-2-yl)benzyl)-7H-pyrrolo[2,3-d]pyrimidine-6-carboxamide | ¹H NMR (400 MHz, DMSO-*d*₆): δ 9.32 (s, 1H), 8.68 (s, 1H), 8.54 (d, *J* = 8.1 Hz, 1H), 7.91 (q, *J* = 1.3 Hz, 1H), 7.61 (d, 2H), 7.36 (s, 1H), 7.31 (d, 2H), 5.91 (s, 2H), 4.79 (s, 1H), 4.07-3.96 (m, 1H), 3.85 (s, 3H), 3.72 (s, 3H), 3.48-3.41 (m, 1H), 1.70-1.59 (m, 1H), 1.12 (d, *J =* 6.7 Hz, 3H), 1.07-0.99 (m, 2H), 0.87-0.77 (m, 2H). | 607.3 |
| 16 7 | 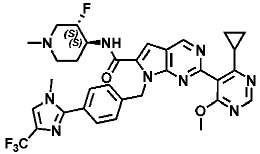 | 2-(4-cyclopropyl-6-methoxypyrimid in-5-yl)-N-((3S,4S)-3-fluoro-1-methylpiperidi n-4-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.35 (s, 1H), 8.92 (d, *J =* 8.4 Hz, 1H), 8.70 (s, 1H), 7.92 (s, 1H), 7.61 (d, *J* = 8.5 Hz, 2H), 7.37 (s, 1H), 7.31 (d, *J* = 8.2 Hz, 2H), 5.91 (s, 2H), 4.64-4.49 (m, 1H), 3.99-3.89 (m, 1H), 3.86 (s, 3H), 3.72 (s, 3H), 3.16-3.10 (m, 1H), 2.75-2.67 (m, 1H), 2.24 (s, 3H), 2.06-1.96 (m, 2H), 1.87-1.79 (m, 1H), 1.69-1.63 (m, 1H), 1.61-1.52 (m, 1H), 1.07-1.02 (m, 2H), 0.86-0.80 (m, 2H). | 664.3 |
| 16 8 | 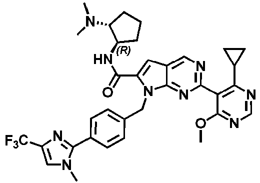 | 2-(4-cyclopropyl-6-methoxypyrimid in-5-yl)-N-((1R,2R)-2-(dimethylamino )cyclopentyl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.33 (s, 1H), 8.83 (s, 1H), 8.69 (s, 1H), 7.91 (d, *J* = 1.3 Hz, 1H), 7.60 (d, 2H), 7.35-7.25 (m, 3H), 5.91 (s, 2H), 4.23 (s, 1H), 3.85 (s, 3H), 3.72 (s, 3H), 2.79-2.69 (m, 1H), 2.15 (s, 6H), 1.99-1.88 (m, 1H), 1.82-1.77 (m, 2H), 1.69-1.57 (m, 2H), 1.56-1.41 (m, 2H), 1.07-0.99 (m, 2H), 0.86-0.78 (m, 2H). | 660.3 |
| 16 9 | 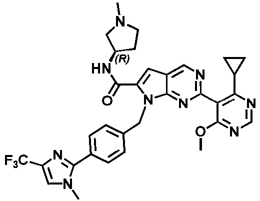 | (R) -2- (4-cyclopropyl-6-methoxypyrimid in-5-yl)-7-(4-(1-methyl-4-(trifluorometh yl)-1H-imidazol-2-yl)benzyl)-N-(1-methylpyrrolid in-3-yl)-7H-pyrrolo[2,3-d]pyrimidine-6-carboxamide | ¹H NMR (400 MHz, DMSO-*d₆*) : δ 9.34 (s, 1H), 8.94 (d, *J =* 6.8 Hz, 1H), 8.68 (s, 1H), 7.91 (s, 1H), 7.61 (d, *J* = 8.3 Hz, 2H), 7.41 (s, 1H), 7.27 (d, *J* = 8.3 Hz, 2H), 5.91 (s, 2H), 4.45-4.37 (m, 1H), 3.84 (s, 3H), 3.72 (s, 3H), 2.53-2.51 (m, 2H), 2.49 (s, 3H), 2.44-2.32 (m, 2H), 2.29-2.15 (m, 1H), 1.88-1.74 (m, 1H), 1.69-1.59 (m, 1H), 1.07-0.99 (m, 2H), 0.87-0.77 (m, 2H) . | 632.6 |
| 17 0 | 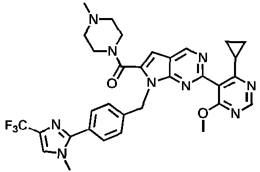 | (2- (4-cyclopropyl-6-methoxypyrimid in-5-yl)-7-(4-(1-methyl-4-(trifluorometh yl)-1H-imidazol-2-yl) benzyl) -7H-pyrrolo[2,3-d]pyrimidin-6-yl) (4-methylpiperazi n-1-yl)methanone | ¹H NMR (400 MHz, DMSO-*d*₆): δ 9.27 (s, 1H), 8.69 (s, 1H), 7.92 (d, *J* = 1.5 Hz, 1H), 7.67 (d, 2H), 7.23 (d, 2H), 6.97 (s, 1H), 5.62 (s, 2H), 3.85 (s, 3H), 3.74 (s, 3H), 3.66-3.52 (m, 2H), 2.49 (s, 3H), 2.33-2.18 (m, 2H), 2.11-2.03 (m, 2H), 1.95-1.80 (m, 2H), 1.73-1.64 (m, 1H), 1.09-1.01 (m, 2H), 0.90-0.81 (m, 2H). | 632.6 |
| 17 1 | 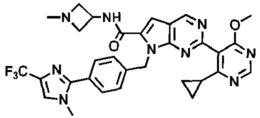 | 2-(4-cyclopropyl-6-methoxypyrimid in-5-yl)-7-(4-(1-methyl-4-(trifluorometh yl)-1H-imidazol-2-yl)benzyl)-N-(1-methylazetidin -3-yl)-7H-pyrrolo[2,3-d]pyrimidine-6-carboxamide | ¹H NMR (400 MHz, DMSO-*d*₆): δ 9.35 (s, 1H), 9.21 (d, *J =* 7.0 Hz, 1H), 8.68 (s, 1H), 7.91 (d, *J* = 1.4 Hz, 1H), 7.64-7.55 (m, 2H), 7.46 (s, 1H), 7.28-7.21 (m, 2H), 5.90 (s, 2H), 4.41 (h, *J =* 6.9 Hz, 1H), 3.84 (s, 3H), 3.72 (s, 3H), 3.59-3.52 (m, 2H), 3.02-2.93 (m, 2H), 2.24 (s, 3H), 1.64 (s, 1H), 1.02 (dd, *J =* 4.7, 2.8 Hz, 2H), 0.85-0.75 (m, 2H). | 618. 6 |
| 17 2 | 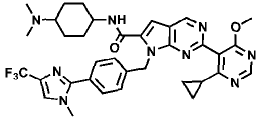 | 2-(4-cyclopropyl-6-methoxypyrimid in-5-yl)-N-(4-(dimethylamino cyclohexyl) - 7-(4-(1-methyl-4-(trifluorometh yl)-1H-imidazol-2-yl)benzyl) -7H-pyrrolo[2,3-d]pyrimidine-6-carboxamide | ¹H NMR (400 MHz, DMSO-*d*₆): δ 9.33 (s, 1H), 8.70 (s, 1H), 8.65 (d, *J* = 8.0 Hz, 1H), 7.93 (d, *J* = 1.4 Hz, 1H), 7.66-7.58 (m, 2H), 7.34 (s, 1H), 7.33-7.27 (m, 2H), 5.92 (s, 2H), 3.86 (s, 3H), 3.73 (s, 3H), 3.73-3.67 (m, 1H), 2.20 (s, 6H), 2.18-2.11 (m, 1H), 1.93-1.87 (m, 2H), 1.86-1.80 (m, 2H), 1.69-1.62 (m, 1H), 1.36-1.30 (m, 2H), 1.30-1.23 (m, 2H), 1.06-1.03 (m, 2H), 0.86-0.81 (m, 2H). | 674.7 |
| 17 3 | 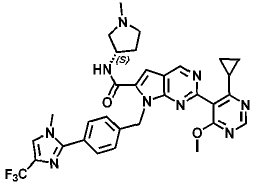 | (S)-2-(4-cyclopropyl-6-methoxypyrimid in-5-yl)-7-(4-(1-methyl-4-(trifluorometh yl)-1H-imidazol-2-yl)benzyl)-N-(1-methylpyrrolidin-3-yl)-7H-pyrrolo[2,3-d]pyrimidine-6-carboxamide | ¹H NMR (400 MHz, DMSO-*d*₆): δ 9.34 (s, 1H), 8.92 (d, *J =* 7.0 Hz, 1H), 8.69 (s, 1H), 7.92 (s, 1H), 7.61 (d, *J* = 8.3 Hz, 2H), 7.41 (s, 1H), 7.27 (d, *J* = 8.3 Hz, 2H), 5.91 (s, 2H), 4.39-4.37 (m, 1H), 3.85 (s, 3H), 3.73 (s, 3H), 2.73-2.67 (m, 1H), 2.63 (s, br, 1H), 2.43 (s, br, 2H), 2.27 (s, 3H), 2.20-2.13 (m, 1H), 1.79-1.73 (m, 1H), 1.68-1.62 (m, 1H), 1.05-1.01 (m, 2H), 0.86-0.81 (m, 2H) | 632.6 |
| 17 4 | 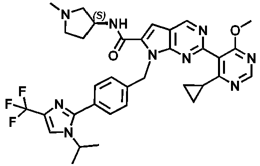 | (S)-2-(4-cyclopropyl-6-methoxypyrimid in-5-yl)-7-(4-(1-isopropyl-4-(trifluorometh yl)-1H-imidazol-2-yl)benzyl)-N-(1-methylpyrrolid in-3-yl)-7H-pyrrolo[2,3-d]pyrimidine-6-carboxamide | ¹H NMR (400 MHz, DMSO-*d*₆): δ 9.34 (s, 1H), 8.91 (d, *J =* 7.1 Hz, 1H), 8.69 (s, 1H), 8.16 (s, 1H), 7.47 (d, *J* = 8.0 Hz, 2H), 7.42 (s, 1H), 7.29 (d, *J* = 8.0 Hz, 2H), 5.92 (s, 2H), 4.39 (p, *J* = 6.7 Hz, 2H), 3.85 (s, 3H), 2.72 (t, *J =* 8.3 Hz, 1H), 2.68-2.60 (m, 1H), 2.48-2.38 (m, 2H), 2.27 (s, 3H), 2.23-2.13 (m, 1H), 1.75 (dq, *J =* 13.1, 6.6 Hz, 1H), 1.65 (tt, *J* = 8.3, 4.7 Hz, 1H), 1.38 (s, 3H), 1.37 (s, 3H), 1.06-1.00 (m, 2H), 0.80 (dd, *J* = 8.0, 3.5 Hz, 2H) . | 660.3 |
| 17 5 | 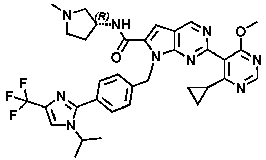 | (R)-2-(4-cyclopropyl-6-methoxypyrimid in-5-yl)-7-(4-(1-isopropyl-4-(trifluorometh yl)-1H-imidazol-2-yl)benzyl)-N-(1-methylpyrrolid in-3-yl)-7H-pyrrolo[2,3-d]pyrimidine-6-carboxamide | ¹H NMR (400 MHz, DMSO-*d*₆): δ 9.34 (s, 1H), 8.90 (d, *J =* 7.1 Hz, 1H), 8.69 (s, 1H), 8.16 (s, 1H), 7.47 (d, *J* = 8.0 Hz, 2H), 7.42 (s, 1H), 7.29 (d, *J* = 8.1 Hz, 2H), 5.92 (s, 2H), 4.39 (h, *J* = 6.7 Hz, 2H), 3.85 (s, 3H), 2.73-2.66 (m, 1H), 2.61 (q, *J* = 8.0, 7.5 Hz, 1H), 2.45-2.37 (m, 2H), 2.26 (s, 3H), 2.18 (tq, *J* = 13.6, 8.4, 6.8 Hz, 1H), 1.74 (tt, *J =* 13.3, 6.2 Hz, 1H), 1.65 (tt, *J =* 8.3, 4.6 Hz, 1H), 1.38 (s, 3H), 1.36 (s, 3H), 1.03 (t, *J =* 3.7 Hz, 2H), 0.80 (dd, *J* = 7.8, 3.5 Hz, 2H). | 660.3 |
| 17 6 | 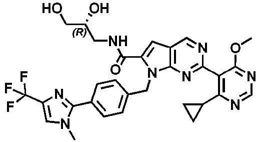 | (R) -2- (4-cyclopropyl-6-methoxypyrimid in-5-yl)-N-(2, 3-dihydroxypropy 1)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.34 (s, 1H), 8.78 (t, *J =* 5.9 Hz, 1H), 8.69 (s, 1H), 7.91 (s, 1H), 7.61 (d, *J* = 8.1 Hz, 2H), 7.39 (s, 1H), 7.31 (d, *J* = 8.1 Hz, 2H), 5.93 (s, 2H), 4.84 (d, *J* = 5.0 Hz, 1H), 4.58 (t, *J* = 5.7 Hz, 1H), 3.85 (s, 3H), 3.73 (s, 3H), 3.68-3.61 (m, 1H), 3.42 (dt, *J* = 13.3, 5.5 Hz, 1H), 3.35 (d, *J* = 5.4 Hz, 2H), 3.21 (dt, *J =* 13.1, 6.3 Hz, 1H), 1.66 (tt, *J =* 8.2, 4.6 Hz, 1H), 1.04 (p, *J* = 3.8 Hz, 2H), 0.82 (dt, *J* = 7.5, 3.1 Hz, 2H). | 623.2 |
| 17 7 | 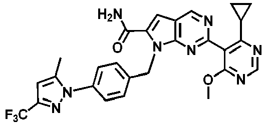 | 2-(4-cyclopropyl-6-methoxypyrimid in-5-yl)-7-(4-(5-methyl-3-(trifluorometh yl)-1H-pyrazol-1-yl)benzyl)-7H-pyrrolo[2,3-d]pyrimidine-6-carboxamide | ¹H NMR (400 MHz, DMSO-*d*₆): δ 9.34 (s, 1H), 8.68 (s, 1H), 8.32 (s, 1H), 7.70 (s, 1H), 7.52-7.42 (m, 3H), 7.36-7.29 (m, 2H), 6.73 (s, 1H), 5.99 (s, 2H), 3.84 (s, 3H), 2.29 (s, 3H), 1.64 (tt, *J =* 8.2, 4.6 Hz, 1H), 1.02 (dq, *J =* 6.0, 3.5 Hz, 2H), 0.81 (dq, *J* = 6.8, 3.4 Hz, 2H). | 549.1 |
| 17 8 | 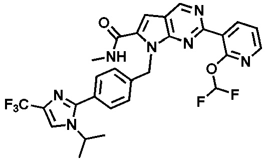 | 2-(2-(difluorometho xy)pyridin-3-yl)-7-(4-(1-isopropyl-4-(trifluorometh yl)-1H-imidazol-2-yl)benzyl)-N-methyl-7H-pyrrolo[2,3-d]pyrimidine-6-carboxamide | ¹H NMR (400 MHz, CDCl₃) : δ 9.14 (s, 1H), 8.40 (dd, *J =* 7.5, 2.0 Hz, 1H), 8.30 (dd, *J =* 4.9, 2.0 Hz, 1H), 7.65 (t, *J =* 73.0 Hz, 1H), 7.54 (d, *J* = 8.1 Hz, 2H), 7.43 (d, *J* = 8.2 Hz, 2H), 7.38 (d, *J =* 1.3 Hz, 1H), 7.31-7.27 (m, 1H), 6.86 (s, 1H), 6.18 (d, *J* = 5.0 Hz, 1H), 6.01 (s, 2H), 4.52 (p, *J* = 6.7 Hz, 1H), 3.00 (d, *J* = 4.9 Hz, 3H), 1.41 (d, *J* = 6.7 Hz, 6H). | 586.1 |
| 17 9 | 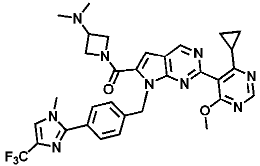 | (2- (4-cyclopropyl-6-methoxypyrimid in-5-yl)-7-(4-(1-methyl-4-(trifluorometh yl)-1H-imidazol-2-yl)benzyl)-7H-pyrrolo[2,3-d]pyrimidin-6-yl) (3-(dimethylamino )azetidin-1-yl)methanone | ¹H NMR (400 MHz, Methanol-*d₄*): δ 9.14 (s, 1H), 8.53 (s, 1H), 7.58 (s, 1H), 7.49 (d, *J* = 8.3 Hz, 2H), 7.26 (d, *J* = 8.2 Hz, 2H), 7.12 (s, 1H), 5.84 (d, *J =* 25.6 Hz, 2H), 4.33-4.16 (m, 4H), 3.97 (s, br, 1H), 3.82 (s, 3H), 3.66 (s, 3H), 2.31 (s, 6H), 1.60-1.55 (m, 1H), 1.06-1.02 (m, 2H), 0.82-0.70 (m, 2H). | 632.6 |
| 18 0 | 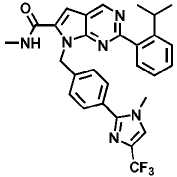 | 2-(2-isopropylpheny l)-N-methyl-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, CDCl₃) : δ 9.16 (s, 1H), 7.72 (dd, *J =* 7.7, 1.4 Hz, 1H), 7.52-7.47 (m, 2H), 7.47-7.41 (m, 2H), 7.33 (dd, *J =* 7.7, 1.6 Hz, 2H), 7.32-7.27 (m, 2H), 6.89 (s, 1H), 6.14 (d, *J* = 5.2 Hz, 1H), 6.01 (s, 2H), 3.71 (s, 3H), 3.60 (p, *J* = 6.9 Hz, 1H), 2.98 (d, *J* = 4.9 Hz, 3H), 1.23 (d, *J* = 6.9 Hz, 6H) | 533.1 |
| 18 1 | 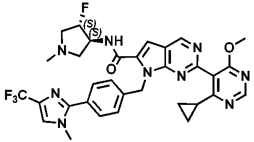 | 2-(4-cyclopropyl-6-methoxypyrimid in-5-yl)-N-((3S,4S)-4-fluoro-1-methylpyrrolid in-3-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d₆*): δ 9.37 (d, *J* = 1.1 Hz, 1H), 9.05 (d, *J* = 7.4 Hz, 1H), 8.69 (s, 1H), 7.92 (d, *J* = 1.3 Hz, 1H), 7.66-7.58 (m, 2H), 7.43 (d, *J* = 1.1 Hz, 1H), 7.28 (d, *J* = 8.1 Hz, 2H), 5.91 (s, 2H), 5.05 (dd, *J* = 54.0, 5.2 Hz, 1H), 4.48-4.34 (m, 1H), 3.85 (d, *J* = 1.1 Hz, 3H), 3.73 (s, 3H), 3.12 (t, *J* = 8.4 Hz, 1H), 2.87 (dd, *J* = 23.2, 11.4 Hz, 1H), 2.67-2.56 (m, 1H), 2.27 (s, 3H), 2.21 (dd, *J =* 9.3, 7.3 Hz, 1H), 1.65 (tt, *J* = 8.1, 4.7 Hz, 1H), 1.03 (dq, *J =* 5.6, 3.5 Hz, 2H), 0.82 (dd, *J* = 8.0, 3.2 Hz, 2H). | 650.3 |
| 18 2 | 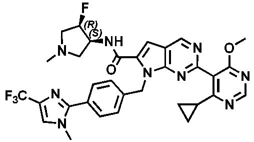 | 2-(4-cyclopropyl-6-methoxypyrimid in-5-yl)-N-((3S,4R)-4-fluoro-1-methylpyrrolid in-3-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d₆*): δ 9.36 (s, 1H), 8.95 (d, *J* = 7.6 Hz, 1H), 8.70 (s, 1H), 7.92 (q, *J* = 1.2 Hz, 1H), 7.65-7.57 (m, 2H), 7.50 (s, 1H), 7.35-7.26 (m, 2H), 5.93 (d, *J* = 1.8 Hz, 2H), 5.13 (dtd, *J* = 55.8, 5.1, 2.0 Hz, 1H), 4.57-4.42 (m, 1H), 3.86 (s, 3H), 3.73 (s, 3H), 3.05 (ddd, *J =* 30.0, 11.6, 4.7 Hz, 1H), 2.83 (dd, J = 9.1, 7.8 Hz, 1H), 2.74-2.61 (m, 2H), 2.31 (s, 3H), 1.66 (tt, J = 8.3, 4.6 Hz, 1H), 1.04 (dd, *J* = 4.7, 2.8 Hz, 2H), 0.83 (dd, *J* = 8.2, 2.9 Hz, 2H). | 650.3 |
| 18 3 | 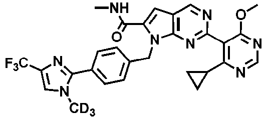 | 2-(4-cyclopropyl-6-methoxypyrimid in-5-yl)-N-methyl-7-(4-(1-(methyl-d₃)-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.34 (s, 1H), 8.83 (d, *J =* 4.7 Hz, 1H), 8.69 (s, 1H), 7.91 (q, *J* = 1.2 Hz, 1H), 7.65-7.58 (m, 2H), 7.35 (s, 1H), 7.31-7.25 (m, 2H), 5.95 (s, 2H), 3.84 (s, 3H), 2.80 (d, *J =* 4.6 Hz, 3H), 1.65 (tt, *J =* 8.3, 4.6 Hz, 1H), 1.02 (dt, *J* = 4.6, 3.0 Hz, 2H), 0.81 (dt, *J* = 8.2, 3.3 Hz, 2H). | 566.1 |
| 18 4 | 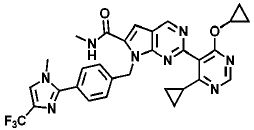 | 2-(4-cyclopropyl-6-cyclopropoxypy rimidin-5-yl)-N-methyl-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, CDCl₃) : δ 9.17 (s, 1H), 8.73 (s, 1H), 7.51-7.47 (m, 2H), 7.40-7.36 (m, 2H), 7.29 (q, *J =* 1.2 Hz, 1H), 6.90 (s, 1H), 5.99 (s, 2H), 4.34 (tt, *J* = 6.2, 3.0 Hz, 1H), 3.72 (s, 3H), 2.97 (d, *J* = 4.9 Hz, 3H), 1.71 (tt, *J* = 8.5, 4.6 Hz, 2H), 0.91-0.84 (m, 4H), 0.73 (dddd, *J* = 7.0, 6.2, 5.2, 0.8 Hz, 2H), 0.61 (ttd, *J =* 5.2, 3.1, 1.6 Hz, 2H). | 589.2 |
| 18 5 | 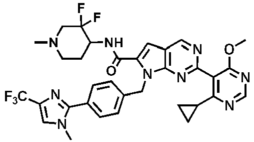 | 2-(4-cyclopropyl-6-methoxypyrimid in-5-yl)-N-(3,3-difluoro-1-methylpiperidi n-4-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d₆*): δ 9.36 (s, 1H), 8.96 (d, *J =* 8.9 Hz, 1H), 8.70 (s, 1H), 7.92 (d, *J* = 1.3 Hz, 1H), 7.64- 7.56 (m, 2H), 7.50 (s, 1H), 7.31 (d, *J* = 8.3 Hz, 2H), 5.90 (s, 2H), 4.39 (s, 1H), 3.86 (s, 3H), 3.72 (s, 3H), 3.09 (s, 1H), 2.81 (s, 1H), 2.27 (s, 5H), 1.87 (s, 1H), 1.78 (s, 1H), 1.66 (tt, *J* = 8.2, 4.6 Hz, 1H), 1.07-1.01 (m, 2H), 0.87-0.80 (m, 2H). | 682.2 |
| 18 6 | 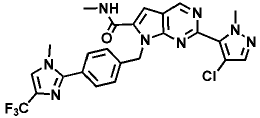 | 2-(4-chloro-1-methyl-1H-pyrazol-5-yl)-N-methyl-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.39 (s, 1H), 8.88 (q, *J =* 4.6 Hz, 1H), 7.91 (d, *J =* 1.4 Hz, 1H), 7.69 (s, 1H), 7.63 (d, *J* = 8.2 Hz, 2H), 7.38-7.30 (m, 3H), 5.99 (s, 2H), 4.09 (s, 3H), 3.73 (s, 3H), 2.81 (d, *J* = 4.5 Hz, 3H). | 529.1 |
| 18 7 | 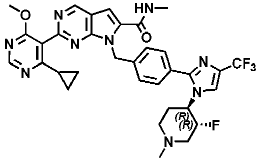 | 2-(4-cyclopropyl-6-methoxypyrimid in-5-yl)-7-(4-(1-((3R,4R)-3-fluoro-1-methylpiperidi n-4-yl)-4-(trifluorometh yl)-1H-imidazol-2-yl)benzyl)-N-methyl-7H-pyrrolo[2,3-d]pyrimidine-6-carboxamide | ¹H NMR (400 MHz, DMSO-*d*₆): δ 9.35 (s, 1H), 8.84 (q, *J =* 4.5 Hz, 1H), 8.69 (s, 1H), 8.38 (d, *J* = 1.5 Hz, 1H), 7.50- 7.43 (m, 2H), 7.36 (s, 1H), 7.29 (d, *J* = 8.2 Hz, 2H), 5.96 (s, 2H), 4.94 (dtd, *J* = 50.4, 9.7, 5.1 Hz, 1H), 4.16-4.05 (m, 1H), 3.84 (s, 3H), 3.20-3.13 (m, 1H), 2.80 (d, *J =* 4.5 Hz, 3H), 2.79-2.70 (m, 1H), 2.23 (s, 3H), 2.10-1.92 (m, 4H), 1.65 (tt, *J =* 8.3, 4.6 Hz, 1H), 1.02(p, *J* = 3.4 Hz, 2H), 0.80 (dd, *J* = 8.1, 3.2 Hz, 2H). | 664.3 |
| 18 8 | 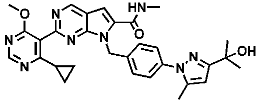 | 2-(4-cyclopropyl-6-methoxypyrimid in-5-yl)-7-(4-(3-(2-hydroxypropan-2-yl)-5-methyl-1H-pyrazol-1-yl)benzyl)-N-methyl-7H-pyrrolo[2,3-d]pyrimidine-6-carboxamide | ¹H NMR (400 MHz, CDCl₃): δ 9.19 (s, 1H), 8.68 (s, 1H), 7.47-7.43 (m, 2H), 7.32-7.28 (m, 2H), 6.90 (s, 1H), 6.18 (d, *J* = 4.7 Hz, 1H), 6.11 (s, 1H), 6.00 (s, 2H), 3.95 (s, 3H), 3.02 (d, *J* = 4.9 Hz, 3H), 2.65 (s, 1H), 2.27 (s, 3H), 1.75-1.70 (m, 1H), 1.56 (s, 6H), 1.23 (dd, *J* = 4.6, 2.8 Hz, 2H), 0.87 (dd, *J = 8.1,* 2.9 Hz, 2H). | 553.2 |
| 18 9 | 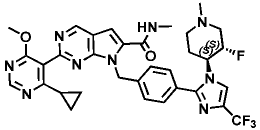 | 2-(4-cyclopropyl-6-methoxypyrimid in-5-yl)-7-(4-(1-((3S,4S)-3-fluoro-1-methylpiperidi n-4-yl)-4-(trifluorometh yl)-1H-imidazol-2-yl)benzyl) -N-methyl-7H-pyrrolo [2,3-d]pyrimidine-6-carboxamide | ¹H NMR (400 MHz, DMSO-*d₆*): δ 9.35 (s, 1H), 8.84 (q, *J* = 4.5 Hz, 1H), 8.69 (s, 1H), 8.38 (d, *J* = 1.4 Hz, 1H), 7.51-7.43 (m, 2H), 7.36 (s, 1H), 7.32-7.26 (m, 2H), 5.96 (s, 2H), 4.94 (dtd, *J* = 50.3, 9.8, 5.0 Hz, 1H), 4.10 (dt, *J* = 18.7, 9.7 Hz, 1H), 3.84 (s, 3H), 3.21 - 3.14 (m, 1H), 2.83-2.73 (m, 4H), 2.23 (s, 3H), 2.10-1.94 (m, 4H), 1.65 (tt, *J =* 8.2, 4.6 Hz, 1H), 1.05-0.99 (m, 2H), 0.80 (dt, *J =* 8.3, 3.3 Hz, 2H). | 664.3 |
| 19 0 | 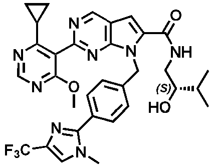 | (S)-2-(4-cyclopropyl-6-methoxypyrimid in-5-yl)-N-(2-hydroxy-3-methylbutyl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, CDCl₃): δ 9.19 (s, 1H), 8.68 (s, 1H), 7.48-7.45 (m, 2H), 7.35-7.31 (m, 2H), 7.29 (q, *J* = 1.2 Hz, 1H), 6.95 (s, 1H), 6.35 (d, *J* = 9.1 Hz, 1H), 6.10 (d, *J* = 14.9 Hz, 1H), 5.84 (d, *J =* 14.9 Hz, 1H), 3.95 (s, 3H), 3.77 (dq, J = 8.7, 4.7, 4.3 Hz, 1H), 3.72 (s, 3H), 3.68 (dd, *J* = 10.8, 4.4 Hz, 1H), 3.43 (dd, *J =* 10.8, 3.3 Hz, 1H), 1.99-1.90 (m, 1H), 1.73 (tt, *J =* 8.3, 4.6 Hz, 2H), 1.24 (dd, *J* = 4.6, 2.6 Hz, 2H), 0.98 (dd, *J* = 10.3, 6.7 Hz, 6H), 0.90-0.87 (m, 2H). | 635.2 |
| 19 1 | 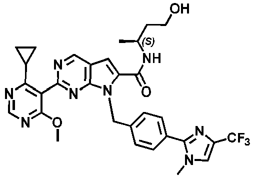 | (S)-2-(4-cyclopropyl-6-methoxypyrimid in-5-yl)-N-(4-hydroxybutan-2-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, CDCl₃) : δ 9.20 (s, 1H), 8.68 (s, 1H), 7.50-7.47 (m, 2H), 7.33 (d, *J* = 8.2 Hz, 2H), 7.29 (d, *J* = 1.2 Hz, 1H), 6.92 (s, 1H), 6.25 (d, *J* = 8.4 Hz, 1H), 6.11 (d, *J* = 14.8 Hz, 1H), 5.88 (d, *J* = 14.8 Hz, 1H), 4.26 (d, *J* = 9.1 Hz, 1H), 3.95 (s, 3H), 3.73 (s, 3H), 3.42 (d, *J* = 5.8 Hz, 3H), 1.86 (d, *J* = 9.1 Hz, 1H), 1.76-1.72 (m, 1H), 1.43 (d, *J =* 4.2 Hz, 1H), 1.29 (d, *J* = 6.7 Hz, 3H), 1.26-1.23 (m, 2H), 0.89 (dd, *J* = 7.9, 2.8 Hz, 2H). | 621.2 |
| 19 2 | 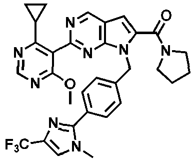 | (2- (4-cyclopropyl-6-methoxypyrimid in-5-yl)-7-(4-(1-methyl-4-(trifluorometh yl)-1H-imidazol-2-yl)benzyl)-7H-pyrrolo[2,3-d]pyrimidin-6-yl) (pyrrolidin -1-yl)methanone | ¹H NMR (400 MHz, CDCl₃) : δ 9.18 (s, 1H), 8.67 (s, 1H), 7.54-7.48 (m, 2H), 7.34 (d, *J* = 8.2 Hz, 2H), 7.31-7.28 (m, 1H), 6.78 (s, 1H), 5.84 (s, 2H), 3.95 (s, 3H), 3.72 (s, 3H), 3.57 (t, *J* = 7.0 Hz, 2H), 3.31 (t, *J* = 6.7 Hz, 2H), 1.95-1.85 (m, 2H), 1.73 (q, *J* = 6.7, 6.3 Hz, 3H), 1.26-1.22 (m, 2H), 0.91-0.85 (m, 2H). | 603.2 |
| 19 3 | 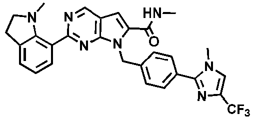 | N-methyl-7-(4-(1-methyl-4-(trifluorometh yl)-1H-imidazol-2-yl)benzyl)-2-(1-methylindolin-7-yl)-7H-pyrrolo[2,3-d]pyrimidine-6-carboxamide | ¹H NMR (400 MHz, DMSO-*d₆*): δ 9.23-9.19 (m, 1H), 8.76 (dd, *J* = 8.9, 4.2 Hz, 1H), 7.87 (d, *J* = 1.0 Hz, 1H), 7.57 (s, 2H), 7.32-7.30 (m, 1H), 7.27 (s, 1H), 7.20 (d, *J* = 8.3 Hz, 2H), 7.08-7.06 (m, 1H), 6.66 (s, 1H), 5.91 (s, 2H), 3.69 (s, 3H), 2.74 (d, *J* = 4.6 Hz, 3H), 2.23 (s, 3H), 2.00-1.88 (m, 2H), 0.91-0.75 (m, 2H). | 546.2 |
| 19 4 | 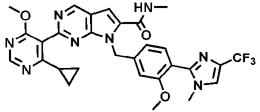 | 2-(4-cyclopropyl-6-methoxypyrimid in-5-yl)-7-(3-methoxy-4-(1-methyl-4-(trifluorometh yl)-1H-imidazol-2-yl)benzyl)-N-methyl-7H-pyrrolo[2,3-d]pyrimidine-6-carboxamide | ¹H NMR (400 MHz, DMSO-*d₆*): δ 9.34 (s, 1H), 8.87 (q, *J* = 4.4 Hz, 1H), 8.69 (s, 1H), 7.88 (d, *J* = 1.1 Hz, 1H), 7.34 (s, 1H), 7.22 (d, *J* = 7.8 Hz, 1H), 7.10 (s, 1H), 6.65 (d, *J* = 7.9 Hz, 1H), 5.95 (s, 2H), 3.82 (s, 3H), 3.71 (s, 3H), 3.44 (s, 3H), 2.82 (d, *J =* 4.6 Hz, 3H), 1.62-1.58 (m, 1H), 1.04-1.00 (m, 2H), 0.82-0.78 (m, 2H). | 593.2 |
| 19 5 | 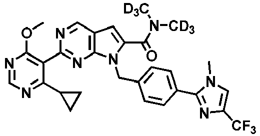 | 2-(4-cyclopropyl-6-methoxypyrimid in-5-yl)-N,N-bis(methyl-d₃)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.26 (s, 1H), 8.69 (s, 1H), 7.93 (d, *J* = 1.1 Hz, 1H), 7.66 (d, *J* = 8.4 Hz, 2H), 7.24 (d, *J* = 8.4 Hz, 2H), 7.01 (s, 1H), 5.61 (s, 2H), 3.85 (s, 3H), 3.73 (s, 3H), 2.04-1.98 (m, 1H), 1.06-1.02 (m, 2H), 0.86-0.83 (m, *J =* 4.8, 3.3 Hz, 2H). | 583.3 |
| 19 6 | 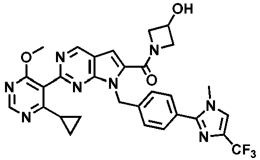 | (2- (4-cyclopropyl-6-methoxypyrimid in-5-yl)-7-(4-(1-methyl-4-(trifluorometh yl)-1H-imidazol-2-yl)benzyl)-7H-pyrrolo[2,3-d]pyrimidin-6-yl) (3-hydroxyazetidi n-1-yl)methanone | ¹H NMR (400 MHz, DMSO-*d₆*): δ 9.29 (s, 1H), 8.69 (s, 1H), 7.92 (d, *J* = 1.1 Hz, 1H), 7.64 (d, *J* = 8.4 Hz, 2H), 7.26 (d, *J* = 8.4 Hz, 2H), 7.20 (s, 1H), 5.86-5.83 (m, 1H), 5.83 (s, 2H), 4.51-4.42 (m, 2H), 4.30-4.22 (m, 1H), 4.08 (d, *J* = 5.6 Hz, 1H), 3.84 (s, 3H), 3.81-3.76 (m, 1H), 3.74 (s, 3H), 2.04-1.96 (m, 1H), 1.04-1.01 (m, 2H), 0.83-0.79 (m, 2H) . | 605.3 |
| 19 7 | 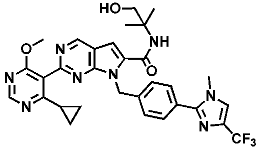 | 2-(4-cyclopropyl-6-methoxypyrimid in-5-yl)-N-(1-hydroxy-2-methylpropan-2-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d₆*): δ 9.30 (s, 1H), 8.69 (s, 1H), 7.94 (d, *J =* 18.1 Hz, 2H), 7.62 (d, *J* = 8.3 Hz, 2H), 7.32 (d, *J* = 8.3 Hz, 2H), 7.28 (s, 1H), 5.87 (s, 2H), 4.88 (t, *J* = 5.9 Hz, 1H), 3.86 (s, 3H), 3.73 (s, 3H), 3.52-3.50 (m, 2H), 1.67-1.62 (m, 1H), 1.31 (s, 6H), 1.07-1.02 (m, 2H), 0.86-0.82 (m, 2H). | 621.3 |
| 19 8 | 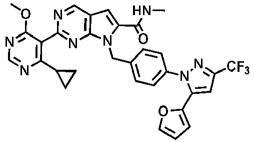 | 2-(4-cyclopropyl-6-methoxypyrimid in-5-yl)-7-(4-(5- (furan-2-yl)-3-(trifluorometh yl)-1H-pyrazol-1-yl)benzyl)-N-methyl-7H-pyrrolo[2,3-d]pyrimidine-6-carboxamide | ¹H NMR (400 MHz, DMSO-d₆): δ 9.35 (s, 1H), 8.84 (d, *J* = 4.7 Hz, 1H), 8.69 (s, 1H), 7.76 (dd, *J* = 1.7, 0.6 Hz, 1H), 7.43-7.39 (m, 2H), 7.36 (s, 1H), 7.29 (d, *J* = 8.5 Hz, 2H), 7.25 (s, 1H), 6.57-6.46 (m, 1H), 6.24-6.14 (m, *J =* 3.5, 0.5 Hz, 1H), 5.97 (s, 2H), 3.82 (s, 3H), 2.80(d, *J* = 4.6 Hz, 3H), 1.65-1.62 (m, 1H), 1.04-1.00 (m, 2H), 0.82-0.77 (m, 2H). | 615.2 |
| 19 9 | 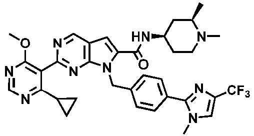 | 2-(4-cyclopropyl-6-methoxypyrimid in-5-yl)-N-((2R,4R)-1,2-dimethylpiperi din-4-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d₆*): δ 9.32 (s, 1H), 8.69 (d, *J =* 6.9 Hz, 1H), 8.68 (s, 1H), 7.91 (d, *J* = 1.3 Hz, 1H), 7.64-7.56 (m, 2H), 7.34 (s, 1H), 7.31-7.24 (m, 2H), 5.90 (s, 2H), 3.84 (s, 3H), 3.79 (s, 1H), 3.72 (s, 3H), 2.83 (s, 1H), 2.18 (s, 3H), 1.75 (s, 2H), 1.64 (dq, J *=* 8.0, 4.0, 3.5 Hz, 1H), 1.55 (ddd, *J =* 24.6, 11.5, 7.6 Hz, 2H), 1.23 (s, 1H), 1.03 (dd, *J* = 5.2, 2.6 Hz, 5H), 0.82 (dt, *J* = 8.3, 3.3 Hz, 3H). | 660.3 |
| 20 0 | 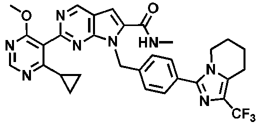 | 2-(4-cyclopropyl-6-methoxypyrimid in-5-yl)-N-methyl-7-(4-(1-(trifluorometh yl) -5, 6, 7, 8-tetrahydroimid azo[1,5-a]pyridin-3-yl)benzyl)-7H-pyrrolo[2,3-d]pyrimidine-6-carboxamide | ¹H NMR (400 MHz, Methanol-d₄): δ 9.21 (s, 1H), 8.62 (s, 1H), 7.58-7.49 (m, 2H), 7.40-7.31 (m, 2H), 7.25 (s, 1H), 6.01 (s, 2H), 4.05-3.98 (m, 2H), 3.92 (s, 3H), 2.98-2.93 (m, 2H), 2.90 (s, 3H), 1.95-1.86 (m, 4H), 1.67 (td, *J =* 8.2, 4.2 Hz, 1H), 1.18-1.09 (m, 2H), 0.89-0.81 (m, 2H). | 603.2 |
| 20 1 | 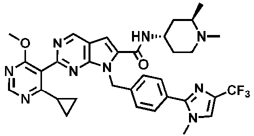 | 2-(4-cyclopropyl-6-methoxypyrimid in-5-yl)-N-((2R,4S)-1,2-dimethylpiperi din-4-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.33 (s, 1H), 8.69 (s, 1H), 8.54 (s, 1H), 7.91 (d, *J =* 1.3 Hz, 1H), 7.65-7.57 (m, 2H), 7.39 (s, 1H), 7.31-7.24 (m, 2H), 5.88 (s, 2H), 3.85 (s, 3H), 3.72 (s, 3H), 2.52 (d, *J* = 1.8 Hz, 4H), 2.24 (s, 2H), 1.90 (s, 2H), 1.64 (tt, *J* = 8.2, 4.6 Hz, 3H), 1.03 (td, *J* = 10.0, 8.7, 5.1 Hz, 5H), 0.82 (tt, *J* = 6.8, 3.3 Hz, 3H). | 660.3 |
| 20 2 | 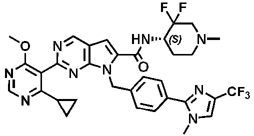 | (S)-2-(4-cyclopropyl-6-methoxypyrimid in-5-yl)-N-(3,3-difluoro-1-methylpiperidi n-4-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d₆*): δ 9.35 (s, 1H), 8.95 (d, *J =* 9.0 Hz, 1H), 8.69 (s, 1H), 7.91 (d, *J* = 1.4 Hz, 1H), 7.63- 7.55 (m, 2H), 7.49 (s, 1H), 7.33-7.27 (m, 2H), 5.90 (s, 2H), 3.85 (s, 3H), 3.71 (s, 3H), 3.08 (d, *J* = 10.5 Hz, 1H), 2.80 (d, *J =* 11.4 Hz, 1H), 2.25 (s, 3H), 2.16 (t, *J =* 11.5 Hz, 2H), 1.94-1.80 (m, 2H), 1.65 (tt, *J =* 8.2, 4.6 Hz, 2H), 1.03 (q, *J* = 3.3 Hz, 2H), 0.82 (dd, *J =* 7.8, 3.4 Hz, 2H) . | 682.3 |
| 20 3 | 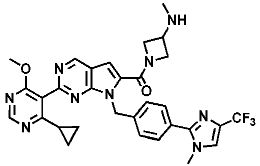 | (2- (4-cyclopropyl-6-methoxypyrimid in-5-yl)-7-(4-(1-methyl-4-(trifluorometh yl)-1H-imidazol-2-yl)benzyl)-7H-pyrrolo[2,3-d]pyrimidin-6-yl) (3-(methylamino)a zetidin-1-yl)methanone | ¹H NMR (400 MHz, DMSO-*d*₆): δ 9.29 (s, 1H), 8.68 (s, 1H), 7.91 (d, *J* = 1.3 Hz, 1H), 7.63 (dd, *J* = 8.3, 6.4 Hz, 2H), 7.28-7.22 (m, 2H), 7.18 (s, 1H), 5.82 (s, 2H), 4.36 (t, *J* = 8.2 Hz, 1H), 4.19-4.10 (m, 1H), 3.98 (dd, *J* = 9. 0, 4.9 Hz, 1H), 3.83 (s, 3H), 3.73 (s, 3H), 3.74-3.68 (m, 1H), 3.46 (p, *J* = 6.7 Hz, 1H), 2.18 (s, 3H), 1.63 (dt, *J* = 8.2, 4.6 Hz, 1H), 1.02 (p, *J* = 3.6 Hz, 2H), 0.80 (dq, *J* = 6.8, 3.4 Hz, 2H). | 618.3 |
| 20 4 | 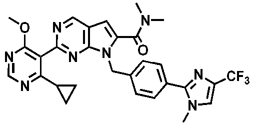 | 2-(4-cyclopropyl-6-methoxypyrimid in-5-yl)-N,N-dimethyl-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.25 (s, 1H), 8.69 (s, 1H), 7.92 (d, *J* = 1.4 Hz, 1H), 7.68-7.61 (m, 2H), 7.24 (d, *J =* 8.2 Hz, 2H), 7.00 (s, 1H), 5.60 (s, 2H), 3.85 (s, 3H), 3.73 (s, 3H), 2.97 (s, 3H), 2.84 (s, 3H), 1.67 (tt, *J =* 8.2, 4.7 Hz, 1H), 1.03 (p, *J* = 3.4 Hz, 2H), 0.86-0.81 (m, 2H). | 577.2 |
| 20 5 | 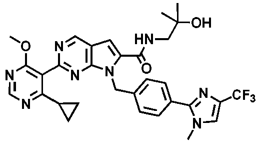 | 2-(4-cyclopropyl-6-methoxypyrimid in-5-yl)-N-(2-hydroxy-2-methylpropyl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.33 (s, 1H), 8.68 (s, 1H), 8.66 (t, *J* = 6.3 Hz, 1H), 7.91 (q, *J* = 1.3 Hz, 1H), 7.63-7.55 (m, 2H), 7.44 (s, 1H), 7.31-7.25 (m, 2H), 5.92 (s, 2H), 4.55 (s, 1H), 3.84 (s, 3H), 3.71 (s, 3H), 3.25 (d, *J* = 6.3 Hz, 2H), 1.65(tt, *J =* 8.2, 4.7 Hz, 1H), 1.06 (s, 6H), 1.03 (ddd, *J =* 7.6, 4.8, 1.5 Hz, 2H), 0.82 (dt, *J* = 8.2, 3.3 Hz, 2H). | 621.3 |
| 20 6 | 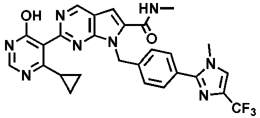 | 2-(4-cyclopropyl-6-hydroxypyrimid in-5-yl)-N-methyl-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d₆*): δ 9.28 (s, 1H), 8.81 (d, *J* = 4.7 Hz, 1H), 8.11 (s, 1H), 7.91 (d, *J* = 1.1 Hz, 1H), 7.60 (d, *J* = 8.3 Hz, 2H), 7.32 (s, 1H), 7.26 (d, *J* = 8.3 Hz, 2H), 5.94 (s, 2H), 3.73 (s, 3H), 2.79 (d, *J* = 4.6 Hz, 3H), 1.47-1.44 (m, 1H), 0.93 (d, *J =* 4.3 Hz, 2H), 0.71-0.66 (m, 2H). | 549.2 |
| 20 7 | 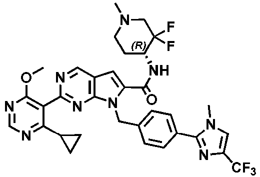 | (R) -2- (4-cyclopropyl-6-methoxypyrimid in-5-yl)-N-(3,3-difluoro-1-methylpiperidi n-4-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d*₆): δ 9.37 (s, 1H), 9.06 (d, *J* = 8.6 Hz, 1H), 8.70 (s, 1H), 7.92 (d, *J* = 1.1 Hz, 1H), 7.62-7.59 (m, 2H), 7.52 (s, 1H), 7.31 (d, *J* = 8.4 Hz, 2H), 5.91 (s, 2H), 4.59 (s, 1H), 3.86 (s, 3H), 3.72 (s, 3H), 2.69 (s, 1H), 2.56 (d, J = 5.5 Hz, 2H), 2.53-2.48 (m, 3H), 2.00 (d, *J* = 15.0 Hz, 2H), 1.69-1.63 (m, 1H), 1.25 (d, *J* = 9.7 Hz, 1H), 1.08-1.02 (m, 2H), 0.86-0.80 (m, 2H). | 682.3 |
| 20 8 | 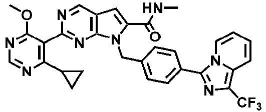 | 2-(4-cyclopropyl-6-methoxypyrimid in-5-yl)-N-methyl-7-(4-(1-(trifluorometh yl)imidazol[1,5 -a]pyridin-3-yl)benzyl)-7H-pyrrolo[2,3-d]pyrimidine-6-carboxamide | ¹H NMR (400 MHz, CDCl₃) : δ 9.20 (d, *J* = 3.0 Hz, 1H), 8.67 (d, *J* = 2.9 Hz, 1H), 8.19 (dq, *J* = 7.5, 2.0, 1.6 Hz, 1H), 7.68 (ddt, *J =* 9.3, 2.5, 1.3 Hz, 1H), 7.66-7.61 (m, 2H), 7.49 (dd, *J =* 8.5, 2.7 Hz, 2H), 6.99 (dd, *J* = 9.3, 6.5 Hz, 1H), 6.91 (d, *J* = 2.9 Hz, 1H), 6.70 (ddt, *J* = 8.5, 6.5, 1.7 Hz, 1H), 6.17 (d, *J* = 5.7 Hz, 1H), 6.03 (d, *J* = 2.7 Hz, 2H), 3.94 (d, *J* = 2.6 Hz, 3H), 3.00 (dd, *J =* 5.1, 2.8 Hz, 3H), 1.74 (dp, *J* = 7.8, 4.4 Hz, 1H), 1.25-1.21 (m, 2H), 0.90-0.84 (m, 2H). | 599.2 |
| 20 9 | 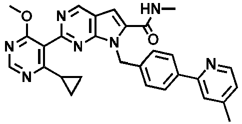 | 2-(4-cyclopropyl-6-methoxypyrimid in-5-yl)-N-methyl-7-(4-(4-methylpyridin-2-yl)benzyl)-7H-pyrrolo[2,3-d]pyrimidine-6-carboxamide | ¹H NMR (400 MHz, DMSO-*d*₆): δ 9.33 (s, 1H), 8.82 (d, *J =* 4.8 Hz, 1H), 8.69 (s, 1H), 8.48 (dd, *J* = 4.9, 0.8 Hz, 1H), 7.99-7.92 (m, 2H), 7.76-7.71 (m, 1H), 7.32 (s, 1H), 7.29-7.22 (m, 2H), 7.16 (ddd, *J* = 5.0, 1.6, 0.8 Hz, 1H), 5.93 (s, 2H), 3.85 (s, 3H), 2.81 (d, *J* = 4.6 Hz, 3H), 2.37 (s, 3H), 1.65 (tt, *J =* 8.3, 4.6 Hz, 1H), 1.02 (dq, *J =* 6.1, 3.5 Hz, 2H), 0.80 (dt, *J* = 8.2, 3.3 Hz, 2H). | 506.2 |
| 21 0 | 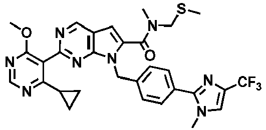 | 2-(4-cyclopropyl-6-methoxypyrimid in-5-yl)-N-methyl-7-(4-(1-methyl-4-(trifluorometh yl)-1H-imidazol-2-yl)benzyl)-N-((methylthio)m ethyl)-7H-pyrrolo[2,3-d]pyrimidine-6-carboxamide | ¹H NMR (400 MHz, DMSO-*d₆*): δ 9.30 (s, 1H), 8.69 (s, 1H), 7.93 (q, *J* = 1.3 Hz, 1H), 7.66-7.61 (m, 2H), 7.29-7.06 (m, 3H), 5.66 (s, 2H), 4.60 (d, *J* = 48.7 Hz, 2H), 3.85 (s, 3H), 3.73 (s, 3H), 3.03 (s, 3H), 2.02 (s, 2H), 1.86 (s, 1H), 1.67 (s, 1H), 1.03 (dt, *J* = 4.5, 2.9 Hz, 2H), 0.83 (dq, *J* = 7.0, 3.4 Hz, 2H). | 623.2 |
| 21 1 | 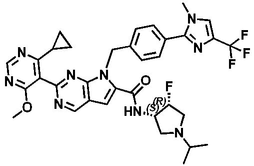 | 2-(4-cyclopropyl-6-methoxypyrimid in-5-yl)-N-((3S,4R)-4-fluoro-1-isopropylpyrro lidin-3-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, CDCl₃): δ 9.21 (s, 1H), 8.67 (s, 1H), 7.51-7.47 (m, 2H), 7.42-7.39 (m, 2H), 7.28 (q, *J* = 1.2 Hz, 1H), 7.01 (s, 1H), 6.67 (dd, *J =* 8.6, 2.4 Hz, 1H), 6.00 (d, *J =* 1.7 Hz, 2H), 5.14 (dddd, *J* = 55.5, 5.7, 4.2, 1.9 Hz, 1H), 4.74-4.60 (m, 1H), 3.94 (s, 3H), 3.71 (s, 3H), 3.05-2.93 (m, 3H), 2.68-2.62 (m, 1H), 2.52 (p, *J* = 6.3 Hz, 1H), 1.72 (tt, *J* = 8.3, 4.6 Hz, 1H), 1.25-1.20 (m, 2H), 1.09 (dd, *J* = 6.3, 4.8 Hz, 6H), 0.89-0.84 (m, 2H). | 678.3 |
| 21 2 | 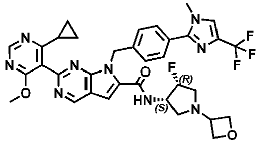 | 2-(4-cyclopropyl-6-methoxypyrimid in-5-yl)-N-((3S,4R)-4-fluoro-1-(oxetan-3-yl)pyrrolidin-3-yl)-7-(4-(1-methyl-4-(trifluorometh yl)-1H-imidazol-2-yl)benzyl)-7H-pyrrolo [2,3-d]pyrimidine-6-carboxamide | ¹H NMR (400 MHz, CDCl₃): δ 9.22 (s, 1H), 8.67 (s, 1H), 7.52-7.48 (m, 2H), 7.40-7.35 (m, 2H), 7.29 (q, *J* = 1.2 Hz, 1H), 7.07 (s, 1H), 6.71 (d, *J* = 8.4 Hz, 1H), 6.05-5.95 (m, 2H), 5.28-5.09 (m, 1H), 4.72 (q, *J* = 8.1, 7.4 Hz, 3H), 4.62 (dd, *J* = 6.7, 5.5 Hz, 2H), 3.94 (s, 3H), 3.87 (p, *J* = 6.1 Hz, 1H), 3.72 (s, 3H), 3.21-3.10 (m, 1H), 3.09-3.05 (m, 1H), 3.05-2.95 (m, 1H), 2.62 (t, *J* = 8.5 Hz, 1H), 1.73-1.69 (m, 1H), 1.25-1.19 (m, 2H), 0.86 (dq, *J =* 6.8, 3.6 Hz, 2H). | 692.2 |
| 21 3 | 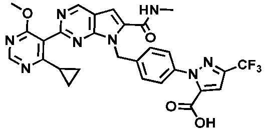 | 1-(4-((2-(4-cyclopropyl-6-methoxypyrimid in-5-yl)-6-(methylcarbamo yl)-7H-pyrrolo[2,3-d]pyrimidin-7-yl)methyl) phen yl)-3-(trifluorometh yl)-1H-pyrazol-5-carboxylic acid | ¹H NMR (400 MHz, Methanol-*d*₄): δ 9.11 (s, 1H), 8.52 (s, 1H), 7.23 (s, 4H), 7.15 (s, 1H), 6.80 (s, 1H), 5.92 (s, 2H), 3.83 (s, 3H), 2.81 (s, 3H), 1.88-1.85 (m, 1H), 1.09-1.07 (m, 2H), 0.94-0.89 (m, 2H). | 593.2 |
| 21 4 | 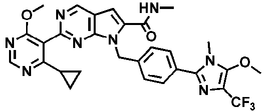 | 2-(4-cyclopropyl-6-methoxypyrimid in-5-yl)-7-(4-(5-methoxy-1-methyl-4-(trifluorometh yl)-1H-imidazol-2-yl)benzyl)-N-methyl-7H-pyrrolo[2,3-d]pyrimidine-6-carboxamide | ¹H NMR (400 MHz, DMSO-*d*₆): δ 9.60 (s, 1H), 8.69 (s, 1H), 8.03 (q, *J* = 4.4 Hz, 1H), 7.91 (s, 1H), 7.60 (d, *J* = 8.2 Hz, 2H), 7.25 (d, *J* = 8.2 Hz, 2H), 5.94 (s, 2H), 4.33 (s, 3H), 3.85 (s, 3H), 3.73 (s, 3H), 2.83 (d, *J* = 4.7 Hz, 3H), 2.04-1.99 (m, 1H), 1.05-1.01 (m, 2H), 0.84-0.80 (m, 3.2 Hz, 2H). | 593.3 |
| 21 5 | 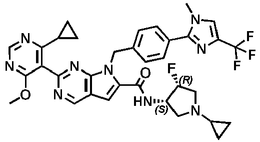 | N-((3S,4R)-1-cyclopropyl-4-fluoropyrrolid in-3-yl)-2-(4-cyclopropyl-6-methoxypyrimid in-5-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, CDCl₃): δ 9.20 (s, 1H), 8.67 (s, 1H), 7.52-7.48 (m, 2H), 7.43-7.39 (m, 2H), 7.28 (d, *J* = 1.3 Hz, 1H), 7.01 (s, 1H), 6.61 (dd, *J =* 8.6, 2.3 Hz, 1H), 6.00 (s, 2H), 5.21-5.03 (m, 1H), 4.70-4.57 (m, 1H), 3.94 (s, 3H), 3.72 (s, 3H), 3.18-2.99 (m, 3H), 2.72 (dd, *J* = 9.4, 7.5 Hz, 1H), 1.74 (ddd, *J* = 15.6, 8.1, 3.6 Hz, 2H), 1.22 (dt, *J* = 4.5, 3.1 Hz, 2H), 0.87 (ddd, *J* = 8.0, 3.9, 2.2 Hz, 2H), 0.44 (ddt, *J* =14.7, 6.3, 2.4 Hz, 4H). | 676.3 |
| 21 6 | 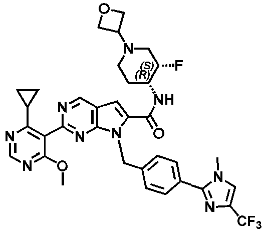 | 2-(4-cyclopropyl-6-methoxypyrimid in-5-yl)-N-((3S,4R)-3-fluoro-1-(oxetan-3-yl)piperidin-4-yl)-7-(4-(1-methyl-4-(trifluorometh yl)-1H-imidazol-2-yl)benzyl) -7H-pyrrolo[2,3-d]pyrimidine-6-carboxamide | ¹H NMR (400 MHz, DMSO-*d*₆): δ 9.35 (s, 1H), 8.89 (d, *J* = 7.5 Hz, 1H), 8.69 (s, 1H), 7.92 (d, *J* = 1.0 Hz, 1H), 7.61 (d, *J* = 8.2 Hz, 2H), 7.48 (s, 1H), 7.29 (d, *J* = 8.3 Hz, 2H), 5.91 (s, 2H), 4.56-4.51 (m, 2H), 4.45 (t, *J* = 6.1 Hz, 1H), 4.39 (t, *J* = 6.1 Hz, 1H), 3.86 (s, 3H), 3.72 (s, 3H), 3.52-3.45 (m, 1H), 2.97 (t, *J =* 10.2 Hz, 2H), 2.76 (s, 1H), 2.21 (d, *J* = 12.5 Hz, 1H), 2.11 (d, *J* = 11.5 Hz, 1H), 1.97 (d, *J =* 14.5 Hz, 2H), 1.68-1.63 (m, 2H), 1.04 (s, 2H), 0.85-0.81 (m, 2H). | 706.4 |
| 21 7 | 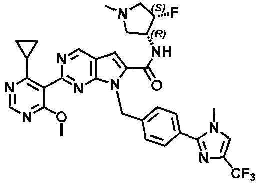 | 2-(4-cyclopropyl-6-methoxypyrimid in-5-yl)-N-((3R,4S)-4-fluoro-1-methylpyrrolid in-3-yl)-7-(4-(1-methyl-4-(trifluorometh yl)-1H-imidazol-2-yl)benzyl) -7H-pyrrolo[2,3-d]pyrimidine-6-carboxamide | ¹H NMR (400 MHz, DMSO-*d₆*): δ 9.35 (s, 1H), 8.94 (d, *J* = 7.6 Hz, 1H), 8.69 (d, J = 5.0 Hz, 1H), 7.91 (d, *J* = 1.2 Hz, 1H), 7.62-7.59 (m, 2H), 7.49 (s, 1H), 7.30 (d, *J* = 8.4 Hz, 2H), 5.92 (s, 2H), 5.21-5.04 (m, 1H), 4.56-4.45 (m, 1H), 3.85 (s, 3H), 3.73 (s, 3H), 3.10-2.99 (m, 1H), 2.83 (t, *J* = 8.5 Hz, 1H), 2.73-2.62 (m, 2H), 2.31 (s, 3H), 1.68-1.62 (m, 1H), 1.06-1.01 (m, 2H), 0.85-0.80 (m, 2H). | 650.3 |
| 21 8 | 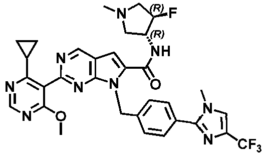 | 2-(4-cyclopropyl-6-methoxypyrimid in-5-yl) -N-((3R,4R)-4-fluoro-1-methylpyrrolid in-3-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d₆*): δ 9.36 (s, 1H), 9.07 (d, *J* = 7.4 Hz, 1H), 8.69 (s, 1H), 7.91 (d, *J* = 1.1 Hz, 1H), 7.64-7.61 (m, 2H), 7.43 (s, 1H), 7.29 (d, *J* = 8.4 Hz, 2H), 5.92 (s, 2H), 5.13-4.98 (m, 1H), 4.47-4.37 (m, 1H), 3.85 (s, 3H), 3.74 (s, 3H), 3.15-3.10 (m, 1H), 2.92-2.83 (m, 1H), 2.70-2.59 (m, 1H), 2.27 (s, 3H), 2.25-2.21 (m, 1H), 1.69-1.63 (m, 1H), 1.06-1.02 (m, 2H), 0.84-0.79 (m, 2H). | 650.3 |
| 21 9 | 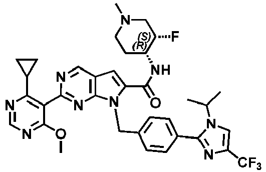 | 2-(4-cyclopropyl-6-methoxypyrimid in-5-yl)-N-((3S,4R)-3-fluoro-1-methylpiperidi n-4-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d₆*): δ 9.39-9.32 (m, 1H), 9.00 (d, *J* = 6.9 Hz, 1H), 8.68 (d, *J* = 4.0 Hz, 1H), 8.15 (s, 1H), 7.51-7.44 (m, 3H), 7.30 (d, *J* = 8.0 Hz, 2H), 5.92 (s, 2H), 4.99 (d, *J =* 48.2 Hz, 1H), 4.42-4.35 (m, 1H), 4.19 (d, *J* = 30.7 Hz, 1H), 3.85 (s, 3H), 2.63 (s, 2H), 2.51 (s, 3H), 2.12 (d, *J* = 13.0 Hz, 1H), 1.83 (d, *J* = 11.7 Hz, 2H), 1.69-1.63 (m, 1H), 1.39-1.35 (m, 6H), 1.24 (s, 1H), 1.04 (s, 2H), 0.84-0.76 (m, 2H) . | 692.3 |
| 22 0 | 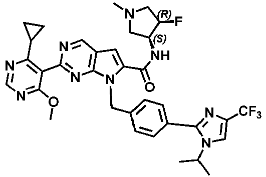 | 2-(4-cyclopropyl-6-methoxypyrimid in-5-yl)-N-((3S,4R)-4-fluoro-1-methylpyrrolid in-3-yl)-7-(4-(1-isopropyl-4-(trifluorometh yl)-1H-imidazol-2-yl)benzyl)-7H-pyrrolo[2,3-d]pyrimidine-6-carboxamide | ¹H NMR (400 MHz, DMSO-*d₆*): δ 9.41-9.29 (m, 1H), 8.93 (d, *J* = 7.5 Hz, 1H), 8.68 (s, 1H), 8.15 (s, 1H), 7.49 (s, 1H), 7.45 (d, *J* = 8.2 Hz, 2H), 7.30 (d, *J* = 8.2 Hz, 2H), 5.93 (s, 2H), 4.57-4.41 (m, 2H), 4.41-4.35 (m, 1H), 3.17 (s, 1H), 2.83 (t, *J* = 8.4 Hz, 1H), 2.72-2.61 (m, 2H), 2.52-2.50 (m, 3H), 2.31 (s, 3H), 1.68-1.62 (m, 1H), 1.37 (m, 6H), 1.03 (d, *J =* 2.4 Hz, 2H), 0.86-0.76 (m, 2H). | 678.2 |
| 22 1 | 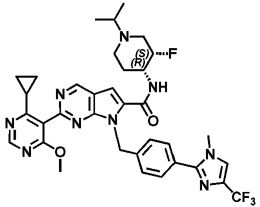 | 2-(4-cyclopropyl-6-methoxypyrimid in-5-yl)-N-((3S,4R)-3-fluoro-1-isopropylpiper idin-4-yl)-7-(4-(1-methyl-4-(trifluorometh yl)-1H-imidazol-2-yl)benzyl)-7H-pyrrolo[2,3-d]pyrimidine-6-carboxamide | ¹H NMR (400 MHz, DMSO-*d₆*): δ 9.34 (s, 1H), 8.84 (d, *J* = 7.4 Hz, 1H), 8.69 (s, 1H), 7.91 (s, 1H), 7.61 (d, *J* = 8.3 Hz, 2H), 7.46 (s, 1H), 7.29 (d, *J* = 8.3 Hz, 2H), 5.91 (s, 2H), 3.85 (s, 3H), 3.72 (s, 3H), 1.80 (s, 6H), 1.05-1.02 (m, 6H), 0.97-0.95 (m, 4H), 0.84-0.82 (m, 4H). | 692.3 |
| 22 2 | 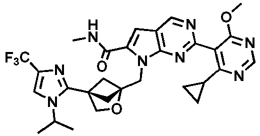 | 2-(4-cyclopropyl-6-methoxypyrimid in-5-yl)-7-(((1s,4s)-4-(1-isopropyl-4-(trifluorometh yl)-1H-imidazol-2-yl)-2-oxabicyclo[2.1 .1]hexan-1-yl)methyl)-N-methyl-7H-pyrrolo[2,3-d]pyrimidine-6-carboxamide | | 597.2 |
| 22 3 | 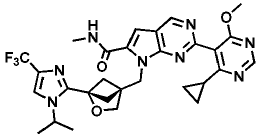 | 2-(4-cyclopropyl-6-methoxypyrimid in-5-yl)-7-(((1s,4s)-1-(1-isopropyl-4-(trifluorometh yl)-1H-imidazol-2-yl)-2-oxabicyclo[2.1 .1]hexan-4-yl)methyl)-N-methyl-7H-pyrrolo[2,3-d]pyrimidine-6-carboxamide | | 597.2 |
| 22 4 | 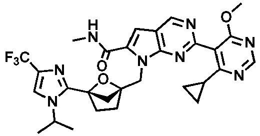 | 2-(4-cyclopropyl-6-methoxypyrimid in-5-yl)-7-(((1R,4S)-4-(1-isopropyl-4-(trifluorometh yl)-1H-imidazol-2-yl)-5-oxabicyclo[2.1 .1]hexan-1-yl)methyl)-N-methyl-7H-pyrrolo[2,3-d]pyrimidine-6-carboxamide | | 597.2 |
| 22 5 | 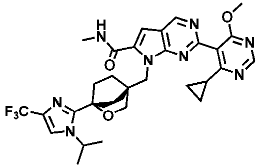 | 2-(4-cyclopropyl-6-methoxypyrimid in-5-yl)-7-(((1s,4s)-1-(1-isopropyl-4-(trifluoromethyl)-1H-imidazol-2-yl)-2-oxabicyclo[2.2 .2]octan-4-yl)methyl)-N-methyl-7H-pyrrolo[2,3-d]pyrimidine-6-carboxamide | | 625.3 |
| 22 6 | 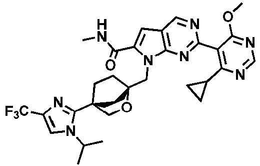 | 2-(4-cyclopropyl-6-methoxypyrimid in-5-yl)-7-(((1s,4s)-4-(1-isopropyl-4-(trifluorometh yl)-1H-imidazol-2-yl)-2-oxabicyclo[2.2 .2]octan-1-yl)methyl)-N-methyl-7H-pyrrolo[2,3-d]pyrimidine-6-carboxamide | ¹H NMR (400 MHz, DMSO-*d*₆): δ 9.25 (s, 1H), 8.71 (d, *J* = 5.1 Hz, 2H), 7.97 (d, *J* = 1.4 Hz, 1H), 7.07 (s, 1H), 4.80 (p, *J* = 6.7 Hz, 1H), 4.70 (s, 2H), 3.92 (s, 2H), 3.84 (s, 3H), 2.83 (d, *J* = 4.5 Hz, 3H), 2.10 (s, 2H), 1.81 (s, 2H), 1.72-1.52 (m, 5H), 1.33 (d, *J* = 6.4 Hz, 6H), 1.06 (p, *J* = 3.4 Hz, 2H), 0.89-0.85 (m, 2H). | 625.3 |

### <Experimental Example 1> Confirmation and evaluation of the binding affinity to recombinant USP1 protein

To evaluate the binding affinity of the compounds of the present invention to the USP1 protein, the following experiment was performed using differential scanning fluorimetry.

Recombinant 0.03 mg/mL USP1 (Δ1-66, Δ125-138, Δ229-408, Δ608-737) protein, dye reagent, and buffer solution (50 mM Tris-HCl pH 8.0, 150 mM NaCl, 2% glycerol, 1 mM DTT) were mixed and 19 µL of the mixture was dispensed into a well plate (MicroAmp Fast 96-Well Reaction plate, 0.1 mL). Each compound was injected at a final concentration of 100 µM in an amount of 1 µL, and the plate containing a total of 20 µL was sealed with MicroAmp Optical Adhesive Film. The melting temperature (Tm) of the protein in each well was measured using a real-time PCR (RT-PCR) device. The difference between the Tm of the sample containing the compound and the Tm containing DMSO is referred to as delta Tm (ΔTm), where a larger ΔTm value indicates stronger binding affinity. The degree of temperature at which the protein is modified in the presence of the compound compared to DMSO was calculated according to Equation 1 below: 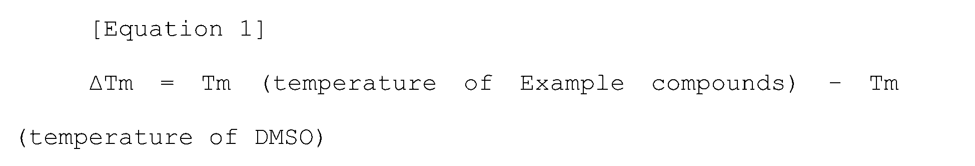 ΔTm = Tm (temperature of Example compounds) - Tm (temperature of DMSO)

The above ΔTm values have a significant correlation with cell activity, and results thereof are shown in Table 2 below.

**[Table 2]**

| Examp le compo und | ΔTm (°C) | Exam ple comp ound | ΔTm ( C) | Exam ple comp ound | ΔTm ( C) | Exam ple comp ound | ΔTm ( C) | Exam ple comp ound | ΔTm ( C) |
|---|---|---|---|---|---|---|---|---|---|
| 1 | A | 46 | A | 91 | A | 136A | A | 181 | A |
| 2 | A | 47 | A | 91 | A | 137 | A | 182 | A |
| 3 | A | 48 | A | 93 | A | 138 | A | 183 | A |
| 4 | A | 49 | A | 94 | A | 139 | A | 184 | A |
| 5 | A | 50 | A | 95 | A | 140 | A | 185 | A |
| 6 | A | 51 | A | 96 | A | 141 | A | 186 | A |
| 7 | A | 52 | A | 97 | A | 142 | A | 187 | A |
| 8 | A | 53 | A | 98 | A | 143 | A | 188 | A |
| 9 | A | 54 | A | 99 | A | 144 | A | 189 | A |
| 10 | A | 55 | A | 100 | A | 145 | A | 190 | A |
| 11 | A | 56 | A | 101 | A | 146 | A | 191 | A |
| 12 | A | 57 | A | 102 | A | 147 | A | 192 | A |
| 13 | A | 58 | A | 103 | A | 14R | A | 193 | B |
| 14 | A | 59 | R | 104 | A | 149 | A | 194 | A |
| 15 | A | 60 | A | 105 | A | 150 | A | 195 | A |
| 16 | A | 61 | A | 106 | A | 151 | A | 196 | A |
| 17 | A | 62 | B | 107 | A | 152 | A | 197 | A |
| 18 | A | 63 | A | 108 | A | 153 | A | 198 | A |
| 19 | A | 64 | A | 109 | A | 154 | A | 199 | A |
| 20 | A | 65 | A | 110 | A | 155 | A | 200 | A |
| 21 | A | 66 | A | 111 | A | 156 | A | 201 | A |
| 22 | A | 67 | A | 112 | A | 157 | A | 202 | A |
| 23 | A | 68 | A | 113 | A | 158 | A | 203 | A |
| 24 | A | 69 | A | 114 | A | 150 | A | 204 | A |
| 25 | A | 70 | A | 115 | A | 160 | A | 205 | A |
| 26 | A | 71 | A | 116 | A | 161 | A | 206 | R |
| 27 | A | 72 | A | 117 | A | 162 | A | 207 | A |
| 28 | A | 73 | A | 118 | A | 163 | A | 208 | A |
| 29 | A | 74 | A | 119 | A | 164 | A | 209 | A |
| 30 | A | 75 | A | 120 | A | 165 | A | 210 | A |
| 31 | A | 76 | A | 121 | A | 166 | A | 211 | A |
| 32 | A | 77 | A | 122 | A | 167 | A | 212 | A |
| 33 | A | 78 | A | 123 | A | 168 | A | 213 | R |
| 34 | A | 79 | A | 124 | A | 169 | A | 214 | A |
| 35 | A | 80 | A | 125 | A | 170 | A | 215 | A |
| 36 | A | 81 | A | 126 | A | 171 | A | 216 | A |
| 37 | A | 82 | A | 127 | A | 172 | A | 217 | A |
| 38 | A | 83 | A | 128 | A | 173 | A | 218 | A |
| 39 | A | R4 | A | 129 | A | 174 | A | 219 | A |
| 40 | A | 85 | A | 130 | A | 175 | A | 220 | A |
| 41 | A | 86 | A | 131 | A | 176 | A | 221 | A |
| 42 | A | 87 | A | 132 | A | 177 | A | 226 | B |
| 43 | A | RR | A | 133 | A | 178 | A | | |
| 44 | B | 89 | A | 134 | A | 179 | A | | |
| 45 | A | 90 | A | 135 | A | 180 | A | | |
| A: **ΔTm** (°C) > 5 °C; B: 5 °C ≥ ΔTm (°C) | | | | | | | | | |

### <Experimental Example 2> Evaluation of Cell Proliferation Inhibitory Activity in BRCA1/2-Mutant or HRD-Positive Cells

To evaluate the inhibitory activity of the compound according to the present invention on the proliferation of BRCA1/2 mutants or homologous recombination deficient (HRD) positive cell lines, the following experiments were performed. UWB1.289 cells were cultured in medium (50% MEBM + 50% RPMI1640), and 1,000 cells were seeded in a well plate (black clear bottom 96-well plate, Corning) 16 to 24 hours prior to compound treatment. The compounds were diluted in dimethyl sulfoxide (3-fold serial dilution starting at 10 µM, for a total of 10 concentrations) and injected at 1 µl per well, resulting a final concentration range of 1.5 nM to 10 µM. Measurements of live cells were performed by a reaction in a 37 °C and CO₂ incubator for 144 hours after compound treatment. The cells were incubated for 10 minutes in an incubator using Cell Titer-Glo luminescent cell-viability reagent (Promega), and luminescence intensity thereof was measured using a reader (SynergyNeo2, Biotek). GI₅₀ values were calculated using the GraphPad Prism version 8.4.3 (GraphPad software Inc., San Diego) program. The results are shown in Table 3 below.

**[Table 3]**

| Examp le Compo und | UWB1.2 89 BRCA1 mut | Exam ple Comp ound | UWB1.2 89 BRCA1 mut | Exam ple Comp ound | UWB1.2 89 BRCA1 mut | Exam ple Comp ound | UWB1.2 89 BRCA1 mut |
|---|---|---|---|---|---|---|---|
| 1 | A | 57 | A | 122 | B | 174 | A |
| 2 | A | 58 | A | 123 | B | 175 | A |
| 3 | A | 60 | A | 124 | A | 176 | B |
| 4 | A | 61 | A | 125 | A | 177 | A |
| 5 | A | 63 | A | 126 | A | 178 | A |
| 6 | A | 64 | B | 127 | A | 179 | A |
| 7 | A | 65 | B | 128 | A | 180 | A |
| R | A | 66 | A | 129 | A | 181 | A |
| 9 | B | 67 | A | 130 | A | 182 | A |
| 10 | A | 68 | A | 131 | A | 183 | A |
| 11 | A | 69 | A | 132 | A | 184 | A |
| 12 | B | 70 | B | 133 | A | 185 | A |
| 13 | A | 71 | A | 134 | A | 186 | A |
| 14 | A | 73 | R | 135 | A | 187 | A |
| 15 | A | 75 | R | 136 | A | 188 | B |
| 17 | A | 78 | B | 137 | A | 189 | A |
| 18 | A | 79 | B | 138 | A | 190 | A |
| 19 | A | 80 | B | 139 | A | 191 | A |
| 20 | R | R1 | A | 140 | A | 109 | A |
| 21 | A | 82 | A | 141 | A | 193 | A |
| 22 | A | 83 | A | 142 | A | 194 | R |
| 23 | A | 84 | B | 143 | B | 195 | R |
| 24 | A | 85 | B | 144 | A | 196 | A |
| 25 | A | 86 | A | 145 | R | 197 | A |
| 26 | A | 87 | B | 146 | A | 198 | R |
| 28 | A | 89 | R | 147 | A | 199 | A |
| 29 | A | 90 | A | 148 | A | 200 | A |
| 30 | A | 92 | A | 149 | A | 201 | A |
| 31 | R | 94 | B | 150 | A | 202 | A |
| 32 | A | 95 | B | 151 | A | 203 | A |
| 33 | A | 97 | B | 152 | B | 204 | A |
| 34 | B | 99 | A | 153 | A | 205 | A |
| 35 | A | 100 | B | 154 | A | 207 | A |
| 36 | B | 101 | B | 155 | A | 208 | A |
| 37 | A | 102 | A | 156 | A | 209 | A |
| 38 | A | 104 | A | 157 | A | 210 | A |
| 39 | B | 105 | B | 158 | A | 211 | A |
| 40 | B | 106 | A | 159 | B | 212 | A |
| 41 | A | 108 | A | 161 | A | 214 | A |
| 42 | B | 109 | A | 162 | A | 215 | A |
| 43 | A | 110 | R | 163 | A | 216 | B |
| 45 | A | 111 | B | 164 | A | 217 | A |
| 47 | A | 112 | A | 165 | A | 218 | A |
| 48 | A | 113 | A | 166 | A | 219 | A |
| 49 | B | 114 | A | 167 | A | 220 | A |
| 50 | B | 115 | A | 168 | A | 221 | A |
| 51 | A | 116 | A | 169 | A | | |
| 52 | B | 117 | A | 170 | B | | |
| 53 | A | 118 | A | 171 | A | | |
| 55 | B | 119 | A | 172 | A | | |
| 56 | B | 121 | A | 173 | A | | |
| **A: GI₅₀ ≤ 0.1 µM; B: 0.1 µM < GI₅₀ ≤ 0.5 µM** | | | | | | | |

As shown in Tables 2 and 3 above, it can be seen that the compounds according to Examples of the present invention exhibited high inhibitory activity against the USP1 protein and high inhibitory activity against cells having BRCA1 mutation/deficiency, BRCA2 mutation/deficiency, or HR deficiency.

As described above, the present invention has been explained in detail through preferred Examples and Experimental Examples, but the scope of the present invention is not limited to the specific compounds according to Examples of the present invention and should be interpreted based on the attached claims. Further, those skilled in the art should understand that various modifications and variations can be made without departing from the scope of the present invention.

## Claims

1. A compound represented by the following Chemical Formula 1, a tautomer thereof, a stereoisomer thereof, or a pharmaceutically acceptable salt thereof: 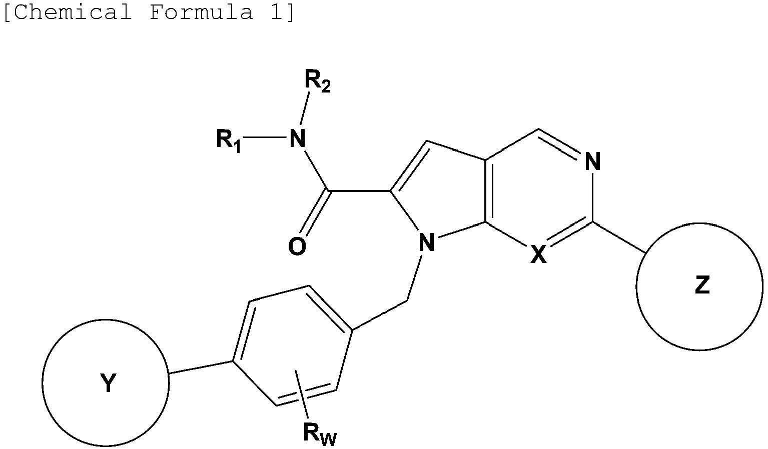
in Chemical Formula 1 above,
X is CH or N;
R_{w} is -H, -C₁₋₆alkyl, -C₁₋₆haloalkyl, -O-C₁₋₆alkyl, or - halo;
R₁ and R₂ are -H, -C₁₋₆alkyl, -C₁₋₆alkenyl, -C₁₋₆alkynyl, -C₁₋₆cyanoalkyl, -C₁₋₆aminoalkyl, -C₁₋₆hydroxyalkyl, -C₁₋₆haloalkyl, -OH, -C₁₋₆alkyl-O-C₁₋₆alkyl, -C₁₋₆alkyl-S-C₁₋₆alkyl, -C₁₋₆alkyl-C(=O) -C₁₋₆alkyl, -C₁₋₆alkyl-C(=O) -OC₁₋₆alkyl, -C₁₋₆alkyl-S(=O) ₂-C₁₋₆alkyl, - (CH₂)n- (3-6 membered cycloalkyl), - (CH₂)n-(4-6 membered heterocycloalkyl), -(CH₂)n-aryl, or - (CH₂)n-(5-6 membered heteroaryl), wherein at least one H of the -C₁₋₆alkyl, -C₁₋₆alkenyl, or -C₁₋₆alkynyl may be substituted with deuterium, and at least one H of the - (CH₂)n- (3-6 membered cycloalkyl), - (CH₂)n- (4-6 membered heterocycloalkyl), -(CH₂)n-aryl, or -(CH₂)n-(5-6 membered heteroaryl) ring may be substituted with -C₁₋₆alkyl, -C₁₋₆aminoalkyl, -C₁₋₆hydroxyalkyl, -C₁₋₆haloalkyl, -halo, -NRₐR_{b}, -O-R_{c}, -C₁₋₆alkyl-O-C₁₋₆alkyl, - (CH₂)n-S (=O)₂-C₁₋₆alkyl, - (CH₂)n-(3-6 membered cycloalkyl), or -(CH₂)n-(4-6 membered heterocycloalkyl), wherein at least one H of the -(CH₂)n-(3-6 membered cycloalkyl) or -(CH₂)n-(4-6 membered heterocycloalkyl) ring may be substituted with -C₁₋₆alkyl, - C₁₋₆haloalkyl, or -halo; or
the R₁ and R₂ may be linked to each other to form a 4-12 membered ring together with the N atom, wherein at least one H of the 4-12 membered ring may be substituted with -C₁₋₆alkyl, -C₁₋₆haloalkyl, -C₁₋₆alkyl-O-C₁₋₆alkyl, -(CH₂)n-S(=O)₂-C₁₋₆alkyl, -CN, -NRₐR_{b}, -O-R_{c}, -halo, -(3-6 membered cycloalkyl), or -(4-6 membered heterocycloalkyl), and - (CH₂)- of the 4-12 membered ring may be substituted with - C(=O)- or -S(=O)₂-;
Rₐ and R_{b} are -H or -C₁₋₆alkyl;
R_{c} is -H or -C₁₋₆alkyl;
n is 0, 1, 2, 3, or 4;
ring Z is -phenyl, -(5-10 membered heteroaryl), or -(5-10 membered heterohydroaryl), wherein the -(5-10 membered heteroaryl) or -(5-10 membered heterohydroaryl) ring comprises one or more N atoms, and at least one H of the - phenyl, -(5-10 membered heteroaryl), or -(5-10 membered heterohydroaryl) ring may be substituted with -C₁₋₆alkyl, - OH, -O-C₁₋₆alkyl, -O-C₁₋₆haloalkyl, -halo, -O-(3-6 membered cycloalkyl), or -(3-6 membered cycloalkyl), wherein at least one H of the -C₁₋₆alkyl or -O-C₁₋₆alkyl may be substituted with deuterium, and -(CH₂)- of the -(5-10 membered heterohydroaryl) ring may be substituted with -C(=O)-; and
ring Y is -(5-10 membered heteroaryl) or -(5-10 membered heterohydroaryl), wherein the -(5-10 membered heteroaryl) or -(5-10 membered heterohydroaryl) ring comprises one or more N atoms, and at least one H of the -(5-10 membered heteroaryl) or -(5-10 membered heterohydroaryl) ring may be substituted with -C₁₋₆alkyl, -C₁₋₆hydroxyalkyl, -C₁₋₆haloalkyl, -O-C₁₋₆alkyl, -COOH, -(3-6 membered cycloalkyl), -(4-6 membered heterocycloalkyl), or -(5-6 membered heteroaryl), wherein at least one H of the -C₁₋₆alkyl or -O-C₁₋₆alkyl may be substituted with deuterium, and at least one H of the -(3-6 membered cycloalkyl), -(4-6 membered heterocycloalkyl), -(5-6 membered heteroaryl) ring may be substituted with -C₁₋₆alkyl, -C₁₋₆haloalkyl, or -halo.

2. The compound represented by Chemical Formula 1, the tautomer thereof, the stereoisomer thereof, or the pharmaceutically acceptable salt thereof of claim 1, wherein X is N.

3. The compound represented by Chemical Formula 1, the tautomer thereof, the stereoisomer thereof, or the pharmaceutically acceptable salt thereof of claim 1, wherein
R₁ and R₂ are -H, -C₁₋₆alkyl, -C₁₋₆alkenyl, -C₁₋₆alkynyl, -C₁₋₆cyanoalkyl, -C₁₋₆aminoalkyl, -C₁₋₆hydroxyalkyl, -C₁₋₆haloalkyl, -OH, -C₁₋₆alkyl-O-C₁₋₆alkyl, -C₁₋₆alkyl-S-C₁₋₆alkyl, -C₁₋₆alkyl-C(=O) -C₁₋₆alkyl, -C₁₋₆alkyl-C(=O) -OC₁₋₆alkyl, -C₁₋₆alkyl-S(=O) ₂-C₁₋₆alkyl, - (CH₂)n- (3-6 membered cycloalkyl), - (CH₂)n-(4-6 membered heterocycloalkyl), -(CH₂)n-phenyl, - (CH₂)n-naphthalenyl, or -(CH₂)n-(5-6 membered heteroaryl), wherein at least one H of the -C₁₋₆alkyl, -C₁₋₆alkenyl, or - C₁₋₆alkynyl may be substituted with deuterium, and -(CH₂)- of the - (CH₂)n- (3-6 membered cycloalkyl) or - (CH₂)n- (4-6 membered heterocycloalkyl) ring may be substituted with - S(=O)₂-, and at least one H of the -(CH₂)n-(3-6 membered cycloalkyl), -(CH₂)n-(4-6 membered heterocycloalkyl), - (CH₂)n-phenyl, -(CH₂)n-naphthalenyl, or -(CH₂)n-(5-6 membered heteroaryl) ring may be substituted with -C₁₋₆alkyl, -C₁₋₆aminoalkyl, -C₁₋₆hydroxyalkyl, -C₁₋₆haloalkyl, -halo, -NRₐR_{b}, -O-R_{c}, -C₁₋₆alkyl-O-C₁₋₆alkyl, - (CH₂)n-S (=O)₂-C₁₋₆alkyl, - (CH₂)n-(3-6 membered cycloalkyl), or -(CH₂)n-(4-6 membered heterocycloalkyl), wherein at least one H of the -(CH₂)n-(3-6 membered cycloalkyl) or -(CH₂)n-(4-6 membered heterocycloalkyl) ring may be substituted with -C₁₋₆alkyl, - C₁₋₆haloalkyl, or -halo; or
the R₁ and R₂ may be linked to each other to form a - (4-6 membered heterocycloalkyl) or -(6-12 membered heterobicycloalkyl) ring together with the N atom, wherein the -(6-12 membered heterobicycloalkyl) ring is a fused ring, a spiro ring, or a bridged ring, and at least one H of the -(4-6 membered heterocycloalkyl), or -(6-12 membered heterobicycloalkyl) ring may be substituted with -C₁₋₆alkyl, -C₁₋₆haloalkyl, -C₁₋₆alkyl-O-C₁₋₆alkyl, -(CH₂)n-S(=O)₂-C₁₋₆alkyl, -CN, -NRₐR_{b}, -O-R_{c}, -halo, -(3-6 membered cycloalkyl), or - (4-6 membered heterocycloalkyl), and -(CH₂)- of the -(4-6 membered heterocycloalkyl) or -(6-12 membered heterobicycloalkyl) ring may be substituted with -S(=O)₂-;
Rₐ and R_{b} are -H or -C₁₋₆alkyl;
R_{c} is -H or -C₁₋₆alkyl; and
n is 0, 1, or 2.

4. The compound represented by Chemical Formula 1, the tautomer thereof, the stereoisomer thereof, or the pharmaceutically acceptable salt thereof of claim 1, wherein the ring Z is -phenyl, 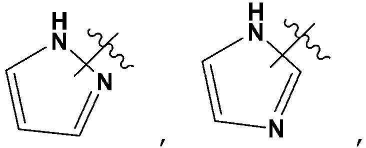 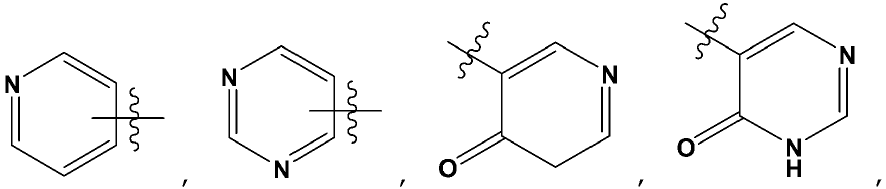 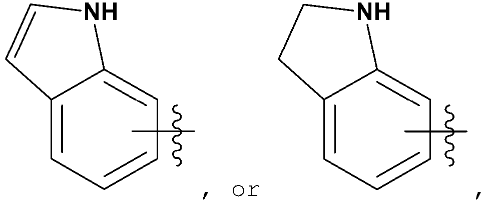 wherein at least one H of the ring Z may be substituted with -C₁₋₆alkyl, -OH, -O-C₁₋₆alkyl, -O-C₁₋₆haloalkyl, -halo, -O-(3-6 membered cycloalkyl), or -(3-6 membered cycloalkyl), wherein at least one H of the -C₁₋₆alkyl or -O-C₁₋₆alkyl may be substituted with deuterium, and -(CH₂)- of the -(5-10 membered heterohydroaryl) ring may be substituted with -C(=O)-.

5. The compound represented by Chemical Formula 1, the tautomer thereof, the stereoisomer thereof, or the pharmaceutically acceptable salt thereof of claim 1, wherein
the ring Y is 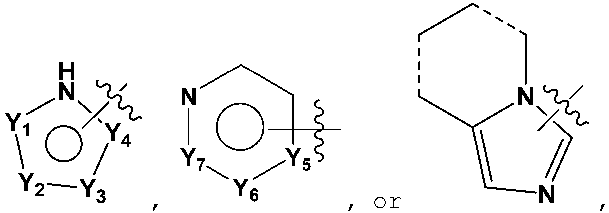 wherein at least one H of the ring Y may be substituted with -C₁₋₆alkyl, -C₁₋₆hydroxyalkyl, -C₁₋₆haloalkyl, -O-C₁₋₆alkyl, - COOH, -(3-6 membered cycloalkyl), -(4-6 membered heterocycloalkyl), or -(5-6 membered heteroaryl), wherein at least one H of the -C₁₋₆alkyl or -O-C₁₋₆alkyl may be substituted with deuterium, and at least one H of the -(3-6 membered cycloalkyl), -(4-6 membered heterocycloalkyl), -(5-6 membered heteroaryl) ring may be substituted with -C₁₋₆alkyl, -C₁₋₆haloalkyl, or -halo;
Y₁ to Y₇ are CH₂, CH, C, NH, N, O, or S; and
----- is a single bond or a double bond.

6. The compound represented by Chemical Formula 1, the tautomer thereof, the stereoisomer thereof, or the pharmaceutically acceptable salt thereof of claim 5, wherein the ring Y is 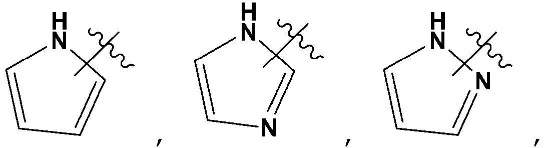 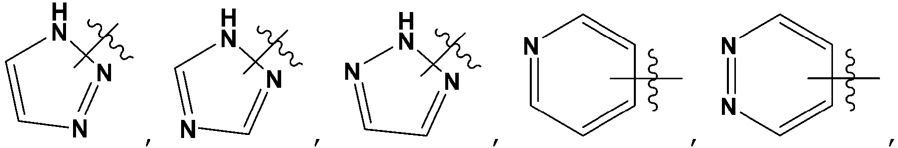 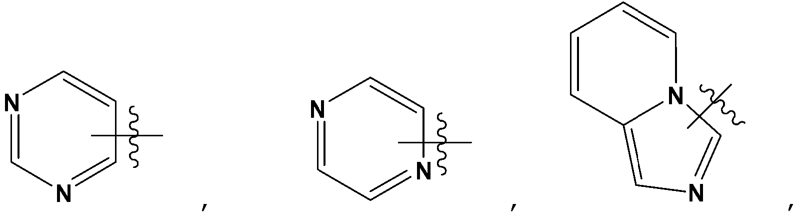 or 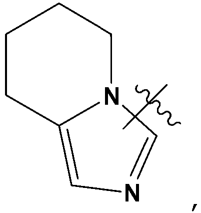 wherein at least one H of the ring Y may be substituted with -C₁₋₆alkyl, -C₁₋₆hydroxyalkyl, -C₁₋₆haloalkyl, -O-C₁₋₆alkyl, -COOH, -(3-6 membered cycloalkyl), -(4-6 membered heterocycloalkyl), or -(5-6 membered heteroaryl), wherein at least one H of the -C₁₋₆alkyl or -O-C₁₋₆alkyl may be substituted with deuterium, and at least one H of the - (3-6 membered cycloalkyl), -(4-6 membered heterocycloalkyl), -(5-6 membered heteroaryl) ring may be substituted with -C₁₋₆alkyl, -C₁₋₆haloalkyl, or -halo.

7. A compound selected from the group consisting of the following compounds, a tautomer thereof, a stereoisomer thereof, or a pharmaceutically acceptable salt thereof:
| | |
|---|---|
| 1 | 2-(4-cyclopropyl-6-methoxypyrimidin-5-yl)-7-(4-(1-isopropyl-4-(trifluoromethyl)-1H-imidazol-2-yl)benzyl)-N-methyl-7H-pyrrolo[2,3-d]pyrimidine-6-carboxamide |
| 2 | 7-(4-(1-isopropyl-4-(trifluoromethyl)-1H-imidazol-2-yl)benzyl)-2-(2-isopropylphenyl)-N-methyl-7H-pyrrolo[2,3-d]pyrimidine-6-carboxamide |
| 3 | 2-(4-cyclopropyl-6-(methoxy-d₃)pyrimidin-5-yl)-7-(4-(1-isopropyl-4-(trifluoromethyl)-1H-imidazol-2-yl)benzyl)-N-methyl-7H-pyrrolo[2,3-d]pyrimidine-6-carboxamide |
| 4 | 2-(4-cyclopropyl-6-methoxypyrimidin-5-yl)-N-ethyl-7-(4-(1-isopropyl-4-(trifluoromethyl)-1H-imidazol-2-yl)benzyl)-7H-pyrrolo[2,3-d]pyrimidine-6-carboxamide |
| 5 | 2-(4-cyclopropyl-6-methoxypyrimidin-5-yl)-7-(4-(1-isopropyl-4-(trifluoromethyl)-1H-imidazol-2-yl)benzyl)-N,N-dimethyl-7H-pyrrolo[2,3-d]pyrimidine-6-carboxamide |
| 6 | 2-(4-cyclopropyl-6-methoxypyrimidin-5-yl)-7-(4-(1-isopropyl-4-(trifluoromethyl)-1H-imidazol-2-yl)benzyl)-N-(2-morpholinoethyl)-7H-pyrrolo[2,3-d]pyrimidine-6-carboxamide |
| 7 | 2-(4-cyclopropyl-6-methoxypyrimidin-5-yl)-N-((1-(hydroxymethyl)cyclopropyl)methyl)-7-(4-(1-isopropyl-4-(trifluoromethyl)-1H-imidazol-2-yl)benzyl)-7H-pyrrolo[2,3-d]pyrimidine-6-carboxamide |
| 8 | 2-(4-cyclopropyl-6-methoxypyrimidin-5-yl)-7-(4-(1-isopropyl-4-(trifluoromethyl)-1H-imidazol-2-yl)benzyl)-N-(2-(methylsulfonyl)ethyl)-7H-pyrrolo[2,3-d]pyrimidine-6-carboxamide |
| 9 | 2-(4-cyclopropyl-6-methoxypyrimidin-5-yl)-7-(4-(1-isopropyl-4-(trifluoromethyl)-1H-imidazol-2-yl)benzyl)-N-(methyl-d₃)-7H-pyrrolo[2,3-d]pyrimidine-6-carboxamide |
| 10 | 2-(4-cyclopropyl-6-methoxypyrimidin-5-yl)-N-(2,2-difluorocyclopropyl)-7-(4-(1-isopropyl-4-(trifluoromethyl)-1H-imidazol-2-yl)benzyl)-7H-pyrrolo[2,3-d]pyrimidine-6-carboxamide |
| 11 | N-cyclopropyl-2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carboxamide |
| 12 | 2-(4-cyclopropyl-6-methoxypyrimidin-5-yl)-N-(2-(dimethylamino)ethyl)-7-(4-(1-isopropyl-4-(trifluoromethyl)-1H-imidazol-2-yl)benzyl)-7H-pyrrolo[2,3-d]pyrimidine-6-carboxamide |
| 13 | 2-(4-cyclopropyl-6-methoxypyrimidin-5-yl)-7-(4-(1-isopropyl-4-(trifluoromethyl)-1H-imidazol-2-yl)benzyl)-N-(oxetan-3-yl)-7H-pyrrolo[2,3-d]pyrimidine-6-carboxamide |
| 14 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(4-morpholinopiperidin-1-yl)methanone |
| 15 | N-cyclobutyl-2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carboxamide |
| 16 | (3,8-diazabicyclo[3.2.1]octan-3-yl)(2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)methanone |
| 17 | 2-(4-cyclopropyl-6-methoxypyrimidin-5-yl)-7-(4-(1-isopropyl-4-(trifluoromethyl)-1H-imidazol-2-yl)benzyl)-N-phenyl-7H-pyrrolo[2,3-d]pyrimidine-6-carboxamide |
| 18 | azetidin-1-yl(2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)methanone |
| 19 | N-(cyclobutylmethyl)-2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carboxamide |
| 20 | 2-(4-cyclopropyl-6-methoxypyrimidin-5-yl)-N-((3,3-difluoro-1-methylcyclobutyl)methyl)-7-(4-(1-isopropyl-4-(trifluoromethyl)-1H-imidazol-2-yl)benzyl)-7H-pyrrolo[2,3-d]pyrimidine-6-carboxamide |
| 21 | 2-(4-cyclopropyl-6-methoxypyrimidin-5-yl)-N-(2-fluoroethyl)-7-(4-(1-isopropyl-4-(trifluoromethyl)-1H-imidazol-2-yl)benzyl)-7H-pyrrolo[2,3-d]pyrimidine-6-carboxamide |
| 22 | N-(cyanomethyl)-2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carboxamide |
| 23 | (S)-2-(4-cyclopropyl-6-methoxypyrimidin-5-yl)-7-(4-(1-isopropyl-4-(trifluoromethyl)-1H-imidazol-2-yl)benzyl)-N-(1-methoxypropan-2-yl)-7H-pyrrolo[2,3-d]pyrimidine-6-carboxamide |
| 24 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(4-methylpiperazin-1-yl)methanone |
| 25 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl) (4-(oxetan-3-yl)piperazin-1-yl)methanone |
| 26 | 2-(4-cyclopropyl-6-methoxypyrimidin-5-yl)-7-(4-(1-isopropyl-4-(trifluoromethyl)-1H-imidazol-2-yl)benzyl)-N-methyl-N-(2-(methylamino)ethyl)-7H-pyrrolo[2,3-d]pyrimidine-6-carboxamide |
| 27 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(1,1-dioxidothiomorpholino)methanone |
| 28 | 2-(4-cyclopropyl-6-methoxypyrimidin-5-yl)-7-(4-(1-isopropyl-4-(trifluoromethyl)-1H-imidazol-2-yl)benzyl)-N-(naphthalen-1-yl)-7H-pyrrolo[2,3-d]pyrimidine-6-carboxamide |
| 29 | 2-(4-cyclopropyl-6-methoxypyrimidin-5-yl)-7-(4-(1-isopropyl-4-(trifluoromethyl)-1H-imidazol-2-yl)benzyl)-N-(2-oxopropyl)-7H-pyrrolo[2,3-d]pyrimidine-6-carboxamide |
| 30 | 2-(4-cyclopropyl-6-methoxypyrimidin-5-yl)-7-(4-(1-isopropyl-4-(trifluoromethyl)-1H-imidazol-2-yl)benzyl)-N-(1-methyl-1H-pyrazol-4-yl)-7H-pyrrolo[2,3-d]pyrimidine-6-carboxamide |
| 31 | 2-(4-cyclopropyl-6-methoxypyrimidin-5-yl)-7-(4-(1-isopropyl-4-(trifluoromethyl)-1H-imidazol-2-yl)benzyl)-N-(thiazol-2-yl)-7H-pyrrolo[2,3-d]pyrimidine-6-carboxamide |
| 32 | 2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carboxamide |
| 33 | 2-(4-cyclopropyl-6-methoxypyrimidin-5-yl)-N-(3-(dimethylamino)propyl)-7-(4-(1-isopropyl-4-(trifluoromethyl)-1H-imidazol-2-yl)benzyl)-7H-pyrrolo[2,3-d]pyrimidine-6-carboxamide |
| 34 | 2-(4-cyclopropyl-6-methoxypyrimidin-5-yl)-7-(4-(1-isopropyl-4-(trifluoromethyl)-1H-imidazol-2-yl)benzyl)-N-(3,3,3-trifluoropropyl)-7H-pyrrolo[2,3-d]pyrimidine-6-carboxamide |
| 35 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(2-oxa-6-azaspiro[3.3]heptan-6-yl)methanone |
| 36 | N-(azetidin-3-yl)-2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carboxamide |
| 37 | 2-(4-cyclopropyl-6-methoxypyrimidin-5-yl)-7-(4-(1-isopropyl-4-(trifluoromethyl)-1H-imidazol-2-yl)benzyl)-N-propyl-7H-pyrrolo[2,3-d]pyrimidine-6-carboxamide |
| 38 | 2-(4-cyclopropyl-6-methoxypyrimidin-5-yl)-N-(2-(dimethylamino)ethyl)-7-(4-(1-isopropyl-4-(trifluoromethyl)-1H-imidazol-2-yl)benzyl)-N-methyl-7H-pyrrolo[2,3-d]pyrimidine-6-carboxamide |
| 39 | (R)-(2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(3-(dimethylamino)pyrrolidin-1-yl)methanone |
| 40 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl) (3,3-difluoroazetidin-1-yl)methanone |
| 41 | 2-(4-cyclopropyl-6-methoxypyrimidin-5-yl)-7-(4-(1-isopropyl-4-(trifluoromethyl)-1H-imidazol-2-yl)benzyl)-N-(pyrrolidin-3-yl)-7H-pyrrolo[2,3-d]pyrimidine-6-carboxamide |
| 42 | 2-(4-cyclopropyl-6-methoxypyrimidin-5-yl)-N,N-diethyl-7-(4-(1-isopropyl-4-(trifluoromethyl)-1H-imidazol-2-yl)benzyl)-7H-pyrrolo[2,3-d]pyrimidine-6-carboxamide |
| 43 | 2-(4-cyclopropyl-6-methoxypyrimidin-5-yl)-N-methyl-7-(4-(1-methyl-4-(trifluoromethyl)-1H-imidazol-2-yl)benzyl)-7H-pyrrolo[2,3-d]pyrimidine-6-carboxamide |
| 44 | 7-(4-(1H-1,2,3-triazol-1-yl)benzyl)-2-(4-cyclopropyl-6-methoxypyrimidin-5-yl)-N-methyl-7H-pyrrolo[2,3-d]pyrimidine-6-carboxamide |
| 45 | methyl (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carbonyl)glycinate |
| 46 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)((1R,5S)-8-cyclopropyl-3,8-diazabicyclo[3.2.1]octan-3-yl)methanone |
| 47 | 2-(4-cyclopropyl-6-methoxypyrimidin-5-yl)-7-(4-(1-isopropyl-4-(trifluoromethyl)-1H-imidazol-2-yl)benzyl)-N-(2-(pyrrolidin-1-yl)ethyl)-7H-pyrrolo[2,3-d]pyrimidine-6-carboxamide |
| 48 | 2-(4-cyclopropyl-6-methoxypyrimidin-5-yl)-N-isopropyl-7-(4-(1-isopropyl-4-(trifluoromethyl)-1H-imidazol-2-yl)benzyl)-7H-pyrrolo[2,3-d]pyrimidine-6-carboxamide |
| 49 | N-benzyl-2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carboxamide |
| 50 | (S)-(2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(3-(dimethylamino)pyrrolidin-1-yl)methanone |
| 51 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(pyrrolidin-1-yl)methanone |
| 52 | 2-(4-cyclopropyl-6-methoxypyrimidin-5-yl)-N-(2-(diethylamino)ethyl)-7-(4-(1-isopropyl-4-(trifluoromethyl)-1H-imidazol-2-yl)benzyl)-7H-pyrrolo[2,3-d]pyrimidine-6-carboxamide |
| 53 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl) (3-(dimethylamino)azetidin-1-yl)methanone |
| 54 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(piperazin-1-yl)methanone |
| 55 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(hexahydropyrrolo[1,2-a]pyrazin-2(1H)-yl)methanone |
| 56 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(5-oxa-8-azaspiro[3.5]nonan-8-yl)methanone |
| 57 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(4-ethylpiperazin-1-yl)methanone |
| 58 | (R)-2-(4-cyclopropyl-6-methoxypyrimidin-5-yl)-7-(4-(1-isopropyl-4-(trifluoromethyl)-1H-imidazol-2-yl)benzyl)-N-(pyrrolidin-2-ylmethyl)-7H-pyrrolo[2,3-d]pyrimidine-6-carboxamide |
| 59 | 2-(4-cyclopropyl-6-oxo-1,6-dihydropyrimidin-5-yl)-7-(4-(1-isopropyl-4-(trifluoromethyl)-1H-imidazol-2-yl)benzyl)-N-methyl-7H-pyrrolo[2,3-d]pyrimidine-6-carboxamide |
| 60 | 2-(4-cyclopropyl-6-methoxypyrimidin-5-yl)-7-(4-(1-isopropyl-4-(trifluoromethyl)-1H-imidazol-2-yl)benzyl)-N-(prop-2-yn-1-yl)-7H-pyrrolo[2,3-d]pyrimidine-6-carboxamide |
| 61 | 2-(4-cyclopropyl-6-methoxypyrimidin-5-yl)-N-(2-hydroxy-2-methylpropyl)-7-(4-(1-isopropyl-4-(trifluoromethyl)-1H-imidazol-2-yl)benzyl)-7H-pyrrolo[2,3-d]pyrimidine-6-carboxamide |
| 62 | 2-(4-cyclopropyl-1-ethyl-6-oxo-1,6-dihydropyrimidin-5-yl)-7-(4-(1-isopropyl-4-(trifluoromethyl)-1H-imidazol-2-yl)benzyl)-N-methyl-7H-pyrrolo[2,3-d]pyrimidine-6-carboxamide |
| 63 | 2-(4-cyclopropyl-6-ethoxypyrimidin-5-yl)-7-(4-(1-isopropyl-4-(trifluoromethyl)-1H-imidazol-2-yl)benzyl)-N-methyl-7H-pyrrolo[2,3-d]pyrimidine-6-carboxamide |
| 64 | N-(but-3-en-1-yl)-2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carboxamide |
| 65 | ((1R,4R)-2-oxa-5-azabicyclo[2.2.1]heptan-5-yl)(2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)methanone |
| 66 | (S)-2-(4-cyclopropyl-6-methoxypyrimidin-5-yl)-7-(4-(1-isopropyl-4-(trifluoromethyl)-1H-imidazol-2-yl)benzyl)-N-(oxetan-2-ylmethyl)-7H-pyrrolo[2,3-d]pyrimidine-6-carboxamide |
| 67 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(3-hydroxypiperidin-1-yl)methanone |
| 68 | 2-(4-cyclopropyl-6-methoxypyrimidin-5-yl)-7-(4-(1-ethyl-4-(trifluoromethyl)-1H-imidazol-2-yl)benzyl)-N-methyl-7H-pyrrolo[2,3-d]pyrimidine-6-carboxamide |
| 69 | 2-(4-cyclopropyl-6-methoxypyrimidin-5-yl)-7-(4-(1-isopropyl-4-(trifluoromethyl)-1H-imidazol-2-yl)benzyl)-N-(1-methylpiperidin-4-yl)-7H-pyrrolo[2,3-d]pyrimidine-6-carboxamide |
| 70 | 2-(4-cyclopropyl-6-methoxypyrimidin-5-yl)-7-(4-(1-isopropyl-4-(trifluoromethyl)-1H-imidazol-2-yl)benzyl)-N-methyl-N-(2,2,2-trifluoroethyl)-7H-pyrrolo[2,3-d]pyrimidine-6-carboxamide |
| 71 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl) (3-(methoxymethyl)azetidin-1-yl)methanone |
| 72 | 1-(2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carbonyl)-3-fluoroazetidine-3-carbonitrile |
| 73 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl) (3,3-difluoropyrrolidin-1-yl)methanone |
| 74 | 2-(4-cyclopropyl-6-methoxypyrimidin-5-yl)-7-(4-(1-isopropyl-4-(trifluoromethyl)-1H-imidazol-2-yl)benzyl)-N-(1-(methylsulfonyl)piperidin-4-yl)-7H-pyrrolo[2,3-d]pyrimidine-6-carboxamide |
| 75 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl) (3-((methylsulfonyl)methyl)azetidin-1-yl)methanone |
| 76 | N-cyclopropyl-2-(4-cyclopropyl-6-methoxypyrimidin-5-yl)-7-(4-(1-isopropyl-4-(trifluoromethyl)-1H-imidazol-2-yl)benzyl)-N-methyl-7H-pyrrolo[2,3-d]pyrimidine-6-carboxamide |
| 77 | 2-(4-cyclopropyl-6-methoxypyrimidin-5-yl)-N-(4-((dimethylamino)methyl)phenyl)-7-(4-(1-isopropyl-4-(trifluoromethyl)-1H-imidazol-2-yl)benzyl)-7H-pyrrolo[2,3-d]pyrimidine-6-carboxamide |
| 78 | 2-(4-cyclopropyl-6-methoxypyrimidin-5-yl)-N-(1,1-dioxidotetrahydrothiophen-3-yl)-7-(4-(1-isopropyl-4-(trifluoromethyl)-1H-imidazol-2-yl)benzyl)-7H-pyrrolo[2,3-d]pyrimidine-6-carboxamide |
| 79 | 2-(4-cyclopropyl-6-methoxypyrimidin-5-yl)-7-(4-(1-isopropyl-4-(trifluoromethyl)-1H-imidazol-2-yl)benzyl)-N-(1-methylcyclopropyl)-7H-pyrrolo[2,3-d]pyrimidine-6-carboxamide |
| 80 | 2-(4-cyclopropyl-6-methoxypyrimidin-5-yl)-N-((3,3-difluorocyclopentyl)methyl)-7-(4-(1-isopropyl-4-(trifluoromethyl)-1H-imidazol-2-yl)benzyl)-7H-pyrrolo[2,3-d]pyrimidine-6-carboxamide |
| 81 | (R)-2-(4-cyclopropyl-6-methoxypyrimidin-5-yl)-N-(1-hydroxypropan-2-yl)-7-(4-(1-isopropyl-4-(trifluoromethyl)-1H-imidazol-2-yl)benzyl)-7H-pyrrolo[2,3-d]pyrimidine-6-carboxamide |
| 82 | (S)-N-(sec-butyl)-2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carboxamide |
| 83 | (R)-2-(4-cyclopropyl-6-methoxypyrimidin-5-yl)-N-(4-hydroxybutan-2-yl)-7-(4-(1-isopropyl-4-(trifluoromethyl)-1H-imidazol-2-yl)benzyl)-7H-pyrrolo[2,3-d]pyrimidine-6-carboxamide |
| 84 | 2-(4-cyclopropyl-6-methoxypyrimidin-5-yl)-N-((3,3-difluorocyclobutyl)methyl)-7-(4-(1-isopropyl-4-(trifluoromethyl)-1H-imidazol-2-yl)benzyl)-7H-pyrrolo[2,3-d]pyrimidine-6-carboxamide |
| 85 | (R)-2-(4-cyclopropyl-6-methoxypyrimidin-5-yl)-7-(4-(1-isopropyl-4-(trifluoromethyl)-1H-imidazol-2-yl)benzyl)-N-(1,1,1-trifluoropropan-2-yl)-7H-pyrrolo[2,3-d]pyrimidine-6-carboxamide |
| 86 | (S)-2-(4-cyclopropyl-6-methoxypyrimidin-5-yl)-7-(4-(1-isopropyl-4-(trifluoromethyl)-1H-imidazol-2-yl)benzyl)-N-(1,1,1-trifluoropropan-2-yl)-7H-pyrrolo[2,3-d]pyrimidine-6-carboxamide |
| 87 | 2-(4-cyclopropyl-6-methoxypyrimidin-5-yl)-7-(4-(1-isopropyl-4-(trifluoromethyl)-1H-imidazol-2-yl)benzyl)-N-((1-methylpiperidin-4-yl)methyl)-7H-pyrrolo[2,3-d]pyrimidine-6-carboxamide |
| 88 | N-(bicyclo[1.1.1]pentan-1-yl)-2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carboxamide |
| 89 | 2-(4-cyclopropyl-6-methoxypyrimidin-5-yl)-N-(3-fluorobicyclo[1.1.1]pentan-1-yl)-7-(4-(1-isopropyl-4-(trifluoromethyl)-1H-imidazol-2-yl)benzyl)-7H-pyrrolo[2,3-d]pyrimidine-6-carboxamide |
| 90 | 2-(4-cyclopropyl-6-methoxypyrimidin-5-yl)-N-(2-(3,3-difluorocyclobutyl)ethyl)-7-(4-(1-isopropyl-4-(trifluoromethyl)-1H-imidazol-2-yl)benzyl)-7H-pyrrolo[2,3-d]pyrimidine-6-carboxamide |
| 91 | (S)-2-(4-cyclopropyl-6-methoxypyrimidin-5-yl)-N-(4-hydroxybutan-2-yl)-7-(4-(1-isopropyl-4-(trifluoromethyl)-1H-imidazol-2-yl)benzyl)-7H-pyrrolo[2,3-d]pyrimidine-6-carboxamide |
| 92 | (R)-N-(sec-butyl)-2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carboxamide |
| 93 | 2-(4-cyclopropyl-6-methoxypyrimidin-5-yl)-N-(1,1-dioxidotetrahydro-2H-thiopyran-4-yl)-7-(4-(1-isopropyl-4-(trifluoromethyl)-1H-imidazol-2-yl)benzyl)-7H-pyrrolo[2,3-d]pyrimidine-6-carboxamide |
| 94 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(1-oxa-4,9-diazaspiro[5.5]undecan-9-yl)methanone |
| 95 | 2-(4-cyclopropyl-6-(difluoromethoxy)pyrimidin-5-yl)-7-(4-(1-isopropyl-4-(trifluoromethyl)-1H-imidazol-2-yl)benzyl)-N-methyl-7H-pyrrolo[2,3-d]pyrimidine-6-carboxamide |
| 96 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(3-(difluoromethyl)azetidin-1-yl)methanone |
| 97 | (S)-(2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl) (3-(methylamino)pyrrolidin-1-yl)methanone |
| 98 | 2-(4-cyclopropyl-6-methoxypyrimidin-5-yl)-7-(4-(1-isopropyl-4-(trifluoromethyl)-1H-imidazol-2-yl)benzyl)-N-(2-(piperazin-1-yl)ethyl)-7H-pyrrolo[2,3-d]pyrimidine-6-carboxamide |
| 99 | (3-aminoazetidin-1-yl)(2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)methanone |
| 100 | N-(2-aminoethyl)-2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carboxamide |
| 101 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(3-fluoroazetidin-1-yl)methanone |
| 102 | 2-(4-cyclopropyl-6-methoxypyrimidin-5-yl)-7-(4-(1-isopropyl-4-(trifluoromethyl)-1H-imidazol-2-yl)benzyl)-N-(2-(4-methylpiperazin-1-yl)ethyl)-7H-pyrrolo[2,3-d]pyrimidine-6-carboxamide |
| 103 | N-(azetidin-3-yl)-2-(4-cyclopropyl-6-methoxypyrimidin-5-yl)-7-(4-(1-isopropyl-4-(trifluoromethyl)-1H-imidazol-2-yl)benzyl)-N-methyl-7H-pyrrolo[2,3-d]pyrimidine-6-carboxamide |
| 104 | 2-(4-cyclopropyl-6-methoxypyrimidin-5-yl)-7-(4-(1-isopropyl-4-(trifluoromethyl)-1H-imidazol-2-yl)benzyl)-N-(3-methyloxetan-3-yl)-7H-pyrrolo[2,3-d]pyrimidine-6-carboxamide |
| 105 | (R)-(2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(3-hydroxypyrrolidin-1-yl)methanone |
| 106 | 2-(4-cyclopropyl-6-methoxypyrimidin-5-yl)-7-(4-(1-isopropyl-4-(trifluoromethyl)-1H-imidazol-2-yl)benzyl)-N-(2-methoxyethyl)-7H-pyrrolo[2,3-d]pyrimidine-6-carboxamide |
| 107 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(2,2-dimethylpyrrolidin-1-yl)methanone |
| 108 | (S)-2-(4-cyclopropyl-6-methoxypyrimidin-5-yl)-7-(4-(1-isopropyl-4-(trifluoromethyl)-1H-imidazol-2-yl)benzyl)-N-((1-methylpyrrolidin-2-yl)methyl)-7H-pyrrolo[2,3-d]pyrimidine-6-carboxamide |
| 109 | (R)-(2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl) (3-(methylamino)pyrrolidin-1-yl)methanone |
| 110 | 2-(4-cyclopropyl-6-methoxypyrimidin-5-yl)-7-(4-(1-isopropyl-4-(trifluoromethyl)-1H-imidazol-2-yl)benzyl)-N-(1-methylazetidin-3-yl)-7H-pyrrolo[2,3-d]pyrimidine-6-carboxamide |
| 111 | 2-(4-cyclopropyl-6-methoxypyrimidin-5-yl)-7-(4-(1-isopropyl-4-(trifluoromethyl)-1H-imidazol-2-yl)benzyl)-N-methyl-N-(oxetan-3-yl)-7H-pyrrolo[2,3-d]pyrimidine-6-carboxamide |
| 112 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl) ((2R,3S)-3-hydroxy-2-methylazetidin-1-yl)methanone |
| 113 | (S)-2-(4-cyclopropyl-6-methoxypyrimidin-5-yl)-N-(1-hydroxypropan-2-yl)-7-(4-(1-isopropyl-4-(trifluoromethyl)-1H-imidazol-2-yl)benzyl)-7H-pyrrolo[2,3-d]pyrimidine-6-carboxamide |
| 114 | 2-(4-cyclopropyl-6-methoxypyrimidin-5-yl)-N-isobutyl-7-(4-(1-isopropyl-4-(trifluoromethyl)-1H-imidazol-2-yl)benzyl)-7H-pyrrolo[2,3-d]pyrimidine-6-carboxamide |
| 115 | 2-(4-cyclopropyl-6-methoxypyrimidin-5-yl)-N-((1R,2R)-2-(dimethylamino)cyclopentyl)-7-(4-(1-isopropyl-4-(trifluoromethyl)-1H-imidazol-2-yl)benzyl)-7H-pyrrolo[2,3-d]pyrimidine-6-carboxamide |
| 116 | 2-(4-cyclopropyl-6-methoxypyrimidin-5-yl)-N-(3,3-difluoro-1-methylcyclobutyl)-7-(4-(1-isopropyl-4-(trifluoromethyl)-1H-imidazol-2-yl)benzyl)-7H-pyrrolo[2,3-d]pyrimidine-6-carboxamide |
| 117 | (S)-(2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(3-hydroxypyrrolidin-1-yl)methanone |
| 118 | N-(2-amino-2-methylpropyl)-2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carboxamide |
| 119 | 2-(4-cyclopropyl-6-isopropoxypyrimidin-5-yl)-7-(4-(1-isopropyl-4-(trifluoromethyl)-1H-imidazol-2-yl)benzyl)-N-methyl-7H-pyrrolo[2,3-d]pyrimidine-6-carboxamide |
| 120 | 2-(4-cyclopropyl-6-methoxypyrimidin-5-yl)-N-hydroxy-7-(4-(1-isopropyl-4-(trifluoromethyl)-1H-imidazol-2-yl)benzyl)-7H-pyrrolo[2,3-d]pyrimidine-6-carboxamide |
| 121 | 2-(4-cyclopropyl-6-methoxypyrimidin-5-yl)-N-(1-cyclopropylpiperidin-4-yl)-7-(4-(1-isopropyl-4-(trifluoromethyl)-1H-imidazol-2-yl)benzyl)-7H-pyrrolo[2,3-d]pyrimidine-6-carboxamide |
| 122 | 2-(4-cyclopropyl-6-methoxypyrimidin-5-yl)-7-(4-(1-isopropyl-4-(trifluoromethyl)-1H-imidazol-2-yl)benzyl)-N-((1r,3r)-3-methoxycyclobutyl)-7H-pyrrolo[2,3-d]pyrimidine-6-carboxamide |
| 123 | (R)-(2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(3-methoxypyrrolidin-1-yl)methanone |
| 124 | (S)-2-(4-cyclopropyl-6-methoxypyrimidin-5-yl)-7-(4-(1-isopropyl-4-(trifluoromethyl)-1H-imidazol-2-yl)benzyl)-N-((tetrahydrofuran-2-yl)methyl)-7H-pyrrolo[2,3-d]pyrimidine-6-carboxamide |
| 125 | 2-(4-cyclopropyl-6-methoxypyrimidin-5-yl)-7-(4-(1-isopropyl-4-(trifluoromethyl)-1H-imidazol-2-yl)benzyl)-N-((3-methyloxetan-3-yl)methyl)-7H-pyrrolo[2,3-d]pyrimidine-6-carboxamide |
| 126 | 2-(4-cyclopropyl-6-methoxypyrimidin-5-yl)-N-(1-(2-fluoro-2-methylpropyl)piperidin-4-yl)-7-(4-(1-isopropyl-4-(trifluoromethyl)-1H-imidazol-2-yl)benzyl)-7H-pyrrolo[2,3-d]pyrimidine-6-carboxamide |
| 127 | (R)-2-(4-cyclopropyl-6-methoxypyrimidin-5-yl)-7-(4-(1-isopropyl-4-(trifluoromethyl)-1H-imidazol-2-yl)benzyl)-N-((tetrahydrofuran-2-yl)methyl)-7H-pyrrolo[2,3-d]pyrimidine-6-carboxamide |
| 128 | (S)-(2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl)(3-methoxypyrrolidin-1-yl)methanone |
| 129 | 2-(4-cyclopropyl-6-methoxypyrimidin-5-yl)-N-((1S,2R)-2-fluorocyclopropyl)-7-(4-(1-isopropyl-4-(trifluoromethyl)-1H-imidazol-2-yl)benzyl)-7H-pyrrolo[2,3-d]pyrimidine-6-carboxamide |
| 130 | 2-(4-cyclopropoxy-6-cyclopropylpyrimidin-5-yl)-7-(4-(1-isopropyl-4-(trifluoromethyl)-1H-imidazol-2-yl)benzyl)-N-methyl-7H-pyrrolo[2,3-d]pyrimidine-6-carboxamide |
| 131 | 2-(4-cyclopropyl-6-methoxypyrimidin-5-yl)-7-(4-(1-isopropyl-4-(trifluoromethyl)-1H-imidazol-2-yl)benzyl)-N-((1s,3s)-3-methoxycyclobutyl)-7H-pyrrolo[2,3-d]pyrimidine-6-carboxamide |
| 132 | 2-(4-cyclopropyl-6-methoxypyrimidin-5-yl)-N-(3-ethyloxetan-3-yl)-7-(4-(1-isopropyl-4-(trifluoromethyl)-1H-imidazol-2-yl)benzyl)-7H-pyrrolo[2,3-d]pyrimidine-6-carboxamide |
| 133 | 2-(4-cyclopropyl-6-methoxypyrimidin-5-yl)-N-(1-((1-fluorocyclopropyl)methyl)piperidin-4-yl)-7-(4-(1-isopropyl-4-(trifluoromethyl)-1H-imidazol-2-yl)benzyl)-7H-pyrrolo[2,3-d]pyrimidine-6-carboxamide |
| 134 | 7-(4-(1-isopropyl-4-(trifluoromethyl)-1H-imidazol-2-yl)benzyl)-2-(2-isopropylpyridin-3-yl)-N-methyl-7H-pyrrolo[2,3-d]pyrimidine-6-carboxamide |
| 135 | (S)-2-(4-cyclopropyl-6-methoxypyrimidin-5-yl)-7-(4-(1-isopropyl-4-(trifluoromethyl)-1H-imidazol-2-yl)benzyl)-N-(2-methoxypropyl)-7H-pyrrolo[2,3-d]pyrimidine-6-carboxamide |
| 136 | (R)-2-(4-cyclopropyl-6-methoxypyrimidin-5-yl)-7-(4-(1-isopropyl-4-(trifluoromethyl)-1H-imidazol-2-yl)benzyl)-N-((1-methylpyrrolidin-2-yl)methyl)-7H-pyrrolo[2,3-d]pyrimidine-6-carboxamide |
| 137 | 2-(4-cyclopropyl-6-methoxypyrimidin-5-yl)-7-(4-(1-isopropyl-4-(trifluoromethyl)-1H-imidazol-2-yl)benzyl)-N-(1-isopropylpiperidin-4-yl)-7H-pyrrolo[2,3-d]pyrimidine-6-carboxamide |
| 138 | 2-(4-cyclopropyl-6-methoxypyrimidin-5-yl)-N-(3-(hydroxymethyl)oxetan-3-yl)-7-(4-(1-isopropyl-4-(trifluoromethyl)-1H-imidazol-2-yl)benzyl)-7H-pyrrolo[2,3-d]pyrimidine-6-carboxamide |
| 139 | 2-(4-cyclopropyl-6-methoxypyrimidin-5-yl)-7-(4-(1-isopropyl-4-(trifluoromethyl)-1H-imidazol-2-yl)benzyl)-N-methyl-N-(oxetan-3-ylmethyl)-7H-pyrrolo[2,3-d]pyrimidine-6-carboxamide |
| 140 | 2-(4-cyclopropyl-6-methoxypyrimidin-5-yl)-N-((3-hydroxyoxetan-3-yl)methyl)-7-(4-(1-isopropyl-4-(trifluoromethyl)-1H-imidazol-2-yl)benzyl)-7H-pyrrolo[2,3-d]pyrimidine-6-carboxamide |
| 141 | 2-(4-cyclopropyl-6-methoxypyrimidin-5-yl)-7-(4-(1-isopropyl-4-(trifluoromethyl)-1H-imidazol-2-yl)benzyl)-N-(oxetan-3-ylmethyl)-7H-pyrrolo[2,3-d]pyrimidine-6-carboxamide |
| 142 | 2-(4-cyclopropyl-6-methoxypyrimidin-5-yl)-N-((3-fluorooxetan-3-yl)methyl)-7-(4-(1-isopropyl-4-(trifluoromethyl)-1H-imidazol-2-yl)benzyl)-7H-pyrrolo[2,3-d]pyrimidine-6-carboxamide |
| 143 | 2-(4-cyclopropyl-6-methoxypyrimidin-5-yl)-7-(4-(1-isopropyl-4-(trifluoromethyl)-1H-imidazol-2-yl)benzyl)-N-(2-(methylamino)ethyl)-7H-pyrrolo[2,3-d]pyrimidine-6-carboxamide |
| 144 | (2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidin-6-yl) (3-(methylamino)azetidin-1-yl)methanone |
| 145 | 7-(4-(1-cyclopropyl-4-(trifluoromethyl)-1H-imidazol-2-yl)benzyl)-2-(4-cyclopropyl-6-methoxypyrimidin-5-yl)-N-methyl-7H-pyrrolo[2,3-d]pyrimidine-6-carboxamide |
| 146 | 2-(4-cyclopropyl-6-methoxypyrimidin-5-yl)-7-(3-fluoro-4-(1-isopropyl-4-(trifluoromethyl)-1H-imidazol-2-yl)benzyl)-N-methyl-7H-pyrrolo[2,3-d]pyrimidine-6-carboxamide |
| 147 | 7-(4-(1-isopropyl-4-(trifluoromethyl)-1H-imidazol-2-yl)benzyl)-2-(2-methoxypyridin-3-yl)-N-methyl-7H-pyrrolo[2,3-d]pyrimidine-6-carboxamide |
| 148 | 2-(4-cyclopropyl-6-methoxypyrimidin-5-yl)-7-(4-(1-methyl-4-(trifluoromethyl)-1H-imidazol-2-yl)benzyl)-7H-pyrrolo[2,3-d]pyrimidine-6-carboxamide |
| 149 | N-(2-(azetidin-1-yl)ethyl)-2-(4-cyclopropyl-6-methoxypyrimidin-5-yl)-7-(4-(1-isopropyl-4-(trifluoromethyl)-1H-imidazol-2-yl)benzyl)-7H-pyrrolo[2,3-d]pyrimidine-6-carboxamide |
| 150 | N-cyclopropyl-2-(4-cyclopropyl-6-methoxypyrimidin-5-yl)-7-(4-(1-methyl-4-(trifluoromethyl)-1H-imidazol-2-yl)benzyl)-7H-pyrrolo[2,3-d]pyrimidine-6-carboxamide |
| 151 | (R)-2-(4-cyclopropyl-6-methoxypyrimidin-5-yl)-N-(1-hydroxypropan-2-yl)-7-(4-(1-methyl-4-(trifluoromethyl)-1H-imidazol-2-yl)benzyl)-7H-pyrrolo[2,3-d]pyrimidine-6-carboxamide |
| 152 | (S)-2-(4-cyclopropyl-6-methoxypyrimidin-5-yl)-N-(2,3-dihydroxypropyl)-7-(4-(1-methyl-4-(trifluoromethyl)-1H-imidazol-2-yl)benzyl)-7H-pyrrolo[2,3-d]pyrimidine-6-carboxamide |
| 153 | 2-(4-cyclopropyl-6-methoxypyrimidin-5-yl)-7-(4-(1-methyl-4-(trifluoromethyl)-1H-imidazol-2-yl)benzyl)-N-(1-methylpiperidin-4-yl)-7H-pyrrolo[2,3-d]pyrimidine-6-carboxamide |
| 154 | 6-(4-cyclopropyl-6-methoxypyrimidin-5-yl)-1-(4-(1-isopropyl-4-(trifluoromethyl)-1H-imidazol-2-yl)benzyl)-N-methyl-1H-pyrrolo[3,2-c]pyridine-2-carboxamide |
| 155 | (S)-2-(4-cyclopropyl-6-methoxypyrimidin-5-yl)-N-(1-hydroxy-3-methylbutan-2-yl)-7-(4-(1-isopropyl-4-(trifluoromethyl)-1H-imidazol-2-yl)benzyl)-7H-pyrrolo[2,3-d]pyrimidine-6-carboxamide |
| 156 | 2-(4-cyclopropyl-6-methoxypyrimidin-5-yl)-N-(1-hydroxy-2-methylpropan-2-yl)-7-(4-(1-isopropyl-4-(trifluoromethyl)-1H-imidazol-2-yl)benzyl)-7H-pyrrolo[2,3-d]pyrimidine-6-carboxamide |
| 157 | 2-(4-cyclopropyl-6-methoxypyrimidin-5-yl)-N-(2-hydroxyethyl)-7-(4-(1-isopropyl-4-(trifluoromethyl)-1H-imidazol-2-yl)benzyl)-7H-pyrrolo[2,3-d]pyrimidine-6-carboxamide |
| 158 | 2-(4-cyclopropyl-6-methoxypyrimidin-5-yl)-N-(4-(dimethylamino)cyclohexyl)-7-(4-(1-isopropyl-4-(trifluoromethyl)-1H-imidazol-2-yl)benzyl)-7H-pyrrolo[2,3-d]pyrimidine-6-carboxamide |
| 159 | 2-(4-cyclopropyl-6-methoxypyrimidin-5-yl)-7-(4-(1-isopropyl-4-(trifluoromethyl)-1H-imidazol-2-yl)benzyl)-N-(piperidin-4-yl)-7H-pyrrolo[2,3-d]pyrimidine-6-carboxamide |
| 160 | 2-(4-cyclopropyl-6-methoxypyrimidin-5-yl)-N-((3S,4R)-3-fluoro-1-(2-methoxyethyl)piperidin-4-yl)-7-(4-(1-methyl-4-(trifluoromethyl)-1H-imidazol-2-yl)benzyl)-7H-pyrrolo[2,3-d]pyrimidine-6-carboxamide |
| 161 | 2-(4-cyclopropyl-6-methoxypyrimidin-5-yl)-N-((3S,4R)-3-fluoro-1-methylpiperidin-4-yl)-7-(4-(1-methyl-4-(trifluoromethyl)-1H-imidazol-2-yl)benzyl)-7H-pyrrolo[2,3-d]pyrimidine-6-carboxamide |
| 162 | 2-(4-cyclopropyl-6-methoxypyrimidin-5-yl)-N-((3R,4S)-3-fluoro-1-methylpiperidin-4-yl)-7-(4-(1-methyl-4-(trifluoromethyl)-1H-imidazol-2-yl)benzyl)-7H-pyrrolo[2,3-d]pyrimidine-6-carboxamide |
| 163 | (R)-2-(4-cyclopropyl-6-methoxypyrimidin-5-yl)-N-(2-hydroxypropyl)-7-(4-(1-methyl-4-(trifluoromethyl)-1H-imidazol-2-yl)benzyl)-7H-pyrrolo[2,3-d]pyrimidine-6-carboxamide |
| 164 | (S)-2-(4-cyclopropyl-6-methoxypyrimidin-5-yl)-N-(2-hydroxypropyl)-7-(4-(1-methyl-4-(trifluoromethyl)-1H-imidazol-2-yl)benzyl)-7H-pyrrolo[2,3-d]pyrimidine-6-carboxamide |
| 165 | 2-(4-cyclopropyl-6-methoxypyrimidin-5-yl)-N-((3R,4R)-3-fluoro-1-methylpiperidin-4-yl)-7-(4-(1-methyl-4-(trifluoromethyl)-1H-imidazol-2-yl)benzyl)-7H-pyrrolo[2,3-d]pyrimidine-6-carboxamide |
| 166 | (S)-2-(4-cyclopropyl-6-methoxypyrimidin-5-yl)-N-(1-hydroxypropan-2-yl)-7-(4-(1-methyl-4-(trifluoromethyl)-1H-imidazol-2-yl)benzyl)-7H-pyrrolo[2,3-d]pyrimidine-6-carboxamide |
| 167 | 2-(4-cyclopropyl-6-methoxypyrimidin-5-yl)-N-((3S,4S)-3-fluoro-1-methylpiperidin-4-yl)-7-(4-(1-methyl-4-(trifluoromethyl)-1H-imidazol-2-yl)benzyl)-7H-pyrrolo[2,3-d]pyrimidine-6-carboxamide |
| 168 | 2-(4-cyclopropyl-6-methoxypyrimidin-5-yl)-N-((1R,2R)-2-(dimethylamino)cyclopentyl)-7-(4-(1-methyl-4-(trifluoromethyl)-1H-imidazol-2-yl)benzyl)-7H-pyrrolo[2,3-d]pyrimidine-6-carboxamide |
| 169 | (R)-2-(4-cyclopropyl-6-methoxypyrimidin-5-yl)-7-(4-(1-methyl-4-(trifluoromethyl)-1H-imidazol-2-yl)benzyl)-N-(1-methylpyrrolidin-3-yl)-7H-pyrrolo[2,3-d]pyrimidine-6-carboxamide |
| 170 | (2-(4-cyclopropyl-6-methoxypyrimidin-5-yl)-7-(4-(1-methyl-4-(trifluoromethyl)-1H-imidazol-2-yl)benzyl)-7H-pyrrolo[2,3-d]pyrimidin-6-yl) (4-methylpiperazin-1-yl)methanone |
| 171 | 2-(4-cyclopropyl-6-methoxypyrimidin-5-yl)-7-(4-(1-methyl-4-(trifluoromethyl)-1H-imidazol-2-yl)benzyl)-N-(1-methylazetidin-3-yl)-7H-pyrrolo[2,3-d]pyrimidine-6-carboxamide |
| 172 | 2-(4-cyclopropyl-6-methoxypyrimidin-5-yl)-N-(4-(dimethylamino)cyclohexyl)-7-(4-(1-methyl-4-(trifluoromethyl)-1H-imidazol-2-yl)benzyl)-7H-pyrrolo[2,3-d]pyrimidine-6-carboxamide |
| 173 | (S)-2-(4-cyclopropyl-6-methoxypyrimidin-5-yl)-7-(4-(1-methyl-4-(trifluoromethyl)-1H-imidazol-2-yl)benzyl)-N-(1-methylpyrrolidin-3-yl)-7H-pyrrolo[2,3-d]pyrimidine-6-carboxamide |
| 174 | (S)-2-(4-cyclopropyl-6-methoxypyrimidin-5-yl)-7-(4-(1-isopropyl-4-(trifluoromethyl)-1H-imidazol-2-yl)benzyl)-N-(1-methylpyrrolidin-3-yl)-7H-pyrrolo[2,3-d]pyrimidine-6-carboxamide |
| 175 | (R)-2-(4-cyclopropyl-6-methoxypyrimidin-5-yl)-7-(4-(1-isopropyl-4-(trifluoromethyl)-1H-imidazol-2-yl)benzyl)-N-(1-methylpyrrolidin-3-yl)-7H-pyrrolo[2,3-d]pyrimidine-6-carboxamide |
| 176 | (R)-2-(4-cyclopropyl-6-methoxypyrimidin-5-yl)-N-(2,3-dihydroxypropyl)-7-(4-(1-methyl-4-(trifluoromethyl)-1H-imidazol-2-yl)benzyl)-7H-pyrrolo[2,3-d]pyrimidine-6-carboxamide |
| 177 | 2-(4-cyclopropyl-6-methoxypyrimidin-5-yl)-7-(4-(5-methyl-3-(trifluoromethyl)-1H-pyrazol-1-yl)benzyl)-7H-pyrrolo[2,3-d]pyrimidine-6-carboxamide |
| 178 | 2-(2-(difluoromethoxy)pyridin-3-yl)-7-(4-(1-isopropyl-4-(trifluoromethyl)-1H-imidazol-2-yl)benzyl)-N-methyl-7H-pyrrolo[2,3-d]pyrimidine-6-carboxamide |
| 179 | (2-(4-cyclopropyl-6-methoxypyrimidin-5-yl)-7-(4-(1-methyl-4-(trifluoromethyl)-1H-imidazol-2-yl)benzyl)-7H-pyrrolo[2,3-d]pyrimidin-6-yl) (3-(dimethylamino)azetidin-1-yl)methanone |
| 180 | 2-(2-isopropylphenyl)-N-methyl-7-(4-(1-methyl-4-(trifluoromethyl)-1H-imidazol-2-yl)benzyl)-7H-pyrrolo[2,3-d]pyrimidine-6-carboxamide |
| 181 | 2-(4-cyclopropyl-6-methoxypyrimidin-5-yl)-N-((3S,4S)-4-fluoro-1-methylpyrrolidin-3-yl)-7-(4-(1-methyl-4-(trifluoromethyl)-1H-imidazol-2-yl)benzyl)-7H-pyrrolo[2,3-d]pyrimidine-6-carboxamide |
| 182 | 2-(4-cyclopropyl-6-methoxypyrimidin-5-yl)-N-((3S,4R)-4-fluoro-1-methylpyrrolidin-3-yl)-7-(4-(1-methyl-4-(trifluoromethyl)-1H-imidazol-2-yl)benzyl)-7H-pyrrolo[2,3-d]pyrimidine-6-carboxamide |
| 183 | 2-(4-cyclopropyl-6-methoxypyrimidin-5-yl)-N-methyl-7-(4-(1-(methyl-d₃)-4-(trifluoromethyl)-1H-imidazol-2-yl)benzyl)-7H-pyrrolo[2,3-d]pyrimidine-6-carboxamide |
| 184 | 2-(4-cyclopropyl-6-cyclopropoxypyrimidin-5-yl)-N-methyl-7-(4-(1-methyl-4-(trifluoromethyl)-1H-imidazol-2-yl)benzyl)-7H-pyrrolo[2,3-d]pyrimidine-6-carboxamide |
| 185 | 2-(4-cyclopropyl-6-methoxypyrimidin-5-yl)-N-(3,3-difluoro-1-methylpiperidin-4-yl)-7-(4-(1-methyl-4-(trifluoromethyl)-1H-imidazol-2-yl)benzyl)-7H-pyrrolo[2,3-d]pyrimidine-6-carboxamide |
| 186 | 2-(4-chloro-1-methyl-1H-pyrazol-5-yl)-N-methyl-7-(4-(1-methyl-4-(trifluoromethyl)-1H-imidazol-2-yl)benzyl)-7H-pyrrolo[2,3-d]pyrimidine-6-carboxamide |
| 187 | 2-(4-cyclopropyl-6-methoxypyrimidin-5-yl)-7-(4-(1-((3R,4R)-3-fluoro-1-methylpiperidin-4-yl)-4-(trifluoromethyl)-1H-imidazol-2-yl)benzyl)-N-methyl-7H-pyrrolo[2,3-d]pyrimidine-6-carboxamide |
| 188 | 2-(4-cyclopropyl-6-methoxypyrimidin-5-yl)-7-(4-(3-(2-hydroxypropan-2-yl)-5-methyl-1H-pyrazol-1-yl)benzyl)-N-methyl-7H-pyrrolo[2,3-d]pyrimidine-6-carboxamide |
| 189 | 2-(4-cyclopropyl-6-methoxypyrimidin-5-yl)-7-(4-(1-((3S,4S)-3-fluoro-1-methylpiperidin-4-yl)-4-(trifluoromethyl)-1H-imidazol-2-yl)benzyl)-N-methyl-7H-pyrrolo[2,3-d]pyrimidine-6-carboxamide |
| 190 | (S)-2-(4-cyclopropyl-6-methoxypyrimidin-5-yl)-N-(2-hydroxy-3-methylbutyl)-7-(4-(1-methyl-4-(trifluoromethyl)-1H-imidazol-2-yl)benzyl)-7H-pyrrolo[2,3-d]pyrimidine-6-carboxamide |
| 191 | (S)-2-(4-cyclopropyl-6-methoxypyrimidin-5-yl)-N-(4-hydroxybutan-2-yl)-7-(4-(1-methyl-4-(trifluoromethyl)-1H-imidazol-2-yl)benzyl)-7H-pyrrolo[2,3-d]pyrimidine-6-carboxamide |
| 192 | (2-(4-cyclopropyl-6-methoxypyrimidin-5-yl)-7-(4-(1-methyl-4-(trifluoromethyl)-1H-imidazol-2-yl)benzyl)-7H-pyrrolo[2,3-d]pyrimidin-6-yl)(pyrrolidin-1-yl)methanone |
| 193 | N-methyl-7-(4-(1-methyl-4-(trifluoromethyl)-1H-imidazol-2-yl)benzyl)-2-(1-methylindolin-7-yl)-7H-pyrrolo[2,3-d]pyrimidine-6-carboxamide |
| 194 | 2-(4-cyclopropyl-6-methoxypyrimidin-5-yl)-7-(3-methoxy-4-(1-methyl-4-(trifluoromethyl)-1H-imidazol-2-yl)benzyl)-N-methyl-7H-pyrrolo[2,3-d]pyrimidine-6-carboxamide |
| 195 | 2-(4-cyclopropyl-6-methoxypyrimidin-5-yl)-N,N-bis(methyl-d₃)-7-(4-(1-methyl-4-(trifluoromethyl)-1H-imidazol-2-yl)benzyl)-7H-pyrrolo[2,3-d]pyrimidine-6-carboxamide |
| 196 | (2-(4-cyclopropyl-6-methoxypyrimidin-5-yl)-7-(4-(1-methyl-4-(trifluoromethyl)-1H-imidazol-2-yl)benzyl)-7H-pyrrolo[2,3-d]pyrimidin-6-yl) (3-hydroxyazetidin-1-yl)methanone |
| 197 | 2-(4-cyclopropyl-6-methoxypyrimidin-5-yl)-N-(1-hydroxy-2-methylpropan-2-yl)-7-(4-(1-methyl-4-(trifluoromethyl)-1H-imidazol-2-yl)benzyl)-7H-pyrrolo[2,3-d]pyrimidine-6-carboxamide |
| 198 | 2-(4-cyclopropyl-6-methoxypyrimidin-5-yl)-7-(4-(5-(furan-2-yl)-3-(trifluoromethyl)-1H-pyrazol-1-yl)benzyl)-N-methyl-7H-pyrrolo[2,3-d]pyrimidine-6-carboxamide |
| 199 | 2-(4-cyclopropyl-6-methoxypyrimidin-5-yl)-N-((2R,4R)-1,2-dimethylpiperidin-4-yl)-7-(4-(1-methyl-4-(trifluoromethyl)-1H-imidazol-2-yl)benzyl)-7H-pyrrolo[2,3-d]pyrimidine-6-carboxamide |
| 200 | 2-(4-cyclopropyl-6-methoxypyrimidin-5-yl)-N-methyl-7-(4-(1-(trifluoromethyl)-5,6,7,8-tetrahydroimidazo[1,5-a]pyridin-3-yl)benzyl)-7H-pyrrolo[2,3-d]pyrimidine-6-carboxamide |
| 201 | 2-(4-cyclopropyl-6-methoxypyrimidin-5-yl)-N-((2R,4S)-1,2-dimethylpiperidin-4-yl)-7-(4-(1-methyl-4-(trifluoromethyl)-1H-imidazol-2-yl)benzyl)-7H-pyrrolo[2,3-d]pyrimidine-6-carboxamide |
| 202 | (S)-2-(4-cyclopropyl-6-methoxypyrimidin-5-yl)-N-(3,3-difluoro-1-methylpiperidin-4-yl)-7-(4-(1-methyl-4-(trifluoromethyl)-1H-imidazol-2-yl)benzyl)-7H-pyrrolo[2,3-d]pyrimidine-6-carboxamide |
| 203 | (2-(4-cyclopropyl-6-methoxypyrimidin-5-yl)-7-(4-(1-methyl-4-(trifluoromethyl)-1H-imidazol-2-yl)benzyl)-7H-pyrrolo[2,3-d]pyrimidin-6-yl) (3-(methylamino)azetidin-1-yl)methanone |
| 204 | 2-(4-cyclopropyl-6-methoxypyrimidin-5-yl)-N,N-dimethyl-7-(4-(1-methyl-4-(trifluoromethyl)-1H-imidazol-2-yl)benzyl)-7H-pyrrolo[2,3-d]pyrimidine-6-carboxamide |
| 205 | 2-(4-cyclopropyl-6-methoxypyrimidin-5-yl)-N-(2-hydroxy-2-methylpropyl)-7-(4-(1-methyl-4-(trifluoromethyl)-1H-imidazol-2-yl)benzyl)-7H-pyrrolo[2,3-d]pyrimidine-6-carboxamide |
| 206 | 2-(4-cyclopropyl-6-hydroxypyrimidin-5-yl)-N-methyl-7-(4-(1-methyl-4-(trifluoromethyl)-1H-imidazol-2-yl)benzyl)-7H-pyrrolo[2,3-d]pyrimidine-6-carboxamide |
| 207 | (R)-2-(4-cyclopropyl-6-methoxypyrimidin-5-yl)-N-(3,3-difluoro-1-methylpiperidin-4-yl)-7-(4-(1-methyl-4-(trifluoromethyl)-1H-imidazol-2-yl)benzyl)-7H-pyrrolo[2,3-d]pyrimidine-6-carboxamide |
| 208 | 2-(4-cyclopropyl-6-methoxypyrimidin-5-yl)-N-methyl-7-(4-(1-(trifluoromethyl)imidazo[1,5-a]pyridin-3-yl)benzyl)-7H-pyrrolo[2,3-d]pyrimidine-6-carboxamide |
| 209 | 2-(4-cyclopropyl-6-methoxypyrimidin-5-yl)-N-methyl-7-(4-(4-methylpyridin-2-yl)benzyl)-7H-pyrrolo[2,3-d]pyrimidine-6-carboxamide |
| 210 | 2-(4-cyclopropyl-6-methoxypyrimidin-5-yl)-N-methyl-7-(4-(1-methyl-4-(trifluoromethyl)-1H-imidazol-2-yl)benzyl)-N-((methylthio)methyl)-7H-pyrrolo[2,3-d]pyrimidine-6-carboxamide |
| 211 | 2-(4-cyclopropyl-6-methoxypyrimidin-5-yl)-N-((3S,4R)-4-fluoro-1-isopropylpyrrolidin-3-yl)-7-(4-(1-methyl-4-(trifluoromethyl)-1H-imidazol-2-yl)benzyl)-7H-pyrrolo[2,3-d]pyrimidine-6-carboxamide |
| 212 | 2-(4-cyclopropyl-6-methoxypyrimidin-5-yl)-N-((3S,4R)-4-fluoro-1-(oxetan-3-yl)pyrrolidin-3-yl)-7-(4-(1-methyl-4-(trifluoromethyl)-1H-imidazol-2-yl)benzyl)-7H-pyrrolo[2,3-d]pyrimidine-6-carboxamide |
| 213 | 1-(4-((2-(4-cyclopropyl-6-methoxypyrimidin-5-yl)-6-(methylcarbamoyl)-7H-pyrrolo[2,3-d]pyrimidin-7-yl)methyl)phenyl)-3-(trifluoromethyl)-1H-pyrazol-5-carboxylic acid |
| 214 | 2-(4-cyclopropyl-6-methoxypyrimidin-5-yl)-7-(4-(5-methoxy-1-methyl-4-(trifluoromethyl)-1H-imidazol-2-yl)benzyl)-N-methyl-7H-pyrrolo[2,3-d]pyrimidine-6-carboxamide |
| 215 | N-((3S,4R)-1-cyclopropyl-4-fluoropyrrolidin-3-yl)-2-(4-cyclopropyl-6-methoxypyrimidin-5-yl)-7-(4-(1-methyl-4-(trifluoromethyl)-1H-imidazol-2-yl)benzyl)-7H-pyrrolo[2,3-d]pyrimidine-6-carboxamide |
| 216 | 2-(4-cyclopropyl-6-methoxypyrimidin-5-yl)-N-((3S,4R)-3-fluoro-1-(oxetan-3-yl)piperidin-4-yl)-7-(4-(1-methyl-4-(trifluoromethyl)-1H-imidazol-2-yl)benzyl)-7H-pyrrolo[2,3-d]pyrimidine-6-carboxamide |
| 217 | 2-(4-cyclopropyl-6-methoxypyrimidin-5-yl)-N-((3R,4S)-4-fluoro-1-methylpyrrolidin-3-yl)-7-(4-(1-methyl-4-(trifluoromethyl)-1H-imidazol-2-yl)benzyl)-7H-pyrrolo[2,3-d]pyrimidine-6-carboxamide |
| 218 | 2-(4-cyclopropyl-6-methoxypyrimidin-5-yl)-N-((3R,4R)-4-fluoro-1-methylpyrrolidin-3-yl)-7-(4-(1-methyl-4-(trifluoromethyl)-1H-imidazol-2-yl)benzyl)-7H-pyrrolo[2,3-d]pyrimidine-6-carboxamide |
| 219 | 2-(4-cyclopropyl-6-methoxypyrimidin-5-yl)-N-((3S,4R)-3-fluoro-1-methylpiperidin-4-yl)-7-(4-(1-isopropyl-4-(trifluoromethyl)-1H-imidazol-2-yl)benzyl)-7H-pyrrolo[2,3-d]pyrimidine-6-carboxamide |
| 220 | 2-(4-cyclopropyl-6-methoxypyrimidin-5-yl)-N-((3S,4R)-4-fluoro-1-methylpyrrolidin-3-yl)-7-(4-(1-isopropyl-4-(trifluoromethyl)-1H-imidazol-2-yl)benzyl)-7H-pyrrolo[2,3-d]pyrimidine-6-carboxamide |
| 221 | 2-(4-cyclopropyl-6-methoxypyrimidin-5-yl)-N-((3S,4R)-3-fluoro-1-isopropylpiperidin-4-yl)-7-(4-(1-methyl-4-(trifluoromethyl)-1H-imidazol-2-yl)benzyl)-7H-pyrrolo[2,3-d]pyrimidine-6-carboxamide |
.

8. A pharmaceutical composition comprising the compound according to any one of claims 1 to 7, a tautomer thereof, a stereoisomer thereof, or a pharmaceutically acceptable salt thereof, and a pharmaceutically acceptable additive.

9. A pharmaceutical composition for preventing or treating cancer, comprising the compound according to any one of claims 1 to 7, a tautomer thereof, a stereoisomer thereof, or a pharmaceutically acceptable salt thereof, as an active ingredient.

10. The pharmaceutical composition of claim 9, wherein the pharmaceutical composition inhibits USP1.

11. The pharmaceutical composition of claim 9, wherein the cancer is a cancer associated with USP1, PARP, BRCA1 mutation/deficiency, BRCA2 mutation/deficiency, or HR deficiency.

12. The pharmaceutical composition of claim 11, wherein the cancer is at least one selected from the group consisting of DNA damage repair pathway deficient cancer, homologous-recombination-deficient cancer, BRCA1 mutant cancer, BRCA2 mutant cancer, PARP inhibitor-resistant or refractory cancer, BRCA1 deficient cancer, BRCA2 deficient cancer, ataxia telangiectasia mutated (ATM) mutant cancer, and mutant cancer in a gene encoding p53.

13. The pharmaceutical composition of claim 12, wherein the cancer is at least one selected from the group consisting of:
breast cancer, ovarian cancer, prostate cancer, pancreatic cancer, triple-negative breast cancer, cervical cancer, testicular cancer, fallopian tube cancer, cholangiocarcinoma, squamous cell carcinoma, lung cancer, lung adenocarcinoma, non-small cell lung cancer, small cell lung cancer, gastric cancer, colon cancer, rectal cancer, thyroid cancer, esophageal cancer, head and neck cancer, oral cancer, nasal cancer, throat cancer, kidney cancer, malignant pancreatic islet tumor, liver cancer, genitourinary tumor, endometrial cancer, peritoneal cancer, melanoma, Hodgkin's disease, non-Hodgkin's lymphoma, acute lymphoblastic leukemia, chronic lymphocytic leukemia, acute myeloid leukemia, hairy cell leukemia, multiple myeloma, neuroblastoma, Wilms' tumor, primary macroglobulinemia, bladder cancer, chronic myeloid leukemia, glioma, primary brain cancer, malignant melanoma, malignant carcinoid tumor, choriocarcinoma, mycosis fungoides, soft tissue sarcoma, osteogenic sarcoma, rhabdomyosarcoma, Kaposi sarcoma, fibrosarcoma, chondrosarcoma, hemangiosarcoma, Ewing sarcoma, bone cancer, malignant hypercalcemia, cervical hyperplasia, renal cell carcinoma, polycythemia vera, idiopathic thrombocythemia, adrenocortical carcinoma, skin cancer, metastatic bone cancer, metastatic brain cancer, meningioma, medulloblastoma, and spinal tumor.

14. Use of the compound according to any one of claims 1 to 7, a tautomer thereof, a stereoisomer thereof, or a pharmaceutically acceptable salt thereof, for use in the manufacture of a medicament for use in the treatment or prevention of diseases associated with USP1, PARP, BRCA1 mutation/deficiency, BRCA2 mutation/deficiency, or HR deficiency.

15. A method for treating or preventing USP1, PARP, BRCA1 mutation/deficiency, BRCA2 mutation/deficiency, or HR deficiency associated diseases, comprising administering to a subject in need thereof a therapeutically effective amount of the compound according to any one of claims 1 to 7, a tautomer thereof, a stereoisomer thereof or a pharmaceutically acceptable salt thereof.

16. A method for treating or preventing one or more selected from the group consisting of the following diseases, comprising administering to a subject in need thereof a therapeutically effective amount of the compound according to any one of claims 1 to 7, a tautomer thereof, a stereoisomer thereof, or a pharmaceutically acceptable salt thereof:
breast cancer, ovarian cancer, prostate cancer, pancreatic cancer, triple-negative breast cancer, cervical cancer, testicular cancer, fallopian tube cancer, cholangiocarcinoma, squamous cell carcinoma, lung cancer, lung adenocarcinoma, non-small cell lung cancer, small cell lung cancer, gastric cancer, colon cancer, rectal cancer, thyroid cancer, esophageal cancer, head and neck cancer, oral cancer, nasal cancer, throat cancer, kidney cancer, malignant pancreatic islet tumor, liver cancer, genitourinary tumor, endometrial cancer, peritoneal cancer, melanoma, Hodgkin's disease, non-Hodgkin's lymphoma, acute lymphoblastic leukemia, chronic lymphocytic leukemia, acute myeloid leukemia, hairy cell leukemia, multiple myeloma, neuroblastoma, Wilms' tumor, primary macroglobulinemia, bladder cancer, chronic myeloid leukemia, glioma, primary brain cancer, malignant melanoma, malignant carcinoid tumor, choriocarcinoma, mycosis fungoides, soft tissue sarcoma, osteogenic sarcoma, rhabdomyosarcoma, Kaposi sarcoma, fibrosarcoma, chondrosarcoma, hemangiosarcoma, Ewing sarcoma, bone cancer, malignant hypercalcemia, cervical hyperplasia, renal cell carcinoma, polycythemia vera, idiopathic thrombocythemia, adrenocortical carcinoma, skin cancer, metastatic bone cancer, metastatic brain cancer, meningioma, medulloblastoma, and spinal tumor.
